# EUROPEAN PATENT SPECIFICATION

(11) **EP 4 003 397 B1**
(45) Date of publication and mention of the grant of the patent: **26.02.2025**
(21) Application number: 20761659.0
(22) Date of filing: 30.07.2020
(51) Int. Cl.: A61K 38/17, A61P 11/00, A61P 43/00

(54) **TREATMENT OF CYSTIC FIBROSIS BY DELIVERY OF NEBULIZED MRNA ENCODING CFTR**
BEHANDLUNG VON ZYSTISCHER FIBROSE DURCH VERABREICHUNG VON VERNEBELTER MRNA, DIE CFTR CODIERT
TRAITEMENT DE LA FIBROSE KYSTIQUE PAR ADMINISTRATION D'ARNM NÉBULISÉ CODANT POUR LA CFTR

(30) Priority: 30.07.2019 US 201962880418 P
(43) Date of publication of application: 01.06.2022
(73) Proprietor: Translate Bio, Inc., Waltham, MA 02451 (US)
(72) Inventor: BARBIER, Ann, Lexington, MA 02421 (US); HEARTLEIN, Michael, Lexington, MA 02421 (US); DEROSA, Frank, Lexington, MA 02421 (US); ABYSALH, Jonathan, Lexington, MA 02421 (US); DIAS, Anusha, Lexington, MA 02421 (US); KARVE, Shrirang, Lexington, MA 02421 (US); PATEL, Zarna, Lexington, MA 02421 (US)
(74) Representative: Elkington and Fife LLP
(86) International application number: PCT/US2020/044158
(87) International publication number: WO 2021/021988

(56) References cited:
- WO-A1-2018/089790
- WO-A1-2018/213476

## Description

### CROSS-REFERENCE TO RELATED APPLICATIONS

The present application claims priority to U.S. Provisional Application Serial No. 62/880,418 filed July 30, 2019.

### INCORPORATION-BY-REFERENCE OF SEQUENCE LISTING

This instant application contains a Sequence Listing which has been submitted electronically in ASCII format and is hereby incorporated by reference in its entirety. Said ASCII copy, created on July 28, 2020, is named MRT-2105WO_ST25.txt and is 167 KB in size. No new matter is hereby added.

### BACKGROUND

Cystic fibrosis (CF) is an autosomal inherited disorder resulting from mutation of the Cystic Fibrosis Transmembrane Conductance Regulator (CFTR) gene, which encodes a chloride ion channel believed to be involved in regulation of multiple other ion channels and transport systems in epithelial cells. Loss of function of CFTR results in chronic lung disease, aberrant mucus production, and dramatically reduced life expectancy. *See generally* Rowe et al., New Engl. J. Med. 352, 1992-2001 (2005).

Currently there is no cure for cystic fibrosis. The literature has documented numerous difficulties encountered in attempting to induce expression of CFTR in the lung. For example, viral vectors comprising CFTR DNA triggered immune responses and CF symptoms persisted after administration. Conese et al., J. Cyst. Fibros. 10 Suppl 2, S114-28 (2011); Rosenecker et al., Curr. Opin. Mol. Ther. 8, 439-45 (2006). Non-viral delivery of DNA, including CFTR DNA, has also been reported to trigger immune responses. Alton et al., Lancet 353, 947-54 (1999); Rosenecker et al., J Gene Med. 5, 49-60 (2003). Furthermore, non-viral DNA vectors encounter the additional problem that the machinery of the nuclear pore complex does not ordinarily import DNA into the nucleus, where transcription would occur. Pearson, Nature 460, 164-69 (2009).

### SUMMARY OF THE INVENTION

The present invention is defined in the claims. The present invention is, in part, based on the surprising discovery that the method of treating CF according to the present invention is effective in improving the lung function of the human CF patients without serious side effects. Notably, an increase in the percent predicted force expiratory volume in one second (ppFEV1) in a patient with a mutation nonamendable to currently available CFTR modulators was observed. Moreover, additional increases in ppFEV1 in patients who were receiving concomitant CFTR modulator therapy were observed, indicating the effectiveness of hCFTR mRNA LNP in improving lung functions.

In one aspect, the present invention relates to a composition comprising an mRNA encoding a Cystic Fibrosis Transmembrane Conductance (CFTR) protein for us in a method of treating cystic fibrosis (CF) in a human subject comprising administration of the composition by nebulization at a dose between 13 mg and 19 mg that provides the human subject with at least a 3% increase in absolute change in ppFEV1 (percent predicted forced expiratory volume in one second) from baseline ppFEV1 at two days following the administration, wherein the mRNA is encapsulated in lipid nanoparticles.

The composition is nebulized at a dose between 13 mg and 19 mg. In some embodiments, the composition is nebulized at a dose of about 13mg. In some embodiments, the composition is nebulized at a dose of about 14 mg. In some embodiments, the composition is nebulized at a dose of about 15mg. In some embodiments, the composition is nebulized at a dose of about 16 mg. In some embodiments, the composition is nebulized at a dose of about 17mg. In some embodiments, the composition is nebulized at a dose of about 18 mg. In some embodiments, the composition is nebulized at a dose of about 19 mg. In some embodiments, a suitable dose for use in the method of the invention is 16 mg.

In some embodiments, a suitable dose provides the human subject with at least a 3% increase in absolute change in ppFEV1 from baseline ppFEV1 at two days following the administration. In some embodiments, a suitable dose provides the human subject with at least a 4% increase in absolute change in ppFEV1 from baseline ppFEV1 at two days following the administration. In some embodiments, a suitable dose provides the human subject with at least a 5% increase in absolute change in ppFEV1 from baseline ppFEV1 at two days following the administration. In some embodiments, a suitable dose provides the human subject with at least a 6% increase in absolute change in ppFEV1 from baseline ppFEV1 at two days following the administration. In some embodiments, a suitable dose provides the human subject with at least a 7% increase in absolute change in ppFEV1 from baseline ppFEV1 at two days following the administration. In some embodiments, a suitable dose provides the human subject with at least a 8% increase in absolute change in ppFEV1 from baseline ppFEV1 at two days following the administration. In some embodiments, a suitable dose provides the human subject with at least a 10% increase in absolute change in ppFEV1 from baseline ppFEV1 at two days following the administration. In some embodiments, a suitable dose provides the human subject with at least a 12% increase in absolute change in ppFEV1 from baseline ppFEV1 at two days following the administration.

In some embodiments, a suitable dose further provides the human subject with at least a 2% increase in absolute change in ppFEV1 from baseline ppFEV1 at one week following the administration. In some embodiments, a suitable dose further provides the human subject with at least a 3% increase in absolute change in ppFEV1 from baseline ppFEV1 at one week following the administration. In some embodiments, a suitable dose further provides the human subject with at least a 4% increase in absolute change in ppFEV1 from baseline ppFEV1 at one week following the administration. In some embodiments, a suitable dose further provides the human subject with at least a 5% increase in absolute change in ppFEV1 from baseline ppFEV1 at one week following the administration. In some embodiments, a suitable dose further provides the human subject with at least a 7% increase in absolute change in ppFEV1 from baseline ppFEV1 at one week following the administration. In some embodiments, a suitable dose further provides the human subject with at least a 10% increase in absolute change in ppFEV1 from baseline ppFEV1 at one week following the administration. In some embodiments, a suitable dose further provides the human subject with at least a 12% increase in absolute change in ppFEV1 from baseline ppFEV1 at one week following the administration.

In some embodiments, the increase in ppFEV1 is the maximum absolute change from baseline through the treatment period. In some embodiments, a suitable dose further provides the human subject with at least a 4% maximum increase in absolute change in ppFEV1 from baseline ppFEV1 at one week following the administration. In some embodiments, a suitable dose further provides the human subject with at least a 5% maximum increase in absolute change in ppFEV1 from baseline ppFEV1 at one week following the administration. In some embodiments, a suitable dose further provides the human subject with at least a 6% maximum increase in absolute change in ppFEV1 from baseline ppFEV1 at one week following the administration. In some embodiments, a suitable dose further provides the human subject with at least a 7% maximum increase in absolute change in ppFEV1 from baseline ppFEV1 at one week following the administration. In some embodiments, a suitable dose further provides the human subject with at least an 8% maximum increase in absolute change in ppFEV1 from baseline ppFEV1 at one week following the administration. In some embodiments, a suitable dose further provides the human subject with at least a 10% maximum increase in absolute change in ppFEV1 from baseline ppFEV1 at one week following the administration. In some embodiments, a suitable dose further provides the human subject with at least a 12% maximum increase in absolute change in ppFEV1 from baseline ppFEV1 at one week following the administration. In some embodiments, a suitable dose further provides the human subject with at least a 15% maximum increase in absolute change in ppFEV1 from baseline ppFEV1 at one week following the administration. In some embodiments, a suitable dose further provides the human subject with at least an 18% maximum increase in absolute change in ppFEV1 from baseline ppFEV1 at one week following the administration. In some embodiments, a suitable dose further provides the human subject with at least a 20% maximum increase in absolute change in ppFEV1 from baseline ppFEV1 at one week following the administration

In one aspect, the present invention provides a method of treating cystic fibrosis (CF) in a human subject comprising nebulizing a composition comprising an mRNA encoding a Cystic Fibrosis Transmembrane Conductance Regulator (CFTR) protein at a dose between 7 mg and 25 mg at a regular interval and/or for a treatment period sufficient to achieve an increase in ppFEV1 (percent predicted forced expiratory volume in one second) from baseline by at least 3%.

In some embodiments, a suitable regular interval is once a week. In some embodiments, a suitable regular interval is twice a week, once every two weeks, once every three weeks, once every four weeks, monthly, once every two months, once every four months, once every six months, or yearly.

In some embodiments, a suitable treatment period is at least a week. In some embodiment, a suitable treatment period is at least two weeks. In some embodiments, a suitable treatment period is at least three weeks. In some embodiments, a suitable treatment period is at least four weeks. In some embodiments, a suitable treatment period is at least five weeks. In some embodiments, a suitable treatment period is at least six weeks. In some embodiments, a suitable treatment period is at least eight weeks. In some embodiments, a suitable treatment period is at least three months. In some embodiments, a suitable treatment period is at least four months. In some embodiments, a suitable treatment period is at least five months. In some embodiments, a suitable treatment period is at least six months. In some embodiments, a suitable treatment period is at least one year. In some embodiments, a suitable treatment period is at least two years. In some embodiments, a suitable treatment period is at least three years. In some embodiments, a suitable treatment period is at least five years. In some embodiments, a suitable treatment period is at least ten years. In some embodiments, a suitable treatment period is at least twenty years. In some embodiments, a suitable treatment period is at least thirty years. In some embodiments, a suitable treatment period is at least fifty years. In some embodiments, a suitable treatment period is during the life of a patient.

In some embodiments, the composition is nebulized for a period to achieve an increase in ppFEV1 from the baseline by at least 4%. In some embodiments, the composition is nebulized for a period to achieve an increase in ppFEV1 from the baseline by at least 5%. In some embodiments, the composition is nebulized for a period to achieve an increase in ppFEV1 from the baseline by at least 6%. In some embodiments, the composition is nebulized for a period to achieve an increase in ppFEV1 from the baseline by at least 7%. In some embodiments, the composition is nebulized for a period to achieve an increase in ppFEV1 from the baseline by at least 8%. In some embodiments, the composition is nebulized for a period to achieve an increase in ppFEV1 from the baseline by at least 9%. In some embodiments, the composition is nebulized for a period to achieve an increase in ppFEV1 from the baseline by at least 10%. In some embodiments, the composition is nebulized for a period to achieve an increase in ppFEV1 from the baseline by at least 11%. In some embodiments, the composition is nebulized for a period to achieve an increase in ppFEV1 from the baseline by at least 12%. In some embodiments, the composition is nebulized for a period to achieve an increase in ppFEV1 from the baseline by at least 13%. In some embodiments, the composition is nebulized for a period to achieve an increase in ppFEV1 from the baseline by at least 14%. In some embodiments, the composition is nebulized for a period to achieve an increase in ppFEV1 from the baseline by at least 15%. In some embodiments, the composition is nebulized for a period to achieve an increase in ppFEV1 from the baseline by at least 20%. In some embodiments, the composition is nebulized for a period to achieve an increase in ppFEV1 from the baseline by at least 25%. In some embodiments, the composition is nebulized for a period to achieve an increase in ppFEV1 from the baseline by at least 30%.

In some embodiments, the increase in ppFEV1 is measured at day two post nebulization. In some embodiments, the increase in ppFEV1 is measured at day three post nebulization. In some embodiments, the increase in ppFEV1 is measured at day four post nebulization. In some embodiments, the increase in ppFEV1 is measured at day five post nebulization. In some embodiments, the increase in ppFEV1 is measured at day six post nebulization. In some embodiments, the increase in ppFEV1 is measured at week one post nebulization. In some embodiments, the increase in ppFEV1 is measured at day eight post nebulization. In some embodiments, the increase in ppFEV1 is measured at day ten post nebulization. In some embodiments, the increase in ppFEV1 is measured at day twelve post nebulization. In some embodiments, the increase in ppFEV1 is measured at week two post nebulization. In some embodiments, the increase in ppFEV1 is measured at week three post nebulization. In some embodiments, the increase in ppFEV1 is measured at one month post nebulization. In some embodiments, the increase in ppFEV1 is measured at the end of the treatment period. In some embodiments, the increase in ppFEV1 is measured at the beginning of the following treatment period.

In some embodiments, the human subject is at risk of cystic fibrosis. In some embodiments, the human subject is suffering from cystic fibrosis. In some embodiments, the human subject is suffering from or at risk of chronic obstructive pulmonary disorder (COPD). In some embodiments, the human subject is suffering from or at risk of cystic fibrosis and chronic obstructive pulmonary disorder (COPD). In some embodiments, the human subject is suffering from or at risk of chronic obstructive pulmonary disorder (COPD) but not cystic fibrosis.

In some embodiments, the human subject has a class I mutation. In some embodiments, the human subject has a class II mutation. In some embodiments, the human subject has a class I mutation and a class II mutation. In some embodiments, the human subject has a mutation selected from the mutations provided in Table 1.

In some embodiments, the human subject has an F508del mutation. In some embodiments, the human subject does not have an F508del mutation. In some embodiments, the F508del mutation is heterozygous. In some embodiments, the F508del mutation is homozygous.

In some embodiments, the method first includes a step of selecting the human subject for treatment based on the presence of a class I and/or class II mutation. In some embodiments, the method first includes a step of selecting the human subject for treatment based on the absence of an F508del mutation.

In some embodiments, the human subject receives concomitant CFTR modulator therapy. In some embodiments, the concomitant CFTR modulator therapy comprises ivacaftor. In some embodiments, the concomitant CFTR modulator therapy comprises lumacaftor. In some embodiments, the concomitant CFTR modulator therapy comprises tezacaftor. In some embodiments, the concomitant CFTR modulator therapy is selected from ivacaftor, lumacaftor, tezacaftor, or a combination. In some embodiments, the concomitant CFTR modulator therapy comprises VX-659. In some embodiments, the concomitant CFTR modulator therapy comprises VX-445. In some embodiments, the concomitant CFTR modulator therapy comprises VX-152. In some embodiments, the concomitant CFTR modulator therapy comprises VX-440. In some embodiments, the concomitant CFTR modulator therapy comprises VX-371. In some embodiments, the concomitant CFTR modulator therapy comprises VX-561. In some embodiments, the concomitant CFTR modulator therapy comprises GLPG1837. In some embodiments, the concomitant CFTR modulator therapy comprises GLPG2222. In some embodiments, the concomitant CFTR modulator therapy comprises GLPG2737. In some embodiments, the concomitant CFTR modulator therapy comprises GLPG2451. In some embodiments, the concomitant CFTR modulator therapy comprises GLPG1837. In some embodiments, the concomitant CFTR modulator therapy comprises PTI-428. In some embodiments, the concomitant CFTR modulator therapy comprises PTI-801. In some embodiments, the concomitant CFTR modulator therapy comprises PTI-808. In some embodiments, the concomitant CFTR modulator therapy comprises eluforsen.

In some embodiments, the human subject is not eligible for treatment with one or more of ivacaftor, lumacaftor, tezacaftor, VX-659, VX-445, VX-152, VX-440, VX-371, VX-561, VX-659 or combinations thereof. In some embodiments, the human subject is not eligible for treatment with one or more of ivacaftor, lumacaftor, tezacaftor, VX-659, VX-445, VX-152, VX-440, VX-371, VX-561, VX-659, GLPG1837, GLPG2222, GLPG2737, GLPG2451, GLPG1837, PTI-428, PTI-801, PTI-808, eluforsen, or combinations thereof.

In some embodiments, the baseline ppFEV1 is measured in the human subject following prior administration to the human subject of the concomitant CFTR modulator therapy.

In some embodiments, the human subject has the baseline ppFEV1 of between about 10% and 95% of predicted normal. In some embodiments, the human subject has the baseline ppFEV1 of between about 20% and 90% of predicted normal. In some embodiments, the human subject has the baseline ppFEV1 of between about 50% and 80% of predicted normal. In some embodiments, the human subject has the baseline ppFEV1 of between about 50% and 60% of predicted normal. In some embodiments, the human subject has the baseline ppFEV1 of between about 60% and 70% of predicted normal. In some embodiments, the human subject has the baseline ppFEV1 of between about 70% and 80% of predicted normal.

In some embodiments, the mRNA comprises a nucleotide sequence of SEQ ID NO: 28.

In some embodiments, the mRNA comprises a 5' Cap with a structure of

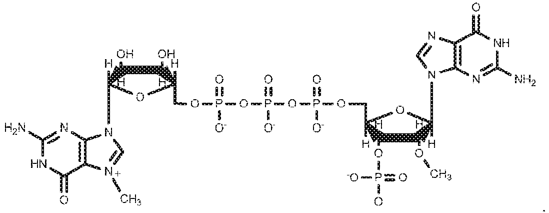

In some embodiments, the mRNA has a capping level of at least 70%. In some embodiments, the mRNA has a capping level of at least 80%. In some embodiments, the mRNA has a capping level of at least 90%. In some embodiments, the mRNA has a capping level of at least 95%. In some embodiments, the mRNA has a capping level of at least 99%.

In some embodiments, the mRNA is unmodified.

In some embodiments, the mRNA is encapsulated in lipid nanoparticle.

In some embodiments, each lipid nanoparticle comprises a PEG-modified lipid. In some embodiments, the lipid nanoparticle comprises the PEG-modified lipid at a molar ratio of 3% or greater of the total lipid content of the lipid nanoparticle. In some embodiments, the lipid nanoparticle comprises the PEG-modified lipid at a molar ratio of 4% or greater of the total lipid content of the lipid nanoparticle. In some embodiments, the lipid nanoparticle comprises the PEG-modified lipid at a molar ratio of 5% or greater of the total lipid content of the lipid nanoparticle.

In some embodiments, the lipid nanoparticles have an encapsulation level of at least 80%. In some embodiments, the lipid nanoparticles have an encapsulation level of at least 90%. In some embodiments, the lipid nanoparticles have an encapsulation level of at least 95%. In some embodiments, the lipid nanoparticles have an encapsulation level of at least 98%.

In some embodiments, the composition is an aqueous solution comprising the lipid nanoparticles.

In some embodiments, the concentration of the mRNA encoding the CFTR protein ranges from 0.1 mg/mL to 1.0 mg/mL. In some embodiments, the concentration of the mRNA encoding the CFTR protein ranges from 0.5 mg/mL to 0.8 mg/mL. In some embodiments, a suitable concentration of the mRNA encoding the CFTR protein is 0.6 mg/mL.

In some embodiments, method comprises first reconstituting lyophilized dry powder into the aqueous solution prior to nebulization.

In some embodiments, each lipid nanoparticle has only three lipid components. In some embodiments, the suitable three lipid components are a cationic lipid, a helper lipid and a PEG-modified lipid.

In some embodiments, a suitable molar ratio of cationic lipid:helper lipid:PEG-modified lipid in each lipid nanoparticle is 60:35:5.

In some embodiments, a suitable cationic lipid is imidazole cholesterol ester (ICE), a suitable helper lipid is 1,2-dioleoyl-sn-glycero-3-phosphoethanolamine (DOPE), and a suitable PEG-modified lipid is 1,2-dimyristoyl-sn-glycerol, methoxypolyethylene glycol (DMG-PEG-2K. In some embodiments, a suitable molar ratio of ICE:DOPE:DMG-PEG-2K in each lipid nanoparticle is 60:35:5.

In some embodiments, the lipid nanoparticles have an average size ranging from 30 nm to 80 nm. In some embodiments, the lipid nanoparticles have an average size ranging from 40 nm to 60 nm. In some embodiments, the lipid nanoparticles have an average size of less than about 80 nm. In some embodiments, the lipid nanoparticles have an average size of less than about 70 nm. In some embodiments, the lipid nanoparticles have an average size of less than about 120 nm. In some embodiments, the lipid nanoparticles have an average size of less than about 110 nm. In some embodiments, the lipid nanoparticles have an average size of less than about 100 nm. In some embodiments, the lipid nanoparticles have an average size of less than about 90 nm. In some embodiments, the lipid nanoparticles have an average size of less than about 80 nm. In some embodiments, the lipid nanoparticles have an average size of less than about 70 nm. In some embodiments, the lipid nanoparticles have an average size of less than about 60 nm. In some embodiments, the lipid nanoparticles have an average size of less than about 50 nm. In some embodiments, the lipid nanoparticles have an average size of less than about 40 nm. In some embodiments, the lipid nanoparticles have an average size of less than about 30 nm.

In some embodiments, the composition comprises trehalose. In some embodiments, the trehalose is present at a concentration of at least 5% (w/v). In some embodiments, the trehalose is present at a concentration of at least 10% (w/v). In some embodiments, the trehalose is present at a concentration of at least 15% (w/v).

In some embodiments, the composition is nebulized at a rate ranging from 0.1 mL/minute to 0.6 mL/minute. In some embodiments, the composition is nebulized at a rate ranging from 0.2 mL/minute to 0.5 mL/minute. In some embodiments, the composition is nebulized at a rate ranging from 0.3 mL/minute to 0.4 mL/minute.

In some embodiments, the composition is nebulized using a vibrating mesh nebulizer.

### BRIEF DESCRIPTION OF THE DRAWINGS

The drawings are for illustration purposes only not for limitation.
**Figure 1** depicts an exemplary graphical representation of mean (SE) ppFEV1 for each dose group by visit through day 8 after administration.
**Figure 2** depicts an exemplary graphical representation of absolute change from baseline in ppFEV1 for each dose group by visit throughout the 8 days after administration.
**Figure 3** depicts an exemplary bar graph representation of an absolute change from baseline in ppFEV1 for each dose group by visit through day 8.

### DEFINITIONS

In order for the present invention to be more readily understood, certain terms are first defined below. Additional definitions for the following terms and other terms are set forth throughout the specification. The publications and other reference materials referenced herein are to describe the background of the invention and to provide additional detail regarding its practice

*Approximately or about*: As used herein, the term "approximately" or "about," as applied to one or more values of interest, refers to a value that is similar to a stated reference value. In certain embodiments, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

*Delivery.* As used herein, the term "delivery" encompasses both local and systemic delivery. For example, delivery of mRNA encompasses situations in which an mRNA is delivered to a target tissue and the encoded protein is expressed and retained within the target tissue (also referred to as "local distribution" or "local delivery"), and situations in which an mRNA is delivered to a target tissue and the encoded protein is expressed and secreted into patient's circulation system (e.g., serum) and systematically distributed and taken up by other tissues (also referred to as "systemic distribution" or "systemic delivery). In some embodiments, delivery is pulmonary delivery, e.g., comprising nebulization.

*Drug product:* As used herein, "drug product" refers to a finished dosage form, e.g., tablet, capsule, or solution that contains the active drug ingredient, generally, but not necessarily, in association with inactive ingredients.

*Encapsulation:* As used herein, the term "encapsulation," or its grammatical equivalent, refers to the process of confining an mRNA molecule within a nanoparticle.

*Expression*: As used herein, "expression" of a nucleic acid sequence refers to translation of an mRNA into a polypeptide, assemble multiple polypeptides (e.g., heavy chain or light chain of antibody) into an intact protein (e.g., antibody) and/or post-translational modification of a polypeptide or fully assembled protein (e.g., antibody). In this application, the terms "expression" and "production," and their grammatical equivalents, are used interchangeably.

*FEV1 or pptEV1*: As used herein, the term "FEV1" means forced expiratory volume in one second. The term "ppFEV 1" refers to percent predicted force expiratory volume in one second compared to normal (*i.e.,* the average FEV1 of non-CF patients). The baseline ppFEV1 is measured in the human subject prior administration of the treatment of the present invention.

*Functional*: As used herein, a "functional" biological molecule is a biological molecule in a form in which it exhibits a property and/or activity by which it is characterized.

*Half-life*: As used herein, the term "half-life" is the time required for a quantity such as nucleic acid or protein concentration or activity to fall to half of its value as measured at the beginning of a time period.

*Homozygous or heterozygous*: As used herein, a patient who is "homozygous" for a particular gene mutation has the same mutation on each allele. The term "heterozygous" as used herein, refers to a patient having a particular gene mutation on one allele, and a different mutation or no mutation on the other allele. Patients that may benefit from the methods of treatment of the invention and from the compositions described herein for use in treating CFTR-mediated diseases include patients who have homozygous or heterozygous mutations on the CFTR gene, but also have a residual function phenotype.

*Improve, increase, or reduce*: As used herein, the terms "improve," "increase" or "reduce," or grammatical equivalents, indicate values that are relative to a baseline measurement, such as a measurement in the same individual prior to initiation of the treatment described herein, or a measurement in a control subject (or multiple control subject) in the absence of the treatment described herein. A "control subject" is a subject afflicted with the same form of disease as the subject being treated, who is about the same age as the subject being treated.

*In Vitro*: As used herein, the term *"in vitro"* refers to events that occur in an artificial environment, e.g., in a test tube or reaction vessel, in cell culture, *etc.,* rather than within a multicellular organism.

*in Vivo*: As used herein, the term *"in vivo"* refers to events that occur within a multicellular organism, such as a human and a non-human animal. In the context of cell-based systems, the term may be used to refer to events that occur within a living cell (as opposed to, for example, *in vitro* systems).

*Isolated*: As used herein, the term "isolated" refers to a substance and/or entity that has been (1) separated from at least some of the components with which it was associated when initially produced (whether in nature and/or in an experimental setting), and/or (2) produced, prepared, and/or manufactured by the hand of man. Isolated substances and/or entities may be separated from about 10%, about 20%, about 30%, about 40%, about 50%, about 60%, about 70%, about 80%, about 90%, about 91%, about 92%, about 93%, about 94%, about 95%, about 96%, about 97%, about 98%, about 99%, or more than about 99% of the other components with which they were initially associated. In some embodiments, isolated agents are about 80%, about 85%, about 90%, about 91%, about 92%, about 93%, about 94%, about 95%, about 96%, about 97%, about 98%, about 99%, or more than about 99% pure. As used herein, a substance is "pure" if it is substantially free of other components. As used herein, calculation of percent purity of isolated substances and/or entities should not include excipients (*e.g.,* buffer, solvent, water, *etc*.).

*messenger RNA (mRNA):* As used herein, the term "messenger RNA (mRNA)" refers to a polynucleotide that encodes at least one polypeptide. mRNA as used herein encompasses both modified and unmodified RNA. mRNA may contain one or more coding and non-coding regions. mRNA can be purified from natural sources, produced using recombinant expression systems and optionally purified, chemically synthesized, *etc.* Where appropriate, *e.g.,* in the case of chemically synthesized molecules, mRNA can comprise nucleoside analogs such as analogs having chemically modified bases or sugars, backbone modifications, *etc.* An mRNA sequence is presented in the 5' to 3' direction unless otherwise indicated.

*Modulator:* As used herein, the term "modulator" refers to a compound that alters or increases the activity of a biological compound such as a protein. For example, a CFTR modulator is a compound that generally increases the activity of CFTR. The increase in activity resulting from a CFTR modulator includes but is not limited to compounds that correct, potentiate, stabilize and/or amplify CFTR.

*Nominal dose:* As used herein, the term "nominal dose" refers to a dose of a mRNA administered to a subject by nebulization. The nominal dose may not be identical to the dose actually delivered to the subject. For example, if a human subject is given a nominal dose of 8 µg of a CFTR mRNA composition disclosed herein, the actual dose that is delivered to the lungs of the subject may vary, *e.g.,* depending on the nebulization parameters used to administer the composition. The actual dose cannot exceed the nominal dose, but typically the actual dose of mRNA delivered by nebulization to the lungs of the human subject is lower than the nominal dose that is administered via the nebulizer.

*N*/*P Ratio:* As used herein, the term "N/P ratio" refers to a molar ratio of positively charged molecular units in the cationic lipids in a lipid nanoparticle relative to negatively charged molecular units in the mRNA encapsulated within that lipid nanoparticle. As such, N/P ratio is typically calculated as the ratio of moles of amine groups in cationic lipids in a lipid nanoparticle relative to moles of phosphate groups in mRNA encapsulated within that lipid nanoparticle.

*Nucleic acid:* As used herein, the term "nucleic acid," in its broadest sense, refers to any compound and/or substance that is or can be incorporated into a polynucleotide chain. In some embodiments, a nucleic acid is a compound and/or substance that is or can be incorporated into a polynucleotide chain via a phosphodiester linkage. In some embodiments, "nucleic acid" refers to individual nucleic acid residues (*e.g*., nucleotides and/or nucleosides). In some embodiments, "nucleic acid" refers to a polynucleotide chain comprising individual nucleic acid residues. In some embodiments, "nucleic acid" encompasses RNA as well as single and/or double-stranded DNA and/or cDNA. Furthermore, the terms "nucleic acid," "DNA," "RNA," and/or similar terms include nucleic acid analogs, *i.e.,* analogs having other than a phosphodiester backbone. For example, the so-called "peptide nucleic acids," which are known in the art and have peptide bonds instead of phosphodiester bonds in the backbone, are considered within the scope of the present invention. The term "nucleotide sequence encoding an amino acid sequence" includes all nucleotide sequences that are degenerate versions of each other and/or encode the same amino acid sequence. Nucleotide sequences that encode proteins and/or RNA may include introns. Nucleic acids can be purified from natural sources, produced using recombinant expression systems and optionally purified, chemically synthesized, *etc.* Where appropriate, *e.g.,* in the case of chemically synthesized molecules, nucleic acids can comprise nucleoside analogs such as analogs having chemically modified bases or sugars, backbone modifications, *etc.* A nucleic acid sequence is presented in the 5' to 3' direction unless otherwise indicated. In some embodiments, a nucleic acid is or comprises natural nucleosides (*e.g*., adenosine, thymidine, guanosine, cytidine, uridine, deoxyadenosine, deoxythymidine, deoxyguanosine, and deoxycytidine); nucleoside analogs (*e.g*., 2-aminoadenosine, 2-thiothymidine, inosine, pyrrolo-pyrimidine, 3-methyl adenosine, 5-methylcytidine, C-5 propynyl-cytidine, C-5 propynyl-uridine, 2-aminoadenosine, C5-bromouridine, C5-fluorouridine, C5-iodouridine, C5-propynyl-uridine, C5-propynyl-cytidine, C5-methylcytidine, 2-aminoadenosine, 7-deazaadenosine, 7-deazaguanosine, 8-oxoadenosine, 8-oxoguanosine, O(6)-methylguanine, and 2-thiocytidine); chemically modified bases; biologically modified bases (*e.g*., methylated bases); intercalated bases; modified sugars (*e.g*., 2'-fluororibose, ribose, 2'-deoxyribose, arabinose, and hexose); and/or modified phosphate groups (*e.g*., phosphorothioates and 5'-N-phosphoramidite linkages). In some embodiments, the present invention is specifically directed to "unmodified nucleic acids," meaning nucleic acids (*e.g*., polynucleotides and residues, including nucleotides and/or nucleosides) that have not been chemically modified in order to facilitate or achieve delivery. In some embodiments, the nucleotides T and U are used interchangeably in sequence descriptions.

*Patient:* As used herein, the term "patient" or "subject" refers to any organism to which a provided composition may be administered, *e.g*., for experimental, diagnostic, prophylactic, cosmetic, and/or therapeutic purposes. Typical patients include animals (*e.g*., mammals such as mice, rats, rabbits, non-human primates, and/or humans). In specific embodiments, a patient is a human. A human includes pre- and post-natal forms.

*Pharmaceutically acceptable:* The term "pharmaceutically acceptable" as used herein, refers to substances that, within the scope of sound medical judgment, are suitable for use in contact with the tissues of human beings and animals without excessive toxicity, irritation, allergic response, or other problem or complication, commensurate with a reasonable benefit/risk ratio.

*Subject*: As used herein, the term "subject" refers to a human or any non-human animal (e.g., mouse, rat, rabbit, dog, cat, cattle, swine, sheep, horse or primate). A human includes pre- and post-natal forms. In many embodiments, a subject is a human being. A subject can be a patient, which refers to a human presenting to a medical provider for diagnosis or treatment of a disease. The term "subject" is used herein interchangeably with "individual" or "patient." A subject can be afflicted with or is susceptible to a disease or disorder but may or may not display symptoms of the disease or disorder.

*Substantially*: As used herein, the term "substantially" refers to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "substantially" is therefore used herein to capture the potential lack of completeness inherent in many biological and chemical phenomena.

*Treating*: As used herein, the term "treat," "treatment," or "treating" refers to any method used to partially or completely alleviate, ameliorate, relieve, inhibit, prevent, delay onset of, reduce severity of and/or reduce incidence of one or more symptoms or features of a particular disease, disorder, and/or condition. Treatment may be administered to a subject who does not exhibit signs of a disease and/or exhibits only early signs of the disease for the purpose of decreasing the risk of developing pathology associated with the disease.

### DETAILED DESCRIPTION

The present invention provides, among other things, an improved method of treating cystic fibrosis (CF) in a human subject. In some embodiments, the invention relates to a composition comprising an mRNA encoding a Cystic Fibrosis Transmembrane Conductance Regulator (CFTR) protein for use in a method of treating cystic fibrosis (CF) in a human subject, wherein the method comprises administration of the composition by nebulization at a dose between 13 mg and 19 mg that it provides the human subject with at least a 3% increase in absolute change in ppFEV1 (percent predicted forced expiratory volume in one second) from baseline ppFEV1 at two days following the administration. In addition or alternatively, the dose is selected to provide the human subject with at least a 2% increase in absolute change in ppFEV1 from baseline ppFEV1 at one week following the administration. In addition or alternatively, the dose is selected to provide the human subject with at least a 4% maximum increase in absolute change in ppFEV1 from baseline ppFEV1 through one week following administration.

Various aspects of the invention are described in detail in the following sections. The use of sections is not meant to limit the invention. Each section can apply to any aspect of the invention. In this application, the use of "or" means "and/or" unless stated otherwise.

### Cystic Fibrosis

Cystic fibrosis, also known as mucoviscidosis, is an autosomal recessive genetic disorder that affects most critically the lungs, and also the pancreas, liver, and intestine (Gibson et al., Am J Respir Crit Care Med. (2003) 168(8):918-951; Ratjen et al.., Lancet Lond Engl. (2003) 361(9358):681-689; O'Sullivan et al., Lancet Lond Engl. (2009) 373(9678):1891-1904). Cystic fibrosis is caused by mutations in the gene encoding for the cystic fibrosis transmembrane conductance regulator (CFTR) protein. This protein functions as a channel that transports chloride ions across the membrane of cells and is required to regulate the components of mucus, sweat, saliva, tears, and digestive enzymes. Disease-causing mutations in the CFTR protein cause dysfunction of its channel activity resulting in abnormal transport of chloride and sodium ions across the epithelium, leading to the thick, viscous secretions in the lung, pancreas and other organs characteristic of CF disease (O'Sulliven et al.., Lancet Lond Engl. (2009) 373(9678):1891-1904; Rowe et al., N Engl J Med. (2005) 352(19):1992-2001). Most CF patients develop severe, chronic lung disease related to airway obstruction partly due to increased levels of sulfated mucins, inflammation, and recurrent infections that are eventually lethal; the median predicted survival age in the US is 40.7 years. Cystic fibrosis is the most frequent lethal genetic disease in the white population.

Symptoms often appear in infancy and childhood, with respiratory symptoms the most frequent followed by failure to thrive, steatorrhea, and meconium ileus (Gibson et al., Am J Respir Crit Care Med. (2003) 168(8):918-951). The most common complications of CF are pulmonary related and include blockages of the narrow passages of affected organs with thickened secretions. These blockages lead to remodeling and infection in the lung, cause damage in the pancreas due to accumulated digestive enzymes, and blockages of the intestines. Diabetes is the most common non-pulmonary complication and is a distinct entity known as CF-related diabetes.

The lungs of individuals with CF are colonized and infected by bacteria from an early age. This leads to chronic airway infection and inflammation, progressing to bronchiectasis, gas trapping, hypoxemia, and hypercarbia. Pulmonary insufficiency is responsible for 68.1% of CF-related deaths in the US. In the initial stage, common bacteria such as *Staphylococcus aureus* and *Hemophilus influenzae* colonize and infect the lungs. Eventually, *Pseudomonas aeruginosa* (and sometimes *Burkholderia cepacia*) dominates. By 18 years of age, 80% of patients with classic CF harbor *P. aeruginosa,* and 3.5% harbor *B. cepacia.* Once within the lungs, these bacteria adapt to the environment and develop resistance to commonly used antibiotics.

The underlying defect causing CF is abnormal epithelial anion transport due to the lack of expression or dysfunction of the CFTR protein. The CFTR protein primarily functions as a chloride channel in epithelial cell membranes; however, it also involved in a number of other cellular membrane functions such as inhibition of sodium transport through the epithelial sodium channel, regulation of the outwardly rectifying chloride channel, and regulation of adenosine triphosphate (ATP) channels (O'Sullivan et al., Lancet Lond Engl. (2009) 373(9678):1891-1904). CF is caused by mutations in the gene encoding for the CFTR protein, of which more than 1,500 disease-causing mutations have been identified (O'Sullivan et al., Lancet Lond Engl. (2009) 373(9678):1891-1904). The more common gene mutations result in the lack of synthesis of the CFTR protein (class I), defective processing and maturation of the CFTR protein (class II), or the expression of a CFTR protein defective in regulation, *e.g.,* diminished ATP binding and hydrolysis (class III) (Rowe et al., N Engl J Med. (2005) 352(19):1992-2001). A deletion of phenylalanine at position 508 (F508del) is the most common CFTR mutation worldwide and is a class II defect in which the misfolded protein is rapidly degraded by the cell soon after synthesis (Rowe et al., N Engl J Med. (2005) 352(19):1992-2001). The lack of a functional CFTR protein causes mucosal obstruction of exocrine glands in CF patients secondary to abnormal transport of chloride and sodium across the epithelium. In the lung, this leads to the development of thick, tenacious secretions that obstruct the airways and submucosal glands, which in turn leads to chronic bacterial infection and inflammation, as described above.

Respiratory symptoms of cystic fibrosis include: a persistent cough that produces thick mucus (sputum), wheezing, breathlessness, exercise intolerance, repeated lung infections and inflamed nasal passages or a stuffy nose. Digestive symptoms of cystic fibrosis include: foulsmelling, greasy stools, poor weight gain and growth, intestinal blockage, particularly in newborns (meconium ileus), and severe constipation.

There are several different methods for assessing symptoms of cystic fibrosis. In one embodiment, one or more symptoms of cystic fibrosis are assessed by forced expiratory volume (FEV), which measures how much air a person can exhale during a forced breath. In one embodiment, the amount of air exhaled in the first second of the forced breath is measured (FEV₁). In one embodiment, the amount of air exhaled in the second of the forced breath is measured (FEV₂). In one embodiment, the amount of air exhaled in the third second of the forced breath is measured (FEV₃). In one embodiment, the forced vital capacity (FVC), which is the total amount of air exhaled during a FEV test, is measured. In one embodiment, one or more symptoms of cystic fibrosis are assessed by Cystic Fibrosis Questionnaire Revise (CFQ-R) respiratory domain score. CFQ-R respiratory domain score is a measure of respiratory symptoms relevant to patients with CF such as cough, sputum production, and difficulty breathing. In one embodiment, one or more symptoms of cystic fibrosis are assessed by relative risk of pulmonary exacerbation. In one embodiment, one or more symptoms of cystic fibrosis are assessed by change in body weight. In one embodiment, one or more symptoms of cystic fibrosis are assessed by change in sweat chloride (mmol/L).

### Chronic Obstructive Pulmonary Disease

Various molecular, cellular and clinical studies have confirmed that CFTR protein dysfunction is common in both the cystic fibrosis (CF) and chronic obstructive pulmonary disease (COPD). Accordingly, without being bound by any particular theory, the inventors believe that human subjects suffering from or at risk of developing COPD benefit from the dosing regimens described herein in the context of treating CF.

Therefore, in certain aspects, the invention also relates to a method of treating chronic obstructive pulmonary disorder (COPD) in a human subject. In particular, the invention relates to a method of treating or preventing chronic obstructive pulmonary disorder (COPD) in a human subject suffering from or at risk of developing COPD comprising administration of a composition comprising an mRNA encoding a Cystic Fibrosis Transmembrane Conductance Regulator (CFTR) protein by nebulization. In some embodiments, the human subject suffers from CF and COPD. Typically, the mRNA encoding the CFTR protein is administered a dose between 7 mg and 25 mg.

### Patient Selection

The present invention is suitable for treatment of human patients with various CFTR defects including, but not limited to, patients with different CFTR symptoms, mutations or classes described herein.

In some embodiments, the human subject is suffering from or at risk of chronic obstructive pulmonary disorder (COPD). In some embodiments, the human subject suffering from or at risk of COPD is not suffering from cystic fibrosis. In some embodiments, the human subject suffering from or at risk of COPD is suffering from cystic fibrosis. In some embodiments, the human subject is at risk of cystic fibrosis. In some embodiments, the human subject is suffering from cystic fibrosis.

In some embodiments, the present invention may be used to treat patients carrying one or more, two or more, three or more, four or more, or five or more mutations from Class I (Defective Protein Synthesis) shown in Table 1. In some embodiments, the present invention may be used to treat patients carrying one or more, two or more, three or more, four or more, or five or more mutations from Class II (Abnormal Processing and Trafficking) shown in Table 1. In some embodiments, the present invention may be used to treat patients carrying one or more, two or more, three or more, four or more, or five or more mutations from Class III (Defective Chanel Regulation/Gating) shown in Table 1. In some embodiments, the present invention may be used to treat patients carrying one or more, two or more, three or more, four or more, or five or more mutations from Class IV (Decreased Channel Conductance) shown in Table 1. In some embodiments, the present invention may be used to treat patients carrying one or more, two or more, three or more, four or more, or five or more mutations from Class V (Reduced Synthesis and/or Trafficking) shown in Table 1. In some embodiments, the present invention may be used to treat patients carrying any combination of specific mutations selected from Table 1 (*e.g.,* one or more, two or more, three or more, four or more, five or more, six or more, seven or more, eight or more, nine or more, or ten or more mutations from different classes shown in Table 1).

**Table 1. Classification of CFTR Gene Mutations**

| Category | Mutation | Specific mutations |
|---|---|---|
| Class I | Defective Protein Synthesis (nonsense, frameshift, aberrant splicing) | 1078delT, 1154 insTC, 1525-2A > G, 1717-1G > A, 1898-1G > A, 2184delA, 2184 insA, 3007delG, 3120+1G > A, 3659delC, 3876delA, 3905insT, 394delTT, 4010del4, 4016insT, 4326delTC, 4374+1G> T, 441delA, 556delA, 621+1G > T, 621-1G > T, 711+1G > T, 875+1G > C, E1104X, E585X, E60X, E822X, G542X, G551D/R553X, Q493X, Q552X, Q814X, R1066C, R1162X, R553X, V520F, W1282X, Y1092X |
| Class II | Abnormal Processing and Trafficking | A559T, D979A, ΔF508 (including F508del), ΔI507, G480C, G85E, N1303K, S549I, S549N, S549R |
| Class III | Defective Chanel Regulation/Gating | G1244E, G1349D, G551D, G551S, G85E, H199R, I1072T, I48T, L1077P, R560T, S1255P, S549N (R75Q) |
| Class IV | Decreased Channel Conductance | A800G, D1152H, D1154G, D614G, delM1140, E822K, G314E, G576A, G622D, G85E, H620Q, I1139V, I1234V, L1335P, M1137V, P67L, R117C, R117P, R117H, R334W, R347H, R347P, R347P/R347H, R792G, S1251N, V232D |
| Class V | Reduced Synthesis and/or Trafficking | 2789+5G > A, 3120G > A, 3272-26A > G, 3849+10kbC > T, 5T variant, 621+3A > G, 711+3A > G, A445E, A455E, IVS8 poly T, P574H, 875+1G > C |

In some embodiments, a patient in need of treatment is a male or female of 2 years or older, or of 3 years or older, or of 6 years or older, or of 7 years or older, or of 12 years or older, or of 13 years or older, or of 18 years or older, or of 19 years or older, or of 25 years or older, or of 25 years or older, or of 30 years or older, or of 35 years or older, or of 40 years or older, or of 45 years or older, or of 50 years or older. In some embodiments, a patient in need of treatment is less than 50 years old, or less than 45 years old, or less than 40 years old, or less than 35 years old, or less than 30 years old, or less than 25 years old, or less than 20 years old, or less than 19 years old, or less than 18 years old, or less than 13 years old, or less than 12 years old, or less than 7 years old, or less than 6 years old, or less than 3 years old, or less than 2 years old. In some embodiments, a patient in need of treatment is a male or female from 2 to 18 years old, or from 2 to 12 years old, or from 2 to 6 years old, or from 6 to 12 years old, or from 6 to 18 years old, or from 12 to 16 years old, or from 2 to 50 years old, or from 6 to 50 years old, or from 12 to 50 years old, or from 18 to 50 years old. In some embodiments, a patient in need of treatment is a female who is pregnant or who may become pregnant.

In some embodiments, a patient is selected for treatment who has an F508del mutation. In some embodiments, the patient who is selected for treatment has a homozygous F508del mutation. In some embodiments, the patient who is selected for treatment has a heterozygous F508del mutation. In some embodiments, the patient who is selected for treatment does not have an F508del mutation.

In some embodiments, a patient in need of treatment has a sweat chloride value of ≥60 mmol/L, ≥65 mmol/L, ≥70 mmol/L, ≥75 mmol/L, ≥80 mmol/L, ≥85 mmol/L, ≥90 mmol/L, ≥95 mmol/L, ≥100 mmol/L, ≥110 mmol/L, ≥120 mmol/L, ≥130 mmol/L, ≥140 mmol/L or ≥150 mmol/L by quantitative pilocarpine iontophoresis (documented in the subject's medical record). In some embodiments, a patient in need of treatment has chronic sinopulmonary disease and/or gastrointestinal/nutritional abnormalities consistent with CF disease. In some embodiments, a patient in need of treatment has chronic sinopulmonary disease and/or gastrointestinal/nutritional abnormalities consistent with CF disease.

In some embodiments, a patient in need of treatment has FEV₁ ≥50% and ≤90% (*e.g.,* ≤85%, ≤80%, ≤75%, ≤70%, ≤65%, ≤60%, or ≤55%) of the predicted normal (*i.e.,* the average FEV of non-CF patients) based on the patient's age, gender, and height. In some embodiments, a patient in need of treatment has resting oxygen saturation ≥92% on room air (pulse oximetry). In some embodiments, a patient in need of treatment has a body mass index ≥17.5 kg/m² and weight ≥40 kg.

In some embodiments, a patient in need of treatment has received or is concurrently receiving other CF medications. For example, a patient in need of treatment may be receiving lumacaftor/ivacaftor combination drug (ORKAMBI^{®}) or may have been on this treatment for at least 28 days prior to commencement of the treatment according to the present invention. Other CF medications may include, but are not limited to, routine inhaled therapies directed at airway clearance and management of respiratory infections, such as bronchodilators, rhDNase (PULMOZYME^{®}), hypertonic saline, antibiotics, and steroids; and other routine CF-related therapies such as systemic antibiotics, pancreatic enzymes, multivitamins, and diabetes and liver medications.

In some embodiments, a patient in need of treatment has been a non-smoker for a minimum of 2 years. In some embodiments, a patient in need of treatment does not receive inhaled rhDNase (PULMOZYME^{®}) treatment for 24 hours before and/or after administration of a composition comprising an mRNA encoding a CFTR protein according to the present invention.

In some embodiments, a patient in need of treatment has been treated or is currently being treated with hormone replacement therapies, thyroid hormone replacement therapy, nonsteroidal inflammatory drugs, and prescription dronabinol (MARINOL^{®}) during treatment.

In some embodiments, a patient in need of treatment has discontinued use of one or more other cystic fibrosis treatments described herein. In some embodiments, the patient has discontinued use of one or more other cystic fibrosis treatments for at least 12 hours, at least 24 hours, at least 36 hours, at least 48 hours, at least 72 hours, at least 1 week, at least 2 weeks, at least 3 weeks, at least 4 weeks, at least 5 weeks, at least 6 weeks, at least 7 weeks, or at least 8 weeks prior to administration of a CFTR mRNA according to the present invention. In some embodiments, the patient has discontinued use of one or more other cystic fibrosis treatments for less than 12 hours, less than 24 hours, less than 36 hours, less than 48 hours, less than 72 hours, less than 1 week, less than 2 weeks, less than 3 weeks, less than 4 weeks, less than 5 weeks, less than 6 weeks, less than 7 weeks, less than 8 weeks, less than 9 weeks, or less than 10 weeks prior to administration of a CFTR mRNA according to the present invention.

### Formulation and Administration

According to the present invention, a suitable formulation for the treatment contains an mRNA encoding any full length, fragment or portion of a CFTR protein which can be substituted for naturally-occurring CFTR protein activity and/or reduce the intensity, severity, and/or frequency of one or more symptoms associated with cystic fibrosis.

In some embodiments, a suitable mRNA sequence is an mRNA sequence encoding a human CFTR (hCFTR) protein. In some embodiments, a suitable mRNA sequence is codon optimized for efficient expression human cells. An exemplary codon-optimized CFTR mRNA coding sequence and the corresponding amino acid sequence are shown in Table 2:

**Table 2. Exemplary CFTR mRNA and Protein Sequences**

| | |
|---|---|
| **Codon-Optimized Human CFTR mRNA** | AUGCAACGCUCUCCUCUUGAAAAGGCCUCGGUGGUGUCCAAGCUCUU CUUCUCGUGGACUAGACCCAUCCUGAGAAAGGGGUACAGACAGCGCU UGGAGCUGUCCGAUAUCUAUCAAAUCCCUUCCGUGGACUCCGCGGAC AACCUGUCCGAGAAGCUCGAGAGAGAAUGGGACAGAGAACUCGCCUC AAAGAAGAACCCGAAGCUGAUUAAUGCGCUUAGGCGGUGCUUUUUC UGGCGGUUCAUGUUCUACGGCAUCUUCCUCUACCUGGGAGAGGUCAC |
| **coding sequence** | CAAGGCCGUGCAGCCCCUGUUGCUGGGACGGAUUAUUGCCUCCUACG ACCCCGACAACAAGGAAGAAAGAAGCAUCGCUAUCUACUUGGGCAUC GGUCUGUGCCUGCUUUUCAUCGUCCGGACCCUCUUGUUGCAUCCUGC UAUUUUCGGCCUGCAUCACAUUGGCAUGCAGAUGAGAAUUGCCAUG UUUUCCCUGAUCUACAAGAAAACUCUGAAGCUCUCGAGCCGCGUGCU UGACAAGAUUUCCAUCGGCCAGCUCGUGUCCCUGCUCUCCAACAAUC UGAACAAGUUCGACGAGGGCCUCGCCCUGGCCCACUUCGUGUGGAUC GCCCCUCUGCAAGUGGCGCUUCUGAUGGGCCUGAUCUGGGAGCUGCU GCAAGCCUCGGCAUUCUGUGGGCUUGGAUUCCUGAUCGUGCUGGCAC UGUUCCAGGCCGGACUGGGGCGGAUGAUGAUGAAGUACAGGGACCA GAGAGCCGGAAAGAUUUCCGAACGGCUGGUGAUCACUUCGGAAAUG AUCGAAAACAUCCAGUCAGUGAAGGCCUACUGCUGGGAAGAGGCCAU GGAAAAGAUGAUUGAAAACCUCCGGCAAACCGAGCUGAAGCUGACCC GCAAGGCCGCUUACGUGCGCUAUUUCAACUCGUCCGCUUUCUUCUUC UCCGGGUUCUUCGUGGUGUUUCUCUCCGUGCUCCCCUACGCCCUGAU UAAGGGAAUCAUCCUCAGGAAGAUCUUCACCACCAUUUCCUUCUGUA UCGUGCUCCGCAUGGCCGUGACCCGGCAGUUCCCAUGGGCCGUGCAG ACUUGGUACGACUCCCUGGGAGCCAUUAACAAGAUCCAGGACUUCCU UCAAAAGCAGGAGUACAAGACCCUCGAGUACAACCUGACUACUACCG AGGUCGUGAUGGAAAACGUCACCGCCUUUUGGGAGGAGGGAUUUGG CGAACUGUUCGAGAAGGCCAAGCAGAACAACAACAACCGCAAGACCU CGAACGGUGACGACUCCCUCUUCUUUUCAAACUUCAGCCUGCUCGGG ACGCCCGUGCUGAAGGACAUUAACUUCAAGAUCGAAAGAGGACAGCU CCUGGCGGUGGCCGGAUCGACCGGAGCCGGAAAGACUUCCCUGCUGA UGGUGAUCAUGGGAGAGCUUGAACCUAGCGAGGGAAAGAUCAAGCA CUCCGGCCGCAUCAGCUUCUGUAGCCAGUUUUCCUGGAUCAUGCCCG GAACCAUUAAGGAAAACAUCAUCUUCGGCGUGUCCUACGAUGAAUAC CGCUACCGGUCCGUGAUCAAAGCCUGCCAGCUGGAAGAGGAUAUUUC AAAGUUCGCGGAGAAAGAUAACAUCGUGCUGGGCGAAGGGGGUAUU ACCUUGUCGGGGGGCCAGCGGGCUAGAAUCUCGCUGGCCAGAGCCGU GUAUAAGGACGCCGACCUGUAUCUCCUGGACUCCCCCUUCGGAUACC UGGACGUCCUGACCGAAAAGGAGAUCUUCGAAUCGUGCGUGUGCAA GCUGAUGGCUAACAAGACUCGCAUCCUCGUGACCUCCAAAAUGGAGC ACCUGAAGAAGGCAGACAAGAUUCUGAUUCUGCAUGAGGGGUCCUCC UACUUUUACGGCACCUUCUCGGAGUUGCAGAACUUGCAGCCCGACUU CUCAUCGAAGCUGAUGGGUUGCGACAGCUUCGACCAGUUCUCCGCCG AAAGAAGGAACUCGAUCCUGACGGAAACCUUGCACCGCUUCUCUUUG GAAGGCGACGCCCCUGUGUCAUGGACCGAGACUAAGAAGCAGAGCUU CAAGCAGACCGGGGAAUUCGGCGAAAAGAGGAAGAACAGCAUCUUG AACCCCAUUAACUCCAUCCGCAAGUUCUCAAUCGUGCAAAAGACGCC ACUGCAGAUGAACGGCAUUGAGGAGGACUCCGACGAACCCCUUGAGA GGCGCCUGUCCCUGGUGCCGGACAGCGAGCAGGGAGAAGCCAUCCUG |
| | CCUCGGAUUUCCGUGAUCUCCACUGGUCCGACGCUCCAAGCCCGGCG GCGGCAGUCCGUGCUGAACCUGAUGACCCACAGCGUGAACCAGGGCC AAAACAUUCACCGCAAGACUACCGCAUCCACCCGGAAAGUGUCCCUG GCACCUCAAGCGAAUCUUACCGAGCUCGACAUCUACUCCCGGAGACU GUCGCAGGAAACCGGGCUCGAAAUUUCCGAAGAAAUCAACGAGGAG GAUCUGAAAGAGUGCUUCUUCGACGAUAUGGAGUCGAUACCCGCCGU GACGACUUGGAACACUUAUCUGCGGUACAUCACUGUGCACAAGUCAU UGAUCUUCGUGCUGAUUUGGUGCCUGGUGAUUUUCCUGGCCGAGGU CGCGGCCUCACUGGUGGUGCUCUGGCUGUUGGGAAACACGCCUCUGC AAGACAAGGGAAACUCCACGCACUCGAGAAACAACAGCUAUGCCGUG AUUAUCACUUCCACCUCCUCUUAUUACGUGUUCUACAUCUACGUCGG AGUGGCGGAUACCCUGCUCGCGAUGGGUUUCUUCAGAGGACUGCCGC UGGUCCACACCUUGAUCACCGUCAGCAAGAUUCUUCACCACAAGAUG UUGCAUAGCGUGCUGCAGGCCCCCAUGUCCACCCUCAACACUCUGAA GGCCGGAGGCAUUCUGAACAGAUUCUCCAAGGACAUCGCUAUCCUGG ACGAUCUCCUGCCGCUUACCAUCUUUGACUUCAUCCAGCUGCUGCUG AUCGUGAUUGGAGCAAUCGCAGUGGUGGCGGUGCUGCAGCCUUACA UUUUCGUGGCCACUGUGCCGGUCAUUGUGGCGUUCAUCAUGCUGCGG GCCUACUUCCUCCAAACCAGCCAGCAGCUGAAGCAACUGGAAUCCGA GGGACGAUCCCCCAUCUUCACUCACCUUGUGACGUCGUUGAAGGGAC UGUGGACCCUCCGGGCUUUCGGACGGCAGCCCUACUUCGAAACCCUC UUCCACAAGGCCCUGAACCUCCACACCGCCAAUUGGUUCCUGUACCU GUCCACCCUGCGGUGGUUCCAGAUGCGCAUCGAGAUGAUUUUCGUCA UCUUCUUCAUCGCGGUCACAUUCAUCAGCAUCCUGACUACCGGAGAG GGAGAGGGACGGGUCGGAAUAAUCCUGACCCUCGCCAUGAACAUUAU GAGCACCCUGCAGUGGGCAGUGAACAGCUCGAUCGACGUGGACAGCC UGAUGCGAAGCGUCAGCCGCGUGUUCAAGUUCAUCGACAUGCCUACU GAGGGAAAACCCACUAAGUCCACUAAGCCCUACAAAAAUGGCCAGCU GAGCAAGGUCAUGAUCAUCGAAAACUCCCACGUGAAGAAGGACGAU AUUUGGCCCUCCGGAGGUCAAAUGACCGUGAAGGACCUGACCGCAAA GUACACCGAGGGAGGAAACGCCAUUCUCGAAAACAUCAGCUUCUCCA UUUCGCCGGGACAGCGGGUCGGCCUUCUCGGGCGGACCGGUUCCGGG AAGUCAACUCUGCUGUCGGCUUUCCUCCGGCUGCUGAAUACCGAGGG GGAAAUCCAAAUUGACGGCGUGUCUUGGGAUUCCAUUACUCUGCAGC AGUGGCGGAAGGCCUUCGGCGUGAUCCCCCAGAAGGUGUUCAUCUUC UCGGGUACCUUCCGGAAGAACCUGGAUCCUUACGAGCAGUGGAGCGA CCAAGAAAUCUGGAAGGUCGCCGACGAGGUCGGCCUGCGCUCCGUGA UUGAACAAUUUCCUGGAAAGCUGGACUUCGUGCUCGUCGACGGGGG AUGUGUCCUGUCGCACGGACAUAAGCAGCUCAUGUGCCUCGCACGGU CCGUGCUCUCCAAGGCCAAGAUUCUGCUGCUGGACGAACCUUCGGCC CACCUGGAUCCGGUCACCUACCAGAUCAUCAGGAGGACCCUGAAGCA GGCCUUUGCCGAUUGCACCGUGAUUCUCUGCGAGCACCGCAUCGAGG |
| | CCAUGCUGGAGUGCCAGCAGUUCCUGGUCAUCGAGGAGAACAAGGUC CGCCAAUACGACUCCAUUCAAAAGCUCCUCAACGAGCGGUCGCUGUU CAGACAAGCUAUUUCACCGUCCGAUAGAGUGAAGCUCUUCCCGCAUC GGAACAGCUCAAAGUGCAAAUCGAAGCCGCAGAUCGCAGCCUUGAAG GAAGAGACUGAGGAAGAGGUGCAGGACACCCGGCUUUAA (SEQ ID NO: 1) |
| **Human CFTR Protein Sequence** |  (SEQ ID NO: 2) |

In one embodiment, a codon-optimized CFTR mRNA sequence includes SEQ ID NO: 1. In some embodiments, a codon-optimized CFTR mRNA sequence suitable for the present invention shares at least 60%, 65%, 70%, 75%, 80%, 85%, 90%, 95%, 96%, 97%, 98%, or 99% identity to SEQ ID NO:1 and encodes a CFTR protein having an amino acid sequence of SEQ ID NO:2.

In some embodiments, a CFTR mRNA suitable for the invention also contains 5' and 3' UTR sequences. Exemplary 5' and 3' UTR sequences are shown below:
Exemplary 5' UTR Sequence 
GGACAGAUCGCCUGGAGACGCCAUCCACGCUGUUUUGACCUCCAUAGAAGACACCGG
GACCGAUCCAGCCUCCGCGGCCGGGAACGGUGCAUUGGAACGCGGAUUCCCCGUGCC
AAGAGUGACUCACCGUCCUUGACACG
(SEQ ID NO: 3)
Exemplary 3' UTR Sequence 
CGGGUGGCAUCCCUGUGACCCCUCCCCAGUGCCUCUCCUGGCCCUGGAAGUUGCCAC
UCCAGUGCCCACCAGCCUUGUCCUAAUAAAAUUAAGUUGCAUCAAGCU
(SEQ ID NO: 4)
 or

GGGUGGCAUCCCUGUGACCCCUCCCCAGUGCCUCUCCUGGCCCUGGAAGUUGCCACU
CCAGUGCCCACCAGCCUUGUCCUAAUAAAAUUAAGUUGCAUCAAAGCU
(SEQ ID NO: 5)

Thus, in one embodiment, an exemplary full-length codon-optimized CFTR mRNA sequence suitable for the invention is:

GGACAGAUCGCCUGGAGACGCCAUCCACGCUGUUUUGACCUCCAUAGAAGACACCGG
GACCGAUCCAGCCUCCGCGGCCGGGAACGGUGCAUUGGAACGCGGAUUCCCCGUGCC
AAGAGUGACUCACCGUCCUUGACACGAUGCAACGCUCUCCUCUUGAAAAGGCCUCGG
UGGUGUCCAAGCUCUUCUUCUCGUGGACUAGACCCAUCCUGAGAAAGGGGUACAGAC
AGCGCUUGGAGCUGUCCGAUAUCUAUCAAAUCCCUUCCGUGGACUCCGCGGACAACC
UGUCCGAGAAGCUCGAGAGAGAAUGGGACAGAGAACUCGCCUCAAAGAAGAACCCGA
AGCUGAUUAAUGCGCUUAGGCGGUGCUUUUUCUGGCGGUUCAUGUUCUACGGCAUC
UUCCUCUACCUGGGAGAGGUCACCAAGGCCGUGCAGCCCCUGUUGCUGGGACGGAUU
AUUGCCUCCUACGACCCCGACAACAAGGAAGAAAGAAGCAUCGCUAUCUACUUGGGC
AUCGGUCUGUGCCUGCUUUUCAUCGUCCGGACCCUCUUGUUGCAUCCUGCUAUUUUC
GGCCUGCAUCACAUUGGCAUGCAGAUGAGAAUUGCCAUGUUUUCCCUGAUCUACAAG

AAAACUCUGAAGCUCUCGAGCCGCGUGCUUGACAAGAUUUCCAUCGGCCAGCUCGUG
UCCCUGCUCUCCAACAAUCUGAACAAGUUCGACGAGGGCCUCGCCCUGGCCCACUUC
GUGUGGAUCGCCCCUCUGCAAGUGGCGCUUCUGAUGGGCCUGAUCUGGGAGCUGCUG
CAAGCCUCGGCAUUCUGUGGGCUUGGAUUCCUGAUCGUGCUGGCACUGUUCCAGGCC
GGACUGGGGCGGAUGAUGAUGAAGUACAGGGACCAGAGAGCCGGAAAGAUUUCCGA
ACGGCUGGUGAUCACUUCGGAAAUGAUCGAAAACAUCCAGUCAGUGAAGGCCUACUG
CUGGGAAGAGGCCAUGGAAAAGAUGAUUGAAAACCUCCGGCAAACCGAGCUGAAGC
UGACCCGCAAGGCCGCUUACGUGCGCUAUUUCAACUCGUCCGCUUUCUUCUUCUCCG
GGUUCUUCGUGGUGUUUCUCUCCGUGCUCCCCUACGCCCUGAUUAAGGGAAUCAUCC
UCAGGAAGAUCUUCACCACCAUUUCCUUCUGUAUCGUGCUCCGCAUGGCCGUGACCC
GGCAGUUCCCAUGGGCCGUGCAGACUUGGUACGACUCCCUGGGAGCCAUUAACAAGA
UCCAGGACUUCCUUCAAAAGCAGGAGUACAAGACCCUCGAGUACAACCUGACUACUA
CCGAGGUCGUGAUGGAAAACGUCACCGCCUUUUGGGAGGAGGGAUUUGGCGAACUG
UUCGAGAAGGCCAAGCAGAACAACAACAACCGCAAGACCUCGAACGGUGACGACUCC
CUCUUCUUUUCAAACUUCAGCCUGCUCGGGACGCCCGUGCUGAAGGACAUUAACUUC
AAGAUCGAAAGAGGACAGCUCCUGGCGGUGGCCGGAUCGACCGGAGCCGGAAAGACU
UCCCUGCUGAUGGUGAUCAUGGGAGAGCUUGAACCUAGCGAGGGAAAGAUCAAGCA
CUCCGGCCGCAUCAGCUUCUGUAGCCAGUUUUCCUGGAUCAUGCCCGGAACCAUUAA
GGAAAACAUCAUCUUCGGCGUGUCCUACGAUGAAUACCGCUACCGGUCCGUGAUCAA
AGCCUGCCAGCUGGAAGAGGAUAUUUCAAAGUUCGCGGAGAAAGAUAACAUCGUGC
UGGGCGAAGGGGGUAUUACCUUGUCGGGGGGCCAGCGGGCUAGAAUCUCGCUGGCCA
GAGCCGUGUAUAAGGACGCCGACCUGUAUCUCCUGGACUCCCCCUUCGGAUACCUGG
ACGUCCUGACCGAAAAGGAGAUCUUCGAAUCGUGCGUGUGCAAGCUGAUGGCUAACA
AGACUCGCAUCCUCGUGACCUCCAAAAUGGAGCACCUGAAGAAGGCAGACAAGAUUC
UGAUUCUGCAUGAGGGGUCCUCCUACUUUUACGGCACCUUCUCGGAGUUGCAGAACU
UGCAGCCCGACUUCUCAUCGAAGCUGAUGGGUUGCGACAGCUUCGACCAGUUCUCCG
CCGAAAGAAGGAACUCGAUCCUGACGGAAACCUUGCACCGCUUCUCUUUGGAAGGCG
ACGCCCCUGUGUCAUGGACCGAGACUAAGAAGCAGAGCUUCAAGCAGACCGGGGAAU
UCGGCGAAAAGAGGAAGAACAGCAUCUUGAACCCCAUUAACUCCAUCCGCAAGUUCU
CAAUCGUGCAAAAGACGCCACUGCAGAUGAACGGCAUUGAGGAGGACUCCGACGAAC
CCCUUGAGAGGCGCCUGUCCCUGGUGCCGGACAGCGAGCAGGGAGAAGCCAUCCUGC
CUCGGAUUUCCGUGAUCUCCACUGGUCCGACGCUCCAAGCCCGGCGGCGGCAGUCCG

UGCUGAACCUGAUGACCCACAGCGUGAACCAGGGCCAAAACAUUCACCGCAAGACUA
CCGCAUCCACCCGGAAAGUGUCCCUGGCACCUCAAGCGAAUCUUACCGAGCUCGACA
UCUACUCCCGGAGACUGUCGCAGGAAACCGGGCUCGAAAUUUCCGAAGAAAUCAACG
AGGAGGAUCUGAAAGAGUGCUUCUUCGACGAUAUGGAGUCGAUACCCGCCGUGACG
ACUUGGAACACUUAUCUGCGGUACAUCACUGUGCACAAGUCAUUGAUCUUCGUGCUG
AUUUGGUGCCUGGUGAUUUUCCUGGCCGAGGUCGCGGCCUCACUGGUGGUGCUCUGG
CUGUUGGGAAACACGCCUCUGCAAGACAAGGGAAACUCCACGCACUCGAGAAACAAC
AGCUAUGCCGUGAUUAUCACUUCCACCUCCUCUUAUUACGUGUUCUACAUCUACGUC
GGAGUGGCGGAUACCCUGCUCGCGAUGGGUUUCUUCAGAGGACUGCCGCUGGUCCAC
ACCUUGAUCACCGUCAGCAAGAUUCUUCACCACAAGAUGUUGCAUAGCGUGCUGCAG
GCCCCCAUGUCCACCCUCAACACUCUGAAGGCCGGAGGCAUUCUGAACAGAUUCUCC
AAGGACAUCGCUAUCCUGGACGAUCUCCUGCCGCUUACCAUCUUUGACUUCAUCCAG
CUGCUGCUGAUCGUGAUUGGAGCAAUCGCAGUGGUGGCGGUGCUGCAGCCUUACAUU
UUCGUGGCCACUGUGCCGGUCAUUGUGGCGUUCAUCAUGCUGCGGGCCUACUUCCUC
CAAACCAGCCAGCAGCUGAAGCAACUGGAAUCCGAGGGACGAUCCCCCAUCUUCACU
CACCUUGUGACGUCGUUGAAGGGACUGUGGACCCUCCGGGCUUUCGGACGGCAGCCC
UACUUCGAAACCCUCUUCCACAAGGCCCUGAACCUCCACACCGCCAAUUGGUUCCUG
UACCUGUCCACCCUGCGGUGGUUCCAGAUGCGCAUCGAGAUGAUUUUCGUCAUCUUC
UUCAUCGCGGUCACAUUCAUCAGCAUCCUGACUACCGGAGAGGGAGAGGGACGGGUC
GGAAUAAUCCUGACCCUCGCCAUGAACAUUAUGAGCACCCUGCAGUGGGCAGUGAAC
AGCUCGAUCGACGUGGACAGCCUGAUGCGAAGCGUCAGCCGCGUGUUCAAGUUCAUC
GACAUGCCUACUGAGGGAAAACCCACUAAGUCCACUAAGCCCUACAAAAAUGGCCAG
CUGAGCAAGGUCAUGAUCAUCGAAAACUCCCACGUGAAGAAGGACGAUAUUUGGCCC
UCCGGAGGUCAAAUGACCGUGAAGGACCUGACCGCAAAGUACACCGAGGGAGGAAAC
GCCAUUCUCGAAAACAUCAGCUUCUCCAUUUCGCCGGGACAGCGGGUCGGCCUUCUC
GGGCGGACCGGUUCCGGGAAGUCAACUCUGCUGUCGGCUUUCCUCCGGCUGCUGAAU
ACCGAGGGGGAAAUCCAAAUUGACGGCGUGUCUUGGGAUUCCAUUACUCUGCAGCAG
UGGCGGAAGGCCUUCGGCGUGAUCCCCCAGAAGGUGUUCAUCUUCUCGGGUACCUUC
CGGAAGAACCUGGAUCCUUACGAGCAGUGGAGCGACCAAGAAAUCUGGAAGGUCGCC
GACGAGGUCGGCCUGCGCUCCGUGAUUGAACAAUUUCCUGGAAAGCUGGACUUCGUG
CUCGUCGACGGGGGAUGUGUCCUGUCGCACGGACAUAAGCAGCUCAUGUGCCUCGCA
CGGUCCGUGCUCUCCAAGGCCAAGAUUCUGCUGCUGGACGAACCUUCGGCCCACCUG

GAUCCGGUCACCUACCAGAUCAUCAGGAGGACCCUGAAGCAGGCCUUUGCCGAUUGC
ACCGUGAUUCUCUGCGAGCACCGCAUCGAGGCCAUGCUGGAGUGCCAGCAGUUCCUG
GUCAUCGAGGAGAACAAGGUCCGCCAAUACGACUCCAUUCAAAAGCUCCUCAACGAG
CGGUCGCUGUUCAGACAAGCUAUUUCACCGUCCGAUAGAGUGAAGCUCUUCCCGCAU
CGGAACAGCUCAAAGUGCAAAUCGAAGCCGCAGAUCGCAGCCUUGAAGGAAGAGACU
GAGGAAGAGGUGCAGGACACCCGGCUUUAACGGGUGGCAUCCCUGUGACCCCUCCCC
AGUGCCUCUCCUGGCCCUGGAAGUUGCCACUCCAGUGCCCACCAGCCUUGUCCUAAU
AAAAUUAAGUUGCAUCAAGCU (SEQ ID NO: 6)

In another embodiment, an exemplary full-length codon-optimized CFTR mRNA sequence is:

GGACAGAUCGCCUGGAGACGCCAUCCACGCUGUUUUGACCUCCAUAGAAGACACCGG
GACCGAUCCAGCCUCCGCGGCCGGGAACGGUGCAUUGGAACGCGGAUUCCCCGUGCC
AAGAGUGACUCACCGUCCUUGACACGAUGCAACGCUCUCCUCUUGAAAAGGCCUCGG
UGGUGUCCAAGCUCUUCUUCUCGUGGACUAGACCCAUCCUGAGAAAGGGGUACAGAC
AGCGCUUGGAGCUGUCCGAUAUCUAUCAAAUCCCUUCCGUGGACUCCGCGGACAACC
UGUCCGAGAAGCUCGAGAGAGAAUGGGACAGAGAACUCGCCUCAAAGAAGAACCCGA
AGCUGAUUAAUGCGCUUAGGCGGUGCUUUUUCUGGCGGUUCAUGUUCUACGGCAUC
UUCCUCUACCUGGGAGAGGUCACCAAGGCCGUGCAGCCCCUGUUGCUGGGACGGAUU
AUUGCCUCCUACGACCCCGACAACAAGGAAGAAAGAAGCAUCGCUAUCUACUUGGGC
AUCGGUCUGUGCCUGCUUUUCAUCGUCCGGACCCUCUUGUUGCAUCCUGCUAUUUUC
GGCCUGCAUCACAUUGGCAUGCAGAUGAGAAUUGCCAUGUUUUCCCUGAUCUACAAG
AAAACUCUGAAGCUCUCGAGCCGCGUGCUUGACAAGAUUUCCAUCGGCCAGCUCGUG
UCCCUGCUCUCCAACAAUCUGAACAAGUUCGACGAGGGCCUCGCCCUGGCCCACUUC
GUGUGGAUCGCCCCUCUGCAAGUGGCGCUUCUGAUGGGCCUGAUCUGGGAGCUGCUG
CAAGCCUCGGCAUUCUGUGGGCUUGGAUUCCUGAUCGUGCUGGCACUGUUCCAGGCC
GGACUGGGGCGGAUGAUGAUGAAGUACAGGGACCAGAGAGCCGGAAAGAUUUCCGA
ACGGCUGGUGAUCACUUCGGAAAUGAUCGAAAACAUCCAGUCAGUGAAGGCCUACUG
CUGGGAAGAGGCCAUGGAAAAGAUGAUUGAAAACCUCCGGCAAACCGAGCUGAAGC
UGACCCGCAAGGCCGCUUACGUGCGCUAUUUCAACUCGUCCGCUUUCUUCUUCUCCG
GGUUCUUCGUGGUGUUUCUCUCCGUGCUCCCCUACGCCCUGAUUAAGGGAAUCAUCC
UCAGGAAGAUCUUCACCACCAUUUCCUUCUGUAUCGUGCUCCGCAUGGCCGUGACCC
GGCAGUUCCCAUGGGCCGUGCAGACUUGGUACGACUCCCUGGGAGCCAUUAACAAGA

UCCAGGACUUCCUUCAAAAGCAGGAGUACAAGACCCUCGAGUACAACCUGACUACUA
CCGAGGUCGUGAUGGAAAACGUCACCGCCUUUUGGGAGGAGGGAUUUGGCGAACUG
UUCGAGAAGGCCAAGCAGAACAACAACAACCGCAAGACCUCGAACGGUGACGACUCC
CUCUUCUUUUCAAACUUCAGCCUGCUCGGGACGCCCGUGCUGAAGGACAUUAACUUC
AAGAUCGAAAGAGGACAGCUCCUGGCGGUGGCCGGAUCGACCGGAGCCGGAAAGACU
UCCCUGCUGAUGGUGAUCAUGGGAGAGCUUGAACCUAGCGAGGGAAAGAUCAAGCA
CUCCGGCCGCAUCAGCUUCUGUAGCCAGUUUUCCUGGAUCAUGCCCGGAACCAUUAA
GGAAAACAUCAUCUUCGGCGUGUCCUACGAUGAAUACCGCUACCGGUCCGUGAUCAA
AGCCUGCCAGCUGGAAGAGGAUAUUUCAAAGUUCGCGGAGAAAGAUAACAUCGUGC
UGGGCGAAGGGGGUAUUACCUUGUCGGGGGGCCAGCGGGCUAGAAUCUCGCUGGCCA
GAGCCGUGUAUAAGGACGCCGACCUGUAUCUCCUGGACUCCCCCUUCGGAUACCUGG
ACGUCCUGACCGAAAAGGAGAUCUUCGAAUCGUGCGUGUGCAAGCUGAUGGCUAACA
AGACUCGCAUCCUCGUGACCUCCAAAAUGGAGCACCUGAAGAAGGCAGACAAGAUUC
UGAUUCUGCAUGAGGGGUCCUCCUACUUUUACGGCACCUUCUCGGAGUUGCAGAACU
UGCAGCCCGACUUCUCAUCGAAGCUGAUGGGUUGCGACAGCUUCGACCAGUUCUCCG
CCGAAAGAAGGAACUCGAUCCUGACGGAAACCUUGCACCGCUUCUCUUUGGAAGGCG
ACGCCCCUGUGUCAUGGACCGAGACUAAGAAGCAGAGCUUCAAGCAGACCGGGGAAU
UCGGCGAAAAGAGGAAGAACAGCAUCUUGAACCCCAUUAACUCCAUCCGCAAGUUCU
CAAUCGUGCAAAAGACGCCACUGCAGAUGAACGGCAUUGAGGAGGACUCCGACGAAC
CCCUUGAGAGGCGCCUGUCCCUGGUGCCGGACAGCGAGCAGGGAGAAGCCAUCCUGC
CUCGGAUUUCCGUGAUCUCCACUGGUCCGACGCUCCAAGCCCGGCGGCGGCAGUCCG
UGCUGAACCUGAUGACCCACAGCGUGAACCAGGGCCAAAACAUUCACCGCAAGACUA
CCGCAUCCACCCGGAAAGUGUCCCUGGCACCUCAAGCGAAUCUUACCGAGCUCGACA
UCUACUCCCGGAGACUGUCGCAGGAAACCGGGCUCGAAAUUUCCGAAGAAAUCAACG
AGGAGGAUCUGAAAGAGUGCUUCUUCGACGAUAUGGAGUCGAUACCCGCCGUGACG
ACUUGGAACACUUAUCUGCGGUACAUCACUGUGCACAAGUCAUUGAUCUUCGUGCUG
AUUUGGUGCCUGGUGAUUUUCCUGGCCGAGGUCGCGGCCUCACUGGUGGUGCUCUGG
CUGUUGGGAAACACGCCUCUGCAAGACAAGGGAAACUCCACGCACUCGAGAAACAAC
AGCUAUGCCGUGAUUAUCACUUCCACCUCCUCUUAUUACGUGUUCUACAUCUACGUC
GGAGUGGCGGAUACCCUGCUCGCGAUGGGUUUCUUCAGAGGACUGCCGCUGGUCCAC
ACCUUGAUCACCGUCAGCAAGAUUCUUCACCACAAGAUGUUGCAUAGCGUGCUGCAG
GCCCCCAUGUCCACCCUCAACACUCUGAAGGCCGGAGGCAUUCUGAACAGAUUCUCC

AAGGACAUCGCUAUCCUGGACGAUCUCCUGCCGCUUACCAUCUUUGACUUCAUCCAG
CUGCUGCUGAUCGUGAUUGGAGCAAUCGCAGUGGUGGCGGUGCUGCAGCCUUACAUU
UUCGUGGCCACUGUGCCGGUCAUUGUGGCGUUCAUCAUGCUGCGGGCCUACUUCCUC
CAAACCAGCCAGCAGCUGAAGCAACUGGAAUCCGAGGGACGAUCCCCCAUCUUCACU
CACCUUGUGACGUCGUUGAAGGGACUGUGGACCCUCCGGGCUUUCGGACGGCAGCCC
UACUUCGAAACCCUCUUCCACAAGGCCCUGAACCUCCACACCGCCAAUUGGUUCCUG
UACCUGUCCACCCUGCGGUGGUUCCAGAUGCGCAUCGAGAUGAUUUUCGUCAUCUUC
UUCAUCGCGGUCACAUUCAUCAGCAUCCUGACUACCGGAGAGGGAGAGGGACGGGUC
GGAAUAAUCCUGACCCUCGCCAUGAACAUUAUGAGCACCCUGCAGUGGGCAGUGAAC
AGCUCGAUCGACGUGGACAGCCUGAUGCGAAGCGUCAGCCGCGUGUUCAAGUUCAUC
GACAUGCCUACUGAGGGAAAACCCACUAAGUCCACUAAGCCCUACAAAAAUGGCCAG
CUGAGCAAGGUCAUGAUCAUCGAAAACUCCCACGUGAAGAAGGACGAUAUUUGGCCC
UCCGGAGGUCAAAUGACCGUGAAGGACCUGACCGCAAAGUACACCGAGGGAGGAAAC
GCCAUUCUCGAAAACAUCAGCUUCUCCAUUUCGCCGGGACAGCGGGUCGGCCUUCUC
GGGCGGACCGGUUCCGGGAAGUCAACUCUGCUGUCGGCUUUCCUCCGGCUGCUGAAU
ACCGAGGGGGAAAUCCAAAUUGACGGCGUGUCUUGGGAUUCCAUUACUCUGCAGCAG
UGGCGGAAGGCCUUCGGCGUGAUCCCCCAGAAGGUGUUCAUCUUCUCGGGUACCUUC
CGGAAGAACCUGGAUCCUUACGAGCAGUGGAGCGACCAAGAAAUCUGGAAGGUCGCC
GACGAGGUCGGCCUGCGCUCCGUGAUUGAACAAUUUCCUGGAAAGCUGGACUUCGUG
CUCGUCGACGGGGGAUGUGUCCUGUCGCACGGACAUAAGCAGCUCAUGUGCCUCGCA
CGGUCCGUGCUCUCCAAGGCCAAGAUUCUGCUGCUGGACGAACCUUCGGCCCACCUG
GAUCCGGUCACCUACCAGAUCAUCAGGAGGACCCUGAAGCAGGCCUUUGCCGAUUGC
ACCGUGAUUCUCUGCGAGCACCGCAUCGAGGCCAUGCUGGAGUGCCAGCAGUUCCUG
GUCAUCGAGGAGAACAAGGUCCGCCAAUACGACUCCAUUCAAAAGCUCCUCAACGAG
CGGUCGCUGUUCAGACAAGCUAUUUCACCGUCCGAUAGAGUGAAGCUCUUCCCGCAU
CGGAACAGCUCAAAGUGCAAAUCGAAGCCGCAGAUCGCAGCCUUGAAGGAAGAGACU
GAGGAAGAGGUGCAGGACACCCGGCUUUAAGGGUGGCAUCCCUGUGACCCCUCCCCA
GUGCCUCUCCUGGCCCUGGAAGUUGCCACUCCAGUGCCCACCAGCCUUGUCCUAAUA
AAAUUAAGUUGCAUCAAAGCU (SEQ ID NO: 7)

In another embodiment, an exemplary codon-optimized CFTR mRNA sequence is:

ATGCAGAGGAGCCCACTGGAGAAAGCCTCCGTGGTGAGTAAACTCTTTTTTAGTTGGAC
CAGACCCATCCTGCGAAAAGGATACAGGCAGCGCCTCGAGTTGTCAGATATCTACCAG

ATTCCTTCTGTGGACTCAGCTGACAATTTGAGTGAGAAGCTGGAGCGGGAGTGGGATAG
AGAGCTGGCGAGCAAAAAAAACCCCAAGCTTATCAATGCTCTGCGCCGCTGCTTTTTCT
GGAGGTTCATGTTTTATGGGATCTTCCTGTACCTGGGGGAGGTCACCAAAGCTGTTCAG
CCGCTCCTTCTTGGCCGCATCATCGCCAGCTATGACCCTGATAATAAAGAAGAAAGGTC
TATTGCTATTTATCTGGGAATTGGCCTCTGCTTGCTCTTCATCGTCCGCACCCTTCTGCTG
CACCCTGCCATTTTTGGCCTTCACCACATCGGCATGCAAATGAGAATTGCCATGTTCTCC
CTCATTTACAAAAAGACCCTGAAACTTTCCTCAAGAGTGTTAGATAAAATATCCATTGG
TCAGCTGGTCAGCCTGCTGTCCAACAATCTTAACAAATTTGATGAAGGCTTGGCGCTGG
CCCACTTCGTGTGGATTGCACCTCTGCAGGTGGCCCTGTTGATGGGACTTATATGGGAG
CTGCTTCAAGCCTCTGCTTTCTGTGGGCTGGGCTTTTTGATTGTACTGGCACTTTTTCAGG
CTGGGCTCGGAAGAATGATGATGAAATACAGAGATCAGCGGGCCGGGAAGATATCAGA
GCGACTTGTGATCACCAGTGAAATGATTGAAAATATTCAGAGCGTGAAAGCCTACTGCT
GGGAAGAAGCCATGGAGAAGATGATTGAGAACCTGAGGCAGACAGAGCTCAAGCTCAC
TCGGAAGGCTGCTTATGTTCGCTATTTCAACAGCAGCGCCTTCTTCTTCAGTGGCTTCTT
TGTTGTCTTCCTGTCTGTTCTGCCATATGCACTGATAAAAGGCATTATTTTACGAAAGAT
CTTCACCACCATCAGTTTTTGCATCGTTCTCAGGATGGCCGTCACAAGACAGTTCCCCTG
GGCTGTGCAGACCTGGTACGATTCCTTGGGGGCCATCAACAAGATTCAAGATTTCTTGC
AAAAACAAGAATATAAAACTTTAGAATACAACCTCACCACCACTGAAGTGGTCATGGA
AAATGTGACAGCCTTTTGGGAGGAGGGTTTTGGAGAATTGTTCGAGAAGGCAAAGCAG
AATAACAACAACAGGAAGACGAGCAATGGGGACGACTCTCTCTTCTTCAGCAACTTTTC
ACTGCTCGGGACCCCTGTGTTGAAAGATATAAACTTCAAGATCGAGAGGGGCCAGCTCT
TGGCTGTGGCAGGCTCCACTGGAGCTGGTAAAACATCTCTTCTCATGGTGATCATGGGG
GAACTGGAGCCTTCCGAAGGAAAAATCAAGCACAGTGGGAGAATCTCATTCTGCAGCC
AGTTTTCCTGGATCATGCCCGGCACCATTAAGGAAAACATCATATTTGGAGTGTCCTAT
GATGAGTACCGCTACCGGTCAGTCATCAAAGCCTGTCAGTTGGAGGAGGACATCTCCAA
GTTTGCAGAGAAAGACAACATTGTGCTTGGAGAGGGGGGTATCACTCTTTCTGGAGGAC
AAAGAGCCAGGATCTCTTTGGCCCGGGCAGTCTACAAGGATGCAGACCTCTACTTGTTG
GACAGTCCCTTCGGCTACCTCGACGTGCTGACTGAAAAAGAAATTTTTGAAAGCTGTGT
GTGCAAACTGATGGCAAACAAGACCAGGATTCTTGTCACCAGCAAGATGGAACATCTG
AAGAAAGCGGACAAAATTCTGATTCTGCATGAAGGGAGCTCCTACTTCTATGGAACATT
TAGCGAGCTTCAGAACCTACAGCCAGACTTCTCCTCCAAATTAATGGGCTGTGACTCCT
TCGACCAGTTCTCTGCAGAAAGAAGAAACTCTATACTCACAGAGACCCTCCACCGCTTC

TCCCTTGAGGGAGATGCCCCAGTTTCTTGGACAGAAACCAAGAAGCAGTCCTTTAAGCA
GACTGGCGAGTTTGGTGAAAAGAGGAAAAATTCAATTCTCAATCCAATTAACAGTATTC
GCAAGTTCAGCATTGTCCAGAAGACACCCCTCCAGATGAATGGCATCGAAGAAGATAG
TGACGAGCCGCTGGAGAGACGGCTGAGTCTGGTGCCAGATTCAGAACAGGGGGAGGCC
ATCCTGCCCCGGATCAGCGTCATTTCCACAGGCCCCACATTACAAGCACGGCGCCGGCA
GAGTGTTTTAAATCTCATGACCCATTCAGTGAACCAGGGCCAAAATATCCACAGGAAGA
CTACAGCTTCTACCCGGAAAGTGTCTCTGGCCCCTCAGGCCAATCTGACCGAGCTGGAC
ATCTACAGCAGGAGGCTCTCCCAGGAAACAGGGCTGGAAATATCTGAAGAGATTAATG
AAGAGGATCTTAAAGAGTGCTTCTTTGATGACATGGAGAGCATCCCCGCGGTGACCACA
TGGAACACCTACCTTAGATATATTACTGTCCACAAGAGCCTCATATTTGTCCTCATCTGG
TGCCTGGTTATTTTCCTCGCTGAGGTGGCGGCCAGTCTTGTTGTGCTCTGGCTGCTGGGC
AACACTCCTCTCCAGGACAAGGGCAATAGTACTCACAGCAGAAATAATTCTTATGCCGT
CATCATTACAAGCACCTCCAGCTACTACGTGTTCTACATCTATGTGGGCGTGGCTGACA
CCCTCCTGGCCATGGGTTTCTTCCGGGGCCTGCCTTTGGTGCACACCCTCATCACAGTGT
CAAAAATTCTGCACCATAAAATGCTTCATTCTGTCCTGCAGGCACCCATGAGCACTTTG
AACACATTGAAGGCTGGCGGCATCCTCAACAGATTTTCTAAAGATATTGCTATCCTGGA
TGATCTCCTCCCCCTGACAATCTTTGACTTTATCCAGCTTCTGCTGATCGTGATTGGAGC
CATAGCAGTGGTTGCTGTCCTGCAGCCCTACATTTTTGTGGCCACCGTGCCCGTGATTGT
TGCCTTTATTATGCTCAGAGCTTACTTCCTGCAAACTTCTCAACAGCTCAAACAGCTAGA
ATCTGAGGGCCGGAGCCCCATTTTTACCCACCTGGTGACTTCCCTGAAGGGACTGTGGA
CTCTGAGAGCATTCGGGCGACAGCCTTACTTTGAGACACTGTTCCACAAGGCCCTGAAC
TTGCACACTGCCAACTGGTTTCTTTACCTGAGCACACTCCGCTGGTTCCAGATGCGGATA
GAGATGATCTTCGTCATCTTTTTTATAGCTGTAACCTTCATTTCTATCCTTACAACAGGA
GAAGGAGAGGGCAGGGTGGGAATCATCCTCACGCTGGCTATGAACATAATGTCCACCT
TGCAGTGGGCCGTGAATTCCAGTATAGATGTGGATTCTCTAATGAGGAGTGTCTCCCGG
GTGTTTAAATTCATTGATATGCCTACTGAGGGGAAACCCACCAAGTCAACAAAACCTTA
TAAGAATGGACAGCTGAGCAAGGTGATGATAATTGAGAACAGCCACGTGAAGAAGGAT
GACATTTGGCCCAGCGGGGGCCAGATGACTGTGAAGGACCTGACGGCCAAGTACACCG
AAGGTGGAAATGCCATTTTGGAAAACATCAGCTTCTCAATCTCTCCTGGGCAGAGAGTT
GGATTGCTGGGTCGCACGGGCAGCGGCAAATCAACCCTGCTCAGTGCCTTCCTTCGGCT
CCTGAATACAGAAGGCGAAATCCAAATTGACGGGGTGAGCTGGGACAGCATCACCCTG
CAGCAGTGGAGAAAAGCATTTGGGGTCATTCCACAGAAAGTTTTCATCTTCTCTGGCAC

TTTCAGAAAGAACCTGGACCCCTATGAGCAGTGGAGCGACCAGGAGATCTGGAAGGTT
GCAGATGAAGTTGGCCTGCGGAGTGTGATAGAACAATTTCCTGGCAAGCTGGATTTTGT
GCTGGTAGATGGAGGCTGCGTGCTGTCCCACGGCCACAAACAGCTGATGTGCCTCGCCC
GCTCCGTTCTTTCAAAGGCCAAAATCTTGCTTTTGGATGAGCCCAGTGCTCACCTCGACC
CAGTGACCTATCAGATAATCCGCAGGACCTTAAAGCAAGCTTTTGCCGACTGCACCGTC
ATACTGTGTGAGCACCGGATTGAAGCAATGCTGGAATGCCAGCAGTTTCTGGTGATCGA
GGAGAATAAGGTCCGGCAGTACGACAGCATCCAGAAGTTGTTGAATGAGCGCAGCCTT
TTCCGCCAGGCCATCTCCCCATCTGACAGAGTCAAGCTGTTTCCACATAGGAACTCCTCT
AAGTGCAAGTCCAAGCCCCAGATCGCTGCCCTCAAGGAGGAAACTGAGGAAGAGGTGC
AGGATACCCGCCTGTGA (SEQ ID NO: 8)

In another embodiment, an exemplary codon-optimized CFTR mRNA sequence is:

ATGCAGAGGAGCCCACTGGAGAAAGCCTCCGTGGTGAGTAAACTCTTTTTTAGTTGGAC
CAGACCCATCCTGCGAAAAGGATACAGGCAGCGCCTCGAGTTGTCTGATATCTACCAGA
TTCCTTCTGTGGACTCAGCTGACAATTTGAGTGAGAAGCTGGAGCGGGAGTGGGATAGA
GAGCTGGCGAGCAAAAAAAACCCCAAGCTTATCAATGCTCTGCGCCGCTGCTTTTTCTG
GAGGTTCATGTTTTATGGGATCTTCCTGTACCTGGGGGAGGTCACCAAAGCTGTTCAGC
CGCTCCTTCTTGGCCGCATCATCGCCAGCTATGACCCTGATAATAAAGAAGAAAGGTCT
ATTGCTATTTATCTGGGAATTGGCCTCTGCTTGCTCTTCATCGTCCGCACCCTTCTGCTGC
ACCCTGCCATTTTTGGCCTTCACCACATCGGCATGCAAATGAGAATTGCCATGTTCTCCC
TCATTTACAAAAAGACCCTGAAACTTTCCTCAAGAGTGTTAGATAAAATATCCATTGGT
CAGCTGGTCAGCCTGCTGTCCAACAATCTTAACAAATTTGATGAAGGCTTGGCGCTGGC
CCACTTCGTGTGGATTGCACCTCTGCAGGTGGCCCTGTTGATGGGACTTATATGGGAGC
TGCTTCAAGCCTCTGCTTTCTGTGGGCTGGGCTTTTTGATTGTACTGGCACTTTTTCAGGC
TGGGCTCGGAAGAATGATGATGAAATACAGAGATCAGCGGGCCGGGAAGATTTCAGAG
CGACTTGTGATCACCAGTGAAATGATTGAAAATATTCAGAGCGTGAAAGCCTACTGCTG
GGAAGAAGCCATGGAGAAGATGATTGAGAACCTGAGGCAGACAGAGCTCAAGCTCACT
CGGAAGGCTGCTTATGTTCGCTATTTCAACAGCAGCGCCTTCTTCTTCAGTGGCTTCTTT
GTTGTCTTCCTGTCTGTTCTGCCATATGCACTGATAAAAGGCATTATTTTACGAAAGATC
TTCACCACCATCAGTTTTTGCATCGTTCTCAGGATGGCCGTCACAAGACAGTTCCCCTGG
GCTGTGCAGACCTGGTACGATTCCTTGGGGGCCATCAACAAGATTCAAGATTTCTTGCA
AAAACAAGAATATAAAACTTTAGAATACAACCTCACCACCACTGAAGTGGTCATGGAA
AATGTGACAGCCTTTTGGGAGGAGGGTTTTGGAGAATTGTTCGAGAAGGCAAAGCAGA

ATAACAACAACAGGAAGACGAGCAATGGGGACGACTCTCTCTTCTTCAGCAACTTTTCA
CTGCTCGGGACCCCTGTGTTGAAAGATATAAACTTCAAGATCGAGAGGGGCCAGCTCTT
GGCTGTGGCAGGCTCCACTGGAGCTGGTAAAACATCTCTTCTCATGGTGATCATGGGGG
AACTGGAGCCTTCCGAAGGAAAAATCAAGCACAGTGGGAGAATCTCATTCTGCAGCCA
GTTTTCCTGGATCATGCCCGGCACCATTAAGGAAAACATCATATTTGGAGTGTCCTATG
ATGAGTACCGCTACCGGTCAGTCATCAAAGCCTGTCAGTTGGAGGAGGACATCTCCAAG
TTTGCAGAGAAAGACAACATTGTGCTTGGAGAGGGGGGTATCACTCTTTCTGGAGGACA
AAGAGCCAGGATCTCTTTGGCCCGGGCAGTCTACAAGGATGCAGACCTCTACTTGTTGG
ACAGTCCCTTCGGCTACCTCGACGTGCTGACTGAAAAAGAAATTTTTGAAAGCTGTGTG
TGCAAACTGATGGCAAACAAGACCAGGATTCTTGTCACCAGCAAGATGGAACATCTGA
AGAAAGCGGACAAAATTCTGATTCTGCATGAAGGGAGCTCCTACTTCTATGGAACATTT
AGCGAGCTTCAGAACCTACAGCCAGACTTCTCCTCCAAATTAATGGGCTGTGACTCCTT
CGACCAGTTCTCTGCAGAAAGAAGAAACTCTATACTCACAGAGACCCTCCACCGCTTCT
CCCTTGAGGGAGATGCCCCAGTTTCTTGGACAGAAACCAAGAAGCAGTCCTTTAAGCAG
ACTGGCGAGTTTGGTGAAAAGAGGAAAAATTCAATTCTCAATCCAATTAACAGTATTCG
CAAGTTCAGCATTGTCCAGAAGACACCCCTCCAGATGAATGGCATCGAAGAAGATAGT
GACGAGCCGCTGGAGAGACGGCTGAGTCTGGTGCCAGATTCAGAACAGGGGGAGGCCA
TCCTGCCCCGGATCAGCGTCATTTCCACAGGCCCCACATTACAAGCACGGCGCCGGCAG
AGTGTTTTAAATCTCATGACCCATTCAGTGAACCAGGGCCAAAATATCCACAGGAAGAC
TACAGCTTCTACCCGGAAAGTGTCTCTGGCCCCTCAGGCCAATCTGACCGAGCTGGACA
TCTACAGCAGGAGGCTCTCCCAGGAAACAGGGCTGGAAATATCTGAAGAGATTAATGA
AGAGGATCTTAAAGAGTGCTTCTTTGATGACATGGAGAGCATCCCCGCGGTGACCACAT
GGAACACCTACCTTAGATATATTACTGTCCACAAGAGCCTCATATTTGTCCTCATCTGGT
GCCTGGTTATTTTCCTCGCTGAGGTGGCGGCCAGTCTTGTTGTGCTCTGGCTGCTGGGCA
ACACTCCTCTCCAGGACAAGGGCAATAGTACTCACAGCAGAAATAATTCTTATGCCGTC
ATCATTACAAGCACCTCCAGCTACTACGTGTTCTACATCTATGTGGGCGTGGCTGACAC
CCTCCTGGCCATGGGTTTCTTCCGGGGCCTGCCTTTGGTGCACACCCTCATCACAGTGTC
AAAAATTCTGCACCATAAAATGCTTCATTCTGTCCTGCAGGCACCCATGAGCACTTTGA
ACACATTGAAGGCTGGCGGCATCCTCAACAGATTTTCTAAAGATATTGCTATCCTGGAT
GATCTCCTCCCCCTGACAATCTTTGACTTTATCCAGCTTCTGCTGATCGTGATTGGAGCC
ATAGCAGTGGTTGCTGTCCTGCAGCCCTACATTTTTGTGGCCACCGTGCCCGTGATTGTT
GCCTTTATTATGCTCAGAGCTTACTTCCTGCAAACTTCTCAACAGCTCAAACAGCTAGAA

TCTGAGGGCCGGAGCCCCATTTTTACCCACCTGGTGACTTCCCTGAAGGGACTGTGGAC
TCTGAGAGCATTCGGGCGACAGCCTTACTTTGAGACACTGTTCCACAAGGCCCTGAACT
TGCACACTGCCAACTGGTTTCTTTACCTGAGCACACTCCGCTGGTTCCAGATGCGGATA
GAGATGATCTTCGTCATCTTTTTTATAGCTGTAACCTTCATTTCTATCCTTACAACAGGA
GAAGGAGAGGGCAGGGTGGGAATCATCCTCACGCTGGCTATGAACATAATGTCCACCT
TGCAGTGGGCCGTGAATTCCAGTATAGATGTGGATTCTCTAATGAGGAGTGTCTCCCGG
GTGTTTAAATTCATTGATATGCCTACTGAGGGGAAACCCACCAAGTCAACAAAACCTTA
TAAGAATGGACAGCTGAGCAAGGTGATGATAATTGAGAACAGCCACGTGAAGAAGGAT
GACATTTGGCCCAGCGGGGGCCAGATGACTGTGAAGGACCTGACGGCCAAGTACACCG
AAGGTGGAAATGCCATTTTGGAAAACATCAGCTTCTCAATCTCTCCTGGGCAGAGAGTT
GGATTGCTGGGTCGCACGGGCAGCGGCAAATCAACCCTGCTCAGTGCCTTCCTTCGGCT
CCTGAATACAGAAGGCGAAATCCAAATTGACGGGGTGAGCTGGGACAGCATCACCCTG
CAGCAGTGGAGAAAAGCATTTGGGGTCATTCCACAGAAAGTTTTCATCTTCTCTGGCAC
TTTCAGAAAGAACCTGGACCCCTATGAGCAGTGGAGCGACCAGGAGATCTGGAAGGTT
GCAGATGAAGTTGGCCTGCGGAGTGTGATAGAACAATTTCCTGGCAAGCTGGATTTTGT
GCTGGTAGATGGAGGCTGCGTGCTGTCCCACGGCCACAAACAGCTGATGTGCCTCGCCC
GCTCCGTTCTTTCAAAGGCCAAAATCTTGCTTTTGGATGAGCCCAGTGCTCACCTTGACC
CAGTGACCTATCAGATAATCCGCAGGACCTTAAAGCAAGCTTTTGCCGACTGCACCGTC
ATACTGTGTGAGCACCGGATTGAAGCAATGCTGGAATGCCAGCAGTTTCTGGTGATCGA
GGAGAATAAGGTCCGGCAGTACGACAGCATCCAGAAGTTGTTGAATGAGCGCAGCCTT
TTCCGCCAGGCCATCTCCCCATCTGACAGAGTCAAGCTGTTTCCACATAGGAACTCCTCT
AAGTGCAAGTCCAAGCCCCAGATCGCTGCCCTCAAGGAGGAAACTGAGGAAGAGGTGC
AGGATACCCGCCTGTGA (SEQ ID NO: 9)

In yet another embodiment, an exemplary codon-optimized CFTR mRNA sequence is:

ATGCAGAGGAGCCCACTGGAGAAAGCCTCCGTGGTGAGTAAACTCTTTTTTAGTTGGAC
CAGACCCATCCTGCGAAAAGGATACAGGCAGCGCCTCGAGTTGTCAGATATCTACCAG
ATTCCTTCTGTGGACTCAGCTGACAATTTGAGTGAGAAGCTGGAGCGGGAGTGGGATAG
AGAGCTGGCGAGCAAAAAAAACCCCAAGCTTATCAATGCTCTGCGCCGCTGCTTTTTCT
GGAGGTTCATGTTTTATGGGATCTTCCTGTACCTGGGGGAGGTCACCAAAGCTGTTCAG
CCGCTCCTTCTTGGCCGCATCATCGCCAGCTATGACCCTGATAATAAAGAAGAAAGGTC
TATTGCTATTTATCTGGGAATTGGCCTCTGCTTGCTCTTCATCGTCCGCACCCTTCTGCTG

CACCCTGCCATTTTTGGCCTTCACCACATCGGCATGCAAATGAGAATTGCCATGTTCTCC
CTCATTTACAAAAAGACCCTGAAACTTTCCTCAAGAGTGTTAGATAAAATATCCATTGG
TCAGCTGGTCAGCCTGCTGTCCAACAATCTTAACAAATTTGATGAAGGCTTGGCGCTGG
CCCACTTCGTGTGGATTGCACCTCTGCAGGTGGCCCTGTTGATGGGACTTATATGGGAG
CTGCTTCAAGCCTCTGCTTTCTGTGGGCTGGGCTTTTTGATTGTACTGGCACTTTTTCAGG
CTGGGCTCGGAAGAATGATGATGAAATACAGAGATCAGCGGGCCGGGAAGATATCAGA
GCGACTTGTGATCACCAGTGAAATGATTGAAAATATTCAGAGCGTGAAAGCCTACTGCT
GGGAAGAAGCCATGGAGAAGATGATTGAGAACCTGAGGCAGACAGAGCTCAAGCTCAC
TCGGAAGGCTGCTTATGTTCGCTATTTCAACAGCAGCGCCTTCTTCTTCAGTGGCTTCTT
TGTTGTCTTCCTGTCTGTTCTGCCATATGCACTGATAAAAGGCATTATTTTACGAAAGAT
CTTCACCACCATCAGTTTTTGCATCGTTCTCAGGATGGCCGTCACAAGACAGTTCCCCTG
GGCTGTGCAGACCTGGTACGATTCCTTGGGGGCCATCAACAAGATTCAAGATTTCTTGC
AAAAACAAGAATATAAAACTTTAGAATACAACCTCACCACCACTGAAGTGGTCATGGA
AAATGTGACAGCCTTTTGGGAGGAGGGTTTTGGAGAATTGTTCGAGAAGGCAAAGCAG
AATAACAACAACAGGAAGACGAGCAATGGGGACGACTCTCTCTTCTTCAGCAACTTTTC
ACTGCTCGGGACCCCTGTGTTGAAAGATATAAACTTCAAGATCGAGAGGGGCCAGCTCT
TGGCTGTGGCAGGCTCCACTGGAGCTGGTAAAACATCTCTTCTCATGGTGATCATGGGG
GAACTGGAGCCTTCCGAAGGAAAAATCAAGCACAGTGGGAGAATCTCATTCTGCAGCC
AGTTTTCCTGGATCATGCCCGGCACCATTAAGGAAAACATCATATTTGGAGTGTCCTAT
GATGAGTACCGCTACCGGTCAGTCATCAAAGCCTGTCAGTTGGAGGAGGACATCTCCAA
GTTTGCAGAGAAAGACAACATTGTGCTTGGAGAGGGGGGTATCACTCTTTCTGGAGGAC
AAAGAGCCAGGATCTCTTTGGCCCGGGCAGTCTACAAGGATGCAGACCTCTACTTGTTG
GACAGTCCCTTCGGCTACCTCGACGTGCTGACTGAAAAAGAAATTTTTGAAAGCTGTGT
GTGCAAACTGATGGCAAACAAGACCAGGATTCTTGTCACCAGCAAGATGGAACATCTG
AAGAAAGCGGACAAAATTCTGATTCTGCATGAAGGGAGCTCCTACTTCTATGGAACATT
TAGCGAGCTTCAGAACCTACAGCCAGACTTCTCCTCCAAATTAATGGGCTGTGACTCCT
TCGACCAGTTCTCTGCAGAAAGAAGAAACTCTATACTCACAGAGACCCTCCACCGCTTC
TCCCTTGAGGGAGATGCCCCAGTTTCTTGGACAGAAACCAAGAAGCAGTCCTTTAAGCA
GACTGGCGAGTTTGGTGAAAAGAGGAAAAATTCAATTCTCAATCCAATTAACAGTATTC
GCAAGTTCAGCATTGTCCAGAAGACACCCCTCCAGATGAATGGCATCGAAGAAGATAG
TGACGAGCCGCTGGAGAGACGGCTGAGTCTGGTGCCAGATTCAGAACAGGGGGAGGCC
ATCCTGCCCCGGATCAGCGTCATTTCCACAGGCCCCACATTACAAGCACGGCGCCGGCA

GAGTGTTTTAAATCTCATGACCCATTCAGTGAACCAGGGCCAAAATATCCACAGGAAGA
CTACAGCTTCTACCCGGAAAGTGTCTCTGGCCCCTCAGGCCAATCTGACCGAGCTGGAC
ATCTACAGCAGGAGGCTCTCCCAGGAAACAGGGCTTGAAATATCTGAAGAGATTAATG
AAGAGGATCTTAAAGAGTGCTTCTTTGATGACATGGAGAGCATCCCCGCGGTGACCACA
TGGAACACCTACCTTAGATATATTACTGTCCACAAGAGCCTCATATTTGTCCTCATCTGG
TGCCTGGTTATTTTCCTCGCTGAGGTGGCGGCCAGTCTTGTTGTGCTCTGGCTGCTGGGC
AACACTCCTCTCCAGGACAAGGGCAATAGTACACACAGCAGAAATAATTCTTATGCCGT
CATCATTACAAGCACCTCCAGCTACTACGTGTTCTACATCTATGTGGGCGTGGCTGACA
CCCTCCTGGCCATGGGTTTCTTCCGGGGCCTGCCTTTGGTGCACACCCTCATCACAGTGT
CAAAAATTCTGCACCATAAAATGCTTCATTCTGTCCTGCAGGCACCCATGAGCACTTTG
AACACATTGAAGGCTGGCGGCATCCTCAACAGATTTTCTAAAGATATTGCTATCCTGGA
TGATCTCCTCCCCCTGACAATCTTTGACTTTATCCAGCTTCTGCTGATCGTGATTGGAGC
CATAGCAGTGGTTGCTGTCCTGCAGCCCTACATTTTTGTGGCCACCGTGCCCGTGATTGT
TGCCTTTATTATGCTCAGAGCTTACTTCCTGCAAACTTCTCAACAGCTCAAACAGCTAGA
ATCTGAGGGCCGGAGCCCCATTTTTACCCACCTGGTGACTTCCCTGAAGGGACTGTGGA
CTCTGAGAGCATTCGGGCGACAGCCTTACTTTGAGACACTGTTCCACAAGGCCCTGAAC
TTGCACACTGCCAACTGGTTTCTTTACCTGAGCACACTCCGCTGGTTCCAGATGCGGATA
GAGATGATCTTCGTCATCTTTTTTATAGCTGTAACCTTCATTTCTATCCTTACAACAGGA
GAAGGAGAGGGCAGGGTGGGAATCATCCTCACGCTGGCTATGAACATAATGTCCACCT
TGCAGTGGGCCGTGAATTCCAGTATAGATGTGGATTCTCTAATGAGGAGTGTCTCCCGG
GTGTTTAAATTCATTGATATGCCTACTGAGGGGAAACCCACCAAGTCAACAAAACCTTA
TAAGAATGGACAGCTGAGCAAGGTGATGATAATTGAGAACAGCCACGTGAAGAAGGAT
GACATTTGGCCCAGCGGGGGCCAGATGACTGTGAAGGACCTGACGGCCAAGTACACCG
AAGGTGGAAATGCCATTTTGGAAAACATCAGCTTCTCAATCTCTCCTGGGCAGAGAGTT
GGATTGCTGGGTCGCACGGGCAGCGGCAAATCAACCCTGCTCAGTGCCTTCCTTCGGCT
CCTGAATACAGAAGGCGAAATCCAAATTGACGGGGTGAGCTGGGACAGCATCACCCTG
CAGCAGTGGAGAAAAGCATTTGGGGTCATTCCACAGAAAGTTTTCATCTTCTCTGGCAC
TTTCAGAAAGAACCTGGACCCCTATGAGCAGTGGAGCGACCAGGAGATCTGGAAGGTT
GCAGATGAAGTTGGCCTGCGGAGTGTGATAGAACAATTTCCTGGCAAGCTGGATTTTGT
GCTGGTAGATGGAGGCTGCGTGCTGTCCCACGGCCACAAACAGCTGATGTGCCTCGCCC
GCTCCGTTCTTTCAAAGGCCAAAATCTTGCTTTTGGATGAGCCCAGTGCTCACCTTGACC
CAGTGACCTATCAGATAATCCGCAGGACCTTAAAGCAAGCTTTTGCCGACTGCACCGTC

ATACTGTGTGAGCACCGGATTGAAGCAATGCTGGAATGCCAGCAGTTTCTGGTGATCGA
GGAGAATAAGGTCCGGCAGTACGACAGCATCCAGAAGTTGTTGAATGAGCGCAGCCTT
TTCCGCCAGGCCATCTCCCCATCTGACAGAGTCAAGCTGTTTCCACATAGGAACTCCTCT
AAGTGCAAGTCCAAGCCCCAGATCGCTGCCCTCAAGGAGGAAACTGAGGAAGAGGTGC
AGGATACCCGCCTGTGA (SEQ ID NO: 10).

In yet another embodiment, an exemplary codon-optimized CFTR mRNA sequence is:

ATGCAGAGGAGCCCACTGGAGAAAGCCTCCGTGGTGAGTAAACTCTTTTTTAGTTGGAC
CAGACCCATCCTGCGAAAAGGATACAGGCAGCGCCTCGAGTTGTCAGATATCTACCAG
ATTCCTTCTGTGGACTCAGCTGACAATTTGAGTGAGAAGCTGGAGCGGGAGTGGGATAG
AGAGCTGGCGAGCAAAAAAAACCCCAAGCTTATCAATGCTCTGCGCCGCTGCTTTTTCT
GGAGGTTCATGTTTTATGGGATCTTCCTGTACCTGGGGGAGGTCACCAAAGCTGTTCAG
CCGCTCCTTCTTGGCCGCATCATCGCCAGCTATGACCCTGATAATAAAGAAGAAAGGTC
TATTGCTATTTATCTGGGAATTGGCCTCTGCTTGCTCTTCATCGTCCGCACCCTTCTGCTG
CACCCTGCCATTTTTGGCCTTCACCACATCGGCATGCAAATGAGAATTGCCATGTTCTCC
CTCATTTACAAAAAGACCCTGAAACTTTCCTCAAGAGTGTTAGATAAAATATCCATTGG
TCAGCTGGTCAGCCTGCTGTCCAACAATCTTAACAAATTTGATGAAGGCTTGGCGCTGG
CCCACTTCGTGTGGATTGCACCTCTGCAGGTGGCCCTGTTGATGGGACTTATATGGGAG
CTGCTTCAAGCCTCTGCTTTCTGTGGGCTGGGCTTTTTGATTGTACTGGCACTTTTTCAGG
CTGGGCTCGGAAGAATGATGATGAAATACAGAGATCAGCGGGCCGGGAAGATATCAGA
GCGACTTGTGATCACCAGTGAAATGATTGAAAATATTCAGAGCGTGAAAGCCTACTGCT
GGGAAGAAGCCATGGAGAAGATGATTGAGAACCTGAGGCAGACAGAGCTCAAGCTCAC
TCGGAAGGCTGCTTATGTTCGCTATTTCAACAGCAGCGCCTTCTTCTTCAGTGGCTTCTT
TGTTGTCTTCCTGTCTGTTCTGCCATATGCACTGATAAAAGGCATTATTTTACGAAAGAT
CTTCACCACCATCAGTTTTTGCATCGTTCTCAGGATGGCCGTCACAAGACAGTTCCCCTG
GGCTGTGCAGACCTGGTACGATTCCTTGGGGGCCATCAACAAGATTCAAGATTTCTTGC
AAAAACAAGAATATAAAACTTTAGAATACAACCTCACCACCACTGAAGTGGTCATGGA
AAATGTGACAGCCTTTTGGGAGGAGGGTTTTGGAGAATTGTTCGAGAAGGCAAAGCAG
AATAACAACAACAGGAAGACGAGCAATGGGGACGACTCTCTCTTCTTCAGCAACTTTTC
ACTGCTCGGGACCCCTGTGTTGAAAGATATAAACTTCAAGATCGAGAGGGGCCAGCTCT
TGGCTGTGGCAGGCTCCACTGGAGCTGGTAAAACATCTCTTCTCATGGTGATCATGGGG
GAACTGGAGCCTTCCGAAGGAAAAATCAAGCACAGTGGGAGAATCTCATTCTGCAGCC

AGTTTTCCTGGATCATGCCCGGCACCATTAAGGAAAACATCATATTTGGAGTGTCCTAT
GATGAGTACCGCTACCGGTCAGTCATCAAAGCCTGTCAGTTGGAGGAGGACATCTCCAA
GTTTGCAGAGAAAGACAACATTGTGCTTGGAGAGGGGGGTATCACTCTTTCTGGAGGAC
AAAGAGCCAGGATCTCTTTGGCCCGGGCAGTCTACAAGGATGCAGACCTCTACTTGTTG
GACAGTCCCTTCGGCTACCTCGACGTGCTGACTGAAAAAGAAATTTTTGAAAGCTGTGT
GTGCAAACTGATGGCAAACAAGACCAGGATTCTTGTCACCAGCAAGATGGAACATCTG
AAGAAAGCGGACAAAATTCTGATTCTGCATGAAGGGAGCTCCTACTTCTATGGAACATT
TAGCGAGCTTCAGAACCTACAGCCAGACTTCTCCTCCAAATTAATGGGCTGTGACTCCT
TCGACCAGTTCTCTGCAGAAAGAAGAAACTCTATACTCACAGAGACCCTCCACCGCTTC
TCCCTTGAGGGAGATGCCCCAGTTTCTTGGACAGAAACCAAGAAGCAGTCCTTTAAGCA
GACTGGCGAGTTTGGTGAAAAGAGGAAAAATTCAATTCTCAATCCAATTAACAGTATTC
GCAAGTTCAGCATTGTCCAGAAGACACCCCTCCAGATGAATGGCATCGAAGAAGATAG
TGACGAGCCGCTGGAGAGACGGCTGAGTCTGGTGCCAGATTCAGAACAGGGGGAGGCC
ATCCTGCCCCGGATCAGCGTCATTTCCACAGGCCCCACATTACAAGCACGGCGCCGGCA
GAGTGTTTTAAATCTCATGACCCATTCAGTGAACCAGGGCCAAAATATCCACAGGAAGA
CTACAGCTTCTACCCGGAAAGTGTCTCTGGCCCCTCAGGCCAATCTGACCGAGCTGGAC
ATCTACAGCAGGAGGCTCTCCCAGGAAACAGGGCTGGAAATATCTGAAGAGATTAATG
AAGAGGATCTTAAAGAGTGCTTCTTTGATGACATGGAGAGCATCCCCGCGGTGACCACA
TGGAACACCTACCTTAGATATATTACTGTCCACAAGAGCCTCATATTTGTCCTCATCTGG
TGCCTGGTTATTTTCCTCGCTGAGGTGGCGGCCAGTCTTGTTGTGCTCTGGCTGCTGGGC
AACACTCCTCTCCAGGACAAGGGCAATAGTACTCACAGCAGAAATAATTCTTATGCCGT
CATCATTACAAGCACCTCCAGCTACTACGTGTTCTACATCTATGTGGGCGTGGCTGACA
CCCTCCTGGCCATGGGTTTCTTCCGGGGCCTGCCTTTGGTGCACACCCTCATCACAGTGT
CAAAAATTCTGCACCATAAAATGCTTCATTCTGTCCTGCAGGCACCCATGAGCACTTTG
AACACATTGAAGGCTGGCGGCATCCTCAACAGATTTTCTAAAGATATTGCTATCCTGGA
TGATCTCCTCCCCCTGACAATCTTTGACTTTATCCAGCTTCTGCTGATCGTGATTGGAGC
CATAGCAGTGGTTGCTGTCCTGCAGCCCTACATTTTTGTGGCCACCGTGCCCGTGATTGT
TGCCTTTATTATGCTCAGAGCTTACTTCCTGCAAACTTCTCAACAGCTCAAACAGCTAGA
ATCTGAGGGCCGGAGCCCCATTTTTACCCACCTGGTGACTTCCCTGAAGGGACTGTGGA
CTCTGAGAGCATTCGGGCGACAGCCTTACTTTGAGACACTGTTCCACAAGGCCCTGAAC
TTGCACACTGCCAACTGGTTTCTTTACCTGAGCACACTCCGCTGGTTCCAGATGCGGATA
GAGATGATCTTCGTCATCTTTTTTATAGCTGTAACCTTCATTTCTATCCTTACAACAGGA

GAAGGAGAGGGCAGGGTGGGAATCATCCTCACGCTGGCTATGAACATAATGTCCACCT
TGCAGTGGGCCGTGAATTCCAGTATAGATGTGGATTCTCTAATGAGGAGTGTCTCCCGG
GTGTTTAAATTCATTGATATGCCAACTGAGGGGAAACCCACCAAGTCAACAAAACCTTA
TAAGAATGGACAGCTGAGCAAGGTGATGATAATTGAGAACAGCCACGTGAAGAAGGAT
GACATTTGGCCCAGCGGGGGCCAGATGACTGTGAAGGACCTGACGGCCAAGTACACCG
AAGGTGGAAATGCCATTTTGGAAAACATCAGCTTCTCAATCTCTCCTGGGCAGAGAGTT
GGATTGCTGGGTCGCACGGGCAGCGGCAAATCAACCCTGCTCAGTGCCTTCCTTCGGCT
CCTGAATACAGAAGGCGAAATCCAAATTGACGGGGTGAGCTGGGACAGCATCACCCTG
CAGCAGTGGAGAAAAGCATTTGGGGTCATTCCACAGAAAGTTTTCATCTTCTCTGGCAC
TTTCAGAAAGAACCTGGACCCCTATGAGCAGTGGAGCGACCAGGAGATCTGGAAGGTT
GCAGATGAAGTTGGCCTGCGGAGTGTGATAGAACAATTTCCTGGCAAGCTGGATTTTGT
GCTGGTAGATGGAGGCTGCGTGCTGTCCCACGGCCACAAACAGCTGATGTGCCTCGCCC
GCTCCGTTCTTTCAAAGGCCAAAATCTTGCTTTTGGATGAGCCCAGTGCTCACCTCGACC
CAGTGACCTATCAGATAATCCGCAGGACCTTAAAGCAAGCTTTTGCCGACTGCACCGTC
ATACTGTGTGAGCACCGGATTGAAGCAATGCTGGAATGCCAGCAGTTTCTGGTGATCGA
GGAGAATAAGGTCCGGCAGTACGACAGCATCCAGAAGTTGTTGAATGAGCGCAGCCTT
TTCCGCCAGGCCATCTCCCCATCTGACAGAGTCAAGCTGTTTCCACATAGGAACTCCTCT
AAGTGCAAGTCCAAGCCCCAGATCGCTGCCCTCAAGGAGGAAACTGAGGAAGAGGTGC
AGGATACCCGCCTGTGA (SEQ ID NO: 11).

In yet another embodiment, an exemplary codon-optimized CFTR mRNA sequence is:

ATGCAGAGGAGCCCACTGGAGAAAGCCTCCGTGGTGAGTAAACTCTTTTTTAGTTGGAC
CAGACCCATCCTGCGAAAAGGATACAGGCAGCGCCTCGAGTTGTCAGATATCTACCAG
ATTCCTTCTGTGGACTCAGCTGACAATTTGAGTGAGAAGCTGGAGCGGGAGTGGGATAG
AGAGCTGGCGAGCAAAAAAAACCCCAAGCTTATCAATGCTCTGCGCCGCTGCTTTTTCT
GGAGGTTCATGTTTTATGGGATCTTCCTGTACCTGGGGGAGGTCACCAAAGCTGTTCAG
CCGCTCCTTCTTGGCCGCATCATCGCCAGCTATGACCCTGATAATAAAGAAGAAAGGTC
TATTGCTATTTATCTGGGAATTGGCCTCTGCTTGCTCTTCATCGTCCGCACCCTTCTGCTG
CACCCTGCCATTTTTGGCCTTCACCACATCGGCATGCAAATGAGAATTGCCATGTTCTCC
CTCATTTACAAAAAGACCCTGAAACTTTCCTCAAGAGTGTTAGATAAAATATCCATTGG
TCAGCTGGTCAGCCTGCTGTCCAACAATCTTAACAAATTTGATGAAGGCTTGGCGCTGG
CCCACTTCGTGTGGATTGCACCTCTGCAGGTGGCCCTGTTGATGGGACTTATATGGGAG

CTGCTTCAAGCCTCTGCTTTCTGTGGGCTGGGCTTTTTGATTGTACTGGCACTTTTTCAGG
CTGGGCTCGGAAGAATGATGATGAAATACAGAGATCAGCGGGCCGGGAAGATATCAGA
GCGACTTGTGATCACCAGTGAAATGATTGAAAATATTCAGAGCGTGAAAGCCTACTGCT
GGGAAGAAGCCATGGAGAAGATGATTGAGAACCTGAGGCAGACAGAGCTCAAGCTCAC
TCGGAAGGCTGCTTATGTTCGCTATTTCAACAGCAGCGCCTTCTTCTTCAGTGGCTTCTT
TGTTGTCTTCCTGTCTGTTCTGCCATATGCACTGATAAAAGGCATTATTTTACGAAAGAT
CTTCACCACCATCAGTTTTTGCATCGTTCTCAGGATGGCCGTCACAAGACAGTTCCCCTG
GGCTGTGCAGACCTGGTACGATTCCTTGGGGGCCATCAACAAGATTCAAGATTTCTTGC
AAAAACAAGAATATAAAACTTTAGAATACAACCTCACCACCACTGAAGTGGTCATGGA
AAATGTGACAGCCTTTTGGGAGGAGGGTTTTGGAGAATTGTTCGAGAAGGCAAAGCAG
AATAACAACAACAGGAAGACGAGCAATGGGGACGACTCTCTCTTCTTCAGCAACTTTTC
ACTGCTCGGGACCCCTGTGTTGAAAGATATAAACTTCAAGATCGAGAGGGGCCAGCTCT
TGGCTGTGGCAGGCTCCACTGGAGCTGGTAAAACATCTCTTCTCATGGTGATCATGGGG
GAACTGGAGCCTTCCGAAGGAAAAATCAAGCACAGTGGGAGAATCTCATTCTGCAGCC
AGTTTTCCTGGATCATGCCCGGCACCATTAAGGAAAACATCATATTTGGAGTGTCCTAT
GATGAGTACCGCTACCGGTCAGTCATCAAAGCCTGTCAGTTGGAGGAGGACATCTCCAA
GTTTGCAGAGAAAGACAACATTGTGCTTGGAGAGGGGGGTATCACTCTTTCTGGAGGAC
AAAGAGCCAGGATCTCTTTGGCCCGGGCAGTCTACAAGGATGCAGACCTCTACTTGTTG
GACAGTCCCTTCGGCTACCTCGACGTGCTGACTGAAAAAGAAATTTTTGAAAGCTGTGT
GTGCAAACTGATGGCAAACAAGACCAGGATTCTTGTCACCAGCAAGATGGAACATCTG
AAGAAAGCGGACAAAATTCTGATTCTGCATGAAGGGAGCTCCTACTTCTATGGAACATT
TAGCGAGCTTCAGAACCTACAGCCAGACTTCTCCTCCAAATTAATGGGCTGTGACTCCT
TCGACCAGTTCTCTGCAGAAAGAAGAAACTCTATACTCACAGAGACCCTCCACCGCTTC
TCCCTTGAGGGAGATGCCCCAGTTTCTTGGACAGAAACCAAGAAGCAGTCCTTTAAGCA
GACTGGCGAGTTTGGTGAAAAGAGGAAAAATTCAATTCTCAATCCTATTAACAGTATTC
GCAAGTTCAGCATTGTCCAGAAGACACCCCTCCAGATGAATGGCATCGAAGAAGATAG
TGACGAGCCGCTGGAGAGACGGCTGAGTCTGGTGCCAGATTCAGAACAGGGGGAGGCC
ATCCTGCCCCGGATCAGCGTCATTTCCACAGGCCCCACATTACAAGCACGGCGCCGGCA
GAGTGTTTTAAATCTCATGACCCATTCAGTGAACCAGGGCCAAAATATCCACAGGAAGA
CTACAGCTTCTACCCGGAAAGTGTCTCTGGCCCCTCAGGCCAATCTGACCGAGCTGGAC
ATCTACAGCAGGAGGCTCTCCCAGGAAACAGGGCTTGAAATATCTGAAGAGATTAATG
AAGAGGATCTTAAAGAGTGCTTCTTTGATGACATGGAGAGCATCCCCGCGGTGACCACA

TGGAACACCTACCTTAGATATATTACTGTCCACAAGAGCCTCATATTTGTCCTCATCTGG
TGCCTGGTTATTTTCCTCGCTGAGGTGGCGGCCAGTCTTGTTGTGCTCTGGCTGCTGGGC
AACACTCCTCTCCAGGACAAGGGCAATAGTACTCACAGCAGAAATAATTCTTATGCCGT
CATCATTACAAGCACCTCCAGCTACTACGTGTTCTACATCTATGTGGGCGTGGCTGACA
CCCTCCTGGCCATGGGTTTCTTCCGGGGCCTGCCTTTGGTGCACACCCTCATCACAGTGT
CAAAAATTCTGCACCATAAAATGCTTCATTCTGTCCTGCAGGCACCCATGAGCACTTTG
AACACATTGAAGGCTGGCGGCATCCTCAACAGATTTTCTAAAGATATTGCTATCCTGGA
TGATCTCCTCCCCCTGACAATCTTTGACTTTATCCAGCTTCTGCTGATCGTGATTGGAGC
CATAGCAGTGGTTGCTGTCCTGCAGCCCTACATTTTTGTGGCCACCGTGCCCGTGATTGT
TGCCTTTATTATGCTCAGAGCTTACTTCCTGCAAACTTCTCAACAGCTCAAACAGCTAGA
ATCTGAGGGCCGGAGCCCCATTTTTACCCACCTGGTGACTTCCCTGAAGGGACTGTGGA
CTCTGAGAGCATTCGGGCGACAGCCTTACTTTGAGACACTGTTCCACAAGGCCCTGAAC
TTGCACACTGCCAACTGGTTTCTTTACCTGAGCACACTCCGCTGGTTCCAGATGCGGATA
GAGATGATCTTCGTCATCTTTTTTATAGCTGTAACCTTCATTTCTATCCTTACAACAGGA
GAAGGAGAGGGCAGGGTGGGAATCATCCTCACGCTGGCTATGAACATAATGTCCACCT
TGCAGTGGGCCGTGAATTCCAGTATAGATGTGGATTCTCTAATGAGGAGTGTCTCCCGG
GTGTTTAAATTCATTGATATGCCTACTGAGGGGAAACCCACCAAGTCAACAAAACCTTA
TAAGAATGGACAGCTGAGCAAGGTGATGATAATTGAGAACAGCCACGTGAAGAAGGAT
GACATTTGGCCCAGCGGGGGCCAGATGACTGTGAAGGACCTGACGGCCAAGTACACCG
AAGGTGGAAATGCCATTTTGGAAAACATCAGCTTCTCAATCTCTCCTGGGCAGAGAGTT
GGATTGCTGGGTCGCACGGGCAGCGGCAAATCAACCCTGCTCAGTGCCTTCCTTCGGCT
CCTGAATACAGAAGGCGAAATCCAAATTGACGGGGTGAGCTGGGACAGCATCACCCTG
CAGCAGTGGAGAAAAGCATTTGGGGTCATTCCACAGAAAGTTTTCATCTTCTCTGGCAC
TTTCAGAAAGAACCTGGACCCCTATGAGCAGTGGAGCGACCAGGAGATCTGGAAGGTT
GCAGATGAAGTTGGCCTGCGGAGTGTGATAGAACAATTTCCTGGCAAGCTGGATTTTGT
GCTGGTAGATGGAGGCTGCGTGCTGTCCCACGGCCACAAACAGCTGATGTGCCTCGCCC
GCTCCGTTCTTTCAAAGGCCAAAATCTTGCTTTTGGATGAGCCCAGTGCTCACCTCGACC
CAGTGACCTATCAGATAATCCGCAGGACCTTAAAGCAAGCTTTTGCCGACTGCACCGTC
ATACTGTGTGAGCACCGGATTGAAGCAATGCTGGAATGCCAGCAGTTTCTGGTGATCGA
GGAGAATAAGGTCCGGCAGTACGACAGCATCCAGAAGTTGTTGAATGAGCGCAGCCTT
TTCCGCCAGGCCATCTCCCCATCTGACAGAGTCAAGCTGTTTCCACATAGGAACTCCTCT

AAGTGCAAGTCCAAGCCCCAGATCGCTGCCCTCAAGGAGGAAACTGAGGAAGAGGTGC
AGGATACCCGCCTGTGA (SEQ ID NO: 12).

In another embodiment, an exemplary codon-optimized CFTR mRNA sequence is:

ATGCAGAGGAGCCCACTGGAGAAAGCCTCCGTGGTGAGTAAACTCTTTTTTAGTTGGAC
CAGACCCATCCTGCGAAAAGGATACAGGCAGCGCCTCGAGTTGTCAGATATCTACCAG
ATTCCTTCTGTGGACTCAGCTGACAATTTGAGTGAGAAGCTGGAGCGGGAGTGGGATAG
AGAGCTGGCGAGCAAAAAAAACCCCAAGCTTATCAATGCTCTGCGCCGCTGCTTTTTCT
GGAGGTTCATGTTTTATGGGATCTTCCTGTACCTGGGGGAGGTCACCAAAGCTGTTCAG
CCGCTCCTTCTTGGCCGCATCATCGCCAGCTATGACCCTGATAATAAAGAAGAAAGGTC
TATTGCTATTTATCTGGGAATTGGCCTCTGCTTGCTCTTCATCGTCCGCACCCTTCTGCTG
CACCCTGCCATTTTTGGCCTTCACCACATCGGCATGCAAATGAGAATTGCCATGTTCTCC
CTCATTTACAAAAAGACCCTGAAACTTTCCTCAAGAGTGTTAGATAAAATATCCATTGG
TCAGCTGGTCAGCCTGCTGTCCAACAATCTTAACAAATTTGATGAAGGCTTGGCGCTGG
CCCACTTCGTGTGGATTGCACCTCTGCAGGTGGCCCTGTTGATGGGACTTATATGGGAG
CTGCTTCAAGCCTCTGCTTTCTGTGGGCTGGGCTTTTTGATTGTACTGGCACTTTTTCAGG
CTGGGCTCGGAAGAATGATGATGAAATACAGAGATCAGCGGGCCGGGAAGATTTCAGA
GCGACTTGTGATCACCAGTGAAATGATTGAAAATATTCAGAGCGTGAAAGCCTACTGCT
GGGAAGAAGCCATGGAGAAGATGATTGAGAACCTGAGGCAGACAGAGCTCAAGCTCAC
TCGGAAGGCTGCTTATGTTCGCTATTTCAACAGCAGCGCCTTCTTCTTCAGTGGCTTCTT
TGTTGTCTTCCTGTCTGTTCTGCCATATGCACTGATAAAAGGCATTATTTTACGAAAGAT
CTTCACCACCATCAGTTTTTGCATCGTTCTCAGGATGGCCGTCACAAGACAGTTCCCCTG
GGCTGTGCAGACCTGGTACGATTCCTTGGGGGCCATCAACAAGATTCAAGATTTCTTGC
AAAAACAAGAATATAAAACTTTAGAATACAACCTCACCACCACTGAAGTGGTCATGGA
AAATGTGACAGCCTTTTGGGAGGAGGGTTTTGGAGAATTGTTCGAGAAGGCAAAGCAG
AATAACAACAACAGGAAGACGAGCAATGGGGACGACTCTCTCTTCTTCAGCAACTTTTC
ACTGCTCGGGACCCCTGTGTTGAAAGATATAAACTTCAAGATCGAGAGGGGCCAGCTCT
TGGCTGTGGCAGGCTCCACTGGAGCTGGTAAAACATCTCTTCTCATGGTGATCATGGGG
GAACTGGAGCCTTCCGAAGGAAAAATCAAGCACAGTGGGAGAATCTCATTCTGCAGCC
AGTTTTCCTGGATCATGCCCGGCACCATTAAGGAAAACATCATATTTGGAGTGTCCTAT
GATGAGTACCGCTACCGGTCAGTCATCAAAGCCTGTCAGTTGGAGGAGGACATCTCCAA
GTTTGCAGAGAAAGACAACATTGTGCTTGGAGAGGGGGGTATCACTCTTTCTGGAGGAC
AAAGAGCCAGGATCTCTTTGGCCCGGGCAGTCTACAAGGATGCAGACCTCTACTTGTTG

GACAGTCCCTTCGGCTACCTCGACGTGCTGACTGAAAAAGAAATTTTTGAAAGCTGTGT
GTGCAAACTGATGGCAAACAAGACCAGGATTCTTGTCACCAGCAAGATGGAACATCTG
AAGAAAGCGGACAAAATTCTGATTCTGCATGAAGGGAGCTCCTACTTCTATGGAACATT
TAGCGAGCTTCAGAACCTACAGCCAGACTTCTCCTCCAAATTAATGGGCTGTGACTCCT
TCGACCAGTTCTCTGCAGAAAGAAGAAACTCTATACTCACAGAGACCCTCCACCGCTTC
TCCCTTGAGGGAGATGCCCCAGTTTCTTGGACAGAAACCAAGAAGCAGTCCTTTAAGCA
GACTGGCGAGTTTGGTGAAAAGAGGAAAAATTCAATTCTCAATCCAATTAACAGTATTC
GCAAGTTCAGCATTGTCCAGAAGACACCCCTCCAGATGAATGGCATCGAAGAAGATAG
TGACGAGCCGCTGGAGAGACGGCTGAGTCTGGTGCCAGATTCAGAACAGGGGGAGGCC
ATCCTGCCCCGGATCAGCGTCATTTCCACAGGCCCCACATTACAAGCACGGCGCCGGCA
GAGTGTTTTAAATCTCATGACCCATTCAGTGAACCAGGGCCAAAATATCCACAGGAAGA
CTACAGCTTCTACCCGGAAAGTGTCTCTGGCCCCTCAGGCCAATCTGACCGAGCTGGAC
ATCTACAGCAGGAGGCTCTCCCAGGAAACAGGGCTGGAAATATCTGAAGAGATTAATG
AAGAGGATCTTAAAGAGTGCTTCTTTGATGACATGGAGAGCATCCCCGCGGTGACCACA
TGGAACACCTACCTTAGATATATTACTGTCCACAAGAGCCTCATATTTGTCCTCATCTGG
TGCCTGGTTATTTTCCTCGCTGAGGTGGCGGCCAGTCTTGTTGTGCTCTGGCTGCTGGGC
AACACTCCTCTCCAGGACAAGGGCAATAGTACTCACAGCAGAAATAATTCTTATGCCGT
CATCATTACAAGCACCTCCAGCTACTACGTGTTCTACATCTATGTGGGCGTGGCTGACA
CCCTCCTGGCCATGGGTTTCTTCCGGGGCCTGCCTTTGGTGCACACCCTCATCACAGTGT
CAAAAATTCTGCACCATAAAATGCTTCATTCTGTCCTGCAGGCACCCATGAGCACTTTG
AACACATTGAAGGCTGGCGGCATCCTCAACAGATTTTCTAAAGATATTGCTATCCTGGA
TGATCTCCTCCCCCTGACAATCTTTGACTTTATCCAGCTTCTGCTGATCGTGATTGGAGC
CATAGCAGTGGTTGCTGTCCTGCAGCCCTACATTTTTGTGGCCACCGTGCCCGTGATTGT
TGCCTTTATTATGCTCAGAGCTTACTTCCTGCAAACTTCTCAACAGCTCAAACAGCTAGA
ATCTGAGGGCCGGAGCCCCATTTTTACCCACCTGGTGACTTCCCTGAAGGGACTGTGGA
CTCTGAGAGCATTCGGGCGACAGCCTTACTTTGAGACACTGTTCCACAAGGCCCTGAAC
TTGCACACTGCCAACTGGTTTCTTTACCTGAGCACACTCCGCTGGTTCCAGATGCGGATA
GAGATGATCTTCGTCATCTTTTTTATAGCTGTAACCTTCATTTCTATCCTTACAACAGGA
GAAGGAGAGGGCAGGGTGGGAATCATCCTCACGCTGGCTATGAACATAATGTCCACCT
TGCAGTGGGCCGTGAATTCCAGTATAGATGTGGATTCTCTAATGAGGAGTGTCTCCCGG
GTGTTTAAATTCATTGATATGCCAACTGAGGGGAAACCCACCAAGTCAACAAAACCTTA
TAAGAATGGACAGCTGAGCAAGGTGATGATAATTGAGAACAGCCACGTGAAGAAGGAT

GACATTTGGCCCAGCGGGGGCCAGATGACTGTGAAGGACCTGACGGCCAAGTACACCG
AAGGTGGAAATGCCATTTTGGAAAACATCAGCTTCTCAATCTCTCCTGGGCAGAGAGTT
GGATTGCTGGGTCGCACGGGCAGCGGCAAATCAACCCTGCTCAGTGCCTTCCTTCGGCT
CCTGAATACAGAAGGCGAAATCCAAATTGACGGGGTGAGCTGGGACAGCATCACCCTG
CAGCAGTGGAGAAAAGCATTTGGGGTCATTCCACAGAAAGTTTTCATCTTCTCTGGCAC
TTTCAGAAAGAACCTGGACCCCTATGAGCAGTGGAGCGACCAGGAGATCTGGAAGGTT
GCAGATGAAGTTGGCCTGCGGAGTGTGATAGAACAATTTCCTGGCAAGCTGGATTTTGT
GCTGGTAGATGGAGGCTGCGTGCTGTCCCACGGCCACAAACAGCTGATGTGCCTCGCCC
GCTCCGTTCTTTCAAAGGCCAAAATCTTGCTTTTGGATGAGCCCAGTGCTCACCTCGACC
CAGTGACCTATCAGATAATCCGCAGGACCTTAAAGCAAGCTTTTGCCGACTGCACCGTC
ATACTGTGTGAGCACCGGATTGAAGCAATGCTGGAATGCCAGCAGTTTCTGGTGATCGA
GGAGAATAAGGTCCGGCAGTACGACAGCATCCAGAAGTTGTTGAATGAGCGCAGCCTT
TTCCGCCAGGCCATCTCCCCATCTGACAGAGTCAAGCTGTTTCCACATAGGAACTCCTCT
AAGTGCAAGTCCAAGCCCCAGATCGCTGCCCTCAAGGAGGAAACTGAGGAAGAGGTGC
AGGATACCCGCCTGTGA (SEQ ID NO: 13).

In another embodiment, an exemplary codon-optimized CFTR mRNA sequence is:

ATGCAGAGGAGCCCACTGGAGAAAGCCTCCGTGGTGAGTAAACTCTTTTTTAGTTGGAC
CAGACCCATCCTGCGAAAAGGATACAGGCAGCGCCTCGAGTTGTCTGATATCTACCAGA
TTCCTTCTGTGGACTCAGCTGACAATTTGAGTGAGAAGCTGGAGCGGGAGTGGGATAGA
GAGCTGGCGAGCAAAAAAAACCCCAAGCTTATCAATGCTCTGCGCCGCTGCTTTTTCTG
GAGGTTCATGTTTTATGGGATCTTCCTGTACCTGGGGGAGGTCACCAAAGCTGTTCAGC
CGCTCCTTCTTGGCCGCATCATCGCCAGCTATGACCCTGATAATAAAGAAGAAAGGTCT
ATTGCTATTTATCTGGGAATTGGCCTCTGCTTGCTCTTCATCGTCCGCACCCTTCTGCTGC
ACCCTGCCATTTTTGGCCTTCACCACATCGGCATGCAAATGAGAATTGCCATGTTCTCCC
TCATTTACAAAAAGACCCTGAAACTTTCCTCAAGAGTGTTAGATAAAATATCCATTGGT
CAGCTGGTCAGCCTGCTGTCCAACAATCTTAACAAATTTGATGAAGGCTTGGCGCTGGC
CCACTTCGTGTGGATTGCACCTCTGCAGGTGGCCCTGTTGATGGGACTTATATGGGAGC
TGCTTCAAGCCTCTGCTTTCTGTGGGCTGGGCTTTTTGATTGTACTGGCACTTTTTCAGGC
TGGGCTCGGAAGAATGATGATGAAATACAGAGATCAGCGGGCCGGGAAGATATCAGAG
CGACTTGTGATCACCAGTGAAATGATTGAAAATATTCAGAGCGTGAAAGCCTACTGCTG
GGAAGAAGCCATGGAGAAGATGATTGAGAACCTGAGGCAGACAGAGCTCAAGCTCACT
CGGAAGGCTGCTTATGTTCGCTATTTCAACAGCAGCGCCTTCTTCTTCAGTGGCTTCTTT

GTTGTCTTCCTGTCTGTTCTGCCATATGCACTGATAAAAGGCATTATTTTACGAAAGATC
TTCACCACCATCAGTTTTTGCATCGTTCTCAGGATGGCCGTCACAAGACAGTTCCCCTGG
GCTGTGCAGACCTGGTACGATTCCTTGGGGGCCATCAACAAGATTCAAGATTTCTTGCA
AAAACAAGAATATAAAACTTTAGAATACAACCTCACCACCACTGAAGTGGTCATGGAA
AATGTGACAGCCTTTTGGGAGGAGGGTTTTGGAGAATTGTTCGAGAAGGCAAAGCAGA
ATAACAACAACAGGAAGACGAGCAATGGGGACGACTCTCTCTTCTTCAGCAACTTTTCA
CTGCTCGGGACCCCTGTGTTGAAAGATATAAACTTCAAGATCGAGAGGGGCCAGCTCTT
GGCTGTGGCAGGCTCCACTGGAGCTGGTAAAACATCTCTTCTCATGGTGATCATGGGGG
AACTGGAGCCTTCCGAAGGAAAAATCAAGCACAGTGGGAGAATCTCATTCTGCAGCCA
GTTTTCCTGGATCATGCCCGGCACCATTAAGGAAAACATCATATTTGGAGTGTCCTATG
ATGAGTACCGCTACCGGTCCGTCATCAAAGCCTGTCAGTTGGAGGAGGACATCTCCAAG
TTTGCAGAGAAAGACAACATTGTGCTTGGAGAGGGGGGTATCACTCTTTCTGGAGGACA
AAGAGCCAGGATCTCTTTGGCCCGGGCAGTCTACAAGGATGCAGACCTCTACTTGTTGG
ACAGTCCCTTCGGCTACCTCGACGTGCTGACTGAAAAAGAAATTTTTGAAAGCTGTGTG
TGCAAACTGATGGCAAACAAGACCAGGATTCTTGTCACCAGCAAGATGGAACATCTGA
AGAAAGCGGACAAAATTCTGATTCTGCATGAAGGGAGCTCCTACTTCTATGGAACATTT
AGCGAGCTTCAGAACCTACAGCCAGACTTCTCCTCCAAATTAATGGGCTGTGACTCCTT
CGACCAGTTCTCTGCAGAAAGAAGAAACTCTATACTCACAGAGACCCTCCACCGCTTCT
CCCTTGAGGGAGATGCCCCAGTTTCTTGGACAGAAACCAAGAAGCAGTCCTTTAAGCAG
ACTGGCGAGTTTGGTGAAAAGAGGAAAAATTCAATTCTCAATCCAATTAACAGTATTCG
CAAGTTCAGCATTGTCCAGAAGACACCCCTCCAGATGAATGGCATCGAAGAAGATAGT
GACGAGCCGCTGGAGAGACGGCTGAGTCTGGTGCCAGATTCAGAACAGGGGGAGGCCA
TCCTGCCCCGGATCAGCGTCATTTCCACAGGCCCCACATTACAAGCACGGCGCCGGCAG
AGTGTTTTAAATCTCATGACCCATTCAGTGAACCAGGGCCAAAATATCCACAGGAAGAC
TACAGCTTCTACCCGGAAAGTGTCTCTGGCCCCTCAGGCCAATCTGACCGAGCTGGACA
TCTACAGCAGGAGGCTCTCCCAGGAAACAGGGCTTGAAATATCTGAAGAGATTAATGA
AGAGGATCTTAAAGAGTGCTTCTTTGATGACATGGAGAGCATCCCCGCGGTGACCACAT
GGAACACCTACCTTAGATATATTACTGTCCACAAGAGCCTCATATTTGTCCTCATCTGGT
GCCTGGTTATTTTCCTCGCTGAGGTGGCGGCCAGTCTTGTTGTGCTCTGGCTGCTGGGCA
ACACTCCTCTCCAGGACAAGGGCAATAGTACTCACAGCAGAAATAATTCTTATGCCGTC
ATCATTACAAGCACCTCCAGCTACTACGTGTTCTACATCTATGTGGGCGTGGCTGACAC
CCTCCTGGCCATGGGTTTCTTCCGGGGCCTGCCTTTGGTGCACACCCTCATCACAGTGTC

AAAAATTCTGCACCATAAAATGCTTCATTCTGTCCTGCAGGCACCCATGAGCACTTTGA
ACACATTGAAGGCTGGCGGCATCCTCAACAGATTTTCTAAAGATATTGCTATCCTGGAT
GATCTCCTCCCCCTGACAATCTTTGACTTTATCCAGCTTCTGCTGATCGTGATTGGAGCC
ATAGCAGTGGTTGCTGTCCTGCAGCCCTACATTTTTGTGGCCACCGTGCCCGTGATTGTT
GCCTTTATTATGCTCAGAGCTTACTTCCTGCAAACTTCTCAACAGCTCAAACAGCTAGAA
TCTGAGGGCCGGAGCCCCATTTTTACCCACCTGGTGACTTCCCTGAAGGGACTGTGGAC
TCTGAGAGCATTCGGGCGACAGCCTTACTTTGAGACACTGTTCCACAAGGCCCTGAACT
TGCACACTGCCAACTGGTTTCTTTACCTGAGCACACTCCGCTGGTTCCAGATGCGGATA
GAGATGATCTTCGTCATCTTTTTTATAGCTGTAACCTTCATTTCTATCCTTACAACAGGA
GAAGGAGAGGGCAGGGTGGGAATCATCCTCACGCTGGCTATGAACATAATGTCCACCT
TGCAGTGGGCCGTGAATTCCAGTATAGATGTGGATTCTCTAATGAGGAGTGTCTCCCGG
GTGTTTAAATTCATTGATATGCCTACTGAGGGGAAACCCACCAAGTCAACAAAGCCTTA
TAAGAATGGACAGCTGAGCAAGGTGATGATAATTGAGAACAGCCACGTGAAGAAGGAT
GACATTTGGCCCAGCGGGGGCCAGATGACTGTGAAGGACCTGACGGCCAAGTACACCG
AAGGTGGAAATGCCATTTTGGAAAACATCAGCTTCTCAATCTCTCCTGGGCAGAGAGTT
GGATTGCTGGGTCGCACGGGCAGCGGCAAATCAACCCTGCTCAGTGCCTTCCTTCGGCT
CCTGAATACAGAAGGCGAAATCCAAATTGACGGGGTGAGCTGGGACAGCATCACCCTG
CAGCAGTGGAGAAAAGCATTTGGGGTCATTCCACAGAAAGTTTTCATCTTCTCTGGCAC
TTTCAGAAAGAACCTGGACCCCTATGAGCAGTGGAGCGACCAGGAGATCTGGAAGGTT
GCAGATGAAGTTGGCCTGCGGAGTGTGATAGAACAATTTCCTGGCAAGCTGGATTTTGT
GCTGGTAGATGGAGGCTGCGTGCTGTCCCACGGCCACAAACAGCTGATGTGCCTCGCCC
GCTCCGTTCTTTCAAAGGCCAAAATCTTGCTTTTGGATGAGCCCAGTGCTCACCTCGACC
CAGTGACCTATCAGATAATCCGCAGGACCTTAAAGCAAGCTTTTGCCGACTGCACCGTC
ATACTGTGTGAGCACCGGATTGAAGCAATGCTGGAATGCCAGCAGTTTCTGGTGATCGA
GGAGAATAAGGTCCGGCAGTACGACAGCATCCAGAAGTTGTTGAATGAGCGCAGCCTT
TTCCGCCAGGCCATCTCCCCATCTGACAGAGTCAAGCTGTTTCCACATAGGAACTCCTCT
AAGTGCAAGTCCAAGCCCCAGATCGCTGCCCTCAAGGAGGAAACTGAGGAAGAGGTGC
AGGATACCCGCCTGTGA (SEQ ID NO: 14).

In another embodiment, an exemplary codon-optimized CFTR mRNA sequence is:

ATGCAGAGGAGCCCACTGGAGAAAGCCTCCGTGGTGAGTAAACTCTTTTTTAGTTGGAC
CAGACCCATCCTGCGAAAAGGATACAGGCAGCGCCTCGAGTTGTCAGATATCTACCAG
ATTCCTTCTGTGGACTCAGCTGACAATTTGAGTGAGAAGCTGGAGCGGGAGTGGGATAG

AGAGCTGGCGAGCAAAAAAAACCCCAAGCTTATCAATGCTCTGCGCCGCTGCTTTTTCT
GGAGGTTCATGTTTTATGGGATCTTCCTGTACCTGGGGGAGGTCACCAAAGCTGTTCAG
CCGCTCCTTCTTGGCCGCATCATCGCCAGCTATGACCCTGATAATAAAGAAGAAAGGTC
TATTGCTATTTATCTGGGAATTGGCCTCTGCTTGCTCTTCATCGTCCGCACCCTTCTGCTG
CACCCTGCCATTTTTGGCCTTCACCACATCGGCATGCAAATGAGAATTGCCATGTTCTCC
CTCATTTACAAAAAGACCCTGAAACTTTCCTCAAGAGTGTTAGATAAAATATCCATTGG
TCAGCTGGTCAGCCTGCTGTCCAACAATCTTAACAAATTTGATGAAGGCTTGGCGCTGG
CCCACTTCGTGTGGATTGCACCTCTGCAGGTGGCCCTGTTGATGGGACTTATATGGGAG
CTGCTTCAAGCCTCTGCTTTCTGTGGGCTGGGCTTTTTGATTGTACTGGCACTTTTTCAGG
CTGGGCTCGGAAGAATGATGATGAAATACAGAGATCAGCGGGCCGGGAAGATATCAGA
GCGACTTGTGATCACCAGTGAAATGATTGAAAATATTCAGAGCGTGAAAGCCTACTGCT
GGGAAGAAGCCATGGAGAAGATGATTGAGAACCTGAGGCAGACAGAGCTCAAGCTCAC
TCGGAAGGCTGCTTATGTTCGCTATTTCAACAGCAGCGCCTTCTTCTTCAGTGGCTTCTT
TGTTGTCTTCCTGTCTGTTCTGCCATATGCACTGATAAAAGGCATTATTTTACGAAAGAT
CTTCACCACCATCAGTTTTTGCATCGTTCTCAGGATGGCCGTCACAAGACAGTTCCCCTG
GGCTGTGCAGACCTGGTACGATTCCTTGGGGGCCATCAACAAGATTCAAGATTTCTTGC
AAAAACAAGAATATAAAACTTTAGAATACAACCTCACCACCACTGAAGTGGTCATGGA
AAATGTGACAGCCTTTTGGGAGGAGGGTTTTGGAGAATTGTTCGAGAAGGCAAAGCAG
AATAACAACAACAGGAAGACGAGCAATGGGGACGACTCTCTCTTCTTCAGCAACTTTTC
ACTGCTCGGGACCCCTGTGTTGAAAGATATAAACTTCAAGATCGAGAGGGGCCAGCTCT
TGGCTGTGGCAGGCTCCACTGGAGCTGGTAAAACATCTCTTCTCATGGTGATCATGGGG
GAACTGGAGCCTTCCGAAGGAAAAATCAAGCACAGTGGGAGAATCTCATTCTGCAGCC
AGTTTTCCTGGATCATGCCCGGCACCATTAAGGAAAACATCATATTTGGAGTGTCCTAT
GATGAGTACCGCTACCGGTCAGTCATCAAAGCCTGTCAGTTGGAGGAGGACATCTCCAA
GTTTGCAGAGAAAGACAACATTGTGCTTGGAGAGGGGGGTATCACTCTTTCTGGAGGAC
AAAGAGCCAGGATCTCTTTGGCCCGGGCAGTCTACAAGGATGCAGACCTCTACTTGTTG
GACAGTCCCTTCGGCTACCTCGACGTGCTGACTGAAAAAGAAATTTTTGAAAGCTGTGT
GTGCAAACTGATGGCAAACAAGACCAGGATTCTTGTCACCAGCAAGATGGAACATCTG
AAGAAAGCGGACAAAATTCTGATTCTGCATGAAGGGAGCTCCTACTTCTATGGAACATT
TAGCGAGCTTCAGAACCTACAGCCAGACTTCTCCTCCAAATTAATGGGCTGTGACTCCT
TCGACCAGTTCTCTGCAGAAAGAAGAAACTCTATACTCACAGAGACCCTCCACCGCTTC
TCCCTTGAGGGAGATGCCCCAGTTTCTTGGACAGAAACCAAGAAGCAGTCCTTTAAGCA

GACTGGCGAGTTTGGTGAAAAGAGGAAAAATTCAATTCTCAATCCTATTAACAGTATTC
GCAAGTTCAGCATTGTCCAGAAGACACCCCTCCAGATGAATGGCATCGAAGAAGATAG
TGACGAGCCGCTGGAGAGACGGCTGAGTCTGGTGCCAGATTCAGAACAGGGGGAGGCC
ATCCTGCCCCGGATCAGCGTCATTTCCACAGGCCCCACATTACAAGCACGGCGCCGGCA
GAGTGTTTTAAATCTCATGACCCATTCAGTGAACCAGGGCCAAAATATCCACAGGAAGA
CTACAGCTTCTACCCGGAAAGTGTCTCTGGCCCCTCAGGCCAATCTGACCGAGCTGGAC
ATCTACAGCAGGAGGCTCTCCCAGGAAACAGGGCTTGAAATATCTGAAGAGATTAATG
AAGAGGATCTTAAAGAGTGCTTCTTTGATGACATGGAGAGCATCCCCGCGGTGACCACA
TGGAACACCTACCTTAGATATATTACTGTCCACAAGAGCCTCATATTTGTCCTCATCTGG
TGCCTGGTTATTTTCCTCGCTGAGGTGGCGGCCAGTCTTGTTGTGCTCTGGCTGCTGGGC
AACACTCCTCTCCAGGACAAGGGCAATAGTACACACAGCAGAAATAATTCTTATGCCGT
CATCATTACAAGCACCTCCAGCTACTACGTGTTCTACATCTATGTGGGCGTGGCTGACA
CCCTCCTGGCCATGGGTTTCTTCCGGGGCCTGCCTTTGGTGCACACCCTCATCACAGTGT
CAAAAATTCTGCACCATAAAATGCTTCATTCTGTCCTGCAGGCACCCATGAGCACTTTG
AACACATTGAAGGCTGGCGGCATCCTCAACAGATTTTCTAAAGATATTGCTATCCTGGA
TGATCTCCTCCCCCTGACAATCTTTGACTTTATCCAGCTTCTGCTGATCGTGATTGGAGC
CATAGCAGTGGTTGCTGTCCTGCAGCCCTACATTTTTGTGGCCACCGTGCCCGTGATTGT
TGCCTTTATTATGCTCAGAGCTTACTTCCTGCAAACTTCTCAACAGCTCAAACAGCTAGA
ATCTGAGGGCCGGAGCCCCATTTTTACCCACCTGGTGACTTCCCTGAAGGGACTGTGGA
CTCTGAGAGCATTCGGGCGACAGCCTTACTTTGAGACACTGTTCCACAAGGCCCTGAAC
TTGCACACTGCCAACTGGTTTCTTTACCTGAGCACACTCCGCTGGTTCCAGATGCGGATA
GAGATGATCTTCGTCATCTTTTTTATAGCTGTAACCTTCATTTCTATCCTTACAACAGGA
GAAGGAGAGGGCAGGGTGGGAATCATCCTCACGCTGGCTATGAACATAATGTCCACCT
TGCAGTGGGCCGTGAATTCCAGTATAGATGTGGATTCTCTAATGAGGAGTGTCTCCCGG
GTGTTTAAATTCATTGATATGCCTACTGAGGGGAAACCCACCAAGTCAACAAAACCTTA
TAAGAATGGACAGCTGAGCAAGGTGATGATAATTGAGAACAGCCACGTGAAGAAGGAT
GACATTTGGCCCAGCGGGGGCCAGATGACTGTGAAGGACCTGACGGCCAAGTACACCG
AAGGTGGAAATGCCATTTTGGAAAACATCAGCTTCTCAATCTCTCCTGGGCAGAGAGTT
GGATTGCTGGGTCGCACGGGCAGCGGCAAATCAACCCTGCTCAGTGCCTTCCTTCGGCT
CCTGAATACAGAAGGCGAAATCCAAATTGACGGGGTGAGCTGGGACAGCATCACCCTG
CAGCAGTGGAGAAAAGCATTTGGGGTCATTCCACAGAAAGTTTTCATCTTCTCTGGCAC
TTTCAGAAAGAACCTGGACCCCTATGAGCAGTGGAGCGACCAGGAGATCTGGAAGGTT

GCAGATGAAGTTGGCCTGCGGAGTGTGATAGAACAATTTCCTGGCAAGCTGGATTTTGT
GCTGGTAGATGGAGGCTGCGTGCTGTCCCACGGCCACAAACAGCTGATGTGCCTCGCCC
GCTCCGTTCTTTCAAAGGCCAAAATCTTGCTTTTGGATGAGCCCAGTGCTCACCTCGACC
CAGTGACCTATCAGATAATCCGCAGGACCTTAAAGCAAGCTTTTGCCGACTGCACCGTC
ATACTGTGTGAGCACCGGATTGAAGCAATGCTGGAATGCCAGCAGTTTCTGGTGATCGA
GGAGAATAAGGTCCGGCAGTACGACAGCATCCAGAAGTTGTTGAATGAGCGCAGCCTT
TTCCGCCAGGCCATCTCCCCATCTGACAGAGTCAAGCTGTTTCCACATAGGAACTCCTCT
AAGTGCAAGTCCAAGCCCCAGATCGCTGCCCTCAAGGAGGAAACTGAGGAAGAGGTGC
AGGATACCCGCCTGTGA (SEQ ID NO: 15).

In yet another embodiment, an exemplary codon-optimized CFTR mRNA sequence is:

ATGCAGAGGAGCCCACTGGAGAAAGCCTCCGTGGTGAGTAAACTCTTTTTTAGTTGGAC
CAGACCCATCCTGCGAAAAGGATACAGGCAGCGCCTCGAGTTGTCAGATATCTACCAG
ATTCCTTCTGTGGACTCAGCTGACAATTTGAGTGAGAAGCTGGAGCGGGAGTGGGATAG
AGAGCTGGCGAGCAAAAAAAACCCCAAGCTTATCAATGCTCTGCGCCGCTGCTTTTTCT
GGAGGTTCATGTTTTATGGGATCTTCCTGTACCTGGGGGAGGTCACCAAAGCTGTTCAG
CCGCTCCTTCTTGGCCGCATCATCGCCAGCTATGACCCTGATAATAAAGAAGAAAGGTC
TATTGCTATTTATCTGGGAATTGGCCTCTGCTTGCTCTTCATCGTCCGCACCCTTCTGCTG
CACCCTGCCATTTTTGGCCTTCACCACATCGGCATGCAAATGAGAATTGCCATGTTCTCC
CTCATTTACAAAAAGACCCTGAAACTTTCCTCAAGAGTGTTAGATAAAATATCCATTGG
TCAGCTGGTCAGCCTGCTGTCCAACAATCTTAACAAATTTGATGAAGGCTTGGCGCTGG
CCCACTTCGTGTGGATTGCACCTCTGCAGGTGGCCCTGTTGATGGGACTTATATGGGAG
CTGCTTCAAGCCTCTGCTTTCTGTGGGCTGGGCTTTTTGATTGTACTGGCACTTTTTCAGG
CTGGGCTCGGAAGAATGATGATGAAATACAGAGATCAGCGGGCCGGGAAGATATCAGA
GCGACTTGTGATCACCAGTGAAATGATTGAAAATATTCAGAGCGTGAAAGCCTACTGCT
GGGAAGAAGCCATGGAGAAGATGATTGAGAACCTGAGGCAGACAGAGCTCAAGCTCAC
TCGGAAGGCTGCTTATGTTCGCTATTTCAACAGCAGCGCCTTCTTCTTCAGTGGCTTCTT
TGTTGTCTTCCTGTCTGTTCTGCCATATGCACTGATAAAAGGCATTATTTTACGAAAGAT
CTTCACCACCATCAGTTTTTGCATCGTTCTCAGGATGGCCGTCACAAGACAGTTCCCCTG
GGCTGTGCAGACCTGGTACGATTCCTTGGGGGCCATCAACAAGATTCAAGATTTCTTGC
AAAAACAAGAATATAAAACTTTAGAATACAACCTCACCACCACTGAAGTGGTCATGGA
AAATGTGACAGCCTTTTGGGAGGAGGGTTTTGGAGAATTGTTCGAGAAGGCAAAGCAG

AATAACAACAACAGGAAGACGAGCAATGGGGACGACTCTCTCTTCTTCAGCAACTTTTC
ACTGCTCGGGACCCCTGTGTTGAAAGATATAAACTTCAAGATCGAGAGGGGCCAGCTCT
TGGCTGTGGCAGGCTCCACTGGAGCTGGTAAAACATCTCTTCTCATGGTGATCATGGGG
GAACTGGAGCCTTCCGAAGGAAAAATCAAGCACAGTGGGAGAATCTCATTCTGCAGCC
AGTTTTCCTGGATCATGCCCGGCACCATTAAGGAAAACATCATATTTGGAGTGTCCTAT
GATGAGTACCGCTACCGGTCCGTCATCAAAGCCTGTCAGTTGGAGGAGGACATCTCCAA
GTTTGCAGAGAAAGACAACATTGTGCTTGGAGAGGGGGGTATCACTCTTTCTGGAGGAC
AAAGAGCCAGGATCTCTTTGGCCCGGGCAGTCTACAAGGATGCAGACCTCTACTTGTTG
GACAGTCCCTTCGGCTACCTCGACGTGCTGACTGAAAAAGAAATTTTTGAAAGCTGTGT
GTGCAAACTGATGGCAAACAAGACCAGGATTCTTGTCACCAGCAAGATGGAACATCTG
AAGAAAGCGGACAAAATTCTGATTCTGCATGAAGGGAGCTCCTACTTCTATGGAACATT
TAGCGAGCTTCAGAACCTACAGCCAGACTTCTCCTCCAAATTAATGGGCTGTGACTCCT
TCGACCAGTTCTCTGCAGAAAGAAGAAACTCTATACTCACAGAGACCCTCCACCGCTTC
TCCCTTGAGGGAGATGCCCCAGTTTCTTGGACAGAAACCAAGAAGCAGTCCTTTAAGCA
GACTGGCGAGTTTGGTGAAAAGAGGAAAAATTCAATTCTCAATCCAATTAACAGTATTC
GCAAGTTCAGCATTGTCCAGAAGACACCCCTCCAGATGAATGGCATCGAAGAAGATAG
TGACGAGCCGCTGGAGAGACGGCTGAGTCTGGTGCCAGATTCAGAACAGGGGGAGGCC
ATCCTGCCCCGGATCAGCGTCATTTCCACAGGCCCCACATTACAAGCACGGCGCCGGCA
GAGTGTTTTAAATCTCATGACCCATTCAGTGAACCAGGGCCAAAATATCCACAGGAAGA
CTACAGCTTCTACCCGGAAAGTGTCTCTGGCCCCTCAGGCCAATCTGACCGAGCTGGAC
ATCTACAGCAGGAGGCTCTCCCAGGAAACAGGGCTGGAAATATCTGAAGAGATTAATG
AAGAGGATCTTAAAGAGTGCTTCTTTGATGACATGGAGAGCATCCCCGCGGTGACCACA
TGGAACACCTACCTTAGATATATTACTGTCCACAAGAGCCTCATATTTGTCCTCATCTGG
TGCCTGGTTATTTTCCTCGCTGAGGTGGCGGCCAGTCTTGTTGTGCTCTGGCTGCTGGGC
AACACTCCTCTCCAGGACAAGGGCAATAGTACTCACAGCAGAAATAATTCTTATGCCGT
CATCATTACAAGCACCTCCAGCTACTACGTGTTCTACATCTATGTGGGCGTGGCTGACA
CCCTCCTGGCCATGGGTTTCTTCCGGGGCCTGCCTTTGGTGCACACCCTCATCACAGTGT
CAAAAATTCTGCACCATAAAATGCTTCATTCTGTCCTGCAGGCACCCATGAGCACTTTG
AACACATTGAAGGCTGGCGGCATCCTCAACAGATTTTCTAAAGATATTGCTATCCTGGA
TGATCTCCTCCCCCTGACAATCTTTGACTTTATCCAGCTTCTGCTGATCGTGATTGGAGC
CATAGCAGTGGTTGCTGTCCTGCAGCCCTACATTTTTGTGGCCACCGTGCCCGTGATTGT
TGCCTTTATTATGCTCAGAGCTTACTTCCTGCAAACTTCTCAACAGCTCAAACAGCTAGA

GTCTGAGGGCCGGAGCCCCATTTTTACCCACCTGGTGACTTCCCTGAAGGGACTGTGGA
CTCTGAGAGCATTCGGGCGACAGCCTTACTTTGAGACACTGTTCCACAAGGCCCTGAAC
TTGCACACTGCCAACTGGTTTCTTTACCTGAGCACACTCCGCTGGTTCCAGATGCGGATA
GAGATGATCTTCGTCATCTTTTTTATAGCTGTAACCTTCATTTCTATCCTTACAACAGGA
GAAGGAGAGGGCAGGGTGGGAATCATCCTCACGCTGGCTATGAACATAATGTCCACCT
TGCAGTGGGCCGTGAATTCCAGTATAGATGTGGATTCTCTAATGAGGAGTGTCTCCCGG
GTGTTTAAATTCATTGATATGCCTACTGAGGGGAAACCCACCAAGTCAACAAAACCTTA
TAAGAATGGACAGCTGAGCAAGGTGATGATAATTGAGAACAGCCACGTGAAGAAGGAT
GACATTTGGCCCAGCGGGGGCCAGATGACTGTGAAGGACCTGACGGCCAAGTACACCG
AAGGTGGAAATGCCATTTTGGAAAACATCAGCTTCTCAATCTCTCCTGGGCAGAGAGTT
GGATTGCTGGGTCGCACGGGCAGCGGCAAATCAACCCTGCTCAGTGCCTTCCTTCGGCT
CCTGAATACAGAAGGCGAAATCCAAATTGACGGGGTGAGCTGGGACAGCATCACCCTG
CAGCAGTGGAGAAAAGCATTTGGGGTCATTCCACAGAAAGTTTTCATCTTCTCTGGCAC
TTTCAGAAAGAACCTGGACCCCTATGAGCAGTGGAGCGACCAGGAGATCTGGAAGGTT
GCAGATGAAGTTGGCCTGCGGAGTGTGATAGAACAATTTCCTGGCAAGCTGGATTTTGT
GCTGGTAGATGGAGGCTGCGTGCTGTCCCACGGCCACAAACAGCTGATGTGCCTCGCCC
GCTCCGTTCTTTCAAAGGCCAAAATCTTGCTTTTGGATGAGCCCAGTGCTCACCTCGACC
CAGTGACCTATCAGATAATCCGCAGGACCTTAAAGCAAGCTTTTGCCGACTGCACCGTC
ATACTGTGTGAGCACCGGATTGAAGCAATGCTGGAATGCCAGCAGTTTCTGGTGATCGA
GGAGAATAAGGTCCGGCAGTACGACAGCATCCAGAAGTTGTTGAATGAGCGCAGCCTT
TTCCGCCAGGCCATCTCCCCATCTGACAGAGTCAAGCTGTTTCCACATAGGAACTCCTCT
AAGTGCAAGTCCAAGCCCCAGATCGCTGCCCTCAAGGAGGAAACTGAGGAAGAGGTGC
AGGATACCCGCCTGTGA (SEQ ID NO: 16).

In another embodiment, an exemplary codon-optimized CFTR mRNA sequence is:

ATGCAGAGGAGCCCACTGGAGAAAGCCTCCGTGGTGAGTAAACTCTTTTTTAGTTGGAC
CAGACCCATCCTGCGAAAAGGATACAGGCAGCGCCTCGAGTTGTCTGATATCTACCAGA
TTCCTTCTGTGGACTCAGCTGACAATTTGAGTGAGAAGCTGGAGCGGGAGTGGGATAGA
GAGCTGGCGAGCAAAAAAAACCCCAAGCTTATCAATGCTCTGCGCCGCTGCTTTTTCTG
GAGGTTCATGTTTTATGGGATCTTCCTGTACCTGGGGGAGGTCACCAAAGCTGTTCAGC
CGCTCCTTCTTGGCCGCATCATCGCCAGCTATGACCCTGATAATAAAGAAGAAAGGTCT
ATTGCTATTTATCTGGGAATTGGCCTCTGCTTGCTCTTCATCGTCCGCACCCTTCTGCTGC
ACCCTGCCATTTTTGGCCTTCACCACATCGGCATGCAAATGAGAATTGCCATGTTCTCCC

TCATTTACAAAAAGACCCTGAAACTTTCCTCAAGAGTGTTAGATAAAATATCCATTGGT
CAGCTGGTCAGCCTGCTGTCCAACAATCTTAACAAATTTGATGAAGGCTTGGCGCTGGC
CCACTTCGTGTGGATTGCACCTCTGCAGGTGGCCCTGTTGATGGGACTTATATGGGAGC
TGCTTCAAGCCTCTGCTTTCTGTGGGCTGGGCTTTTTGATTGTACTGGCACTTTTTCAGGC
TGGGCTCGGAAGAATGATGATGAAATACAGAGATCAGCGGGCCGGGAAGATTTCAGAG
CGACTTGTGATCACCAGTGAAATGATTGAAAATATTCAGAGCGTGAAAGCCTACTGCTG
GGAAGAAGCCATGGAGAAGATGATTGAGAACCTGAGGCAGACAGAGCTCAAGCTCACT
CGGAAGGCTGCTTATGTTCGCTATTTCAACAGCAGCGCCTTCTTCTTCAGTGGCTTCTTT
GTTGTCTTCCTGTCTGTTCTGCCATATGCACTGATAAAAGGCATTATTTTACGAAAGATC
TTCACCACCATCAGTTTTTGCATCGTTCTCAGGATGGCCGTCACAAGACAGTTCCCCTGG
GCTGTGCAGACCTGGTACGATTCCTTGGGGGCCATCAACAAGATTCAAGATTTCTTGCA
AAAACAAGAATATAAAACTTTAGAATACAACCTCACCACCACTGAAGTGGTCATGGAA
AATGTGACAGCCTTTTGGGAGGAGGGTTTTGGAGAATTGTTCGAGAAGGCAAAGCAGA
ATAACAACAACAGGAAGACGAGCAATGGGGACGACTCTCTCTTCTTCAGCAACTTTTCA
CTGCTCGGGACCCCTGTGTTGAAAGATATAAACTTCAAGATCGAGAGGGGCCAGCTCTT
GGCTGTGGCAGGCTCCACTGGAGCTGGTAAAACATCTCTTCTCATGGTGATCATGGGGG
AACTGGAGCCTTCCGAAGGAAAAATCAAGCACAGTGGGAGAATCTCATTCTGCAGCCA
GTTTTCCTGGATCATGCCCGGCACCATTAAGGAAAACATCATATTTGGAGTGTCCTATG
ATGAGTACCGCTACCGGTCAGTCATCAAAGCCTGTCAGTTGGAGGAGGACATCTCCAAG
TTTGCAGAGAAAGACAACATTGTGCTTGGAGAGGGGGGTATCACTCTTTCTGGAGGACA
AAGAGCCAGGATCTCTTTGGCCCGGGCAGTCTACAAGGATGCAGACCTCTACTTGTTGG
ACAGTCCCTTCGGCTACCTCGACGTGCTGACTGAAAAAGAAATTTTTGAAAGCTGTGTG
TGCAAACTGATGGCAAACAAGACCAGGATTCTTGTCACCAGCAAGATGGAACATCTGA
AGAAAGCGGACAAAATTCTGATTCTGCATGAAGGGAGCTCCTACTTCTATGGAACATTT
AGCGAGCTTCAGAACCTACAGCCAGACTTCTCCTCCAAATTAATGGGCTGTGACTCCTT
CGACCAGTTCTCTGCAGAAAGAAGAAACTCTATACTCACAGAGACCCTCCACCGCTTCT
CCCTTGAGGGAGATGCCCCAGTTTCTTGGACAGAAACCAAGAAGCAGTCCTTTAAGCAG
ACTGGCGAGTTTGGTGAAAAGAGGAAAAATTCAATTCTCAATCCTATTAACAGTATTCG
CAAGTTCAGCATTGTCCAGAAGACACCCCTCCAGATGAATGGCATCGAAGAAGATAGT
GACGAGCCGCTGGAGAGACGGCTGAGTCTGGTGCCAGATTCAGAACAGGGGGAGGCCA
TCCTGCCCCGGATCAGCGTCATTTCCACAGGCCCCACATTACAAGCACGGCGCCGGCAG
AGTGTTTTAAATCTCATGACCCATTCAGTGAACCAGGGCCAAAATATCCACAGGAAGAC

TACAGCTTCTACCCGGAAAGTGTCTCTGGCCCCTCAGGCCAATCTGACCGAGCTGGACA
TCTACAGCAGGAGGCTCTCCCAGGAAACAGGGCTGGAAATATCTGAAGAGATTAATGA
AGAGGATCTTAAAGAGTGCTTCTTTGATGACATGGAGAGCATCCCCGCGGTGACCACAT
GGAACACCTACCTTAGATATATTACTGTCCACAAGAGCCTCATATTTGTCCTCATCTGGT
GCCTGGTTATTTTCCTCGCTGAGGTGGCGGCCAGTCTTGTTGTGCTCTGGCTGCTGGGCA
ACACTCCTCTCCAGGACAAGGGCAATAGTACACACAGCAGAAATAATTCTTATGCCGTC
ATCATTACAAGCACCTCCAGCTACTACGTGTTCTACATCTATGTGGGCGTGGCTGACAC
CCTCCTGGCCATGGGTTTCTTCCGGGGCCTGCCTTTGGTGCACACCCTCATCACAGTGTC
AAAAATTCTGCACCATAAAATGCTTCATTCTGTCCTGCAGGCACCCATGAGCACTTTGA
ACACATTGAAGGCTGGCGGCATCCTCAACAGATTTTCTAAAGATATTGCTATCCTGGAT
GATCTCCTCCCCCTGACAATCTTTGACTTTATCCAGCTTCTGCTGATCGTGATTGGAGCC
ATAGCAGTGGTTGCTGTCCTGCAGCCCTACATTTTTGTGGCCACCGTGCCCGTGATTGTT
GCCTTTATTATGCTCAGAGCTTACTTCCTGCAAACTTCTCAACAGCTCAAACAGCTAGAA
TCTGAGGGCCGGAGCCCCATTTTTACCCACCTGGTGACTTCCCTGAAGGGACTGTGGAC
TCTGAGAGCATTCGGGCGACAGCCTTACTTTGAGACACTGTTCCACAAGGCCCTGAACT
TGCACACTGCCAACTGGTTTCTTTACCTGAGCACACTCCGCTGGTTCCAGATGCGGATA
GAGATGATCTTCGTCATCTTTTTTATAGCTGTAACCTTCATTTCTATCCTTACAACAGGA
GAAGGAGAGGGCAGGGTGGGAATCATCCTCACGCTGGCTATGAACATAATGTCCACCT
TGCAGTGGGCCGTGAATTCCAGTATAGATGTGGATTCTCTAATGAGGAGTGTCTCCCGG
GTGTTTAAATTCATTGATATGCCTACTGAGGGGAAACCCACCAAGTCAACAAAACCTTA
TAAGAATGGACAGCTGAGCAAGGTGATGATAATTGAGAACAGCCACGTGAAGAAGGAT
GACATTTGGCCCAGCGGGGGCCAGATGACTGTGAAGGACCTGACGGCCAAGTACACCG
AAGGTGGAAATGCCATTTTGGAAAACATCAGCTTCTCAATCTCTCCTGGGCAGAGAGTT
GGATTGCTGGGTCGCACGGGCAGCGGCAAATCAACCCTGCTCAGTGCCTTCCTTCGGCT
CCTGAATACAGAAGGCGAAATCCAAATTGACGGGGTGAGCTGGGACAGCATCACCCTG
CAGCAGTGGAGAAAAGCATTTGGGGTCATTCCACAGAAAGTTTTCATCTTCTCTGGCAC
TTTCAGAAAGAACCTGGACCCCTATGAGCAGTGGAGCGACCAGGAGATCTGGAAGGTT
GCAGATGAAGTTGGCCTGCGGAGTGTGATAGAACAATTTCCTGGCAAGCTGGATTTTGT
GCTGGTAGATGGAGGCTGCGTGCTGTCCCACGGCCACAAACAGCTGATGTGCCTCGCCC
GCTCCGTTCTTTCAAAGGCCAAAATCTTGCTTTTGGATGAGCCCAGTGCTCACCTCGACC
CAGTGACCTATCAGATAATCCGCAGGACCTTAAAGCAAGCTTTTGCCGACTGCACCGTC
ATACTGTGTGAGCACCGGATTGAAGCAATGCTGGAATGCCAGCAGTTTCTGGTGATCGA

GGAGAATAAGGTCCGGCAGTACGACAGCATCCAGAAGTTGTTGAATGAGCGCAGCCTT
TTCCGCCAGGCCATCTCCCCATCTGACAGAGTCAAGCTGTTTCCACATAGGAACTCCTCT
AAGTGCAAGTCCAAGCCCCAGATCGCTGCCCTCAAGGAGGAAACTGAGGAAGAGGTGC
AGGATACCCGCCTGTGA (SEQ ID NO: 17).

In another embodiment, an exemplary codon-optimized CFTR mRNA sequence is:

ATGCAGAGAAGCCCCCTGGAGAAGGCCTCTGTGGTGAGCAAGCTGTTCTTCAGCTGGAC
CAGACCCATCCTGAGAAAGGGCTACAGACAGAGACTGGAGCTGTCTGACATCTACCAG
ATCCCCTCTGTGGACTCTGCCGACAACCTGTCTGAGAAGCTGGAGAGAGAGTGGGACA
GAGAGCTGGCCAGCAAGAAGAACCCCAAGCTGATCAATGCCCTGAGAAGATGCTTCTT
CTGGAGATTCATGTTCTATGGCATCTTCCTGTACCTGGGAGAGGTGACCAAGGCCGTGC
AGCCCCTGCTGCTGGGCAGGATCATTGCCAGCTATGACCCTGACAACAAGGAGGAGAG
AAGCATTGCCATCTACCTGGGCATTGGCCTGTGCCTGCTGTTCATTGTGAGAACCCTGCT
GCTGCACCCTGCCATCTTTGGCCTGCACCACATTGGCATGCAGATGAGAATTGCCATGT
TCAGCCTGATCTACAAGAAGACCCTGAAGCTGAGCAGCAGAGTGCTGGACAAGATCAG
CATTGGCCAGCTGGTGAGCCTGCTGAGCAACAACCTGAACAAGTTTGATGAGGGCCTGG
CCCTGGCCCACTTTGTGTGGATTGCCCCCCTGCAGGTGGCCCTGCTGATGGGCCTGATCT
GGGAGCTGCTGCAGGCCTCTGCCTTCTGTGGCCTGGGCTTCCTGATTGTGCTGGCCCTGT
TCCAGGCCGGCCTGGGCAGAATGATGATGAAGTACAGAGACCAGAGAGCCGGCAAGAT
CTCTGAGAGACTGGTGATCACCTCTGAGATGATTGAGAACATCCAGTCTGTGAAGGCCT
ACTGCTGGGAGGAGGCCATGGAGAAGATGATTGAGAACCTGAGACAGACAGAGCTGAA
GCTGACCAGGAAGGCCGCCTATGTGAGATACTTCAACAGCTCTGCCTTCTTCTTCTCTGG
CTTCTTTGTGGTGTTCCTGTCTGTGCTGCCCTATGCCCTGATCAAGGGCATCATCCTGAG
GAAGATCTTCACCACCATCAGCTTCTGCATTGTGCTGAGGATGGCCGTGACCAGGCAGT
TCCCCTGGGCCGTGCAGACCTGGTATGACAGCCTGGGGGCCATCAACAAGATCCAGGA
CTTCCTGCAGAAGCAGGAGTACAAGACCCTGGAGTACAACCTGACCACCACAGAGGTG
GTGATGGAGAATGTGACAGCCTTCTGGGAGGAGGGCTTTGGAGAGCTGTTTGAGAAGG
CCAAGCAGAACAACAACAACAGAAAGACCAGCAATGGAGATGACAGCCTGTTCTTCAG
CAACTTCAGCCTGCTGGGCACCCCTGTGCTGAAGGACATCAACTTCAAGATTGAGAGGG
GCCAGCTGCTGGCCGTGGCCGGCAGCACAGGAGCCGGCAAGACCAGCCTGCTGATGGT
GATCATGGGAGAGCTGGAGCCCTCTGAGGGCAAGATCAAGCACTCTGGCAGAATCAGC
TTCTGCAGCCAGTTCAGCTGGATCATGCCTGGCACCATCAAGGAGAACATCATCTTTGG
GGTGAGCTATGATGAGTACAGGTACAGATCTGTGATCAAGGCCTGCCAGCTGGAGGAG

GACATCTCCAAGTTTGCCGAGAAGGACAACATTGTGCTGGGGGAGGGAGGCATCACCC
TGTCTGGGGGCCAGAGAGCCAGAATCAGCCTGGCCAGAGCCGTGTACAAGGATGCCGA
CCTGTACCTGCTGGACAGCCCCTTTGGCTACCTGGATGTGCTGACAGAGAAGGAGATCT
TTGAGAGCTGTGTGTGCAAGCTGATGGCCAACAAGACCAGGATCCTGGTGACCAGCAA
GATGGAGCACCTGAAGAAGGCCGACAAGATCCTGATCCTGCATGAGGGCAGCAGCTAC
TTCTATGGCACCTTCTCTGAGCTGCAGAACCTGCAGCCTGACTTCAGCAGCAAGCTGAT
GGGCTGTGACAGCTTTGACCAGTTCTCTGCTGAGAGAAGAAACAGCATCCTGACAGAG
ACCCTGCACAGGTTCAGCCTGGAGGGGGATGCCCCTGTGAGCTGGACAGAGACCAAGA
AGCAGAGCTTCAAGCAGACAGGAGAGTTTGGGGAGAAGAGGAAGAACAGCATCCTGA
ACCCCATCAACAGCATCAGGAAGTTCAGCATTGTGCAGAAGACCCCCCTGCAGATGAA
TGGCATTGAGGAGGACTCTGATGAGCCCCTGGAGAGAAGACTGAGCCTGGTGCCAGAC
TCTGAGCAGGGAGAGGCCATCCTGCCCAGGATCTCTGTGATCAGCACAGGCCCCACCCT
GCAGGCCAGAAGAAGACAGTCTGTGCTGAACCTGATGACCCACTCTGTGAACCAGGGC
CAGAATATCCACAGAAAGACCACAGCCAGCACCAGAAAGGTGAGCCTGGCCCCCCAGG
CCAACCTGACAGAGCTGGACATCTACAGCAGAAGGCTGAGCCAGGAGACAGGCCTGGA
GATCTCTGAGGAGATCAATGAGGAGGACCTGAAGGAGTGCTTCTTTGATGACATGGAG
AGCATCCCTGCCGTGACCACCTGGAACACCTACCTGAGATACATCACAGTGCACAAGA
GCCTGATCTTTGTGCTGATCTGGTGCCTGGTGATCTTCCTGGCCGAGGTGGCCGCCAGCC
TGGTGGTGCTGTGGCTGCTGGGCAACACCCCCCTGCAGGACAAGGGCAACAGCACCCA
CAGCAGAAACAACAGCTATGCTGTGATCATCACCAGCACCAGCAGCTACTATGTGTTCT
ACATCTATGTGGGAGTGGCTGACACCCTGCTGGCCATGGGCTTCTTCAGAGGCCTGCCC
CTGGTGCACACCCTGATCACAGTGAGCAAGATCCTGCACCACAAGATGCTGCACTCTGT
GCTGCAGGCCCCCATGAGCACCCTGAACACCCTGAAGGCTGGAGGCATCCTGAACAGA
TTCAGCAAGGACATTGCCATCCTGGATGACCTGCTGCCCCTGACCATCTTTGACTTCATC
CAGCTGCTGCTGATTGTGATTGGAGCCATTGCCGTGGTGGCCGTGCTGCAGCCCTACAT
CTTTGTGGCCACAGTGCCTGTGATTGTGGCCTTCATCATGCTGAGGGCCTACTTCCTGCA
GACCAGCCAGCAGCTGAAGCAGCTGGAGTCTGAGGGCAGAAGCCCCATCTTCACCCAC
CTGGTGACCAGCCTGAAGGGCCTGTGGACCCTGAGGGCCTTTGGCAGACAGCCCTACTT
TGAGACCCTGTTCCACAAGGCCCTGAACCTGCACACAGCCAACTGGTTCCTGTACCTGA
GCACCCTGAGATGGTTCCAGATGAGGATTGAGATGATCTTTGTGATCTTCTTCATTGCCG
TGACCTTCATCAGCATCCTGACCACAGGGGAGGGCGAGGGCAGAGTGGGCATCATCCT
GACCCTGGCCATGAACATCATGAGCACCCTGCAGTGGGCCGTGAACAGCAGCATTGAT

GTGGACAGCCTGATGAGATCTGTGAGCAGAGTGTTCAAGTTCATTGACATGCCCACAGA
GGGCAAGCCCACCAAGAGCACCAAGCCCTACAAGAATGGCCAGCTGAGCAAGGTGATG
ATCATTGAGAACAGCCATGTGAAGAAGGATGACATCTGGCCCTCTGGAGGCCAGATGA
CAGTGAAGGACCTGACAGCCAAGTACACAGAGGGGGGCAATGCCATCCTGGAGAACAT
CAGCTTCAGCATCAGCCCTGGCCAGAGGGTGGGCCTGCTGGGCAGAACAGGCTCTGGC
AAGAGCACCCTGCTGTCTGCCTTCCTGAGGCTGCTGAACACAGAGGGAGAGATCCAGA
TTGATGGGGTGAGCTGGGACAGCATCACCCTGCAGCAGTGGAGGAAGGCCTTTGGGGT
GATCCCCCAGAAGGTGTTCATCTTCTCTGGCACCTTCAGGAAGAACCTGGACCCCTATG
AGCAGTGGTCTGACCAGGAGATCTGGAAGGTGGCCGATGAGGTGGGCCTGAGATCTGT
GATTGAGCAGTTCCCTGGCAAGCTGGACTTTGTGCTGGTGGATGGAGGCTGTGTGCTGA
GCCATGGCCACAAGCAGCTGATGTGCCTGGCCAGATCTGTGCTGAGCAAGGCCAAGAT
CCTGCTGCTGGATGAGCCCTCTGCCCACCTGGACCCTGTGACCTACCAGATCATCAGAA
GAACCCTGAAGCAGGCCTTTGCCGACTGCACAGTGATCCTGTGTGAGCACAGAATTGAG
GCCATGCTGGAGTGCCAGCAGTTCCTGGTGATTGAGGAGAACAAGGTGAGGCAGTATG
ACAGCATCCAGAAGCTGCTGAATGAGAGAAGCCTGTTCAGACAGGCCATCAGCCCCTC
TGACAGAGTGAAGCTGTTCCCCCACAGGAACAGCAGCAAGTGCAAGAGCAAGCCCCAG
ATTGCCGCCCTGAAGGAGGAGACAGAGGAGGAGGTGCAGGACACCAGACTGTGA (SEQ
ID NO: 18).

In yet another embodiment, an exemplary codon-optimized CFTR mRNA sequence is:

ATGCAGAGGAGCCCCCTGGAGAAGGCCAGCGTGGTGAGCAAGCTGTTCTTCAGCTGGA
CCAGGCCCATCCTGAGGAAGGGCTACAGGCAGAGGCTGGAGCTGAGCGACATCTACCA
GATCCCCAGCGTGGACAGCGCCGACAACCTGAGCGAGAAGCTGGAGAGGGAGTGGGA
CAGGGAGCTGGCCAGCAAGAAGAACCCCAAGCTGATCAACGCCCTGAGGAGGTGCTTC
TTCTGGAGGTTCATGTTCTACGGCATCTTCCTGTACCTGGGCGAGGTGACCAAGGCCGT
GCAGCCCCTGCTGCTGGGCAGGATCATCGCCAGCTACGACCCCGACAACAAGGAGGAG
AGGAGCATCGCCATCTACCTGGGCATCGGCCTGTGCCTGCTGTTCATCGTGAGGACCCT
GCTGCTGCACCCCGCCATCTTCGGCCTGCACCACATCGGCATGCAGATGAGGATCGCCA
TGTTCAGCCTGATCTACAAGAAGACCCTGAAGCTGAGCAGCAGGGTGCTGGACAAGAT
CAGCATCGGCCAGCTGGTGAGCCTGCTGAGCAACAACCTGAACAAGTTCGACGAGGGC
CTGGCCCTGGCCCACTTCGTGTGGATCGCCCCCCTGCAGGTGGCCCTGCTGATGGGCCT
GATCTGGGAGCTGCTGCAGGCCAGCGCCTTCTGCGGCCTGGGCTTCCTGATCGTGCTGG

CCCTGTTCCAGGCCGGCCTGGGCAGGATGATGATGAAGTACAGGGACCAGAGGGCCGG
CAAGATCAGCGAGAGGCTGGTGATCACCAGCGAGATGATCGAGAACATCCAGAGCGTG
AAGGCCTACTGCTGGGAGGAGGCCATGGAGAAGATGATCGAGAACCTGAGGCAGACCG
AGCTGAAGCTGACCAGGAAGGCCGCCTACGTGAGGTACTTCAACAGCAGCGCCTTCTTC
TTCAGCGGCTTCTTCGTGGTGTTCCTGAGCGTGCTGCCCTACGCCCTGATCAAGGGCATC
ATCCTGAGGAAGATCTTCACCACCATCAGCTTCTGCATCGTGCTGAGGATGGCCGTGAC
CAGGCAGTTCCCCTGGGCCGTGCAGACCTGGTACGACAGCCTGGGCGCCATCAACAAG
ATCCAGGACTTCCTGCAGAAGCAGGAGTACAAGACCCTGGAGTACAACCTGACCACCA
CCGAGGTGGTGATGGAGAACGTGACCGCCTTCTGGGAGGAGGGCTTCGGCGAGCTGTT
CGAGAAGGCCAAGCAGAACAACAACAACAGGAAGACCAGCAACGGCGACGACAGCCT
GTTCTTCAGCAACTTCAGCCTGCTGGGCACCCCCGTGCTGAAGGACATCAACTTCAAGA
TCGAGAGGGGCCAGCTGCTGGCCGTGGCCGGCAGCACCGGCGCCGGCAAGACCAGCCT
GCTGATGGTGATCATGGGCGAGCTGGAGCCCAGCGAGGGCAAGATCAAGCACAGCGGC
AGGATCAGCTTCTGCAGCCAGTTCAGCTGGATCATGCCCGGCACCATCAAGGAGAACAT
CATCTTCGGCGTGAGCTACGACGAGTACAGGTACAGGAGCGTGATCAAGGCCTGCCAG
CTGGAGGAGGACATCAGCAAGTTCGCCGAGAAGGACAACATCGTGCTGGGCGAGGGCG
GCATCACCCTGAGCGGCGGCCAGAGGGCCAGGATCAGCCTGGCCAGGGCCGTGTACAA
GGACGCCGACCTGTACCTGCTGGACAGCCCCTTCGGCTACCTGGACGTGCTGACCGAGA
AGGAGATCTTCGAGAGCTGCGTGTGCAAGCTGATGGCCAACAAGACCAGGATCCTGGT
GACCAGCAAGATGGAGCACCTGAAGAAGGCCGACAAGATCCTGATCCTGCACGAGGGC
AGCAGCTACTTCTACGGCACCTTCAGCGAGCTGCAGAACCTGCAGCCCGACTTCAGCAG
CAAGCTGATGGGCTGCGACAGCTTCGACCAGTTCAGCGCCGAGAGGAGGAACAGCATC
CTGACCGAGACCCTGCACAGGTTCAGCCTGGAGGGCGACGCCCCCGTGAGCTGGACCG
AGACCAAGAAGCAGAGCTTCAAGCAGACCGGCGAGTTCGGCGAGAAGAGGAAGAACA
GCATCCTGAACCCCATCAACAGCATCAGGAAGTTCAGCATCGTGCAGAAGACCCCCCTG
CAGATGAACGGCATCGAGGAGGACAGCGACGAGCCCCTGGAGAGGAGGCTGAGCCTG
GTGCCCGACAGCGAGCAGGGCGAGGCCATCCTGCCCAGGATCAGCGTGATCAGCACCG
GCCCCACCCTGCAGGCCAGGAGGAGGCAGAGCGTGCTGAACCTGATGACCCACAGCGT
GAACCAGGGCCAGAACATCCACAGGAAGACCACCGCCAGCACCAGGAAGGTGAGCCT
GGCCCCCCAGGCCAACCTGACCGAGCTGGACATCTACAGCAGGAGGCTGAGCCAGGAG
ACCGGCCTGGAGATCAGCGAGGAGATCAACGAGGAGGACCTGAAGGAGTGCTTCTTCG
ACGACATGGAGAGCATCCCCGCCGTGACCACCTGGAACACCTACCTGAGGTACATCAC

CGTGCACAAGAGCCTGATCTTCGTGCTGATCTGGTGCCTGGTGATCTTCCTGGCCGAGG
TGGCCGCCAGCCTGGTGGTGCTGTGGCTGCTGGGCAACACCCCCCTGCAGGACAAGGG
CAACAGCACCCACAGCAGGAACAACAGCTACGCCGTGATCATCACCAGCACCAGCAGC
TACTACGTGTTCTACATCTACGTGGGCGTGGCCGACACCCTGCTGGCCATGGGCTTCTTC
AGGGGCCTGCCCCTGGTGCACACCCTGATCACCGTGAGCAAGATCCTGCACCACAAGAT
GCTGCACAGCGTGCTGCAGGCCCCCATGAGCACCCTGAACACCCTGAAGGCCGGCGGC
ATCCTGAACAGGTTCAGCAAGGACATCGCCATCCTGGACGACCTGCTGCCCCTGACCAT
CTTCGACTTCATCCAGCTGCTGCTGATCGTGATCGGCGCCATCGCCGTGGTGGCCGTGCT
GCAGCCCTACATCTTCGTGGCCACCGTGCCCGTGATCGTGGCCTTCATCATGCTGAGGG
CCTACTTCCTGCAGACCAGCCAGCAGCTGAAGCAGCTGGAGAGCGAGGGCAGGAGCCC
CATCTTCACCCACCTGGTGACCAGCCTGAAGGGCCTGTGGACCCTGAGGGCCTTCGGCA
GGCAGCCCTACTTCGAGACCCTGTTCCACAAGGCCCTGAACCTGCACACCGCCAACTGG
TTCCTGTACCTGAGCACCCTGAGGTGGTTCCAGATGAGGATCGAGATGATCTTCGTGAT
CTTCTTCATCGCCGTGACCTTCATCAGCATCCTGACCACCGGCGAGGGCGAGGGCAGGG
TGGGCATCATCCTGACCCTGGCCATGAACATCATGAGCACCCTGCAGTGGGCCGTGAAC
AGCAGCATCGACGTGGACAGCCTGATGAGGAGCGTGAGCAGGGTGTTCAAGTTCATCG
ACATGCCCACCGAGGGCAAGCCCACCAAGAGCACCAAGCCCTACAAGAACGGCCAGCT
GAGCAAGGTGATGATCATCGAGAACAGCCACGTGAAGAAGGACGACATCTGGCCCAGC
GGCGGCCAGATGACCGTGAAGGACCTGACCGCCAAGTACACCGAGGGCGGCAACGCCA
TCCTGGAGAACATCAGCTTCAGCATCAGCCCCGGCCAGAGGGTGGGCCTGCTGGGCAG
GACCGGCAGCGGCAAGAGCACCCTGCTGAGCGCCTTCCTGAGGCTGCTGAACACCGAG
GGCGAGATCCAGATCGACGGCGTGAGCTGGGACAGCATCACCCTGCAGCAGTGGAGGA
AGGCCTTCGGCGTGATCCCCCAGAAGGTGTTCATCTTCAGCGGCACCTTCAGGAAGAAC
CTGGACCCCTACGAGCAGTGGAGCGACCAGGAGATCTGGAAGGTGGCCGACGAGGTGG
GCCTGAGGAGCGTGATCGAGCAGTTCCCCGGCAAGCTGGACTTCGTGCTGGTGGACGG
CGGCTGCGTGCTGAGCCACGGCCACAAGCAGCTGATGTGCCTGGCCAGGAGCGTGCTG
AGCAAGGCCAAGATCCTGCTGCTGGACGAGCCCAGCGCCCACCTGGACCCCGTGACCT
ACCAGATCATCAGGAGGACCCTGAAGCAGGCCTTCGCCGACTGCACCGTGATCCTGTGC
GAGCACAGGATCGAGGCCATGCTGGAGTGCCAGCAGTTCCTGGTGATCGAGGAGAACA
AGGTGAGGCAGTACGACAGCATCCAGAAGCTGCTGAACGAGAGGAGCCTGTTCAGGCA
GGCCATCAGCCCCAGCGACAGGGTGAAGCTGTTCCCCCACAGGAACAGCAGCAAGTGC

AAGAGCAAGCCCCAGATCGCCGCCCTGAAGGAGGAGACCGAGGAGGAGGTGCAGGAC
ACCAGGCTGTGA (SEQ ID NO: 19).

In another embodiment, an exemplary codon-optimized CFTR mRNA sequence is:

ATGCAGAGATCCCCTCTGGAGAAGGCCTCAGTGGTGTCCAAGCTTTTCTTCTCCTGGAC
CAGGCCCATTTTAAGAAAGGGCTACAGGCAGAGACTTGAGCTGTCTGACATCTATCAGA
TCCCTTCTGTGGATTCTGCTGACAATCTTAGTGAAAAATTGGAAAGGGAGTGGGACAGA
GAGCTGGCAAGTAAAAAGAACCCCAAGCTGATTAATGCCCTGAGGCGCTGCTTTTTTTG
GAGATTCATGTTCTATGGCATATTCCTCTACCTTGGAGAAGTAACCAAAGCTGTACAGC
CTCTCCTCCTTGGCAGAATCATTGCCTCCTATGATCCTGATAACAAGGAGGAGAGAAGC
ATAGCCATCTACCTGGGCATTGGGCTGTGCCTCTTGTTTATTGTGAGGACCCTTCTCTTG
CACCCTGCCATCTTTGGCCTTCATCACATTGGCATGCAAATGAGAATAGCAATGTTTAGT
CTTATTTACAAAAAAACATTAAAACTCTCTTCCAGGGTGTTGGACAAGATCAGTATTGG
ACAACTGGTCAGCCTGCTGAGCAACAACCTGAACAAGTTTGATGAAGGACTGGCCCTG
GCCCACTTTGTCTGGATTGCCCCCCTTCAGGTGGCTCTTTTGATGGGCCTGATCTGGGAA
CTCCTGCAGGCCTCTGCCTTCTGTGGGTTAGGCTTCCTGATAGTGCTAGCTCTCTTTCAG
GCAGGGTTGGGTAGAATGATGATGAAGTACAGAGACCAGAGGGCTGGGAAGATATCTG
AGAGGCTGGTCATTACTTCTGAAATGATAGAAAACATCCAGTCTGTTAAAGCTTACTGC
TGGGAGGAGGCTATGGAAAAGATGATTGAGAACTTGAGGCAAACAGAGCTCAAGCTGA
CTAGGAAGGCAGCCTATGTCAGGTATTTCAACAGCAGTGCTTTCTTCTTCTCAGGCTTTT
TCGTGGTCTTCTTGAGTGTTCTGCCCTATGCCCTCATCAAGGGGATAATTTTGAGAAAGA
TTTTCACCACTATTTCCTTTTGCATTGTCCTGAGGATGGCTGTCACCAGGCAATTCCCCT
GGGCTGTGCAGACATGGTATGACTCTCTGGGGGCCATCAACAAAATCCAAGATTTCCTG
CAGAAGCAGGAGTACAAGACCCTGGAATACAACCTCACCACCACAGAAGTTGTGATGG
AGAATGTGACTGCATTCTGGGAGGAAGGATTTGGGGAGCTGTTTGAGAAAGCAAAACA
AAACAATAATAACAGGAAAACCAGCAATGGAGATGACTCCCTGTTCTTTTCCAACTTCT
CTTTGTTGGGCACCCCTGTCCTGAAAGATATAAACTTTAAAATTGAAAGAGGGCAGCTG
TTGGCAGTTGCTGGCTCCACAGGAGCTGGAAAAACTTCACTACTGATGGTGATCATGGG
GGAGTTAGAACCCTCTGAAGGGAAAATAAAACATTCTGGGAGGATTAGTTTCTGCAGCC
AGTTCAGCTGGATCATGCCTGGGACCATTAAAGAAAATATTATATTTGGAGTGAGCTAT
GATGAATATAGATATAGGAGTGTCATCAAAGCCTGTCAGTTGGAGGAAGACATCAGCA
AATTTGCAGAGAAAGACAACATTGTTCTGGGTGAAGGTGGCATCACCCTGTCAGGAGG
GCAAAGGGCCAGGATCAGCTTGGCCAGAGCAGTCTATAAAGATGCTGATCTGTACCTCC

TGGATAGCCCTTTTGGCTATCTGGATGTTTTGACAGAGAAGGAAATTTTTGAGTCCTGTG
TCTGCAAGTTAATGGCAAATAAAACAAGGATACTTGTGACCTCAAAAATGGAACACCT
GAAGAAGGCTGACAAAATTCTGATCCTGCATGAGGGCAGCAGCTACTTTTATGGAACAT
TTTCTGAACTGCAGAATTTGCAACCAGACTTTTCATCAAAGCTCATGGGATGTGACAGT
TTTGATCAGTTTTCTGCAGAAAGGAGAAACTCCATTTTGACTGAGACCCTGCACAGGTT
CAGTCTGGAGGGGGATGCCCCAGTGAGTTGGACTGAGACAAAGAAACAGAGCTTCAAG
CAGACTGGAGAGTTTGGAGAAAAGAGGAAAAACTCAATTCTCAATCCCATCAATAGCA
TCAGGAAGTTCAGCATAGTTCAGAAGACTCCTTTGCAGATGAATGGGATTGAAGAGGA
CTCAGATGAGCCCCTGGAAAGGAGACTCTCCTTGGTGCCAGATTCAGAGCAGGGGGAA
GCCATACTGCCAAGGATCTCTGTGATTTCTACAGGGCCCACCCTCCAAGCAAGAAGGAG
ACAGTCAGTTTTAAACCTGATGACCCACTCTGTCAACCAGGGACAGAACATTCATAGAA
AGACAACAGCATCTACAAGAAAAGTTTCACTGGCCCCTCAAGCCAATTTAACTGAACTA
GATATCTACAGCAGGAGGCTCAGCCAAGAAACAGGCCTGGAGATCTCAGAAGAAATAA
ATGAGGAGGATTTGAAGGAATGCTTCTTTGATGATATGGAGAGCATCCCAGCTGTCACA
ACCTGGAACACCTACCTGAGATACATCACAGTGCACAAATCCCTCATCTTTGTACTTAT
ATGGTGCCTTGTCATCTTCTTAGCTGAGGTGGCTGCTTCCCTGGTGGTGCTGTGGCTGCT
GGGAAACACACCCCTCCAGGATAAAGGGAACTCTACTCACAGCAGGAACAACAGTTAT
GCTGTGATCATCACCAGTACCTCCTCCTACTATGTGTTCTACATTTATGTTGGAGTTGCA
GACACATTGCTTGCCATGGGTTTTTTTAGAGGACTCCCCCTGGTGCATACTCTCATCACT
GTTTCCAAAATCCTTCACCACAAGATGCTGCACAGTGTACTACAGGCTCCCATGAGCAC
CCTCAACACTCTTAAAGCAGGAGGAATCTTGAACAGATTTAGCAAGGACATTGCAATTC
TTGATGACCTGCTTCCACTGACCATCTTTGACTTCATCCAGCTTCTGCTCATTGTAATTG
GTGCCATTGCTGTGGTAGCAGTGCTCCAGCCATATATTTTTGTGGCCACTGTGCCTGTTA
TTGTGGCCTTCATTATGTTGAGAGCCTACTTCCTGCAGACCTCTCAGCAGCTCAAGCAAC
TTGAAAGTGAGGGCAGGAGCCCCATATTTACACACTTGGTCACTTCCCTCAAAGGCCTC
TGGACACTCAGAGCTTTTGGAAGACAACCTTATTTTGAAACTCTCTTCCACAAGGCTCTG
AATCTCCACACAGCCAACTGGTTTCTGTATCTTTCAACACTGCGCTGGTTCCAGATGAGG
ATTGAGATGATCTTTGTTATCTTCTTCATAGCTGTTACCTTCATCTCTATTCTGACAACTG
GTGAGGGGGAAGGGAGAGTAGGCATCATCCTCACACTAGCCATGAACATAATGTCTAC
CTTACAATGGGCCGTGAACAGCTCCATAGATGTGGACAGCCTCATGAGAAGTGTGTCAA
GAGTTTTCAAATTCATTGACATGCCCACAGAAGGCAAACCAACCAAGAGCACAAAACC
CTACAAGAATGGCCAGCTGAGTAAGGTCATGATCATTGAAAATTCTCATGTGAAGAAG

GATGATATTTGGCCCAGTGGGGGCCAGATGACAGTCAAGGACCTCACTGCCAAATACA
CAGAGGGTGGAAATGCTATCCTAGAGAACATCTCCTTCTCCATCTCCCCAGGCCAAAGA
GTTGGCTTGCTGGGCAGGACTGGCAGTGGCAAGTCCACCTTGCTCTCAGCATTTCTCAG
GCTTTTAAATACAGAGGGAGAGATTCAAATTGATGGGGTGTCTTGGGATAGTATAACAC
TTCAACAGTGGAGGAAAGCCTTTGGTGTGATTCCTCAGAAAGTGTTTATCTTCTCTGGCA
CTTTCAGAAAAAATCTGGACCCCTATGAACAGTGGAGTGACCAGGAAATCTGGAAGGT
GGCAGATGAAGTGGGCCTAAGATCAGTCATAGAGCAGTTTCCTGGAAAGTTGGATTTTG
TGCTTGTAGATGGAGGCTGTGTGCTGTCCCATGGCCATAAACAGCTAATGTGCCTGGCT
AGGTCAGTGCTGAGCAAGGCCAAGATCCTGCTGTTAGATGAGCCTTCAGCCCATCTGGA
CCCTGTGACATACCAGATTATCAGAAGAACTCTGAAGCAGGCCTTTGCTGACTGCACTG
TCATCCTGTGTGAGCACAGAATTGAGGCCATGCTGGAGTGCCAGCAGTTCCTTGTTATA
GAAGAGAATAAGGTTAGGCAGTATGACAGCATTCAGAAACTGCTAAATGAAAGATCTC
TCTTCAGGCAAGCTATTTCACCATCTGATAGAGTGAAACTTTTTCCCCACAGAAATTCCT
CTAAATGTAAATCTAAGCCCCAGATAGCTGCCTTGAAAGAGGAGACTGAAGAAGAAGT
CCAGGACACCAGACTGTGA (SEQ ID NO: 20).

In another embodiment, an exemplary codon-optimized CFTR mRNA sequence is:

ATGCAGAGATCCCCGCTGGAGAAGGCATCTGTGGTGTCAAAACTGTTCTTTAGCTGGAC
AAGGCCCATCCTTAGGAAAGGGTACAGACAGAGGTTGGAGCTGTCAGACATATATCAG
ATCCCTTCAGTGGACTCTGCAGACAACCTCTCTGAAAAGCTGGAGAGGGAATGGGACA
GGGAACTGGCCAGCAAAAAAAACCCTAAACTGATTAATGCCCTGAGGAGGTGCTTCTTT
TGGAGATTCATGTTCTATGGGATCTTCCTTTACCTGGGGGAGGTGACTAAAGCTGTTCA
GCCTCTTCTTCTGGGGAGGATTATTGCCTCCTATGACCCAGACAACAAAGAAGAAAGAA
GCATAGCCATTTACTTAGGCATAGGCCTCTGCTTGCTCTTCATAGTTAGAACCCTCCTAC
TCCACCCAGCCATCTTTGGTCTCCACCACATAGGTATGCAGATGAGAATAGCAATGTTC
TCCTTGATCTACAAGAAGACCCTCAAGCTGTCCAGCAGGGTGCTGGACAAGATCTCCAT
AGGCCAGTTAGTCAGTCTACTGTCCAATAACTTAAATAAGTTTGATGAGGGACTGGCAC
TGGCACATTTTGTGTGGATTGCCCCCCTCCAAGTGGCCCTTCTTATGGGCCTTATCTGGG
AGCTGTTGCAGGCCTCTGCTTTCTGTGGCCTGGGTTTCCTCATAGTCCTAGCCTTATTCC
AGGCTGGACTGGGCAGAATGATGATGAAGTATAGGGACCAAAGAGCAGGGAAGATTTC
TGAAAGGCTGGTTATAACTTCTGAGATGATTGAGAACATTCAGTCAGTGAAAGCTTACT
GCTGGGAAGAAGCTATGGAAAAAATGATTGAAAATCTCAGACAGACTGAATTAAAGTT
GACCAGGAAAGCTGCTTATGTCAGATACTTCAACTCCTCAGCCTTCTTTTTTTCTGGCTT

CTTTGTTGTATTCCTTTCAGTCCTCCCCTATGCCCTGATTAAGGGCATTATCTTGAGGAA
AATTTTCACAACCATCTCCTTTTGTATTGTCCTCAGGATGGCTGTTACAAGGCAATTTCC
TTGGGCTGTGCAAACTTGGTATGATAGCCTTGGAGCAATCAACAAGATCCAGGATTTCC
TGCAAAAGCAGGAGTACAAGACATTGGAATACAACCTTACCACCACTGAGGTGGTGAT
GGAAAATGTGACTGCCTTCTGGGAGGAGGGGTTTGGAGAGCTGTTTGAGAAAGCCAAA
CAGAACAACAACAATAGAAAGACCTCTAATGGTGATGATTCCCTGTTCTTTTCTAACTTT
AGTCTTCTGGGGACCCCAGTTCTGAAAGATATTAACTTTAAAATTGAAAGGGGACAGTT
GCTGGCTGTGGCTGGGTCCACTGGGGCTGGGAAGACAAGCCTGCTCATGGTGATCATGG
GAGAGCTGGAACCCAGTGAAGGAAAGATCAAACACTCAGGCAGGATCTCCTTCTGCAG
CCAGTTCTCATGGATTATGCCAGGCACTATTAAAGAAAATATCATCTTTGGTGTAAGCT
ATGATGAGTACAGGTATAGATCTGTAATTAAAGCCTGCCAGCTGGAGGAAGACATCTCT
AAGTTTGCTGAGAAGGATAACATTGTGTTGGGGGAAGGGGGCATCACCCTTTCTGGTGG
GCAGAGGGCTAGGATCTCCCTTGCTAGGGCAGTATACAAGGATGCTGACTTGTACCTCT
TGGATAGTCCTTTTGGCTACCTAGATGTGCTGACAGAGAAAGAAATATTTGAAAGCTGT
GTGTGTAAGCTCATGGCTAACAAGACCAGGATCCTGGTCACCAGTAAAATGGAACACCT
CAAAAAAGCAGACAAGATCCTTATTCTCCATGAGGGCTCCTCCTACTTCTATGGGACCT
TCAGTGAGCTGCAGAATCTGCAGCCAGACTTCTCCTCAAAACTTATGGGCTGTGACTCC
TTTGACCAATTCTCTGCAGAAAGAAGGAATAGCATACTGACAGAAACACTGCATAGATT
CTCCCTGGAAGGAGATGCCCCAGTGAGTTGGACAGAAACCAAAAAGCAGAGCTTCAAG
CAGACTGGTGAGTTTGGTGAAAAGAGGAAGAATTCTATCCTGAACCCCATCAATAGCAT
CAGGAAATTTAGCATAGTCCAAAAGACCCCCCTCCAGATGAATGGAATAGAGGAGGAT
AGTGATGAGCCTCTTGAGAGAAGGCTGTCCCTGGTTCCAGACAGTGAACAGGGTGAAG
CCATTCTTCCGAGGATCAGTGTCATCTCCACTGGGCCCACATTGCAGGCCAGAAGAAGA
CAGTCTGTTCTGAATTTGATGACACATTCTGTGAATCAAGGCCAGAATATCCATAGAAA
AACCACTGCCAGCACCAGAAAAGTTTCTCTAGCCCCCCAGGCTAACCTGACTGAGTTAG
ACATCTACAGCAGAAGGCTGAGCCAAGAGACTGGCTTGGAAATATCTGAGGAGATCAA
TGAGGAGGACCTCAAGGAGTGCTTCTTTGATGACATGGAGTCAATCCCTGCAGTCACTA
CATGGAACACTTACCTAAGGTACATCACAGTTCATAAGAGCCTCATCTTTGTCCTCATAT
GGTGTCTGGTCATCTTTTTAGCAGAAGTGGCTGCCAGCCTAGTTGTGCTGTGGTTACTGG
GCAATACACCTCTTCAGGACAAAGGCAATAGCACACACAGCAGAAACAACTCCTATGC
AGTGATCATCACCTCTACAAGCTCTTACTATGTATTCTATATATATGTGGGAGTGGCAGA
TACTCTCCTGGCCATGGGATTCTTCAGGGGATTACCTCTAGTTCACACATTGATCACAGT

GTCAAAAATTCTCCACCACAAGATGTTACACAGTGTCCTGCAAGCCCCAATGTCTACTC
TGAACACACTTAAGGCAGGTGGAATTTTGAATAGGTTTAGCAAGGACATAGCTATCCTG
GATGATCTCCTCCCTCTGACCATCTTTGACTTCATCCAGTTACTGCTCATTGTAATTGGA
GCCATTGCAGTGGTAGCAGTCCTACAGCCTTACATTTTTGTGGCTACTGTTCCTGTTATT
GTGGCCTTCATTATGCTAAGAGCTTACTTCCTGCAAACAAGCCAACAGTTGAAACAGCT
AGAAAGTGAGGGAAGGTCCCCCATCTTCACCCACCTGGTGACATCACTCAAGGGGCTAT
GGACTCTTAGGGCTTTTGGGAGACAGCCGTACTTTGAGACCTTATTCCATAAGGCCCTT
AACCTCCATACAGCAAACTGGTTCTTATACCTGAGTACTCTGAGGTGGTTTCAAATGAG
GATTGAAATGATTTTTGTGATCTTCTTCATTGCTGTGACCTTCATCTCAATCTTGACCAC
AGGAGAGGGGGAGGGCAGGGTGGGCATCATACTGACCTTGGCCATGAACATTATGTCA
ACCCTGCAGTGGGCTGTCAATAGCTCCATTGATGTGGACAGTCTGATGAGGAGTGTCTC
CAGGGTCTTCAAGTTTATTGACATGCCAACTGAGGGCAAACCCACCAAAAGCACTAAG
CCATATAAAAATGGCCAACTGTCCAAAGTGATGATCATTGAAAATTCACATGTAAAGAA
GGATGATATCTGGCCCTCTGGAGGACAGATGACAGTGAAAGACCTGACTGCCAAGTAC
ACAGAGGGTGGTAATGCCATTCTTGAGAACATTAGTTTCAGTATTTCCCCGGGGCAAAG
GGTGGGCCTCCTTGGCAGAACAGGCTCTGGCAAGAGTACCCTGCTGTCAGCCTTTTTAA
GACTGTTGAACACTGAGGGAGAAATTCAGATTGATGGTGTCTCCTGGGATAGCATCACC
CTCCAGCAGTGGAGAAAAGCTTTTGGAGTGATCCCGCAAAAGGTTTTCATCTTTTCAGG
CACCTTCCGGAAGAACCTGGACCCCTATGAGCAGTGGTCTGACCAGGAAATATGGAAG
GTAGCTGATGAAGTTGGGCTTAGGTCAGTCATAGAGCAGTTCCCAGGCAAACTGGACTT
TGTCCTGGTGGATGGTGGATGTGTACTGAGTCATGGGCACAAACAGCTGATGTGCCTAG
CCAGGTCTGTGCTCAGCAAGGCAAAGATATTGCTGCTTGATGAACCCAGTGCCCATCTG
GACCCAGTCACATATCAGATCATCAGAAGAACATTGAAGCAGGCCTTTGCTGATTGCAC
AGTTATCCTCTGTGAGCACAGGATTGAGGCCATGCTGGAGTGCCAGCAGTTTCTGGTGA
TTGAGGAGAATAAAGTAAGGCAGTATGACTCCATCCAGAAGCTGCTCAATGAAAGAAG
CCTCTTTAGACAAGCTATCTCCCCCTCAGACAGGGTCAAATTGTTCCCTCACAGAAACA
GCAGCAAGTGCAAGAGCAAGCCCCAAATTGCAGCCTTGAAAGAGGAGACAGAGGAAG
AGGTGCAGGACACCAGACTCTGA (SEQ ID NO: 21).

In another embodiment, an exemplary codon-optimized CFTR mRNA sequence is:

ATGCAGAGAAGCCCCCTGGAGAAGGCCAGCGTGGTGAGCAAGCTGTTCTTCAGCTGGA
CCAGACCCATCCTGAGAAAGGGCTACAGACAGAGACTGGAGCTGAGCGACATCTACCA
GATCCCCAGCGTGGACAGCGCCGACAACCTGAGCGAGAAGCTGGAGAGAGAGTGGGA

CAGAGAGCTGGCCAGCAAGAAGAACCCCAAGCTGATCAACGCCCTGAGAAGATGCTTC
TTCTGGAGATTCATGTTCTACGGCATCTTCCTGTACCTGGGCGAGGTGACCAAGGCCGT
GCAGCCCCTGCTGCTGGGCAGAATCATCGCCAGCTACGACCCCGACAACAAGGAGGAG
AGAAGCATCGCCATCTACCTGGGCATCGGCCTGTGCCTGCTGTTCATCGTGAGAACCCT
GCTGCTGCACCCCGCCATCTTCGGCCTGCACCACATCGGCATGCAGATGAGAATCGCCA
TGTTCAGCCTGATCTACAAGAAGACCCTGAAGCTGAGCAGCAGAGTGCTGGACAAGAT
CAGCATCGGCCAGCTGGTGAGCCTGCTGAGCAACAACCTGAACAAGTTCGACGAGGGC
CTGGCCCTGGCCCACTTCGTGTGGATCGCCCCCCTGCAGGTGGCCCTGCTGATGGGCCT
GATCTGGGAGCTGCTGCAGGCCAGCGCCTTCTGCGGCCTGGGCTTCCTGATCGTGCTGG
CCCTGTTCCAGGCCGGCCTGGGCAGAATGATGATGAAGTACAGAGACCAGAGAGCCGG
CAAGATCAGCGAGAGACTGGTGATCACCAGCGAGATGATCGAGAACATCCAGAGCGTG
AAGGCCTACTGCTGGGAGGAGGCCATGGAGAAGATGATCGAGAACCTGAGACAGACCG
AGCTGAAGCTGACCAGAAAGGCCGCCTACGTGAGATACTTCAACAGCAGCGCCTTCTTC
TTCAGCGGCTTCTTCGTGGTGTTCCTGAGCGTGCTGCCCTACGCCCTGATCAAGGGCATC
ATCCTGAGAAAGATCTTCACCACCATCAGCTTCTGCATCGTGCTGAGAATGGCCGTGAC
CAGACAGTTCCCCTGGGCCGTGCAGACCTGGTACGACAGCCTGGGCGCCATCAACAAG
ATCCAGGACTTCCTGCAGAAGCAGGAGTACAAGACCCTGGAGTACAACCTGACCACCA
CCGAGGTGGTGATGGAGAACGTGACCGCCTTCTGGGAGGAGGGCTTCGGCGAGCTGTT
CGAGAAGGCCAAGCAGAACAACAACAACAGAAAGACCAGCAACGGCGACGACAGCCT
GTTCTTCAGCAACTTCAGCCTGCTGGGCACCCCCGTGCTGAAGGACATCAACTTCAAGA
TCGAGAGAGGCCAGCTGCTGGCCGTGGCCGGCAGCACCGGCGCCGGCAAGACCAGCCT
GCTGATGGTGATCATGGGCGAGCTGGAGCCCAGCGAGGGCAAGATCAAGCACAGCGGC
AGAATCAGCTTCTGCAGCCAGTTCAGCTGGATCATGCCCGGCACCATCAAGGAGAACAT
CATCTTCGGCGTGAGCTACGACGAGTACAGATACAGAAGCGTGATCAAGGCCTGCCAG
CTGGAGGAGGACATCAGCAAGTTCGCCGAGAAGGACAACATCGTGCTGGGCGAGGGCG
GCATCACCCTGAGCGGCGGCCAGAGAGCCAGAATCAGCCTGGCCAGAGCCGTGTACAA
GGACGCCGACCTGTACCTGCTGGACAGCCCCTTCGGCTACCTGGACGTGCTGACCGAGA
AGGAGATCTTCGAGAGCTGCGTGTGCAAGCTGATGGCCAACAAGACCAGAATCCTGGT
GACCAGCAAGATGGAGCACCTGAAGAAGGCCGACAAGATCCTGATCCTGCACGAGGGC
AGCAGCTACTTCTACGGCACCTTCAGCGAGCTGCAGAACCTGCAGCCCGACTTCAGCAG
CAAGCTGATGGGCTGCGACAGCTTCGACCAGTTCAGCGCCGAGAGAAGAAACAGCATC
CTGACCGAGACCCTGCACAGATTCAGCCTGGAGGGCGACGCCCCCGTGAGCTGGACCG

AGACCAAGAAGCAGAGCTTCAAGCAGACCGGCGAGTTCGGCGAGAAGAGAAAGAACA
GCATCCTGAACCCCATCAACAGCATCAGAAAGTTCAGCATCGTGCAGAAGACCCCCCTG
CAGATGAACGGCATCGAGGAGGACAGCGACGAGCCCCTGGAGAGAAGACTGAGCCTG
GTGCCCGACAGCGAGCAGGGCGAGGCCATCCTGCCCAGAATCAGCGTGATCAGCACCG
GCCCCACCCTGCAGGCCAGAAGAAGACAGAGCGTGCTGAACCTGATGACCCACAGCGT
GAACCAGGGCCAGAACATCCACAGAAAGACCACCGCCAGCACCAGAAAGGTGAGCCT
GGCCCCCCAGGCCAACCTGACCGAGCTGGACATCTACAGCAGAAGACTGAGCCAGGAG
ACCGGCCTGGAGATCAGCGAGGAGATCAACGAGGAGGACCTGAAGGAGTGCTTCTTCG
ACGACATGGAGAGCATCCCCGCCGTGACCACCTGGAACACCTACCTGAGATACATCAC
CGTGCACAAGAGCCTGATCTTCGTGCTGATCTGGTGCCTGGTGATCTTCCTGGCCGAGG
TGGCCGCCAGCCTGGTGGTGCTGTGGCTGCTGGGCAACACCCCCCTGCAGGACAAGGG
CAACAGCACCCACAGCAGAAACAACAGCTACGCCGTGATCATCACCAGCACCAGCAGC
TACTACGTGTTCTACATCTACGTGGGCGTGGCCGACACCCTGCTGGCCATGGGCTTCTTC
AGAGGCCTGCCCCTGGTGCACACCCTGATCACCGTGAGCAAGATCCTGCACCACAAGAT
GCTGCACAGCGTGCTGCAGGCCCCCATGAGCACCCTGAACACCCTGAAGGCCGGCGGC
ATCCTGAACAGATTCAGCAAGGACATCGCCATCCTGGACGACCTGCTGCCCCTGACCAT
CTTCGACTTCATCCAGCTGCTGCTGATCGTGATCGGCGCCATCGCCGTGGTGGCCGTGCT
GCAGCCCTACATCTTCGTGGCCACCGTGCCCGTGATCGTGGCCTTCATCATGCTGAGAG
CCTACTTCCTGCAGACCAGCCAGCAGCTGAAGCAGCTGGAGAGCGAGGGCAGAAGCCC
CATCTTCACCCACCTGGTGACCAGCCTGAAGGGCCTGTGGACCCTGAGAGCCTTCGGCA
GACAGCCCTACTTCGAGACCCTGTTCCACAAGGCCCTGAACCTGCACACCGCCAACTGG
TTCCTGTACCTGAGCACCCTGAGATGGTTCCAGATGAGAATCGAGATGATCTTCGTGAT
CTTCTTCATCGCCGTGACCTTCATCAGCATCCTGACCACCGGCGAGGGCGAGGGCAGAG
TGGGCATCATCCTGACCCTGGCCATGAACATCATGAGCACCCTGCAGTGGGCCGTGAAC
AGCAGCATCGACGTGGACAGCCTGATGAGAAGCGTGAGCAGAGTGTTCAAGTTCATCG
ACATGCCCACCGAGGGCAAGCCCACCAAGAGCACCAAGCCCTACAAGAACGGCCAGCT
GAGCAAGGTGATGATCATCGAGAACAGCCACGTGAAGAAGGACGACATCTGGCCCAGC
GGCGGCCAGATGACCGTGAAGGACCTGACCGCCAAGTACACCGAGGGCGGCAACGCCA
TCCTGGAGAACATCAGCTTCAGCATCAGCCCCGGCCAGAGAGTGGGCCTGCTGGGCAG
AACCGGCAGCGGCAAGAGCACCCTGCTGAGCGCCTTCCTGAGACTGCTGAACACCGAG
GGCGAGATCCAGATCGACGGCGTGAGCTGGGACAGCATCACCCTGCAGCAGTGGAGAA
AGGCCTTCGGCGTGATCCCCCAGAAGGTGTTCATCTTCAGCGGCACCTTCAGAAAGAAC

CTGGACCCCTACGAGCAGTGGAGCGACCAGGAGATCTGGAAGGTGGCCGACGAGGTGG
GCCTGAGAAGCGTGATCGAGCAGTTCCCCGGCAAGCTGGACTTCGTGCTGGTGGACGG
CGGCTGCGTGCTGAGCCACGGCCACAAGCAGCTGATGTGCCTGGCCAGAAGCGTGCTG
AGCAAGGCCAAGATCCTGCTGCTGGACGAGCCCAGCGCCCACCTGGACCCCGTGACCT
ACCAGATCATCAGAAGAACCCTGAAGCAGGCCTTCGCCGACTGCACCGTGATCCTGTGC
GAGCACAGAATCGAGGCCATGCTGGAGTGCCAGCAGTTCCTGGTGATCGAGGAGAACA
AGGTGAGACAGTACGACAGCATCCAGAAGCTGCTGAACGAGAGAAGCCTGTTCAGACA
GGCCATCAGCCCCAGCGACAGAGTGAAGCTGTTCCCCCACAGAAACAGCAGCAAGTGC
AAGAGCAAGCCCCAGATCGCCGCCCTGAAGGAGGAGACCGAGGAGGAGGTGCAGGAC
ACCAGACTGTGA (SEQ ID NO: 22).

In another embodiment, an exemplary codon-optimized CFTR mRNA sequence is:

ATGCAGCGCAGCCCCCTGGAGAAGGCCAGCGTGGTGAGCAAGCTGTTCTTCAGCTGGA
CCCGCCCCATCCTGCGCAAGGGCTACCGCCAGCGCCTGGAGCTGAGCGACATCTACCAG
ATCCCCAGCGTGGACAGCGCCGACAACCTGAGCGAGAAGCTGGAGCGCGAGTGGGACC
GCGAGCTGGCCAGCAAGAAGAACCCCAAGCTGATCAACGCCCTGCGCCGCTGCTTCTTC
TGGCGCTTCATGTTCTACGGCATCTTCCTGTACCTGGGCGAGGTGACCAAGGCCGTGCA
GCCCCTGCTGCTGGGCCGCATCATCGCCAGCTACGACCCCGACAACAAGGAGGAGCGC
AGCATCGCCATCTACCTGGGCATCGGCCTGTGCCTGCTGTTCATCGTGCGCACCCTGCTG
CTGCACCCCGCCATCTTCGGCCTGCACCACATCGGCATGCAGATGCGCATCGCCATGTT
CAGCCTGATCTACAAGAAGACCCTGAAGCTGAGCAGCCGCGTGCTGGACAAGATCAGC
ATCGGCCAGCTGGTGAGCCTGCTGAGCAACAACCTGAACAAGTTCGACGAGGGCCTGG
CCCTGGCCCACTTCGTGTGGATCGCCCCCCTGCAGGTGGCCCTGCTGATGGGCCTGATC
TGGGAGCTGCTGCAGGCCAGCGCCTTCTGCGGCCTGGGCTTCCTGATCGTGCTGGCCCT
GTTCCAGGCCGGCCTGGGCCGCATGATGATGAAGTACCGCGACCAGCGCGCCGGCAAG
ATCAGCGAGCGCCTGGTGATCACCAGCGAGATGATCGAGAACATCCAGAGCGTGAAGG
CCTACTGCTGGGAGGAGGCCATGGAGAAGATGATCGAGAACCTGCGCCAGACCGAGCT
GAAGCTGACCCGCAAGGCCGCCTACGTGCGCTACTTCAACAGCAGCGCCTTCTTCTTCA
GCGGCTTCTTCGTGGTGTTCCTGAGCGTGCTGCCCTACGCCCTGATCAAGGGCATCATCC
TGCGCAAGATCTTCACCACCATCAGCTTCTGCATCGTGCTGCGCATGGCCGTGACCCGC
CAGTTCCCCTGGGCCGTGCAGACCTGGTACGACAGCCTGGGCGCCATCAACAAGATCCA
GGACTTCCTGCAGAAGCAGGAGTACAAGACCCTGGAGTACAACCTGACCACCACCGAG
GTGGTGATGGAGAACGTGACCGCCTTCTGGGAGGAGGGCTTCGGCGAGCTGTTCGAGA

AGGCCAAGCAGAACAACAACAACCGCAAGACCAGCAACGGCGACGACAGCCTGTTCTT
CAGCAACTTCAGCCTGCTGGGCACCCCCGTGCTGAAGGACATCAACTTCAAGATCGAGC
GCGGCCAGCTGCTGGCCGTGGCCGGCAGCACCGGCGCCGGCAAGACCAGCCTGCTGAT
GGTGATCATGGGCGAGCTGGAGCCCAGCGAGGGCAAGATCAAGCACAGCGGCCGCATC
AGCTTCTGCAGCCAGTTCAGCTGGATCATGCCCGGCACCATCAAGGAGAACATCATCTT
CGGCGTGAGCTACGACGAGTACCGCTACCGCAGCGTGATCAAGGCCTGCCAGCTGGAG
GAGGACATCAGCAAGTTCGCCGAGAAGGACAACATCGTGCTGGGCGAGGGCGGCATCA
CCCTGAGCGGCGGCCAGCGCGCCCGCATCAGCCTGGCCCGCGCCGTGTACAAGGACGC
CGACCTGTACCTGCTGGACAGCCCCTTCGGCTACCTGGACGTGCTGACCGAGAAGGAGA
TCTTCGAGAGCTGCGTGTGCAAGCTGATGGCCAACAAGACCCGCATCCTGGTGACCAGC
AAGATGGAGCACCTGAAGAAGGCCGACAAGATCCTGATCCTGCACGAGGGCAGCAGCT
ACTTCTACGGCACCTTCAGCGAGCTGCAGAACCTGCAGCCCGACTTCAGCAGCAAGCTG
ATGGGCTGCGACAGCTTCGACCAGTTCAGCGCCGAGCGCCGCAACAGCATCCTGACCG
AGACCCTGCACCGCTTCAGCCTGGAGGGCGACGCCCCCGTGAGCTGGACCGAGACCAA
GAAGCAGAGCTTCAAGCAGACCGGCGAGTTCGGCGAGAAGCGCAAGAACAGCATCCTG
AACCCCATCAACAGCATCCGCAAGTTCAGCATCGTGCAGAAGACCCCCCTGCAGATGA
ACGGCATCGAGGAGGACAGCGACGAGCCCCTGGAGCGCCGCCTGAGCCTGGTGCCCGA
CAGCGAGCAGGGCGAGGCCATCCTGCCCCGCATCAGCGTGATCAGCACCGGCCCCACC
CTGCAGGCCCGCCGCCGCCAGAGCGTGCTGAACCTGATGACCCACAGCGTGAACCAGG
GCCAGAACATCCACCGCAAGACCACCGCCAGCACCCGCAAGGTGAGCCTGGCCCCCCA
GGCCAACCTGACCGAGCTGGACATCTACAGCCGCCGCCTGAGCCAGGAGACCGGCCTG
GAGATCAGCGAGGAGATCAACGAGGAGGACCTGAAGGAGTGCTTCTTCGACGACATGG
AGAGCATCCCCGCCGTGACCACCTGGAACACCTACCTGCGCTACATCACCGTGCACAAG
AGCCTGATCTTCGTGCTGATCTGGTGCCTGGTGATCTTCCTGGCCGAGGTGGCCGCCAG
CCTGGTGGTGCTGTGGCTGCTGGGCAACACCCCCCTGCAGGACAAGGGCAACAGCACC
CACAGCCGCAACAACAGCTACGCCGTGATCATCACCAGCACCAGCAGCTACTACGTGTT
CTACATCTACGTGGGCGTGGCCGACACCCTGCTGGCCATGGGCTTCTTCCGCGGCCTGC
CCCTGGTGCACACCCTGATCACCGTGAGCAAGATCCTGCACCACAAGATGCTGCACAGC
GTGCTGCAGGCCCCCATGAGCACCCTGAACACCCTGAAGGCCGGCGGCATCCTGAACC
GCTTCAGCAAGGACATCGCCATCCTGGACGACCTGCTGCCCCTGACCATCTTCGACTTC
ATCCAGCTGCTGCTGATCGTGATCGGCGCCATCGCCGTGGTGGCCGTGCTGCAGCCCTA
CATCTTCGTGGCCACCGTGCCCGTGATCGTGGCCTTCATCATGCTGCGCGCCTACTTCCT

GCAGACCAGCCAGCAGCTGAAGCAGCTGGAGAGCGAGGGCCGCAGCCCCATCTTCACC
CACCTGGTGACCAGCCTGAAGGGCCTGTGGACCCTGCGCGCCTTCGGCCGCCAGCCCTA
CTTCGAGACCCTGTTCCACAAGGCCCTGAACCTGCACACCGCCAACTGGTTCCTGTACC
TGAGCACCCTGCGCTGGTTCCAGATGCGCATCGAGATGATCTTCGTGATCTTCTTCATCG
CCGTGACCTTCATCAGCATCCTGACCACCGGCGAGGGCGAGGGCCGCGTGGGCATCATC
CTGACCCTGGCCATGAACATCATGAGCACCCTGCAGTGGGCCGTGAACAGCAGCATCG
ACGTGGACAGCCTGATGCGCAGCGTGAGCCGCGTGTTCAAGTTCATCGACATGCCCACC
GAGGGCAAGCCCACCAAGAGCACCAAGCCCTACAAGAACGGCCAGCTGAGCAAGGTG
ATGATCATCGAGAACAGCCACGTGAAGAAGGACGACATCTGGCCCAGCGGCGGCCAGA
TGACCGTGAAGGACCTGACCGCCAAGTACACCGAGGGCGGCAACGCCATCCTGGAGAA
CATCAGCTTCAGCATCAGCCCCGGCCAGCGCGTGGGCCTGCTGGGCCGCACCGGCAGC
GGCAAGAGCACCCTGCTGAGCGCCTTCCTGCGCCTGCTGAACACCGAGGGCGAGATCC
AGATCGACGGCGTGAGCTGGGACAGCATCACCCTGCAGCAGTGGCGCAAGGCCTTCGG
CGTGATCCCCCAGAAGGTGTTCATCTTCAGCGGCACCTTCCGCAAGAACCTGGACCCCT
ACGAGCAGTGGAGCGACCAGGAGATCTGGAAGGTGGCCGACGAGGTGGGCCTGCGCA
GCGTGATCGAGCAGTTCCCCGGCAAGCTGGACTTCGTGCTGGTGGACGGCGGCTGCGTG
CTGAGCCACGGCCACAAGCAGCTGATGTGCCTGGCCCGCAGCGTGCTGAGCAAGGCCA
AGATCCTGCTGCTGGACGAGCCCAGCGCCCACCTGGACCCCGTGACCTACCAGATCATC
CGCCGCACCCTGAAGCAGGCCTTCGCCGACTGCACCGTGATCCTGTGCGAGCACCGCAT
CGAGGCCATGCTGGAGTGCCAGCAGTTCCTGGTGATCGAGGAGAACAAGGTGCGCCAG
TACGACAGCATCCAGAAGCTGCTGAACGAGCGCAGCCTGTTCCGCCAGGCCATCAGCC
CCAGCGACCGCGTGAAGCTGTTCCCCCACCGCAACAGCAGCAAGTGCAAGAGCAAGCC
CCAGATCGCCGCCCTGAAGGAGGAGACCGAGGAGGAGGTGCAGGACACCCGCCTGTAA
(SEQ ID NO: 23).

In yet another embodiment, an exemplary codon-optimized CFTR mRNA sequence is:

ATGCAGAGAAGCCCCCTGGAGAAGGCCAGCGTGGTGAGCAAGCTGTTCTTCAGCTGGA
CCAGACCCATCCTGAGAAAGGGCTACAGACAGAGACTGGAGCTGAGCGACATCTACCA
GATCCCCAGCGTGGACAGCGCCGACAACCTGAGCGAGAAGCTGGAGAGAGAGTGGGA
CAGAGAGCTGGCCAGCAAGAAGAACCCCAAGCTGATCAACGCCCTGAGAAGATGCTTC
TTCTGGAGATTCATGTTCTACGGCATCTTCCTGTACCTGGGCGAGGTGACCAAGGCCGT
GCAGCCCCTGCTGCTGGGCAGAATCATCGCCAGCTACGACCCCGACAACAAGGAGGAG

AGAAGCATCGCCATCTACCTGGGCATCGGCCTGTGCCTGCTGTTCATCGTGAGAACCCT
GCTGCTGCACCCCGCCATCTTCGGCCTGCACCACATCGGCATGCAGATGAGAATCGCCA
TGTTCAGCCTGATCTACAAGAAGACCCTGAAGCTGAGCAGCAGAGTGCTGGACAAGAT
CAGCATCGGCCAGCTGGTGAGCCTGCTGAGCAACAACCTGAACAAGTTCGACGAGGGC
CTGGCCCTGGCCCACTTCGTGTGGATCGCCCCCCTGCAGGTGGCCCTGCTGATGGGCCT
GATCTGGGAGCTGCTGCAGGCCAGCGCCTTCTGCGGCCTGGGCTTCCTGATCGTGCTGG
CCCTGTTCCAGGCCGGCCTGGGCAGAATGATGATGAAGTACAGGGACCAGAGAGCCGG
CAAGATCAGCGAGAGACTGGTGATCACCAGCGAGATGATCGAGAACATCCAGAGCGTG
AAGGCCTACTGCTGGGAGGAGGCCATGGAGAAGATGATCGAGAACCTGAGACAGACCG
AGCTGAAGCTGACCAGAAAGGCCGCCTACGTGAGATACTTCAACAGCAGCGCCTTCTTC
TTCAGCGGCTTCTTCGTGGTGTTCCTGAGCGTGCTGCCCTACGCCCTGATCAAGGGCATC
ATCCTGAGAAAGATCTTCACCACCATCAGCTTCTGCATCGTGCTGAGAATGGCCGTGAC
CAGACAGTTCCCCTGGGCCGTGCAGACCTGGTACGACAGCCTGGGCGCCATCAACAAG
ATCCAGGACTTCCTGCAGAAGCAGGAGTACAAGACCCTGGAGTACAACCTGACCACCA
CCGAGGTGGTGATGGAGAACGTGACCGCCTTCTGGGAGGAGGGCTTCGGCGAGCTGTT
CGAGAAGGCCAAGCAGAACAACAACAACAGAAAGACCAGCAACGGCGACGACAGCCT
GTTCTTCAGCAACTTCAGCCTGCTGGGCACCCCCGTGCTGAAGGACATCAACTTCAAGA
TCGAGAGAGGCCAGCTGCTGGCCGTGGCCGGCAGCACCGGCGCCGGCAAGACCAGCCT
GCTGATGGTGATCATGGGCGAGCTGGAGCCCAGCGAGGGCAAGATCAAGCACAGCGGC
AGAATCAGCTTCTGCAGCCAGTTCAGCTGGATCATGCCCGGCACCATCAAGGAGAACAT
CATCTTCGGCGTGAGCTACGACGAGTACAGATACAGAAGCGTGATCAAGGCCTGCCAG
CTGGAGGAGGACATCAGCAAGTTCGCCGAGAAGGACAACATCGTGCTGGGCGAGGGCG
GCATCACCCTGAGCGGCGGCCAGAGAGCCAGAATCAGCCTGGCCAGAGCCGTGTACAA
GGACGCCGACCTGTACCTGCTGGACAGCCCCTTCGGCTACCTGGACGTGCTGACCGAGA
AGGAGATCTTCGAGAGCTGCGTGTGCAAGCTGATGGCCAACAAGACCAGAATCCTGGT
GACCAGCAAGATGGAGCACCTGAAGAAGGCCGACAAGATCCTGATCCTGCACGAGGGC
AGCAGCTACTTCTACGGCACCTTCAGCGAGCTGCAGAACCTGCAGCCCGACTTCAGCAG
CAAGCTGATGGGCTGCGACAGCTTCGACCAGTTCAGCGCCGAGAGAAGAAACAGCATC
CTGACCGAGACCCTGCACAGATTCAGCCTGGAGGGCGACGCCCCCGTGAGCTGGACCG
AGACCAAGAAGCAGAGCTTCAAGCAGACCGGCGAGTTCGGCGAGAAGAGAAAGAACA
GCATCCTGAACCCCATCAACAGCATCAGAAAGTTCAGCATCGTGCAGAAGACCCCCCTG
CAGATGAACGGCATCGAGGAGGACAGCGACGAGCCCCTGGAGAGAAGACTGAGCCTG

GTGCCCGACAGCGAGCAGGGCGAGGCCATCCTGCCCAGAATCAGCGTGATCAGCACCG
GCCCCACCCTGCAGGCCAGAAGACAGAGCGTGCTGAACCTGATGACCCACAGCGT
GAACCAGGGCCAGAACATCCACAGAAAGACCACCGCCAGCACCAGAAAGGTGAGCCT
GGCCCCCCAGGCCAACCTGACCGAGCTGGACATCTACAGCAGAAGACTGAGCCAGGAG
ACCGGCCTGGAGATCAGCGAGGAGATCAACGAGGAGGACCTGAAGGAGTGCTTCTTCG
ACGACATGGAGAGCATCCCCGCCGTGACCACCTGGAACACCTACCTGAGATACATCAC
CGTGCACAAGAGCCTGATCTTCGTGCTGATCTGGTGCCTGGTGATCTTCCTGGCCGAGG
TGGCCGCCAGCCTGGTGGTGCTGTGGCTGCTGGGCAACACCCCCCTGCAGGACAAGGG
CAACAGCACCCACAGCAGAAACAACAGCTACGCCGTGATCATCACCAGCACCAGCAGC
TACTACGTGTTCTACATCTACGTGGGCGTGGCCGACACCCTGCTGGCCATGGGCTTCTTC
AGAGGCCTGCCCCTGGTGCACACCCTGATCACCGTGAGCAAGATCCTGCACCACAAGAT
GCTGCACAGCGTGCTGCAGGCCCCCATGAGCACCCTGAACACCCTGAAGGCCGGCGGC
ATCCTGAACAGATTCAGCAAGGACATCGCCATCCTGGACGACCTGCTGCCCCTGACCAT
CTTCGACTTCATCCAGCTGCTGCTGATCGTGATCGGCGCCATCGCCGTGGTGGCCGTGCT
GCAGCCCTACATCTTCGTGGCCACCGTGCCCGTGATCGTGGCCTTCATCATGCTGAGAG
CCTACTTCCTGCAGACCAGCCAGCAGCTGAAGCAGCTGGAGAGCGAGGGCAGGAGCCC
CATCTTCACCCACCTGGTGACCAGCCTGAAGGGCCTGTGGACCCTGAGAGCCTTCGGCA
GACAGCCCTACTTCGAGACCCTGTTCCACAAGGCCCTGAACCTGCACACCGCCAACTGG
TTCCTGTACCTGAGCACCCTGAGATGGTTCCAGATGAGAATCGAGATGATCTTCGTGAT
CTTCTTCATCGCCGTGACCTTCATCAGCATCCTGACCACCGGCGAGGGCGAGGGCAGAG
TGGGCATCATCCTGACCCTGGCCATGAACATCATGAGCACCCTGCAGTGGGCCGTGAAC
AGCAGCATCGACGTGGACAGCCTGATGAGAAGCGTGAGCAGAGTGTTCAAGTTCATCG
ACATGCCCACCGAGGGCAAGCCCACCAAGAGCACCAAGCCCTACAAGAACGGCCAGCT
GAGCAAGGTGATGATCATCGAGAACAGCCACGTGAAGAAGGACGACATCTGGCCCAGC
GGCGGCCAGATGACCGTGAAGGACCTGACCGCCAAGTACACCGAGGGCGGCAACGCCA
TCCTGGAGAACATCAGCTTCAGCATCAGCCCCGGCCAGAGAGTGGGCCTGCTGGGCAG
AACCGGCAGCGGCAAGAGCACCCTGCTGAGCGCCTTCCTGAGACTGCTGAACACCGAG
GGCGAGATCCAGATCGACGGCGTGAGCTGGGACAGCATCACCCTGCAGCAGTGGAGAA
AGGCCTTCGGCGTGATCCCCCAGAAGGTGTTCATCTTCAGCGGCACCTTCAGAAAGAAC
CTGGACCCCTACGAGCAGTGGAGCGACCAGGAGATCTGGAAGGTGGCCGACGAGGTGG
GCCTGAGAAGCGTGATCGAGCAGTTCCCCGGCAAGCTGGACTTCGTGCTGGTGGACGG
CGGCTGCGTGCTGAGCCACGGCCACAAGCAGCTGATGTGCCTGGCCAGAAGCGTGCTG

AGCAAGGCCAAGATCCTGCTGCTGGACGAGCCCAGCGCCCACCTGGACCCCGTGACCT
ACCAGATCATCAGAAGAACCCTGAAGCAGGCCTTCGCCGACTGCACCGTGATCCTGTGC
GAGCACAGAATCGAGGCCATGCTGGAGTGCCAGCAGTTCCTGGTGATCGAGGAGAACA
AGGTGAGACAGTACGACAGCATCCAGAAGCTGCTGAACGAGAGAAGCCTGTTCAGACA
GGCCATCAGCCCCAGCGACAGAGTGAAGCTGTTCCCCCACAGAAACAGCAGCAAGTGC
AAGAGCAAGCCCCAGATCGCCGCCCTGAAGGAGGAGACCGAGGAGGAGGTGCAGGAC
ACCAGACTGTGA (SEQ ID NO: 24).

In another embodiment, an exemplary codon-optimized CFTR mRNA sequence is:

ATGCAGAGGTCACCTCTGGAAAAGGCTAGCGTGGTCAGCAAGCTATTTTTTTCCTGGAC
CCGCCCGATACTCAGGAAGGGCTACCGACAGCGGCTGGAGCTGAGTGACATTTATCAG
ATTCCCTCCGTCGATTCCGCTGACAACCTGTCTGAGAAACTGGAGCGGGAATGGGATAG
GGAACTGGCGTCCAAAAAAAACCCCAAACTCATCAATGCACTCCGCAGATGCTTCTTCT
GGCGGTTTATGTTTTATGGCATATTCCTGTATCTGGGGGAGGTGACGAAAGCCGTGCAG
CCGCTGCTGCTTGGTCGCATTATCGCGTCATACGATCCAGATAACAAGGAGGAAAGAAG
TATCGCTATCTATCTCGGGATAGGGCTGTGCCTGCTCTTCATTGTGCGGACTCTTCTCTT
GCACCCCGCCATTTTCGGTCTGCATCATATAGGTATGCAGATGAGAATTGCGATGTTCTC
ATTGATTTACAAAAAAACGCTTAAGCTAAGTTCAAGGGTGCTAGATAAGATATCGATCG
GCCAGCTGGTGTCTCTGCTTAGCAACAACCTCAATAAATTCGACGAAGGCCTTGCACTG
GCCCACTTCGTGTGGATCGCCCCTCTGCAGGTGGCTCTGCTGATGGGGTTAATATGGGA
GCTGTTGCAGGCCTCCGCTTTTTGTGGCCTGGGGTTTCTCATCGTGTTGGCCTTGTTTCA
GGCAGGGCTGGGACGTATGATGATGAAATATAGGGATCAGAGGGCTGGCAAAATCTCT
GAGCGCCTGGTTATTACGAGTGAAATGATTGAGAACATCCAGTCAGTGAAGGCCTATTG
CTGGGAGGAGGCCATGGAAAAAATGATTGAGAACCTACGCCAGACTGAGCTGAAGTTA
ACCAGAAAAGCCGCCTATGTGCGCTACTTTAACAGTAGCGCATTTTTCTTCTCCGGTTTT
TTCGTGGTGTTTCTTAGTGTGTTGCCGTATGCCTTAATCAAGGGAATAATACTCCGGAAG
ATTTTCACTACCATCAGCTTCTGTATCGTGTTGCGGATGGCCGTCACCCGGCAGTTTCCC
TGGGCAGTACAGACTTGGTACGATTCTCTCGGAGCAATTAACAAAATCCAAGACTTTCT
ACAAAAGCAGGAGTACAAGACCCTGGAGTACAATCTGACCACCACAGAAGTCGTAATG
GAGAATGTAACTGCCTTCTGGGAAGAGGGCTTTGGCGAACTCTTTGAAAAGGCCAAGC
AGAACAATAACAACCGGAAGACCTCCAACGGGGACGACAGCTTATTTTTCAGCAATTTT
TCTTTGCTCGGGACCCCTGTACTGAAAGATATTAACTTTAAGATCGAGCGCGGACAACT
CCTGGCTGTCGCCGGCAGCACTGGAGCTGGAAAAACATCACTGCTTATGGTGATAATGG

GAGAACTCGAACCAAGCGAGGGAAAAATAAAGCACTCTGGACGGATTAGTTTTTGCTC
CCAGTTCTCGTGGATAATGCCTGGCACCATTAAGGAGAATATCATCTTTGGAGTGAGTT
ACGACGAATACCGGTACCGGTCCGTTATCAAGGCTTGTCAACTCGAGGAGGACATTTCT
AAATTCGCCGAAAAAGATAATATAGTGCTGGGCGAAGGAGGCATTACACTGAGCGGGG
GTCAGAGAGCTCGAATTAGCCTCGCCCGAGCAGTCTATAAAGACGCCGATCTTTACCTG
CTGGATTCCCCTTTTGGGTATTTGGATGTTCTGACAGAGAAGGAAATCTTTGAATCATGT
GTCTGTAAACTGATGGCCAATAAGACTAGGATTCTAGTGACTTCGAAAATGGAGCACCT
GAAAAAAGCGGACAAAATTCTGATACTCCATGAAGGGTCTTCCTACTTCTACGGCACCT
TCTCAGAGTTGCAGAACTTACAACCTGATTTTTCATCTAAGCTTATGGGGTGCGACTCGT
TTGACCAGTTCTCCGCTGAAAGACGAAACAGCATCTTAACGGAAACTCTTCACAGGTTC
TCATTAGAGGGAGATGCGCCGGTGTCCTGGACAGAGACAAAAAAACAGTCTTTCAAAC
AGACAGGAGAGTTTGGCGAGAAGAGAAAAAACTCAATCCTCAATCCCATCAATTCTATT
AGAAAGTTTAGCATCGTCCAAAAAACACCATTGCAGATGAATGGGATTGAGGAGGACA
GTGATGAGCCTTTGGAACGAAGACTGTCCCTGGTACCCGATAGCGAACAGGGTGAGGC
CATCCTTCCTAGGATCTCGGTCATAAGTACAGGGCCCACACTGCAGGCCAGGCGACGTC
AAAGTGTCCTCAATCTTATGACGCACAGTGTGAATCAGGGGCAGAACATCCATCGTAAG
ACGACAGCTTCAACTCGAAAGGTCAGTCTAGCTCCACAAGCCAATCTTACAGAGCTGGA
CATTTATTCCCGCCGCCTCAGTCAGGAGACCGGATTGGAAATATCAGAGGAAATTAATG
AAGAGGATCTGAAGGAATGCTTCTTTGATGACATGGAATCGATCCCCGCTGTTACTACC
TGGAACACATATCTGAGATATATTACCGTCCATAAGAGCTTAATCTTTGTACTGATATG
GTGCTTGGTGATTTTCCTGGCAGAGGTTGCGGCGAGTTTGGTCGTGCTATGGCTCCTTGG
AAACACTCCCCTGCAGGATAAGGGGAACTCCACTCATAGCAGGAATAACAGCTATGCC
GTGATCATCACCTCTACCTCCTCTTATTACGTGTTTTACATATACGTCGGTGTTGCGGAT
ACCCTGTTGGCAATGGGGTTCTTTAGAGGACTACCCCTAGTTCACACCCTGATCACCGTT
TCGAAGATCTTGCACCACAAGATGCTTCATAGCGTTCTCCAAGCTCCTATGAGCACCCT
TAATACACTGAAAGCAGGAGGTATCCTTAACCGCTTTTCCAAAGACATCGCTATACTCG
ACGATTTGCTCCCATTGACCATCTTCGACTTCATTCAGCTGCTCCTCATTGTGATCGGCG
CCATTGCCGTGGTCGCAGTGTTACAGCCATATATTTTCGTAGCCACCGTGCCCGTCATCG
TGGCATTTATCATGCTGCGCGCATATTTCTTACAGACATCTCAGCAACTGAAGCAGCTG
GAATCTGAGGGCAGATCTCCTATTTTTACACACCTGGTTACCAGCCTGAAGGGCCTGTG
GACCCTGCGTGCTTTCGGTCGCCAACCCTACTTTGAGACTCTCTTCCATAAGGCTCTGAA
TTTACATACTGCCAATTGGTTCCTATACCTTAGTACCCTTCGGTGGTTCCAGATGCGGAT

AGAAATGATCTTCGTGATTTTCTTCATCGCAGTCACTTTCATCTCTATTTTGACGACCGG
TGAGGGCGAGGGCAGGGTGGGCATCATTCTGACTTTGGCCATGAACATTATGTCAACAC
TCCAGTGGGCCGTTAATTCAAGCATTGATGTGGATTCCTTGATGCGTTCCGTCAGCAGG
GTATTTAAATTCATAGACATGCCCACCGAGGGCAAGCCAACAAAATCTACCAAGCCAT
ACAAAAATGGCCAACTAAGCAAGGTCATGATTATCGAGAATTCTCATGTGAAAAAGGA
CGACATTTGGCCTTCCGGGGGTCAAATGACTGTAAAGGACCTGACGGCTAAATACACTG
AGGGCGGTAATGCTATCTTGGAGAACATCTCTTTCAGCATCTCCCCTGGCCAGAGAGTG
GGACTGCTCGGGCGGACAGGCTCCGGAAAGTCTACGCTCCTTTCAGCATTCCTTAGACT
TCTGAACACCGAAGGTGAGATTCAGATTGACGGGGTCTCTTGGGACTCCATCACACTTC
AGCAATGGAGGAAGGCATTCGGTGTAATCCCCCAAAAGGTTTTTATCTTCTCCGGAACA
TTTCGTAAGAATCTGGACCCGTACGAGCAGTGGTCAGATCAGGAGATCTGGAAAGTAG
CAGACGAGGTCGGGCTACGGAGCGTTATTGAACAGTTTCCTGGCAAACTGGACTTCGTT
TTGGTGGACGGAGGCTGTGTGCTGAGTCACGGCCATAAACAACTGATGTGCTTAGCTAG
GTCTGTTCTCAGCAAGGCAAAGATTTTACTGCTGGATGAACCAAGCGCCCACCTTGATC
CAGTGACATATCAAATCATCAGAAGAACTCTTAAACAGGCGTTCGCCGACTGCACAGTG
ATCCTGTGTGAGCACAGAATAGAAGCCATGCTGGAATGTCAACAGTTTCTCGTGATTGA
GGAGAACAAGGTGCGCCAGTACGATAGCATCCAGAAGTTACTCAATGAAAGGTCACTC
TTCAGGCAGGCCATCTCACCCAGCGACCGCGTTAAGCTGTTTCCACACCGAAACAGTTC
CAAGTGCAAAAGTAAGCCACAGATTGCTGCACTGAAGGAAGAGACAGAAGAAGAAGTT
CAGGACACTCGGCTCTGA (SEQ ID NO: 25).

In another embodiment, an exemplary codon-optimized CFTR mRNA sequence is:

ATGCAGAGGAGCCCACTGGAGAAAGCCTCCGTGGTGAGTAAACTCTTTTTTAGTTGGAC
CAGACCCATCCTGCGAAAAGGATACAGGCAGCGCCTCGAGTTGTCAGATATCTACCAG
ATTCCTTCTGTGGACTCAGCTGACAATTTGAGTGAGAAGCTGGAGCGGGAGTGGGATAG
AGAGCTGGCGAGCAAAAAAAACCCCAAGCTTATCAATGCTCTGCGCCGCTGCTTTTTCT
GGAGGTTCATGTTTTATGGGATCTTCCTGTACCTGGGGGAGGTCACCAAAGCTGTTCAG
CCGCTCCTTCTTGGCCGCATCATCGCCAGCTATGACCCTGATAATAAAGAAGAAAGGTC
TATTGCTATTTATCTGGGAATTGGCCTCTGCTTGCTCTTCATCGTCCGCACCCTTCTGCTG
CACCCTGCCATTTTTGGCCTTCACCACATCGGCATGCAAATGAGAATTGCCATGTTCTCC
CTCATTTACAAAAAGACCCTGAAACTTTCCTCAAGAGTGTTAGATAAAATATCCATTGG
TCAGCTGGTCAGCCTGCTGTCCAACAATCTTAACAAATTTGATGAAGGCTTGGCGCTGG
CCCACTTCGTGTGGATTGCACCTCTGCAGGTGGCCCTGTTGATGGGACTTATATGGGAG

CTGCTTCAAGCCTCTGCTTTCTGTGGGCTGGGCTTTTTGATTGTACTGGCACTTTTTCAGG
CTGGGCTCGGAAGAATGATGATGAAATACAGAGATCAGCGGGCCGGGAAGATATCAGA
GCGACTTGTGATCACCAGTGAAATGATTGAAAATATTCAGAGCGTGAAAGCCTACTGCT
GGGAAGAAGCCATGGAGAAGATGATTGAGAACCTGAGGCAGACAGAGCTCAAGCTCAC
TCGGAAGGCTGCTTATGTTCGCTATTTCAACAGCAGCGCCTTCTTCTTCAGTGGCTTCTT
TGTTGTCTTCCTGTCTGTTCTGCCATATGCACTGATAAAAGGCATTATTTTACGAAAGAT
CTTCACCACCATCAGTTTTTGCATCGTTCTCAGGATGGCCGTCACAAGACAGTTCCCCTG
GGCTGTGCAGACCTGGTACGATTCCTTGGGGGCCATCAACAAGATTCAAGATTTCTTGC
AAAAACAAGAATATAAAACTTTAGAATACAACCTCACCACCACTGAAGTGGTCATGGA
AAATGTGACAGCCTTTTGGGAGGAGGGTTTTGGAGAATTGTTCGAGAAGGCAAAGCAG
AATAACAACAACAGGAAGACGAGCAATGGGGACGACTCTCTCTTCTTCAGCAACTTTTC
ACTGCTCGGGACCCCTGTGTTGAAAGATATAAACTTCAAGATCGAGAGGGGCCAGCTCT
TGGCTGTGGCAGGCTCCACTGGAGCTGGTAAAACATCTCTTCTCATGGTGATCATGGGG
GAACTGGAGCCTTCCGAAGGAAAAATCAAGCACAGTGGGAGAATCTCATTCTGCAGCC
AGTTTTCCTGGATCATGCCCGGCACCATTAAGGAAAACATCATATTTGGAGTGTCCTAT
GATGAGTACCGCTACCGGTCAGTCATCAAAGCCTGTCAGTTGGAGGAGGACATCTCCAA
GTTTGCAGAGAAAGACAACATTGTGCTTGGAGAGGGGGGTATCACTCTTTCTGGAGGAC
AAAGAGCCAGGATCTCTTTGGCCCGGGCAGTCTACAAGGATGCAGACCTCTACTTGTTG
GACAGTCCCTTCGGCTACCTCGACGTGCTGACTGAAAAAGAAATTTTTGAAAGCTGTGT
GTGCAAACTGATGGCAAACAAGACCAGGATTCTTGTCACCAGCAAGATGGAACATCTG
AAGAAAGCGGACAAAATTCTGATTCTGCATGAAGGGAGCTCCTACTTCTATGGAACATT
TAGCGAGCTTCAGAACCTACAGCCAGACTTCTCCTCCAAATTAATGGGCTGTGACTCCT
TCGACCAGTTCTCTGCAGAAAGAAGAAACTCTATACTCACAGAGACCCTCCACCGCTTC
TCCCTTGAGGGAGATGCCCCAGTTTCTTGGACAGAAACCAAGAAGCAGTCCTTTAAGCA
GACTGGCGAGTTTGGTGAAAAGAGGAAAAATTCAATTCTCAATCCAATTAACAGTATTC
GCAAGTTCAGCATTGTCCAGAAGACACCCCTCCAGATGAATGGCATCGAAGAAGATAG
TGACGAGCCGCTGGAGAGACGGCTGAGTCTGGTGCCAGATTCAGAACAGGGGGAGGCC
ATCCTGCCCCGGATCAGCGTCATTTCCACAGGCCCCACATTACAAGCACGGCGCCGGCA
GAGTGTTTTAAATCTCATGACCCATTCAGTGAACCAGGGCCAAAATATCCACAGGAAGA
CTACAGCTTCTACCCGGAAAGTGTCTCTGGCCCCTCAGGCCAATCTGACCGAGCTGGAC
ATCTACAGCAGGAGGCTCTCCCAGGAAACAGGGCTGGAAATATCTGAAGAGATTAATG
AAGAGGATCTTAAAGAGTGCTTCTTTGATGACATGGAGAGCATCCCCGCGGTGACCACA

TGGAACACCTACCTTAGATATATTACTGTCCACAAGAGCCTCATATTTGTCCTCATCTGG
TGCCTGGTTATTTTCCTCGCTGAGGTGGCGGCCAGTCTTGTTGTGCTCTGGCTGCTGGGC
AACACTCCTCTCCAGGACAAGGGCAATAGTACTCACAGCAGAAATAATTCTTATGCCGT
CATCATTACAAGCACCTCCAGCTACTACGTGTTCTACATCTATGTGGGCGTGGCTGACA
CCCTCCTGGCCATGGGTTTCTTCCGGGGCCTGCCTTTGGTGCACACCCTCATCACAGTGT
CAAAAATTCTGCACCATAAAATGCTTCATTCTGTCCTGCAGGCACCCATGAGCACTTTG
AACACATTGAAGGCTGGCGGCATCCTCAACAGATTTTCTAAAGATATTGCTATCCTGGA
TGATCTCCTCCCCCTGACAATCTTTGACTTTATCCAGCTTCTGCTGATCGTGATTGGAGC
CATAGCAGTGGTTGCTGTCCTGCAGCCCTACATTTTTGTGGCCACCGTGCCCGTGATTGT
TGCCTTTATTATGCTCAGAGCTTACTTCCTGCAAACTTCTCAACAGCTCAAACAGCTAGA
ATCTGAGGGCCGGAGCCCCATTTTTACCCACCTGGTGACTTCCCTGAAGGGACTGTGGA
CTCTGAGAGCATTCGGGCGACAGCCTTACTTTGAGACACTGTTCCACAAGGCCCTGAAC
TTGCACACTGCCAACTGGTTTCTTTACCTGAGCACACTCCGCTGGTTCCAGATGCGGATA
GAGATGATCTTCGTCATCTTTTTTATAGCTGTAACCTTCATTTCTATCCTTACAACAGGA
GAAGGAGAGGGCAGGGTGGGAATCATCCTCACGCTGGCTATGAACATAATGTCCACCT
TGCAGTGGGCCGTGAATTCCAGTATAGATGTGGATTCTCTAATGAGGAGTGTCTCCCGG
GTGTTTAAATTCATTGATATGCCTACTGAGGGGAAACCCACCAAGTCAACAAAACCTTA
TAAGAATGGACAGCTGAGCAAGGTGATGATAATTGAGAACAGCCACGTGAAGAAGGAT
GACATTTGGCCCAGCGGGGGCCAGATGACTGTGAAGGACCTGACGGCCAAGTACACCG
AAGGTGGAAATGCCATTTTGGAAAACATCAGCTTCTCAATCTCTCCTGGGCAGAGAGTT
GGATTGCTGGGTCGCACGGGCAGCGGCAAATCAACCCTGCTCAGTGCCTTCCTTCGGCT
CCTGAATACAGAAGGCGAAATCCAAATTGACGGGGTGAGCTGGGACAGCATCACCCTG
CAGCAGTGGAGAAAAGCATTTGGGGTCATTCCACAGAAAGTTTTCATCTTCTCTGGCAC
TTTCAGAAAGAACCTGGACCCCTATGAGCAGTGGAGCGACCAGGAGATCTGGAAGGTT
GCAGATGAAGTTGGCCTGCGGAGTGTGATAGAACAATTTCCTGGCAAGCTGGATTTTGT
GCTGGTAGATGGAGGCTGCGTGCTGTCCCACGGCCACAAACAGCTGATGTGCCTCGCCC
GCTCCGTTCTTTCAAAGGCCAAAATCTTGCTTTTGGATGAGCCCAGTGCTCACCTCGACC
CAGTGACCTATCAGATAATCCGCAGGACCTTAAAGCAAGCTTTTGCCGACTGCACCGTC
ATACTGTGTGAGCACCGGATTGAAGCAATGCTGGAATGCCAGCAGTTTCTGGTGATCGA
GGAGAATAAGGTCCGGCAGTACGACAGCATCCAGAAGTTGTTGAATGAGCGCAGCCTT
TTCCGCCAGGCCATCTCCCCATCTGACAGAGTCAAGCTGTTTCCACATAGGAACTCCTCT

AAGTGCAAGTCCAAGCCCCAGATCGCTGCCCTCAAGGAGGAAACTGAGGAAGAGGTGC
AGGATACCCGCCTGTGA (SEQ ID NO: 26).

In another embodiment, an exemplary codon-optimized CFTR mRNA sequence is:

ATGCAACGGAGTCCTCTGGAAAAAGCCTCTGTCGTATCTAAGCTTTTCTTCAGTTGGAC
ACGCCCGATTTTGAGAAAGGGTTATCGGCAACGCTTGGAACTTAGTGACATCTACCAAA
TTCCAAGTGTAGACTCAGCCGATAACTTGAGCGAAAAGCTCGAACGAGAGTGGGATCG
AGAACTGGCTAGCAAAAAAAATCCCAAACTCATAAATGCCCTGCGACGCTGTTTCTTTT
GGCGATTTATGTTTTACGGTATTTTCCTTTATTTGGGTGAGGTCACGAAGGCTGTACAGC
CACTGCTGCTGGGTCGCATCATTGCCTCTTACGACCCTGACAACAAAGAGGAGCGGTCA
ATAGCTATCTACCTTGGTATAGGACTTTGCTTGCTCTTCATAGTCCGCACGTTGCTTCTC
CACCCTGCTATATTTGGTCTCCATCACATTGGGATGCAAATGCGGATCGCGATGTTCAGT
CTTATATATAAAAAGACTCTTAAACTTTCCAGCCGGGTTCTGGATAAGATCTCTATTGGT
CAACTGGTATCTCTTTTGTCTAACAACCTGAATAAGTTCGACGAGGGCCTTGCATTGGCC
CATTTTGTATGGATTGCCCCTTTGCAAGTCGCCCTCCTGATGGGATTGATCTGGGAACTC
CTGCAAGCTAGTGCTTTTTGCGGATTGGGATTCCTCATAGTCCTTGCGCTCTTTCAGGCG
GGACTTGGACGCATGATGATGAAGTATCGCGACCAACGAGCTGGCAAGATCAGTGAAC
GGCTTGTAATAACCAGTGAAATGATAGAGAACATCCAGAGCGTAAAAGCTTACTGTTG
GGAAGAAGCGATGGAAAAGATGATTGAGAACCTTCGCCAGACAGAACTTAAACTTACA
CGAAAGGCCGCTTATGTCCGGTACTTCAACTCTTCAGCATTTTTTTTTAGTGGCTTCTTTG
TAGTGTTCCTGTCCGTCCTTCCGTATGCACTTATCAAGGGTATAATACTTAGGAAAATCT
TCACAACAATCAGTTTTTGCATAGTCCTTCGCATGGCAGTAACTCGCCAATTTCCCTGGG
CAGTTCAGACGTGGTACGACTCACTTGGCGCAATTAACAAAATTCAAGATTTCCTCCAA
AAGCAAGAGTATAAAACCTTGGAATACAACCTTACCACCACAGAAGTTGTAATGGAAA
ATGTCACAGCCTTCTGGGAGGAAGGTTTCGGCGAACTTTTTGAGAAGGCGAAGCAAAAT
AACAATAATCGGAAAACATCAAACGGTGACGATTCACTGTTCTTTTCTAACTTTAGCCTT
CTTGGGACGCCCGTCCTGAAGGACATAAACTTTAAGATTGAACGGGGTCAACTTCTCGC
GGTCGCAGGGAGTACTGGAGCGGGAAAAACGAGCCTGCTGATGGTGATAATGGGGGAG
TTGGAGCCCTCAGAAGGCAAGATCAAGCATAGTGGTAGAATTAGCTTCTGCAGTCAATT
TAGTTGGATTATGCCGGGCACGATCAAAGAAAATATAATCTTTGGGGTATCCTACGATG
AATACAGGTACCGATCAGTGATAAAAGCGTGCCAGCTTGAAGAAGACATTTCAAAGTTT
GCTGAGAAGGATAATATCGTACTTGGAGAAGGAGGTATCACCCTGTCTGGGGGTCAAC
GAGCGAGGATCTCCCTGGCACGCGCCGTCTACAAGGACGCGGACCTCTATCTGTTGGAT

TCACCGTTCGGATATTTGGACGTGCTTACGGAGAAAGAAATATTTGAGAGCTGTGTTTG
CAAGCTCATGGCAAATAAAACCAGAATATTGGTTACAAGCAAGATGGAGCATCTTAAG
AAAGCAGATAAAATCCTGATATTGCACGAGGGCTCTTCATACTTCTACGGGACGTTTTC
TGAGTTGCAGAACCTCCAGCCGGATTTCAGCTCTAAGCTGATGGGCTGTGATTCCTTTG
ATCAGTTTAGTGCGGAAAGACGAAACAGTATACTCACCGAAACACTGCACAGGTTCTCT
CTGGAGGGCGACGCCCCGGTTTCCTGGACAGAGACGAAGAAGCAGTCCTTCAAACAGA
CAGGCGAGTTTGGGGAGAAAAGGAAAAATAGCATACTCAACCCGATTAACAGCATTCG
CAAGTTCAGTATAGTACAAAAGACCCCGTTGCAGATGAACGGTATAGAGGAAGATTCT
GATGAGCCACTGGAAAGACGGCTTTCTCTCGTTCCGGACAGTGAACAGGGAGAGGCAA
TACTGCCTCGGATCAGCGTTATCTCTACAGGACCTACTTTGCAAGCTCGGCGCCGACAG
TCAGTCTTGAATCTTATGACTCATAGTGTTAATCAAGGCCAGAATATCCATCGCAAGAC
CACCGCAAGTACAAGGAAAGTGAGCTTGGCACCTCAAGCAAACCTTACTGAACTTGAT
ATCTACTCACGGCGACTTTCACAGGAGACCGGACTTGAAATTAGTGAAGAAATTAACGA
GGAGGACCTCAAGGAGTGCTTCTTCGATGACATGGAATCAATCCCCGCAGTCACAACCT
GGAACACTTATCTGAGGTATATAACAGTTCACAAGAGCCTCATTTTTGTACTTATTTGGT
GTTTGGTAATTTTCCTGGCGGAGGTTGCTGCTTCTTTGGTCGTCCTTTGGCTCCTCGGGA
ATACACCGCTCCAAGACAAAGGCAACTCTACCCATAGTAGGAACAATTCATATGCAGT
GATTATAACCAGTACATCATCTTATTACGTTTTCTATATTTATGTCGGGGTAGCTGACAC
GCTGTTGGCGATGGGCTTCTTTAGGGGCCTCCCCTTGGTACACACCCTTATCACGGTGAG
TAAAATCCTGCATCACAAAATGCTTCATTCTGTACTCCAAGCGCCGATGAGTACGCTTA
ATACGCTGAAAGCAGGAGGGATACTGAATCGGTTCAGCAAGGACATCGCCATTCTGGA
TGACCTGCTTCCATTGACAATATTTGATTTCATTCAGCTCCTTCTCATAGTTATTGGAGC
CATAGCGGTGGTGGCTGTGCTTCAGCCTTATATATTCGTTGCCACAGTTCCCGTTATAGT
GGCATTTATAATGCTCAGGGCCTACTTTCTCCAGACTTCCCAGCAGTTGAAGCAACTCG
AATCAGAAGGAAGGTCACCTATTTTCACACATCTTGTGACTTCCTTGAAGGGCTTGTGG
ACGCTGCGGGCCTTCGGAAGACAACCATATTTTGAAACTCTCTTCCACAAAGCTTTGAA
TCTTCATACTGCGAACTGGTTCCTGTATTTGAGTACTTTGCGCTGGTTCCAGATGAGGAT
AGAAATGATATTCGTTATCTTCTTTATCGCGGTTACGTTCATAAGTATCCTCACTACGGG
GGAGGGTGAGGGTAGAGTGGGCATAATACTGACCCTCGCCATGAACATTATGTCCACCC
TGCAGTGGGCGGTAAACAGCAGCATAGATGTGGATTCTTTGATGCGCAGTGTGAGCAG
GGTTTTTAAGTTTATCGATATGCCGACGGAAGGAAAGCCCACTAAAAGCACGAAACCCT
ATAAAAATGGACAGCTTAGCAAAGTAATGATAATCGAGAATAGCCATGTGAAAAAGGA

TGACATATGGCCTTCCGGAGGCCAAATGACTGTTAAAGATCTGACCGCTAAATATACCG
AGGGCGGCAACGCAATACTCGAAAACATAAGCTTTTCCATAAGCCCCGGCCAACGCGT
GGGTCTTCTGGGGAGGACTGGCTCCGGAAAATCAACGTTGCTTAGCGCGTTTTTGCGGC
TCCTTAACACTGAAGGTGAGATCCAAATAGATGGCGTTAGTTGGGACTCTATAACACTG
CAACAATGGCGGAAAGCTTTCGGCGTCATACCTCAGAAGGTGTTCATCTTTAGCGGAAC
GTTCAGGAAGAACTTGGATCCCTACGAACAATGGAGTGATCAAGAAATATGGAAAGTG
GCAGATGAGGTAGGCTTGCGCAGTGTCATTGAACAATTCCCAGGGAAACTCGACTTTGT
ACTGGTGGACGGCGGTTGCGTCTTGTCACACGGGCACAAACAGTTGATGTGTTTGGCCC
GCAGTGTTTTGTCTAAGGCGAAGATTCTGTTGCTCGACGAACCGAGTGCTCATCTTGATC
CCGTCACCTACCAAATCATCAGAAGGACGTTGAAGCAAGCTTTCGCCGACTGCACTGTA
ATCCTTTGTGAGCATAGGATCGAAGCAATGCTCGAGTGCCAACAGTTCTTGGTTATAGA
GGAGAATAAGGTTCGGCAATACGACTCAATACAGAAACTGCTTAATGAGCGGTCACTCT
TTCGACAAGCTATCTCTCCTAGTGACAGGGTAAAGCTTTTTCCTCATCGGAATTCCAGCA
AGTGTAAGAGTAAACCACAGATCGCCGCCCTTAAAGAGGAGACCGAAGAAGAGGTGCA
GGATACGAGACTTTAG (SEQ ID NO: 27).

In some embodiments, a codon-optimized CFTR mRNA sequence suitable for the present invention shares at least 60%, 65%, 70%, 75%, 80%, 85%, 90%, 95%, 96%, 97%, 98%, or 99% identity to SEQ ID NO:6 or SEQ ID NO:7 and encodes a CFTR protein having an amino acid sequence of SEQ ID NO:2. In a specific embodiment, a codon-optimized CFTR mRNA sequence suitable for the present invention has the nucleotide sequence of SEQ ID NO:6.

In some embodiments, a suitable mRNA sequence may be an mRNA sequence encoding a homolog or an analog of human CFTR (hCFTR) protein. For example, a homolog or an analog of hCFTR protein may be a modified hCFTR protein containing one or more amino acid substitutions, deletions, and/or insertions as compared to a wild-type or naturally-occurring hCFTR protein while retaining substantial hCFTR protein activity. In some embodiments, an mRNA suitable for the present invention encodes an amino acid sequence at least 50%, 55%, 60%, 65%, 70%, 75%, 80%, 85%, 90%, 91%, 92%, 93%, 94%, 95%, 96%, 97%, 98%, 99% or more homologous to SEQ ID NO: 2. In some embodiments, an mRNA suitable for the present invention encodes a protein substantially identical to hCFTR protein. In some embodiments, an mRNA suitable for the present invention encodes an amino acid sequence at least 50%, 55%, 60%, 65%, 70%, 75%, 80%, 85%, 90%, 91%, 92%, 93%, 94%, 95%, 96%, 97%, 98%, 99% or more identical to SEQ ID NO: 2. In some embodiments, an mRNA suitable for the present invention encodes a fragment or a portion of hCFTR protein. In some embodiments, an mRNA suitable for the present invention encodes a fragment or a portion of hCFTR protein, wherein the fragment or portion of the protein still maintains CFTR activity similar to that of the wild-type protein. Thus, in some embodiments, an mRNA suitable for the present invention has a nucleotide sequence at least 50%, 55%, 60%, 65%, 70%, 75%, 80%, 85%, 90%, 91%, 92%, 93%, 94%, 95%, 96%, 97%, 98%, 99% or more identical SEQ ID NO: 1, SEQ ID NO: 6 or SEQ ID NO: 7.

In some embodiments, an mRNA suitable for the present invention has a nucleotide sequence at least 50%, 55%, 60%, 65%, 70%, 75%, 80%, 85%, 90%, 91%, 92%, 93%, 94%, 95%, 96%, 97%, 98%, 99% or more identical to any one of SEQ ID NO: 8, SEQ ID NO: 29, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, or SEQ ID NO: 27.

In some embodiments, a suitable mRNA encodes a fusion protein comprising a full length, fragment or portion of an hCFTR protein fused to another protein *(e.g.,* an N or C terminal fusion). In some embodiments, the protein fused to the mRNA encoding a full length, fragment or portion of an HCFTR protein encodes a signal or a cellular targeting sequence.

mRNAs according to the present invention may be synthesized according to any of a variety of known methods. For example, mRNAs according to the present invention may be synthesized via *in vitro* transcription (IVT). Briefly, IVT is typically performed with a linear or circular DNA template containing a promoter, a pool of ribonucleotide triphosphates, a buffer system that may include DTT and magnesium ions, and an appropriate RNA polymerase (e.g., T3, T7, or SP6 RNA polymerase), DNAse I, pyrophosphatase, and/or RNAse inhibitor. The exact conditions will vary according to the specific application.

Typically, mRNA synthesis includes the addition of a "cap" on the N-terminal (5') end, and a "tail" on the C-terminal (3') end. The presence of the cap is important in providing resistance to nucleases found in most eukaryotic cells. The presence of a "tail" serves to protect the mRNA from exonuclease degradation.

Thus, in some embodiments, mRNAs (e.g., mRNAs encoding CFTR) include a 5' cap structure. A 5' cap is typically added as follows: first, an RNA terminal phosphatase removes one of the terminal phosphate groups from the 5' nucleotide, leaving two terminal phosphates; guanosine triphosphate (GTP) is then added to the terminal phosphates via a guanylyl transferase, producing a 5'-5' triphosphate linkage; and the 7-nitrogen of guanine is then methylated by a methyltransferase. In some embodiments, the nucleotide forming the cap is further methylated at the 3' position. In some embodiments, the nucleotide directly adjacent to the cap is further methylated at the 2' position. Examples of cap structures include, but are not limited to, m7G(5')ppp(5")(2'OMeG), m7G(5')ppp(5')(2'OMeA), m7(3'OMeG)(5')ppp(5')(2'OMeG), m7(3'OMeG)(5')ppp(5')(2'OMeA), m7G(5')ppp (5'(A,G(5')ppp(5')A and G(5')ppp(5')G. In a specific embodiment, the cap structure is m7G(5')ppp(5')(2'OMeG). Additional cap structures are described in published US Application No. US 2016/0032356 and U.S. Provisional Application 64/464,327, filed February 27, 2017.

In some embodiments, mRNAs (e.g., mRNAs encoding CFTR) include a 3' tail structure. Typically, a tail structure includes a poly(A) and/or poly(C) tail. A poly-A or poly-C tail on the 3' terminus of mRNA typically includes at least 50 adenosine or cytosine nucleotides, at least 100 adenosine or cytosine nucleotides, at least 150 adenosine or cytosine nucleotides, at least 200 adenosine or cytosine nucleotides, at least 250 adenosine or cytosine nucleotides, at least 300 adenosine or cytosine nucleotides, at least 350 adenosine or cytosine nucleotides, at least 400 adenosine or cytosine nucleotides, at least 450 adenosine or cytosine nucleotides, at least 500 adenosine or cytosine nucleotides, at least 550 adenosine or cytosine nucleotides, at least 600 adenosine or cytosine nucleotides, at least 650 adenosine or cytosine nucleotides, at least 700 adenosine or cytosine nucleotides, at least 750 adenosine or cytosine nucleotides, at least 800 adenosine or cytosine nucleotides, at least 850 adenosine or cytosine nucleotides, at least 900 adenosine or cytosine nucleotides, at least 950 adenosine or cytosine nucleotides, or at least 1 kb adenosine or cytosine nucleotides, respectively. In some embodiments, a poly-A or poly-C tail may be about 10 to 800 adenosine or cytosine nucleotides (*e*.*g*., about 10 to 200 adenosine or cytosine nucleotides, about 10 to 300 adenosine or cytosine nucleotides, about 10 to 400 adenosine or cytosine nucleotides, about 10 to 500 adenosine or cytosine nucleotides, about 10 to 550 adenosine or cytosine nucleotides, about 10 to 600 adenosine or cytosine nucleotides, about 50 to 600 adenosine or cytosine nucleotides, about 100 to 600 adenosine or cytosine nucleotides, about 150 to 600 adenosine or cytosine nucleotides, about 200 to 600 adenosine or cytosine nucleotides, about 250 to 600 adenosine or cytosine nucleotides, about 300 to 600 adenosine or cytosine nucleotides, about 350 to 600 adenosine or cytosine nucleotides, about 400 to 600 adenosine or cytosine nucleotides, about 450 to 600 adenosine or cytosine nucleotides, about 500 to 600 adenosine or cytosine nucleotides, about 10 to 150 adenosine or cytosine nucleotides, about 10 to 100 adenosine or cytosine nucleotides, about 20 to 70 adenosine or cytosine nucleotides, or about 20 to 60 adenosine or cytosine nucleotides) respectively. In some embodiments, a tail structure includes is a combination of poly(A) and poly(C) tails with various lengths described herein. In some embodiments, a tail structure includes at least 50%, 55%, 65%, 70%, 75%, 80%, 85%, 90%, 92%, 94%, 95%, 96%, 97%, 98%, or 99% adenosine nucleotides. In some embodiments, a tail structure includes at least 50%, 55%, 65%, 70%, 75%, 80%, 85%, 90%, 92%, 94%, 95%, 96%, 97%, 98%, or 99% cytosine nucleotides.

In a specific embodiment, an mRNA encoding CFTR that has a poly(A) tail of between 200 and 1000 adenosine nucleotides (*e.g.,* as determined using agarose gel electrophoresis) is particularly suitable for practicing the invention. Typically, an mRNA encoding CFTR for use with the invention has a poly(A) tail that is between 400 and 700 adenosine nucleotides (*e.g.,* as determined using agarose gel electrophoresis).

In a specific embodiment, the mRNA encoding CFTR has the following sequence and structural elements:

GGACAGAUCGCCUGGAGACGCCAUCCACGCUGUUUUGACCUCCAUAGAAGACACCGG
GACCGAUCCAGCCUCCGCGGCCGGGAACGGUGCAUUGGAACGCGGAUUCCCCGUGCC
AAGAGUGACUCACCGUCCUUGACACGAUGCAACGCUCUCCUCUUGAAAAGGCCUCGG
UGGUGUCCAAGCUCUUCUUCUCGUGGACUAGACCCAUCCUGAGAAAGGGGUACAGAC
AGCGCUUGGAGCUGUCCGAUAUCUAUCAAAUCCCUUCCGUGGACUCCGCGGACAACC
UGUCCGAGAAGCUCGAGAGAGAAUGGGACAGAGAACUCGCCUCAAAGAAGAACCCGA
AGCUGAUUAAUGCGCUUAGGCGGUGCUUUUUCUGGCGGUUCAUGUUCUACGGCAUC
UUCCUCUACCUGGGAGAGGUCACCAAGGCCGUGCAGCCCCUGUUGCUGGGACGGAUU
AUUGCCUCCUACGACCCCGACAACAAGGAAGAAAGAAGCAUCGCUAUCUACUUGGGC
AUCGGUCUGUGCCUGCUUUUCAUCGUCCGGACCCUCUUGUUGCAUCCUGCUAUUUUC
GGCCUGCAUCACAUUGGCAUGCAGAUGAGAAUUGCCAUGUUUUCCCUGAUCUACAAG
AAAACUCUGAAGCUCUCGAGCCGCGUGCUUGACAAGAUUUCCAUCGGCCAGCUCGUG
UCCCUGCUCUCCAACAAUCUGAACAAGUUCGACGAGGGCCUCGCCCUGGCCCACUUC
GUGUGGAUCGCCCCUCUGCAAGUGGCGCUUCUGAUGGGCCUGAUCUGGGAGCUGCUG
CAAGCCUCGGCAUUCUGUGGGCUUGGAUUCCUGAUCGUGCUGGCACUGUUCCAGGCC
GGACUGGGGCGGAUGAUGAUGAAGUACAGGGACCAGAGAGCCGGAAAGAUUUCCGA
ACGGCUGGUGAUCACUUCGGAAAUGAUCGAAAACAUCCAGUCAGUGAAGGCCUACUG

CUGGGAAGAGGCCAUGGAAAAGAUGAUUGAAAACCUCCGGCAAACCGAGCUGAAGC
UGACCCGCAAGGCCGCUUACGUGCGCUAUUUCAACUCGUCCGCUUUCUUCUUCUCCG
GGUUCUUCGUGGUGUUUCUCUCCGUGCUCCCCUACGCCCUGAUUAAGGGAAUCAUCC
UCAGGAAGAUCUUCACCACCAUUUCCUUCUGUAUCGUGCUCCGCAUGGCCGUGACCC
GGCAGUUCCCAUGGGCCGUGCAGACUUGGUACGACUCCCUGGGAGCCAUUAACAAGA
UCCAGGACUUCCUUCAAAAGCAGGAGUACAAGACCCUCGAGUACAACCUGACUACUA
CCGAGGUCGUGAUGGAAAACGUCACCGCCUUUUGGGAGGAGGGAUUUGGCGAACUG
UUCGAGAAGGCCAAGCAGAACAACAACAACCGCAAGACCUCGAACGGUGACGACUCC
CUCUUCUUUUCAAACUUCAGCCUGCUCGGGACGCCCGUGCUGAAGGACAUUAACUUC
AAGAUCGAAAGAGGACAGCUCCUGGCGGUGGCCGGAUCGACCGGAGCCGGAAAGACU
UCCCUGCUGAUGGUGAUCAUGGGAGAGCUUGAACCUAGCGAGGGAAAGAUCAAGCA
CUCCGGCCGCAUCAGCUUCUGUAGCCAGUUUUCCUGGAUCAUGCCCGGAACCAUUAA
GGAAAACAUCAUCUUCGGCGUGUCCUACGAUGAAUACCGCUACCGGUCCGUGAUCAA
AGCCUGCCAGCUGGAAGAGGAUAUUUCAAAGUUCGCGGAGAAAGAUAACAUCGUGC
UGGGCGAAGGGGGUAUUACCUUGUCGGGGGGCCAGCGGGCUAGAAUCUCGCUGGCCA
GAGCCGUGUAUAAGGACGCCGACCUGUAUCUCCUGGACUCCCCCUUCGGAUACCUGG
ACGUCCUGACCGAAAAGGAGAUCUUCGAAUCGUGCGUGUGCAAGCUGAUGGCUAACA
AGACUCGCAUCCUCGUGACCUCCAAAAUGGAGCACCUGAAGAAGGCAGACAAGAUUC
UGAUUCUGCAUGAGGGGUCCUCCUACUUUUACGGCACCUUCUCGGAGUUGCAGAACU
UGCAGCCCGACUUCUCAUCGAAGCUGAUGGGUUGCGACAGCUUCGACCAGUUCUCCG
CCGAAAGAAGGAACUCGAUCCUGACGGAAACCUUGCACCGCUUCUCUUUGGAAGGCG
ACGCCCCUGUGUCAUGGACCGAGACUAAGAAGCAGAGCUUCAAGCAGACCGGGGAAU
UCGGCGAAAAGAGGAAGAACAGCAUCUUGAACCCCAUUAACUCCAUCCGCAAGUUCU
CAAUCGUGCAAAAGACGCCACUGCAGAUGAACGGCAUUGAGGAGGACUCCGACGAAC
CCCUUGAGAGGCGCCUGUCCCUGGUGCCGGACAGCGAGCAGGGAGAAGCCAUCCUGC
CUCGGAUUUCCGUGAUCUCCACUGGUCCGACGCUCCAAGCCCGGCGGCGGCAGUCCG
UGCUGAACCUGAUGACCCACAGCGUGAACCAGGGCCAAAACAUUCACCGCAAGACUA
CCGCAUCCACCCGGAAAGUGUCCCUGGCACCUCAAGCGAAUCUUACCGAGCUCGACA
UCUACUCCCGGAGACUGUCGCAGGAAACCGGGCUCGAAAUUUCCGAAGAAAUCAACG
AGGAGGAUCUGAAAGAGUGCUUCUUCGACGAUAUGGAGUCGAUACCCGCCGUGACG
ACUUGGAACACUUAUCUGCGGUACAUCACUGUGCACAAGUCAUUGAUCUUCGUGCUG
AUUUGGUGCCUGGUGAUUUUCCUGGCCGAGGUCGCGGCCUCACUGGUGGUGCUCUGG

CUGUUGGGAAACACGCCUCUGCAAGACAAGGGAAACUCCACGCACUCGAGAAACAAC
AGCUAUGCCGUGAUUAUCACUUCCACCUCCUCUUAUUACGUGUUCUACAUCUACGUC
GGAGUGGCGGAUACCCUGCUCGCGAUGGGUUUCUUCAGAGGACUGCCGCUGGUCCAC
ACCUUGAUCACCGUCAGCAAGAUUCUUCACCACAAGAUGUUGCAUAGCGUGCUGCAG
GCCCCCAUGUCCACCCUCAACACUCUGAAGGCCGGAGGCAUUCUGAACAGAUUCUCC
AAGGACAUCGCUAUCCUGGACGAUCUCCUGCCGCUUACCAUCUUUGACUUCAUCCAG
CUGCUGCUGAUCGUGAUUGGAGCAAUCGCAGUGGUGGCGGUGCUGCAGCCUUACAUU
UUCGUGGCCACUGUGCCGGUCAUUGUGGCGUUCAUCAUGCUGCGGGCCUACUUCCUC
CAAACCAGCCAGCAGCUGAAGCAACUGGAAUCCGAGGGACGAUCCCCCAUCUUCACU
CACCUUGUGACGUCGUUGAAGGGACUGUGGACCCUCCGGGCUUUCGGACGGCAGCCC
UACUUCGAAACCCUCUUCCACAAGGCCCUGAACCUCCACACCGCCAAUUGGUUCCUG
UACCUGUCCACCCUGCGGUGGUUCCAGAUGCGCAUCGAGAUGAUUUUCGUCAUCUUC
UUCAUCGCGGUCACAUUCAUCAGCAUCCUGACUACCGGAGAGGGAGAGGGACGGGUC
GGAAUAAUCCUGACCCUCGCCAUGAACAUUAUGAGCACCCUGCAGUGGGCAGUGAAC
AGCUCGAUCGACGUGGACAGCCUGAUGCGAAGCGUCAGCCGCGUGUUCAAGUUCAUC
GACAUGCCUACUGAGGGAAAACCCACUAAGUCCACUAAGCCCUACAAAAAUGGCCAG
CUGAGCAAGGUCAUGAUCAUCGAAAACUCCCACGUGAAGAAGGACGAUAUUUGGCCC
UCCGGAGGUCAAAUGACCGUGAAGGACCUGACCGCAAAGUACACCGAGGGAGGAAAC
GCCAUUCUCGAAAACAUCAGCUUCUCCAUUUCGCCGGGACAGCGGGUCGGCCUUCUC
GGGCGGACCGGUUCCGGGAAGUCAACUCUGCUGUCGGCUUUCCUCCGGCUGCUGAAU
ACCGAGGGGGAAAUCCAAAUUGACGGCGUGUCUUGGGAUUCCAUUACUCUGCAGCAG
UGGCGGAAGGCCUUCGGCGUGAUCCCCCAGAAGGUGUUCAUCUUCUCGGGUACCUUC
CGGAAGAACCUGGAUCCUUACGAGCAGUGGAGCGACCAAGAAAUCUGGAAGGUCGCC
GACGAGGUCGGCCUGCGCUCCGUGAUUGAACAAUUUCCUGGAAAGCUGGACUUCGUG
CUCGUCGACGGGGGAUGUGUCCUGUCGCACGGACAUAAGCAGCUCAUGUGCCUCGCA
CGGUCCGUGCUCUCCAAGGCCAAGAUUCUGCUGCUGGACGAACCUUCGGCCCACCUG
GAUCCGGUCACCUACCAGAUCAUCAGGAGGACCCUGAAGCAGGCCUUUGCCGAUUGC
ACCGUGAUUCUCUGCGAGCACCGCAUCGAGGCCAUGCUGGAGUGCCAGCAGUUCCUG
GUCAUCGAGGAGAACAAGGUCCGCCAAUACGACUCCAUUCAAAAGCUCCUCAACGAG
CGGUCGCUGUUCAGACAAGCUAUUUCACCGUCCGAUAGAGUGAAGCUCUUCCCGCAU
CGGAACAGCUCAAAGUGCAAAUCGAAGCCGCAGAUCGCAGCCUUGAAGGAAGAGACU
GAGGAAGAGGUGCAGGACACCCGGCUUUAACGGGUGGCAUCCCUGUGACCCCUCCCC

AGUGCCUCUCCUGGCCCUGGAAGUUGCCACUCCAGUGCCCACCAGCCUUGUCCUAAU
AAAAUUAAGUUGCAUCAAGCU (SEQ ID NO: 28)

**Table A. mRNA Structural Elements**

| **Structural Element** | **Description** | **Sequence Coordinates** |
|---|---|---|
| Cap Structure | 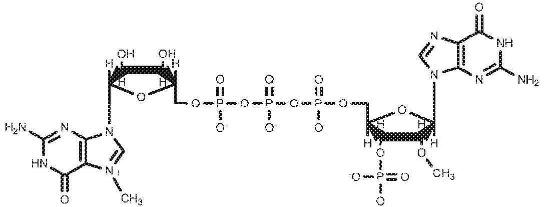 | 7mG is attached to the nucleotide in position 1 of the CFTR mRNA where the first nucleotide (underlined) is methylated at the 2' position |
| 5' UTR | GGAC...CACG | 1-140 |
| Start Codon hCFTR | AUG (Bold) | 141-143 |
| Stop Codon hCFTR | UAA (Bold) | 4581-4583 |
| 3' UTR | CGGG ... AGCU | 4584-4688 |
| Poly A tail | (A)ₓ, x=200-1000* | 4689-ff |

In another embodiment, the mRNA encoding CFTR has the following sequence and structural elements:

GGACAGAUCGCCUGGAGACGCCAUCCACGCUGUUUUGACCUCCAUAGA
AGACACCGGGACCGAUCCAGCCUCCGCGGCCGGGAACGGUGCAUUGGAACGCGGAUU
CCCCGUGCCAAGAGUGACUCACCGUCCUUGACACGAUGCAGCGGUCCCCGCUCGAAA
AGGCCAGUGUCGUGUCCAAACUCUUCUUCUCAUGGACUCGGCCUAUCCUUAGAAAGG
GGUAUCGGCAGAGGCUUGAGUUGUCUGACAUCUACCAGAUCCCCUCGGUAGAUUCGG
CGGAUAACCUCUCGGAGAAGCUCGAACGGGAAUGGGACCGCGAACUCGCGUCUAAGA
AAAACCCGAAGCUCAUCAACGCACUGAGAAGGUGCUUCUUCUGGCGGUUCAUGUUCU
ACGGUAUCUUCUUGUAUCUCGGGGAGGUCACAAAAGCAGUCCAACCCCUGUUGUUGG
GUCGCAUUAUCGCCUCGUACGACCCCGAUAACAAAGAAGAACGGAGCAUCGCGAUCU

ACCUCGGGAUCGGACUGUGUUUGCUUUUCAUCGUCAGAACACUUUUGUUGCAUCCAG
CAAUCUUCGGCCUCCAUCACAUCGGUAUGCAGAUGCGAAUCGCUAUGUUUAGCUUGA
UCUACAAAAAGACACUGAAACUCUCGUCGCGGGUGUUGGAUAAGAUUUCCAUCGGUC
AGUUGGUGUCCCUGCUUAGUAAUAACCUCAACAAAUUCGAUGAGGGACUGGCGCUG
GCACAUUUCGUGUGGAUUGCCCCGUUGCAAGUCGCCCUUUUGAUGGGCCUUAUUUGG
GAGCUGUUGCAGGCAUCUGCCUUUUGUGGCCUGGGAUUUCUGAUUGUGUUGGCAUU
GUUUCAGGCUGGGCUUGGGCGGAUGAUGAUGAAGUAUCGCGACCAGAGAGCGGGUA
AAAUCUCGGAAAGACUCGUCAUCACUUCGGAAAUGAUCGAAAACAUCCAGUCGGUCA
AAGCCUAUUGCUGGGAAGAAGCUAUGGAGAAGAUGAUUGAAAACCUCCGCCAAACU
GAGCUGAAACUGACCCGCAAGGCGGCGUAUGUCCGGUAUUUCAAUUCGUCAGCGUUC
UUCUUUUCCGGGUUCUUCGUUGUCUUUCUCUCGGUUUUGCCUUAUGCCUUGAUUAAG
GGGAUUAUCCUCCGCAAGAUUUUCACCACGAUUUCGUUCUGCAUUGUAUUGCGCAUG
GCAGUGACACGGCAAUUUCCGUGGGCCGUGCAGACAUGGUAUGACUCGCUUGGAGCG
AUCAACAAAAUCCAAGACUUCUUGCAAAAGCAAGAGUACAAGACCCUGGAGUACAAU
CUUACUACUACGGAGGUAGUAAUGGAGAAUGUGACGGCUUUUUGGGAAGAGGGUUU
UGGAGAACUGUUUGAGAAAGCAAAGCAGAAUAACAACAACCGCAAGACCUCAAAUG
GGGACGAUUCCCUGUUUUUCUCGAACUUCUCCCUGCUCGGAACACCCGUGUUGAAGG
ACAUCAAUUUCAAGAUUGAGAGGGGACAGCUUCUCGCGGUAGCGGGAAGCACUGGU
GCGGGAAAAACUAGCCUCUUGAUGGUGAUUAUGGGGGAGCUUGAGCCCAGCGAGGG
GAAGAUUAAACACUCCGGGCGUAUCUCAUUCUGUAGCCAGUUUUCAUGGAUCAUGCC
CGGAACCAUUAAAGAGAACAUCAUUUUCGGAGUAUCCUAUGAUGAGUACCGAUACA
GAUCGGUCAUUAAGGCGUGCCAGUUGGAAGAGGACAUUUCUAAGUUCGCCGAGAAG
GAUAACAUCGUCUUGGGAGAAGGGGGUAUUACAUUGUCGGGAGGGCAGCGAGCGCG
GAUCAGCCUCGCGAGAGCGGUAUACAAAGAUGCAGAUUUGUAUCUGCUUGAUUCACC
GUUUGGAUACCUCGACGUAUUGACAGAAAAAGAAAUCUUCGAGUCGUGCGUGUGUA
AACUUAUGGCUAAUAAGACGAGAAUCCUGGUGACAUCAAAAAUGGAACACCUUAAG
AAGGCGGACAAGAUCCUGAUCCUCCACGAAGGAUCGUCCUACUUUUACGGCACUUUC
UCAGAGUUGCAAAACUUGCAGCCGGACUUCUCAAGCAAACUCAUGGGGUGUGACUCA
UUCGACCAGUUCAGCGCGGAACGGCGGAACUCGAUCUUGACGGAAACGCUGCACCGA
UUCUCGCUUGAGGGUGAUGCCCCGGUAUCGUGGACCGAGACAAAGAAGCAGUCGUUU
AAGCAGACAGGAGAAUUUGGUGAGAAAAGAAAGAACAGUAUCUUGAAUCCUAUUAA
CUCAAUUCGCAAGUUCUCAAUCGUCCAGAAAACUCCACUGCAGAUGAAUGGAAUUGA

AGAGGAUUCGGACGAACCCCUGGAGCGCAGGCUUAGCCUCGUGCCGGAUUCAGAGCA
AGGGGAGGCCAUUCUUCCCCGGAUUUCGGUGAUUUCAACCGGACCUACACUUCAGGC
GAGGCGAAGGCAAUCCGUGCUCAACCUCAUGACGCAUUCGGUAAACCAGGGGCAAAA
CAUUCACCGCAAAACGACGGCCUCAACGAGAAAAGUGUCACUUGCACCCCAGGCGAA
UUUGACUGAACUCGACAUCUACAGCCGUAGGCUUUCGCAAGAAACCGGACUUGAGAU
CAGCGAAGAAAUCAAUGAAGAAGAUUUGAAAGAGUGUUUCUUUGAUGACAUGGAAU
CAAUCCCAGCGGUGACAACGUGGAACACAUACUUGCGUUACAUCACGGUGCACAAGU
CCUUGAUUUUCGUCCUCAUCUGGUGUCUCGUGAUCUUUCUCGCUGAGGUCGCAGCGU
CACUUGUGGUCCUCUGGCUGCUUGGUAAUACGCCCUUGCAAGACAAAGGCAAUUCUA
CACACUCAAGAAACAAUUCCUAUGCCGUGAUUAUCACUUCUACAAGCUCGUAUUACG
UGUUUUACAUCUACGUAGGAGUGGCCGACACUCUGCUCGCGAUGGGUUUCUUCCGAG
GACUCCCACUCGUUCACACGCUUAUCACUGUCUCCAAGAUUCUCCACCAUAAGAUGC
UUCAUAGCGUACUGCAGGCUCCCAUGUCCACCUUGAAUACGCUCAAGGCGGGAGGUA
UUUUGAAUCGCUUCUCAAAAGAUAUUGCAAUUUUGGAUGACCUUCUGCCCCUGACGA
UCUUCGACUUCAUCCAGUUGUUGCUGAUCGUGAUUGGGGCUAUUGCAGUAGUCGCU
GUCCUCCAGCCUUACAUUUUUGUCGCGACCGUUCCGGUGAUCGUGGCGUUUAUCAUG
CUGCGGGCCUAUUUCUUGCAGACGUCACAGCAGCUUAAGCAACUGGAGUCUGAAGGG
AGGUCGCCUAUCUUUACGCAUCUUGUGACCAGUUUGAAGGGAUUGUGGACGUUGCG
CGCCUUUGGCAGGCAGCCCUACUUUGAAACACUGUUCCACAAAGCGCUGAAUCUCCA
UACGGCAAAUUGGUUUUUGUAUUUGAGUACCCUCCGAUGGUUUCAGAUGCGCAUUG
AGAUGAUUUUUGUGAUCUUCUUUAUCGCGGUGACUUUUAUCUCCAUCUUGACCACG
GGAGAGGGCGAGGGACGGGUCGGUAUUAUCCUGACACUCGCCAUGAACAUUAUGAG
CACUUUGCAGUGGGCAGUGAACAGCUCGAUUGAUGUGGAUAGCCUGAUGAGGUCCG
UUUCGAGGGUCUUUAAGUUCAUCGACAUGCCGACGGAGGGAAAGCCCACAAAAAGU
ACGAAACCCUAUAAGAAUGGGCAAUUGAGUAAGGUAAUGAUCAUCGAGAACAGUCA
CGUGAAGAAGGAUGACAUCUGGCCUAGCGGGGGUCAGAUGACCGUGAAGGACCUGA
CGGCAAAAUACACCGAGGGAGGGAACGCAAUCCUUGAAAACAUCUCGUUCAGCAUUA
GCCCCGGUCAGCGUGUGGGGUUGCUCGGGAGGACCGGGUCAGGAAAAUCGACGUUGC
UGUCGGCCUUCUUGAGACUUCUGAAUACAGAGGGUGAGAUCCAGAUCGACGGCGUU
UCGUGGGAUAGCAUCACCUUGCAGCAGUGGCGGAAAGCGUUUGGAGUAAUCCCCCAA
AAGGUCUUUAUCUUUAGCGGAACCUUCCGAAAGAAUCUCGAUCCUUAUGAACAGUG
GUCAGAUCAAGAGAUUUGGAAAGUCGCGGACGAGGUUGGCCUUCGGAGUGUAAUCG

AGCAGUUUCCGGGAAAACUCGACUUUGUCCUUGUAGAUGGGGGAUGCGUCCUGUCGC
AUGGGCACAAGCAGCUCAUGUGCCUGGCGCGAUCCGUCCUCUCUAAAGCGAAAAUUC
UUCUCUUGGAUGAACCUUCGGCCCAUCUGGACCCGGUAACGUAUCAGAUCAUCAGAA
GGACACUUAAGCAGGCGUUUGCCGACUGCACGGUGAUUCUCUGUGAGCAUCGUAUCG
AGGCCAUGCUCGAAUGCCAGCAAUUUCUUGUCAUCGAAGAGAAUAAGGUCCGCCAGU
ACGACUCCAUCCAGAAGCUGCUUAAUGAGAGAUCAUUGUUCCGGCAGGCGAUUUCAC
CAUCCGAUAGGGUGAAACUUUUUCCACACAGAAAUUCGUCGAAGUGCAAGUCCAAAC
CGCAGAUCGCGGCCUUGAAAGAAGAGACUGAAGAAGAAGUUCAAGACACGCGUCUU
UAACGGGUGGCAUCCCUGUGACCCCUCCCCAGUGCCUCUCCUGGCCCUGGAAGUUGC
CACUCCAGUGCCCACCAGCCUUGUCCUAAUAAAAUUAAGUUGCAUCAAGCU (SEQ ID
NO: 29)

**Table B. mRNA Structural Elements**

| **Structural Element** | **Description** | **Sequence Coordinates** |
|---|---|---|
| Cap Structure | 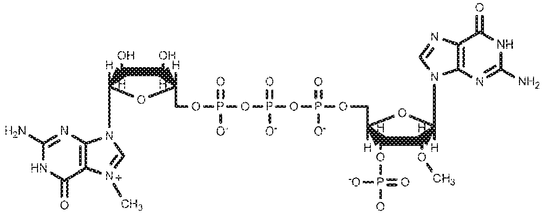 | 7mG is attached to the nucleotide in position 1 of the CFTR mRNA where the first nucleotide (underlined) is methylated at the 2' position |
| 5' UTR | GGAC...CACG | 1-140 |
| Start Codon hCFTR | AUG (Bold) | 141-143 |
| Stop Codon hCFTR | UAA (Bold) | 4581-4583 |
| 3' UTR | CCTGG...AGCU | 4584-4689 |
| Poly A tail | (A)ₓ, x=200-1000* | 4690-ff |

In another embodiment, the mRNA encoding CFTR has the following sequence and structural elements:

GGACAGAUCGCCUGGAGACGCCAUCCACGCUGUUUUGACCUCCAUAGA
AGACACCGGGACCGAUCCAGCCUCCGCGGCCGGGAACGGUGCAUUGGAACGCGGAUU
CCCCGUGCCAAGAGUGACUCACCGUCCUUGACACGAUGCAGAGGAGCCCACUGGAGA
AAGCCUCCGUGGUGAGUAAACUCUUUUUUAGUUGGACCAGACCCAUCCUGCGAAAAG
GAUACAGGCAGCGCCUCGAGUUGUCUGAUAUCUACCAGAUUCCUUCUGUGGACUCAG
CUGACAAUUUGAGUGAGAAGCUGGAGCGGGAGUGGGAUAGAGAGCUGGCGAGCAAA
AAAAACCCCAAGCUUAUCAAUGCUCUGCGCCGCUGCUUUUUCUGGAGGUUCAUGUUU
UAUGGGAUCUUCCUGUACCUGGGGGAGGUCACCAAAGCUGUUCAGCCGCUCCUUCUU
GGCCGCAUCAUCGCCAGCUAUGACCCUGAUAAUAAAGAAGAAAGGUCUAUUGCUAUU
UAUCUGGGAAUUGGCCUCUGCUUGCUCUUCAUCGUCCGCACCCUUCUGCUGCACCCU
GCCAUUUUUGGCCUUCACCACAUCGGCAUGCAAAUGAGAAUUGCCAUGUUCUCCCUC
AUUUACAAAAAGACCCUGAAACUUUCCUCAAGAGUGUUAGAUAAAAUAUCCAUUGG
UCAGCUGGUCAGCCUGCUGUCCAACAAUCUUAACAAAUUUGAUGAAGGCUUGGCGCU
GGCCCACUUCGUGUGGAUUGCACCUCUGCAGGUGGCCCUGUUGAUGGGACUUAUAUG
GGAGCUGCUUCAAGCCUCUGCUUUCUGUGGGCUGGGCUUUUUGAUUGUACUGGCACU
UUUUCAGGCUGGGCUCGGAAGAAUGAUGAUGAAAUACAGAGAUCAGCGGGCCGGGA
AGAUUUCAGAGCGACUUGUGAUCACCAGUGAAAUGAUUGAAAAUAUUCAGAGCGUG
AAAGCCUACUGCUGGGAAGAAGCCAUGGAGAAGAUGAUUGAGAACCUGAGGCAGAC
AGAGCUCAAGCUCACUCGGAAGGCUGCUUAUGUUCGCUAUUUCAACAGCAGCGCCUU
CUUCUUCAGUGGCUUCUUUGUUGUCUUCCUGUCUGUUCUGCCAUAUGCACUGAUAAA
AGGCAUUAUUUUACGAAAGAUCUUCACCACCAUCAGUUUUUGCAUCGUUCUCAGGAU
GGCCGUCACAAGACAGUUCCCCUGGGCUGUGCAGACCUGGUACGAUUCCUUGGGGGC
CAUCAACAAGAUUCAAGAUUUCUUGCAAAAACAAGAAUAUAAAACUUUAGAAUACA
ACCUCACCACCACUGAAGUGGUCAUGGAAAAUGUGACAGCCUUUUGGGAGGAGGGU
UUUGGAGAAUUGUUCGAGAAGGCAAAGCAGAAUAACAACAACAGGAAGACGAGCAA
UGGGGACGACUCUCUCUUCUUCAGCAACUUUUCACUGCUCGGGACCCCUGUGUUGAA
AGAUAUAAACUUCAAGAUCGAGAGGGGCCAGCUCUUGGCUGUGGCAGGCUCCACUGG
AGCUGGUAAAACAUCUCUUCUCAUGGUGAUCAUGGGGGAACUGGAGCCUUCCGAAG
GAAAAAUCAAGCACAGUGGGAGAAUCUCAUUCUGCAGCCAGUUUUCCUGGAUCAUGC
CCGGCACCAUUAAGGAAAACAUCAUAUUUGGAGUGUCCUAUGAUGAGUACCGCUACC
GGUCAGUCAUCAAAGCCUGUCAGUUGGAGGAGGACAUCUCCAAGUUUGCAGAGAAA
GACAACAUUGUGCUUGGAGAGGGGGGUAUCACUCUUUCUGGAGGACAAAGAGCCAG

GAUCUCUUUGGCCCGGGCAGUCUACAAGGAUGCAGACCUCUACUUGUUGGACAGUCC
CUUCGGCUACCUCGACGUGCUGACUGAAAAAGAAUUUUGAAAGCUGUGUGUGCA
AACUGAUGGCAAACAAGACCAGGAUUCUUGUCACCAGCAAGAUGGAACAUCUGAAG
AAAGCGGACAAAAUUCUGAUUCUGCAUGAAGGGAGCUCCUACUUCUAUGGAACAUU
UAGCGAGCUUCAGAACCUACAGCCAGACUUCUCCUCCAAAUUAAUGGGCUGUGACUC
CUUCGACCAGUUCUCUGCAGAAAGAAGAAACUCUAUACUCACAGAGACCCUCCACCG
CUUCUCCCUUGAGGGAGAUGCCCCAGUUUCUUGGACAGAAACCAAGAAGCAGUCCUU
UAAGCAGACUGGCGAGUUUGGUGAAAAGAGGAAAAAUUCAAUUCUCAAUCCAAUUA
ACAGUAUUCGCAAGUUCAGCAUUGUCCAGAAGACACCCCUCCAGAUGAAUGGCAUCG
AAGAAGAUAGUGACGAGCCGCUGGAGAGACGGCUGAGUCUGGUGCCAGAUUCAGAA
CAGGGGGAGGCCAUCCUGCCCCGGAUCAGCGUCAUUUCCACAGGCCCCACAUUACAA
GCACGGCGCCGGCAGAGUGUUUUAAAUCUCAUGACCCAUUCAGUGAACCAGGGCCAA
AAUAUCCACAGGAAGACUACAGCUUCUACCCGGAAAGUGUCUCUGGCCCCUCAGGCC
AAUCUGACCGAGCUGGACAUCUACAGCAGGAGGCUCUCCCAGGAAACAGGGCUGGAA
AUAUCUGAAGAGAUUAAUGAAGAGGAUCUUAAAGAGUGCUUCUUUGAUGACAUGGA
GAGCAUCCCCGCGGUGACCACAUGGAACACCUACCUUAGAUAUAUUACUGUCCACAA
GAGCCUCAUAUUUGUCCUCAUCUGGUGCCUGGUUAUUUUCCUCGCUGAGGUGGCGGC
CAGUCUUGUUGUGCUCUGGCUGCUGGGCAACACUCCUCUCCAGGACAAGGGCAAUAG
UACUCACAGCAGAAAUAAUUCUUAUGCCGUCAUCAUUACAAGCACCUCCAGCUACUA
CGUGUUCUACAUCUAUGUGGGCGUGGCUGACACCCUCCUGGCCAUGGGUUUCUUCCG
GGGCCUGCCUUUGGUGCACACCCUCAUCACAGUGUCAAAAAUUCUGCACCAUAAAAU
GCUUCAUUCUGUCCUGCAGGCACCCAUGAGCACUUUGAACACAUUGAAGGCUGGCGG
CAUCCUCAACAGAUUUUCUAAAGAUAUUGCUAUCCUGGAUGAUCUCCUCCCCCUGAC
AAUCUUUGACUUUAUCCAGCUUCUGCUGAUCGUGAUUGGAGCCAUAGCAGUGGUUG
CUGUCCUGCAGCCCUACAUUUUUGUGGCCACCGUGCCCGUGAUUGUUGCCUUUAUUA
UGCUCAGAGCUUACUUCCUGCAAACUUCUCAACAGCUCAAACAGCUAGAAUCUGAGG
GCCGGAGCCCCAUUUUUACCCACCUGGUGACUUCCCUGAAGGGACUGUGGACUCUGA
GAGCAUUCGGGCGACAGCCUUACUUUGAGACACUGUUCCACAAGGCCCUGAACUUGC
ACACUGCCAACUGGUUUCUUUACCUGAGCACACUCCGCUGGUUCCAGAUGCGGAUAG
AGAUGAUCUUCGUCAUCUUUUUUAUAGCUGUAACCUUCAUUUCUAUCCUUACAACAG
GAGAAGGAGAGGGCAGGGUGGGAAUCAUCCUCACGCUGGCUAUGAACAUAAUGUCC
ACCUUGCAGUGGGCCGUGAAUUCCAGUAUAGAUGUGGAUUCUCUAAUGAGGAGUGU

CUCCCGGGUGUUUAAAUUCAUUGAUAUGCCUACUGAGGGGAAACCCACCAAGUCAAC
AAAACCUUAUAAGAAUGGACAGCUGAGCAAGGUGAUGAUAAUUGAGAACAGCCACG
UGAAGAAGGAUGACAUUUGGCCCAGCGGGGGCCAGAUGACUGUGAAGGACCUGACG
GCCAAGUACACCGAAGGUGGAAAUGCCAUUUUGGAAAACAUCAGCUUCUCAAUCUCU
CCUGGGCAGAGAGUUGGAUUGCUGGGUCGCACGGGCAGCGGCAAAUCAACCCUGCUC
AGUGCCUUCCUUCGGCUCCUGAAUACAGAAGGCGAAAUCCAAAUUGACGGGGUGAGC
UGGGACAGCAUCACCCUGCAGCAGUGGAGAAAAGCAUUUGGGGUCAUUCCACAGAAA
GUUUUCAUCUUCUCUGGCACUUUCAGAAAGAACCUGGACCCCUAUGAGCAGUGGAGC
GACCAGGAGAUCUGGAAGGUUGCAGAUGAAGUUGGCCUGCGGAGUGUGAUAGAACA
AUUUCCUGGCAAGCUGGAUUUUGUGCUGGUAGAUGGAGGCUGCGUGCUGUCCCACG
GCCACAAACAGCUGAUGUGCCUCGCCCGCUCCGUUCUUUCAAAGGCCAAAAUCUUGC
UUUUGGAUGAGCCCAGUGCUCACCUUGACCCAGUGACCUAUCAGAUAAUCCGCAGGA
CCUUAAAGCAAGCUUUUGCCGACUGCACCGUCAUACUGUGUGAGCACCGGAUUGAAG
CAAUGCUGGAAUGCCAGCAGUUUCUGGUGAUCGAGGAGAAUAAGGUCCGGCAGUAC
GACAGCAUCCAGAAGUUGUUGAAUGAGCGCAGCCUUUUCCGCCAGGCCAUCUCCCCA
UCUGACAGAGUCAAGCUGUUUCCACAUAGGAACUCCUCUAAGUGCAAGUCCAAGCCC
CAGAUCGCUGCCCUCAAGGAGGAAACUGAGGAAGAGGUGCAGGAUACCCGCCUGUGA
CGGGUGGCAUCCCUGUGACCCCUCCCCAGUGCCUCUCCUGGCCCUGGAAGUUGCCAC
UCCAGUGCCCACCAGCCUUGUCCUAAUAAAAUUAAGUUGCAUCAAGCU (SEQ ID NO:
30)

**Table C. mRNA Structural Elements**

| **Structural Element** | **Description** | **Sequence Coordinates** |
|---|---|---|
| Cap Structure | 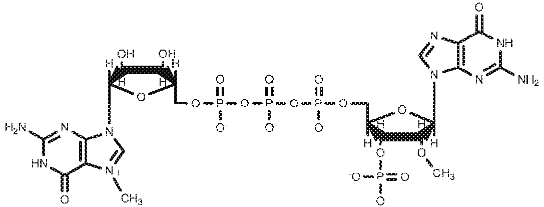 | 7mG is attached to the nucleotide in position 1 of the CFTR mRNA where the first nucleotide (underlined) is methylated at the 2' position |
| 5' UTR | GGAC...CACG | 1-140 |
| Start Codon hCFTR | AUG (Bold) | 141-143 |
| Stop Codon hCFTR | UGA (Bold) | 4581-4583 |
| 3' UTR | CGGG...AGCU | 4584-4688 |
| Poly A tail | (A)ₓ, x=200-1000* | 4689-ff |

### Modified mRNA

A CFTR mRNA may contain only naturally-occurring nucleotides (or unmodified nucleotides). In some embodiments, however, a suitable CFTR mRNA may contain backbone modifications, sugar modifications and/or base modifications. For example, modified nucleotides may include, but not be limited to, modified purines (adenine (A), guanine (G)) or pyrimidines (thymine (T), cytosine (C), uracil (U)), and as modified nucleotides analogues or derivatives of purines and pyrimidines, such as *e.g.* 1-methyl-adenine, 2-methyl-adenine, 2-methylthio-N-6-isopentenyl-adenine, N6-methyl-adenine, N6-isopentenyl-adenine, 2-thio-cytosine, 3-methyl-cytosine, 4-acetyl-cytosine, 5-methyl-cytosine, 2,6-diaminopurine, 1-methyl-guanine, 2-methyl-guanine, 2,2-dimethyl-guanine, 7-methyl-guanine, inosine, 1-methyl-inosine, pseudouracil (5-uracil), dihydro-uracil, 2-thio-uracil, 4-thio-uracil, 5-carboxymethylaminomethyl-2-thio-uracil, 5-(carboxyhydroxymethyl)-uracil, 5-fluoro-uracil, 5-bromo-uracil, 5-carboxymethylaminomethyl-uracil, 5-methyl-2-thio-uracil, 5-methyl-uracil, N-uracil-5-oxyacetic acid methyl ester, 5-methylaminomethyl-uracil, 5-methoxyaminomethyl-2-thio-uracil, 5'-methoxycarbonylmethyl-uracil, 5-methoxy-uracil, uracil-5-oxyacetic acid methyl ester, uracil-5-oxyacetic acid (v), 1-methyl-pseudouracil, queosine, .beta.-D-mannosyl-queosine, wybutoxosine, and phosphoramidates, phosphorothioates, peptide nucleotides, methylphosphonates, 7-deazaguanosine, 5-methylcytosine and inosine. The preparation of such analogues is known to a person skilled in the art *e.g.,* from the U.S. Pat. No. 4,373,071, U.S. Pat. No. 4,401,796, U.S. Pat. No. 4,415,732, U.S. Pat. No. 4,458,066, U.S. Pat. No. 4,500,707, U.S. Pat. No. 4,668,777, U.S. Pat. No. 4,973,679, U.S. Pat. No. 5,047,524, U.S. Pat. No. 5,132,418, U.S. Pat. No. 5,153,319, U.S. Pat. Nos. 5,262,530 and 5,700,642.

In some embodiments, mRNAs (*e.g.,* mRNAs encoding CFTR) may contain RNA backbone modifications. Typically, a backbone modification is a modification in which the phosphates of the backbone of the nucleotides contained in the RNA are modified chemically. Exemplary backbone modifications typically include, but are not limited to, modifications from the group consisting of methylphosphonates, methylphosphoramidates, phosphoramidates, phosphorothioates (*e.g.,* cytidine 5'-O-(1-thiophosphate)), boranophosphates, positively charged guanidinium groups etc., which means by replacing the phosphodiester linkage by other anionic, cationic or neutral groups.

In some embodiments, mRNAs (*e.g.,* mRNAs encoding CFTR) may contain sugar modifications. A typical sugar modification is a chemical modification of the sugar of the nucleotides it contains including, but not limited to, sugar modifications chosen from the group consisting of 2'-deoxy-2'-fluoro-oligoribonucleotide (2'-fluoro-2'-deoxycytidine 5'-triphosphate, 2'-fluoro-2'-deoxyuridine 5'-triphosphate), 2'-deoxy-2'-deamine-oligoribonucleotide (2'-amino-2'-deoxycytidine 5'-triphosphate, 2'-amino-2'-deoxyuridine 5'-triphosphate), 2'-O-alkyloligoribonucleotide, 2'-deoxy-2'-C-alkyloligoribonucleotide (2'-O-methylcytidine 5'-triphosphate, 2'-methyluridine 5'-triphosphate), 2'-C-alkyloligoribonucleotide, and isomers thereof (2'-aracytidine 5'-triphosphate, 2'-arauridine 5'-triphosphate), or azidotriphosphates (2'-azido-2'-deoxycytidine 5'-triphosphate, 2'-azido-2'-deoxyuridine 5'-triphosphate).

In a specific embodiment of the invention, mRNAs encoding CFTR are unmodified.

### Delivery Vehicles

According to the present invention, mRNA encoding a CFTR protein (*e.g.,* a full length, fragment, or portion of a CFTR protein) as described herein may be delivered as naked mRNA (unpackaged) or via delivery vehicles. As used herein, the terms "delivery vehicle," "transfer vehicle," "nanoparticle" or grammatical equivalent, are used interchangeably.

Delivery vehicles can be formulated in combination with one or more additional nucleic acids, carriers, targeting ligands or stabilizing reagents, or in pharmacological compositions where it is mixed with suitable excipients. Techniques for formulation and administration of drugs may be found in "Remington's Pharmaceutical Sciences," Mack Publishing Co., Easton, Pa., latest edition. A particular delivery vehicle is selected based upon its ability to facilitate the transfection of a nucleic acid to a target cell.

According to various embodiments, suitable delivery vehicles include, but are not limited to polymer based carriers, such as polyethyleneimine (PEI), lipid nanoparticles (LNPs) and liposomes, nanoliposomes, ceramide-containing nanoliposomes, proteoliposomes, both natural and synthetically-derived exosomes, natural, synthetic and semi-synthetic lamellar bodies, nanoparticulates, calcium phosphor-silicate nanoparticulates, calcium phosphate nanoparticulates, silicon dioxide nanoparticulates, nanocrystalline particulates, semiconductor nanoparticulates, poly(D-arginine), sol-gels, nanodendrimers, starch-based delivery systems, micelles, emulsions, niosomes, multi-domain-block polymers (vinyl polymers, polypropyl acrylic acid polymers, dynamic polyconjugates), dry powder formulations, plasmids, viruses, calcium phosphate nucleotides, aptamers, peptides and other vectorial tags.

### Liposomal delivery vehicles

In some embodiments, a suitable delivery vehicle is a liposomal delivery vehicle, *e.g.,* a lipid nanoparticle (LNP) or liposome. In some embodiments, liposomes may comprise one or more cationic lipids. In some embodiments, a liposome comprises one or more cationic lipids, one or more non-cationic lipids, one or more cholesterol-based lipids and one or more PEG-modified lipids. In some embodiments, a liposome comprises one or more cationic lipids, one or more non-cationic lipids, and one or more PEG-modified lipids. In some embodiments, a liposome comprises no more than four distinct lipid components. In some embodiments, a liposome comprises no more than three distinct lipid components. In some embodiments, one distinct lipid component is a sterol-based cationic lipid.

As used herein, the phrase "cationic lipid" refers to any of a number of lipid species that have a net positive charge at a selected pH, such as physiological pH. Several cationic lipids have been described in the literature, many of which are commercially available. An example of suitable cationic lipids for use in the compositions and methods of the invention include those described in international patent publications WO 2010/053572 (for example, C12-200 described at paragraph [00225]) and WO 2012/170930. In certain embodiments, the compositions and methods of the invention employ a lipid nanoparticles comprising an ionizable cationic lipid described in U.S. provisional patent application 61/617,468, filed March 29, 2012, such as, *e.g.,* (15Z, 18Z)-N,N-dimethyl-6-(9Z, 12Z)-octadeca-9, 12-dien-1-yl)tetracosa-15,18-dien-1-amine (HGT5000), (15Z, 18Z)-N,N-dimethyl-6-((9Z, 12Z)-octadeca-9,12-dien-1-yl)tetracosa-4,15,18-trien-l-amine (HGTS5001), and (15Z,18Z)-N,N-dimethyl-6-((9Z, 12Z)-octadeca-9, 12-dien-1-yl)tetracosa-5, 15, 18-trien-1-amine (HGT5002).

In some embodiments, provided liposomes include a cationic lipid described in international patent publications WO 2013/063468 and WO 2015/061467.

In particular embodiments, provided liposomes include a cationic lipid cKK-E12, or (3,6-bis(4-(bis(2-hydroxydodecyl)amino)butyl)piperazine-2,5-dione, OF-00, OF-01, OF-02, or OF-*03* (*see, e.g.,* Fenton, Owen S., et al. "Bioinspired Alkenyl Amino Alcohol Ionizable Lipid Materials for Highly Potent In Vivo mRNA Delivery." Advanced materials (2016)).

In some embodiments, suitable cationic lipids may be N-[1-(2,3-dioleyloxy)propyl]-N,N,N-trimethylammonium chloride or "DOIMA" (Felgner et al. (Proc. Nat'1 Acad. Sci. 84, 7413 (1987); U.S. Pat. No. 4,897,355). DOTMA can be formulated alone or can be combined with the neutral lipid, dioleoylphosphatidyl-ethanolamine or "DOPE" or other cationic or non-cationic lipids into a liposomal transfer vehicle or a lipid nanoparticle, and such liposomes can be used to enhance the delivery of nucleic acids into target cells. Other suitable cationic lipids include, for example, 5-carboxyspermylglycinedioctadecylamide or "DOGS," 2,3-dioleyloxy-N-[2(spermine-carboxamido)ethyl]-N,N-dimethyl-1-propanaminium or "DOSPA" (Behr et al. Proc. Nat.'l Acad. Sci. 86, 6982 (1989); U.S. Pat. No. 5,171,678; U.S. Pat. No. 5,334,761), 1,2-Dioleoyl-3-Dimethylammonium-Propane or "DODAP", 1,2-Dioleoyl-3-Trimethylammonium-Propane or "DOTAP".

Additional exemplary cationic lipids also include 1,2-distearyloxy-N,N-dimethyl-3-aminopropane or "DSDMA", 1,2-dioleyloxy-N,N-dimethyl-3-aminopropane or "DODMA," 1 ,2-dilinoleyloxy-N,N-dimethyl-3-aminopropane or "DLinDMA," 1,2-dilinolenyloxy-N,N-dimethyl-3-aminopropane or "DLenDMA," N-dioleyl-N,N-dimethylammonium chloride or "DODAC," N,N-distearyl-N,N-dimethylarnrnonium bromide or "DDAB," N-(1,2-dimyristyloxyprop-3-yl)-N,N-dimethyl-N-hydroxyethyl ammonium bromide or "DMRIE," 3-dimethylamino-2-(cholest-5-en-3-beta-oxybutan-4-oxy)-1-(cis,cis-9,12-octadecadienoxy)propane or "CLinDMA," 2-[5'-(cholest-5-en-3-beta-oxy)-3'-oxapentoxy)-3-dimethy 1-1-(cis,cis-9', 1-2'-octadecadienoxy)propane or "CpLinDMA," N,N-dimethyl-3,4-dioleyloxybenzylamine or"DMOBA," 1,2-N,N'-dioleylcarbamyl-3-dimethylaminopropane or "DOcarbDAP," 2,3-Dilinoleoyloxy-N,N-dimethylpropylamine or "DLinDAP," 1,2-N,N'-Dilinoleylcarbamyl-3-dimethylaminopropane or "DLincarbDAP," 1,2-Dilinoleoylcarbamyl-3-dimethylaminopropane or "DLinCDAP," 2,2-dilinoley1-4-dimethylaminomethyl-[1,3]-dioxolane or "DLinDMA," 2,2-dilinoleyl-4-dimethylaminoethyl-[1,3]-dioxolane or "DLin-K-XTC2-DMA," and 2-(2,2-di((9Z,12Z)-octadeca-9,1 2-dien-1-yl)-1,3-dioxolan-4-yl)-N,N-dimethylethanamine (DLin-KC2-DMA)) (*See* WO 2010/042877; Semple et al., Nature Biotech. 28: 172-176 (2010)), or mixtures thereof. (Heyes, J., et al.., J Controlled Release 107: 276-287 (2005); Morrissey, DV., et al., Nat. Biotechnol. 23(8): 1003-1007 (2005); PCT Publication WO2005/121348A1). In some embodiments, one or more of the cationic lipids comprise at least one of an imidazole, dialkylamino, or guanidinium moiety.

In some embodiments, the one or more cationic lipids may be chosen from XTC (2,2-Dilinoley1-4-dimethylaminoethy1-[1,3]-dioxolane), MC3 (((6Z,9Z,28Z,31Z)-heptatriaconta-6,9,28,31-tetraen-19-yl 4-(dimethylamino)butanoate), ALNY-100 ((3aR,5s,6aS)-N,N-dimethyl-2,2-di((9Z,12Z)-octadeca-9,12-dienyl)tetrahydro-3aH-cyclopenta[d] [1 ,3]dioxol-5-amine)), NC98-5 (4,7,13-tris(3-oxo-3-(undecylamino)propyl)-N1,N16-diundecyl-4,7,10,13-tetraazahexadecane-1,16-diamide), HGT4003 (WO 2012/170889, ICE (WO 2011/068810), and aminoalcohol lipidoids such as those disclosed in WO2010/053572.

In some embodiments, sterol-based cationic lipids may be use instead or in addition to cationic lipids described herein. Suitable sterol-based cationic lipids are dialkylamino-, imidazole-, and guanidinium-containing sterol-based cationic lipids. For example, certain embodiments are directed to a composition comprising one or more sterol-based cationic lipids comprising an imidazole, for example, the imidazole cholesterol ester or "ICE" lipid (3S, 10R, 13R, 17R)-10, 13-dimethyl-17-((R)-6-methylheptan-2-yl)-2, 3, 4, 7, 8, 9, 10, 11, 12, 13, 14, 15, 16, 17-tetradecahydro-1H-cyclopenta[a]phenanthren-3-yl 3-(1H-imidazol-4-yl)propanoate, as represented by structure (I) below. In certain embodiments, a lipid nanoparticle for delivery of RNA (*e.g.,* mRNA) encoding a functional protein may comprise one or more imidazole-based cationic lipids, for example, the imidazole cholesterol ester or "ICE" lipid (3S, 10R, 13R, 17R)-10, 13-dimethyl-17-((R)-6-methylheptan-2-yl)-2, 3, 4, 7, 8, 9, 10, 11, 12, 13, 14, 15, 16, 17-tetradecahydro-1H-cyclopenta[a]phenanthren-3-yl 3-(1H-imidazol-4-yl)propanoate, as represented by structure (I).

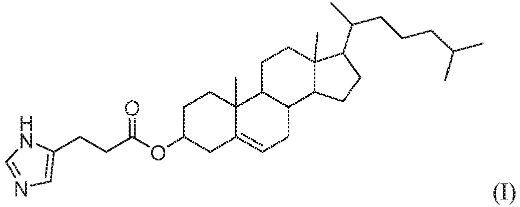

In some embodiments, the percentage of cationic lipid in a liposome may be greater than 10%, greater than 20%, greater than 30%, greater than 40%, greater than 50%, greater than 60%, or greater than 70%. In some embodiments, cationic lipid(s) constitute(s) about 30-50 % (*e.g.,* about 30-45%, about 30-40%, about 35-50%, about 35-45%, or about 35-40%) of the liposome by weight. In some embodiments, the cationic lipid *(e.g.,* ICE lipid) constitutes about 30%, about 35%, about 40 %, about 45%, or about 50% of the liposome by molar ratio.

As used herein, the phrase "non-cationic lipid" refers to any neutral, zwitterionic or anionic lipid. As used herein, the phrase "anionic lipid" refers to any of a number of lipid species that carry a net negative charge at a selected H, such as physiological pH. Non-cationic lipids include, but are not limited to, distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoylphosphatidylethanolamine (DOPE), palmitoyloleoylphosphatidylcholine (POPC), palmitoyloleoyl-phosphatidylethanolamine (POPE), dioleoyl-phosphatidylethanolamine 4-(N-maleimidomethyl)-cyclohexane-1-carboxylate (DOPE-mal), dipalmitoyl phosphatidyl ethanolamine (DPPE), dimyristoylphosphoethanolamine (DMPE), distearoyl-phosphatidyl-ethanolamine (DSPE), phosphatidylserine, sphingolipids, cerebrosides, gangliosides, 16-O-monomethyl PE, 16-O-dimethyl PE, 18-1-trans PE, 1-stearoyl-2-oleoyl-phosphatidyethanolamine (SOPE), or a mixture thereof.

In some embodiments, such non-cationic lipids may be used alone, but are preferably used in combination with other lipids, for example, cationic lipids. In some embodiments, the non-cationic lipid may comprise a molar ratio of about 5% to about 90%, or about 10 % to about 70% of the total lipid present in a liposome. In some embodiments, a non-cationic lipid is a neutral lipid, *i.e.,* a lipid that does not carry a net charge in the conditions under which the composition is formulated and/or administered. In some embodiments, the percentage of non-cationic lipid in a liposome may be greater than 5%, greater than 10%, greater than 20%, greater than 30%, or greater than 40%.

Suitable cholesterol-based cationic lipids include, for example, DC-Chol (N,N-dimethyl-N-ethylcarboxamidocholesterol), 1,4-bis(3-N-oleylamino-propyl)piperazine (Gao, et al. Biochem. Biophys. Res. Comm. 179, 280 (1991); Wolf et al. BioTechniques 23, 139 (1997); U.S. Pat. No. 5,744,335), or ICE. In some embodiments, the cholesterol-based lipid may comprise a molar ration of about 2% to about 30%, or about 5% to about 20% of the total lipid present in a liposome. In some embodiments, the percentage of cholesterol-based lipid in the lipid nanoparticle may be greater than 5%, greater than 10%, greater than 20%, greater than 30%, or greater than 40%.

The use of polyethylene glycol (PEG)-modified phospholipids and derivatized lipids such as derivatized cerarmides (PEG-CER), including N-Octanoyl-Sphingosine-1-[Succinyl(Methoxy Polyethylene Glycol)-2000] (C8 PEG-2000 ceramide) is also contemplated by the present invention, either alone or preferably in combination with other lipid formulations together which comprise the transfer vehicle *(e.g.,* a lipid nanoparticle). Contemplated PEG-modified lipids include, but are not limited to, a polyethylene glycol chain of up to 5 kDa in length covalently attached to a lipid with alkyl chain(s) of C₆-C₂₀ length. The addition of such components may prevent complex aggregation and may also provide a means for increasing circulation lifetime and increasing the delivery of the lipid-nucleic acid composition to the target tissues, (Klibanov et al. (1990) FEBS Letters, 268 (1): 235-237), or they may be selected to rapidly exchange out of the formulation *in vivo* (*see* U.S. Pat. No. 5,885,613). Particularly useful exchangeable lipids are PEG-ceramides having shorter acyl chains (e.g., C14 or C18). For example, 1,2-dimyristoyl-rac-glycero-3-methoxypolyethylene glycol-2000 (DMG-PEG2K) is a suitable lipid for use in the compositions of the invention. The PEG-modified phospholipid and derivitized lipids of the present invention may comprise a molar ratio from about 0% to about 20%, about 0.5% to about 20%, about 1% to about 15%, about 4% to about 10%, or about 2% of the total lipid present in the liposomal transfer vehicle.

The compositions of the inventions are administered to a human subject via nebulization. The liposomes encapsulating the CFTR mRNA in these compositions may comprise a PEG-modified lipid for greater stability and/or enhanced mucopenetration to gain access to the lung epithelium. For instance, the liposome may comprise a PEG-modified lipid at a molar ratio of 3% or greater of the total lipid content of the liposome. In a specific embodiment, the liposome comprises the PEG-modified lipid at a molar ratio of 4% or greater of the total lipid content of the liposome. In a particular embodiment, the liposome comprises the PEG-modified lipid at a molar ratio of 5% or greater of the total lipid content of the liposome.

According to various embodiments, the selection of cationic lipids, non-cationic lipids and/or PEG-modified lipids which comprise the lipid nanoparticle, as well as the relative molar ratio of such lipids to each other, is based upon the characteristics of the selected lipid(s), the nature of the intended target cells, the characteristics of the mRNA to be delivered. Additional considerations include, for example, the saturation of the alkyl chain, as well as the size, charge, pH, pKa, fusogenicity and toxicity of the selected lipid(s). Thus, the molar ratios may be adjusted accordingly.

In some embodiments, a suitable delivery vehicle is formulated using a polymer as a carrier, alone or in combination with other carriers including various lipids described herein. Thus, in some embodiments, liposomal delivery vehicles, as used herein, also encompass nanoparticles comprising polymers. Suitable polymers may include, for example, polyacrylates, polyalkycyanoacrylates, polylactide, polylactide-polyglycolide copolymers, polycaprolactones, dextran, albumin, gelatin, alginate, collagen, chitosan, cyclodextrins, protamine, PEGylated protamine, PLL, PEGylated PLL and polyethylenimine (PEI). When PEI is present, it may be branched PEI of a molecular weight ranging from 10 to 40 kDa, *e.g.,* 25 kDa branched PEI (Sigma #408727).

A suitable liposome for the present invention may include one or more of any of the cationic lipids, non-cationic lipids, cholesterol lipids, PEG-modified lipids and/or polymers described herein at various ratios. As non-limiting examples, a suitable liposome formulation may include a combination selected from cKK-E12, DOPE, cholesterol and DMG-PEG2K; C12-200, DOPE, cholesterol and DMG-PEG2K; HGT4003, DOPE, cholesterol and DMG-PEG2K; ICE, DOPE, cholesterol and DMG-PEG2K; or ICE, DOPE, and DMG-PEG2K.

In various embodiments, cationic lipids (*e.g.,* cKK-E12, C12-200, ICE, and/or HGT4003) constitute about 30-60 % (*e.g.,* about 30-55%, about 30-50%, about 30-45%, about 30-40%, about 35-50%, about 35-45%, or about 35-40%) of the liposome by molar ratio. In some embodiments, the percentage of cationic lipids (*e.g.,* cKK-E12, C12-200, ICE, and/or HGT4003) is or greater than about 30%, about 35%, about 40 %, about 45%, about 50%, about 55%, or about 60% of the liposome by molar ratio.

In some embodiments, the ratio of cationic lipid(s) to non-cationic lipid(s) to cholesterol-based lipid(s) to PEG-modified lipid(s) may be between about 30-60:25-35:20-30:1-15, respectively. In some embodiments, the ratio of cationic lipid(s) to non-cationic lipid(s) to cholesterol-based lipid(s) to PEG-modified lipid(s) is approximately 40:30:20:10, respectively. In some embodiments, the ratio of cationic lipid(s) to non-cationic lipid(s) to cholesterol-based lipid(s) to PEG-modified lipid(s) is approximately 40:30:25:5, respectively. In some embodiments, the ratio of cationic lipid(s) to non-cationic lipid(s) to cholesterol-based lipid(s) to PEG-modified lipid(s) is approximately 40:32:25:3, respectively. In some embodiments, the ratio of cationic lipid(s) to non-cationic lipid(s) to cholesterol-based lipid(s) to PEG-modified lipid(s) is approximately 50:25:20:5. In some embodiments, the ratio of sterol lipid(s) to non-cationic lipid(s) to PEG-modified lipid(s) is 50:45:5. In some embodiments, the ratio of sterol lipid(s) to non-cationic lipid(s) to PEG-modified lipid(s) is 50:40:10. In some embodiments, the ratio of sterol lipid(s) to non-cationic lipid(s) to PEG-modified lipid(s) is 55:40:5. In some embodiments, the ratio of sterol lipid(s) to non-cationic lipid(s) to PEG-modified lipid(s) is 55:35:10. In some embodiments, the ratio of sterol lipid(s) to non-cationic lipid(s) to PEG-modified lipid(s) is 60:35:5. In some embodiments, the ratio of sterol lipid(s) to non-cationic lipid(s) to PEG-modified lipid(s) is 60:30:10.

In some embodiments, the nominal nitrogen/phosphorus (N/P) charge ratio which refers to the positively charged nitrogens in the cationic lipid and the negatively charged phosphodiester linkages within mRNA is about between 1 and 10. In some embodiments, the N/P is about 1. In some embodiments, the N/P is about 2. In some embodiments, the N/P is about 3. In some embodiments, the N/P is about 4. In some embodiments, the N/P is about 5. In some embodiments, the N/P is about 6. In some embodiments, the N/P is about 7. In some embodiments, the N/P is about 8. In some embodiments, the N/P is about 9. In some embodiments, the N/P is about 10.

Liposomes suitable for the administration to human subjects via nebulization may have an average particle size (Zₐᵥₑ) of less than 500 nm *(e.g.,* less than about 400 nm, 300 nm, 200 nm, 175 nm, 150 nm, 125 nm, 100 nm, 75 nm, 50 nm, 25 nm, or smaller in a PBS solution). The average particle size (Zₐᵥₑ) of liposomes for use with the invention is typically less than 150 nm, more typically less than 100 nm (e.g. less than 80 nm). For instance, liposomes with an average particle size (Zₐᵥₑ) of between 40 nm and 60 nm are particularly suitable for use in the compositions of the invention.

In a specific embodiment, the liposome encapsulating the CFTR mRNA has only three lipid components. For instance, the three lipid components may be a cationic lipid, a helper lipid and a PEG-modified lipid. In some embodiments, the molar ratio of cationic lipid:helper lipid:PEG-modified lipid in each lipid nanoparticle is 50-60:35-45:5-10. In a specific embodiment, the cationic lipid is a sterol lipid (e.g. ICE).

In a particular embodiment, the three lipid components of the liposome are imidazole cholesterol ester (ICE) as the cationic lipid, 1,2-dioleoyl-sn-glycero-3-phosphoethanolamine (DOPE) as the helper lipid, and 1,2-dimyristoyl-sn-glycerol, methoxypolyethylene glycol (DMG-PEG-2K) as the PEG-modified lipid. A liposome comprising ICE, DOPE, and DMG-PEG2K has been found to be particularly suitable for use with the present invention. In some embodiments, a suitable liposome for the present invention comprises ICE and DOPE at an ICE:DOPE molar ratio of >1:1. In some embodiments, the ICE: DOPE molar ratio is <2.5:1. In some embodiments, the ICE: DOPE molar ratio is between 1:1 and 2.5:1. In some embodiments, the ICE:DOPE molar ratio is approximately 1.5:1. In some embodiments, the ICE:DOPE molar ratio is approximately 1.7:1. In some embodiments, the ICE:DOPE molar ratio is approximately 2:1. In some embodiments, a suitable liposome for the present invention comprises ICE and DMG-PEG-2K at an ICE:DMG-PEG-2K molar ratio of >10:1. In some embodiments, the ICE:DMG-PEG-2K molar ratio is <16:1. In some embodiments, the ICE:DMG-PEG-2K molar ratio is approximately 12:1. In some embodiments, the ICE:DMG-PEG-2K molar ratio is approximately 14:1. In some embodiments, a suitable liposome for the present invention comprises DOPE and DMG-PEG-2K at a DOPE: DMG-PEG-2K molar ratio of >5:1. In some embodiments, the DOPE: DMG-PEG-2K molar ratio is <11:1. In some embodiments, the DOPE: DMG-PEG-2K molar ratio is approximately 7:1. In some embodiments, the DOPE: DMG-PEG-2K molar ratio is approximately 10:1. In some embodiments, a suitable liposome for the present invention comprises ICE, DOPE and DMG-PEG-2K at a molar ratio of 50%-60% ICE, 30%-40% DOPE and 5%-10% DMG-PEG-2K. In some embodiments, a suitable liposome for the present invention comprises ICE, DOPE and DMG-PEG-2K at an ICE:DOPE:DMG-PEG-2K molar ratio of 50:45:5. In some embodiments, a suitable liposome for the present invention comprises ICE, DOPE and DMG-PEG-2K at an ICE:DOPE:DMG-PEG-2K molar ratio of 50:40:10. In some embodiments, a suitable liposome for the present invention comprises ICE, DOPE and DMG-PEG-2K at an ICE:DOPE:DMG-PEG-2K molar ratio of 55:40:5. In some embodiments, a suitable liposome for the present invention comprises ICE, DOPE and DMG-PEG-2K at an ICE:DOPE:DMG-PEG-2K molar ratio of 55:35:10. In some embodiments, a suitable liposome for the present invention comprises ICE, DOPE and DMG-PEG-2K at an ICE:DOPE:DMG-PEG-2K molar ratio of 60:35:5. In some embodiments, a suitable liposome for the present invention comprises ICE, DOPE and DMG-PEG-2K at an ICE:DOPE:DMG-PEG-2K molar ratio of 60:30:10. In a particular embodiment of the invention, the liposome encapsulating the CFTR mRNA comprises ICE, DOPE and DMG-PEG-2K as the only lipid components in a molar ratio of 60:35:5. Liposomes suitable for the administration to human subjects via nebulization may have an average size (Zₐᵥₑ) of less than 100 nm. For instance, liposomes may range from 40 nm to 60 nm in size.

The liposomal transfer vehicles for use in the compositions of the invention can be prepared by various techniques which are presently known in the art. Various methods are described in published U.S. Application No. US 2011/0244026, published U.S. Application No. US 2016/0038432 and provisional U.S. Application No. 62/580,155, filed November 1, 2017 and can be used to practice the present invention.

Briefly, the process of preparing improved CFTR-mRNA lipid liposomes includes a step of heating one or more of the solutions (*i*.*e*., applying heat from a heat source to the solution) to a temperature (or to maintain at a temperature) greater than ambient temperature, the one more solutions being the solution comprising the pre-formed lipid nanoparticles, the solution comprising the mRNA and the mixed solution comprising the lipid nanoparticle encapsulated mRNA. In some embodiments, the process includes the step of heating one or both of the mRNA solution and the pre-formed lipid nanoparticle solution, prior to the mixing step. In some embodiments, the process includes heating one or more one or more of the solution comprising the pre-formed lipid nanoparticles, the solution comprising the mRNA and the solution comprising the lipid nanoparticle encapsulated mRNA, during the mixing step. In some embodiments, the process includes the step of heating the lipid nanoparticle encapsulated mRNA, after the mixing step. In some embodiments, the temperature to which one or more of the solutions is heated (or at which one or more of the solutions is maintained) is or is greater than about 30 °C, 37 °C, 40 °C, 45 °C, 50 °C, 55 °C, 60 °C, 65 °C, or 70 °C. In some embodiments, the temperature to which one or more of the solutions is heated ranges from about 25-70 °C, about 30-70 °C, about 35-70 °C, about 40-70 °C, about 45-70 °C, about 50-70 °C, or about 60-70 °C. In some embodiments, the temperature greater than ambient temperature to which one or more of the solutions is heated is about 65 °C.

To facilitate expression of mRNA *in vivo,* delivery vehicles such as liposomes can be formulated in combination with one or more additional nucleic acids, carriers, targeting ligands or stabilizing reagents, or in pharmacological compositions where it is mixed with suitable excipients. Techniques for formulation and administration of drugs may be found in "Remington's Pharmaceutical Sciences," Mack Publishing Co., Easton, Pa., latest edition.

As used herein, the term "therapeutically effective amount" is largely determined based on the total amount of the therapeutic agent contained in the pharmaceutical compositions of the present invention. Generally, a therapeutically effective amount is sufficient to achieve a meaningful benefit to the subject (e.g., treating, modulating, curing, preventing and/or ameliorating cystic fibrosis). For example, a therapeutically effective amount may be an amount sufficient to achieve a desired therapeutic and/or prophylactic effect.

In some embodiments, the composition comprising an mRNA encoding CFTR comprises mRNA at a concentration of at least 0.1 mg/mL. In some embodiments, the composition comprising an mRNA encoding CFTR comprises mRNA at a concentration of at least 0.2 mg/mL. In some embodiments, the composition comprising an mRNA encoding CFTR comprises mRNA at a concentration of at least 0.3 mg/mL. In some embodiments, the composition comprising an mRNA encoding CFTR comprises mRNA at a concentration of at least 0.4 mg/mL. In some embodiments, the mRNA encoding a CFTR protein is at a concentration of at least 0.5 mg/mL. In some embodiments, the mRNA encoding a CFTR protein is at a concentration of at least 0.6 mg/mL. In some embodiments, the mRNA encoding a CFTR protein is at a concentration of at least 0.7 mg/mL. In some embodiments, the mRNA encoding a CFTR protein is at a concentration of at least 0.8 mg/mL. In some embodiments, the mRNA encoding a CFTR protein is at a concentration of at least 0.9 mg/mL. In some embodiments, the mRNA encoding a CFTR protein is at a concentration of at least 1.0 mg/mL. In some embodiments, the mRNA encoding a CFTR protein is at a concentration of at least 2.0 mg/mL. In some embodiments, the mRNA encoding a CFTR protein is at a concentration of at least 3.0 mg/mL. In some embodiments, the mRNA encoding a CFTR protein is at a concentration of at least 4.0 mg/mL. In some embodiments, the mRNA encoding a CFTR protein is at a concentration of at least 5.0 mg/mL. In some embodiments, the mRNA encoding a CFTR protein is at a concentration of at least 6.0 mg/mL. In some embodiments, the mRNA encoding a CFTR protein is at a concentration of at least 7.0 mg/mL. In some embodiments, the mRNA encoding a CFTR protein is at a concentration of at least 8.0 mg/mL. In some embodiments, the mRNA encoding a CFTR protein is at a concentration of at least 9.0 mg/mL. In some embodiments, the mRNA encoding a CFTR protein is at a concentration of at least 10.0 mg/mL. In some embodiments, the mRNA encoding a CFTR protein is at a concentration ranging from 0.1 mg/mL to 10.0 mg/mL. Typically, in the compositions of the invention, the mRNA encoding a CFTR protein is at a concentration ranging from 0.5 mg/mL to 0.8 mg/mL, *e.g.,* 0.6 mg/mL.

In some embodiments, the composition comprising an mRNA encoding CFTR is formulated with a diluent. In some embodiments, the diluent is selected from a group consisting of DMSO, ethylene glycol, glycerol, 2-Methyl-2,4-pentanediol (MPD), propylene glycol, sucrose, and trehalose. In some embodiments, the formulation comprises 1%, 2%, 3%, 4%, 5%, 6%, 7%, 8%, 9%, 10%, 11%, 12%, 13%, 14%, 15%, 16%, 17%, 18%, 19% or 20% diluent.

Trehalose as a diluent has been shown to be particularly effective in providing a stable composition comprising liposome-encapsulated mRNA encoding CFTR. A suitable trehalose concentration is between about 5% and about 15% (w/v), *e.g*., about 10% (w/v).

### Lyophilization

The liposomal CFTR mRNA compositions of the invention may be provided in form of a dry powder. In some embodiments, CFTR mRNA dry powder is formed by lyophilization of the mRNA-lipid complex. Applicant hereby fully incorporates by reference their earlier patent application US14/124615 filed on 06/08/2012, which was granted a U. S. patent 9,717,690 on 08.01.2017. The lyophilized dry powder is suitable for long term storage. It can be reconstituted with purified water for administration to a subject in need thereof. In certain embodiments, upon reconstitution with an appropriate rehydration media (*e*.*g*., purified water, deionized water, 5% dextrose (w/v), 10% trehalose (w/v) and/or normal saline, the reconstituted composition demonstrates pharmacological or biological activity comparable with that observed prior to lyophilization. For example, in certain embodiments, the pharmacological and biological activity of an encapsulated polynucleotide is equivalent to that observed prior to lyophilization of the composition, or alternatively demonstrates a negligible reduction in pharmacological and biological activity (*e.g.,* less than about a 1%, 2%, 2.5%, 3%, 4%, 5%, 6%, 7%, 8% 9% or 10% reduction in the biological or pharmacological activity of an encapsulated polynucleotide).

In certain embodiments, the pharmaceutical compositions comprising lyophilized nanoparticles or liposomal delivery vehicles are characterized as being stable (e.g., as stable as pharmaceutical compositions comprising an equivalent unlyophilized vehicles). Lyophilization of the lipid nanoparticles does not appreciably change or alter the particle size of the lipid nanoparticles following lyophilizaiton and/or reconstitution. For example, disclosed herein are pharmaceutical compositions comprising lyophilized lipid delivery vehicles, wherein upon reconstitution (*e.g.,* with purified water) the lipid nanoparticles do not flocculate or aggregate, or alternatively demonstrated limited or negligible flocculation or aggregation (e.g., as determined by the particle size of the reconstituted lipid nanoparticles).

Accordingly, in certain embodiments, upon reconstitution of a lyophilized lipid nanoparticle the lipid nanoparticles have a D_{V50} of less than about 500 nm *(e.g.,* less than about 300 nm, 200 nm, 150 nm, 125 nm, 120 nm, 100 nm, 75 nm, 50 nm, 25 nm, or smaller). Similarly, in certain embodiments, upon reconstitution of a lyophilized lipid nanoparticle the lipid nanoparticles have a D_{V90} of less than about 750 nm *(e.g.,* less than about 700 nm, 500 nm, 300 nm, 200 nm, 150 nm, 125 nm, 100 nm, 75 nm, 50 nm, 25 nm, or smaller).

In other embodiments, the pharmaceutical compositions comprising lyophilized lipid delivery vehicles are characterized as having a polydispersion index of less than about 1 *(e.g.,* less than 0.95, 0.9, 0.8, 0.75, 0.7, 0.6, 0.5, 0.4, 0.3, 0.25, 0.2, 0.1, 0.05, or less). In some embodiments, the pharmaceutical compositions comprising lyophilized lipid delivery vehicles demonstrate a reduced tendency to flocculate or otherwise aggregate (e.g., during lyophilization or upon reconstitution). For example, upon reconstitution the lipid delivery vehicles may have an average particle size (Zₐᵥₑ) of less than 500 nm (*e.g.,* less than about 400 nm, 300 nm, 200 nm, 175 nm, 150 nm, 125 nm, 100 nm, 75 nm, 50 nm, 25 nm, or smaller in a PBS solution). Typically, the average particle size (Zₐᵥₑ) of lipid delivery vehicles for use with the invention is between 40 nm and 60 nm.

In some embodiments, the lyophilized lipid delivery vehicles (*e.g*., lyophilized lipid nanoparticles) further comprise or are alternatively prepared using one or more lyoprotectants (*e.g*., sugars and/or carbohydrates). In certain embodiments, the inclusion of one or more lyoprotectants in the lipid nanoparticle may improve or otherwise enhance the stability of the lyophilized lipid delivery vehicles (*e.g*., under normal storage conditions) and/or facilitate reconstitution of the lyophilized lipid delivery vehicles using a rehydration media, thereby preparing an aqueous formulation. For example, in certain embodiments the lipid nanoparticles are prepared and prior to lyophilization the buffer present in the liposomal formulation may be replaced *(e.g.,* via centrifugation) with a lyoprotectant such as a sucrose solution or suspension (e.g., an aqueous solution comprising between about 1-50% (w/v) or 10-25% (w/v) sucrose). In some embodiments, the lyoprotectant in trehalose. In some embodiments, the lyoprotectant comprises 10-50% (w/v), or 10-25% (w/v) or 10-20% (w/v) or 10-15% (w/v) trehalose. Other lyoprotectants that may be used to prepare the lyophilized compositions described herein include, for example, dextran *(e.g.,* 1.5 kDa, 5 kDa and/or 40 kDa) and inulin *(e.g.,* 1.8 kDa and/or 4 kDa). The lyophilized lipid delivery vehicles have an encapsulation efficiency of greater than about 80%.

A pharmaceutical composition comprising a lyophilized lipid nanoparticle comprising CFTR-encoding mRNA is stable at 4°C for at least 1 month, at least 2 months, at least 3 months, at least 4 months, at least 5 months, at least 6 months, or for at least 1 year. In some embodiments, the lyophilized lipid delivery vehicles may be stored under refrigeration and remain stable (*e.g.,* as demonstrated by minimal or no losses in their intended pharmaceutical or biological activity) for extended periods of time (e.g., stable for at least about 1, 2, 3, 4, 5, 6, 9, 12, 18, 24, 36 months or longer upon storage at about 4°C). In other embodiments, the lyophilized lipid delivery vehicles may be stored without refrigeration and remain stable for extended periods of time (*e.g.,* stable for at least about 1, 2, 3, 4, 5, 6, 9, 12, 18, 24, 36 months or longer upon storage at about 25°C).

The pharmaceutical composition in lyophilized form can be stored in frozen condition for 1, 2, 3, 4, 5 or 10 years without loss of pharmacological or biological activity.

Accordingly, also provided herein are methods for treating disease in a subject by administering an effective amount of pharmaceutical compositions comprising lyophilized CFTR mRNA- lipid delivery vehicles to a subject (e.g., upon reconstitution with a rehydrating media such as sterile water for injection).

In some embodiments, the formulation is administered by a metered-dose inhaler.

In some embodiments, the formulation is administered by a nebulizer.

Suitable CFTR mRNA formulation for nebulization may be stored as a frozen liquid, or sterile liquid, or lyophilized or dry powder and reconstituted prior to nebulization. In some embodiments, the composition is stored in a single-use vial prior to nebulization. In some embodiments, the single-use vial comprises 50 mL or less of the composition. In some embodiments, the single-use vial comprises 40 mL or less of the composition. In some embodiments, the single-use vial comprises 30 mL or less of the composition. In some embodiments, the single-use vial comprises 20 mL or less of the composition. In some embodiments, the single-use vial comprises 10 mL or less of the composition. In some embodiments, the single-use vial comprises 9.0 mL or less of the composition. In some embodiments, the single-use vial comprises 8.0 mL or less of the composition. In some embodiments, the single-use vial comprises 7.0 mL or less of the composition. In some embodiments, the single-use vial comprises 6.0 mL or less of the composition. In some embodiments, the single-use vial comprises 5.0 mL or less of the composition. In some embodiments, the single-use vial comprises between 4.0 mL and 5.0 mL of the composition. More typically, the single-use vial comprises between 3.0 and 4.0 mL of the composition. In a specific embodiment, the single-use vial comprises 3.2 mL of the composition.

### Exemplary Formulations

### Compositions Comprising SEQ ID NO: 28

In embodiments, a composition comprises:
a. an mRNA encoding the CFTR protein, wherein the mRNA comprises:
   i. the sequence as set out in SEQ ID NO: 28;
   ii. a 5' cap structure, wherein the 5' cap structure is 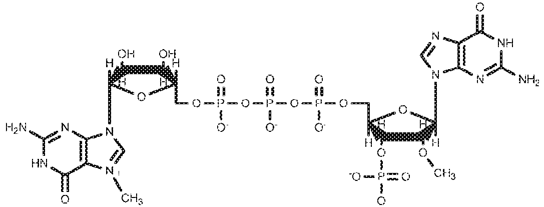 ("m7G(5')ppp(5')(2°OMeG)"); and
   iii. a poly A tail of between 200 and 1000 adenosine nucleotides;
b. imidazole cholesterol ester (ICE);
c. 1,2-dioleoyl-SN-glycero-3-phosphoethanolamine (DOPE)
d. 1,2-dimyristoyl-rac-glycero-3-methylpolyoxyethylene (DMG-PEG-2K); and
e. trehalose.

In embodiments, the mRNA of SEQ ID NO: 28 has an average molecular weight of about 1.63 megadaltons. In embodiments, the 5' UTR, hCFTR start codon, hCFTR stop codon, and 3' UTR of the mRNA of SEQ ID NO: 28 are as set forth in **Table A.** In embodiments, the concentration of mRNA is about 0.6 mg/mL.

In embodiments, the nitrogen/phosphorus (N/P) ratio (*i.e.,* the ratio of positively-charged nitrogens within ICE and the negatively charged phosphodiester lipids with the mRNA) is about 4. In embodiments, the average particle size range for the LNP formulation is about 40-60 nm.

Exemplary compositions comprising the mRNA of SEQ ID NO: 28 also include those described in **Table D.**

**Table D. Exemplary Formulations of SEQ ID NO: 28**

| | **Formulation 1** | **Formulation 2** | **Formulation 3** | **Formulation 4** |
|---|---|---|---|---|
| CFTR mRNA | SEQ ID NO: 28 | SEQ ID NO: 28 | SEQ ID NO: 28 | SEQ ID NO: 28 |
| 5' Cap | m7G(5')ppp(5') (2'OMeG) | m7G(5')ppp(5') (2'OMeG) | m7G(5')ppp(5') (2'OMeG) | m7G(5')ppp(5') (2'OMeG) |
| 5' UTR | as described in Table A | as described in Table A | as described in Table A | as described in Table A |
| Start Codon hCFTR | AUG | AUG | AUG | AUG |
| Stop Codon hCFTR | UAA | UAA | UAA | UAA |
| 3'UTR | as described in Table A | as described in Table A | as described in Table A | as described in Table A |
| PolyA Tail | ~ 200-1000 adenosine nucleotides | ~ 200-1000 adenosine nucleotides | ~ 200-1000 adenosine nucleotides | ~ 200-1000 adenosine nucleotides |
| ICE:DOPE: DMG-PEG-2K | 60:35:5 | 60:30:10 | 60:35:5 | 60:35:5 |
| Diluent | 10% trehalose | 10% trehalose | 10% sucrose | 10% glucose |
| Average particle size | 40-60 nm | 40-60 nm | 40-60 nm | 40-60 nm |
| N/P charge ratio | 4 | 4 | 4 | 4 |

In embodiments, a formulation is Formulation 1. In embodiments, Formulation 1 is further characterized by a concentration of the mRNA that is about 0.6 mg/ml.

In embodiments, a formulation is Formulation 2. In embodiments, Formulation 2 is further characterized by a concentration of the mRNA that is about 0.6 mg/ml.

In embodiments, a formulation is Formulation 3. In embodiments, Formulation 3 is further characterized by a concentration of the mRNA that is about 0.6 mg/ml.

In embodiments, a formulation is Formulation 4. In embodiments, Formulation 4 is further characterized by a concentration of the mRNA that is about 0.6 mg/ml.

In the clinical studies described in the exemplified embodiments of the invention, a single dose of formulation 1 in Table D was found to result in an improvement of ppFEV1 (forced expiratory volume in one second) from baseline ppFEV1 at two days following administration to the lungs of human CF patient via nebulization.

### Compositions Comprising SEQ ID NO: 29

In embodiments, a composition comprises:
a. an mRNA encoding the CFTR protein, wherein the mRNA comprises:
   i. the sequence as set out in SEQ ID NO: 29;
   ii. a 5' cap structure, wherein the 5' cap structure is 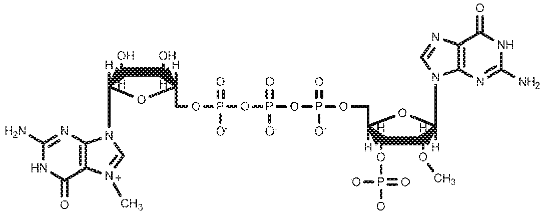 ("m7G(5')ppp(5')(2'OMeG)"); and
   iii. a poly A tail of between 200 and 1000 adenosine nucleotides;
b. imidazole cholesterol ester (ICE);
c. 1,2-dioleoyl-SN-glycero-3-phosphoethanolamine (DOPE)
d. 1,2-dimyristoyl-rac-glycero-3-methylpolyoxyethylene (DMG-PEG-2K); and
e. trehalose.

In embodiments, the mRNA of SEQ ID NO: 29 has an average molecular weight of about 1.63 megadaltons. In embodiments, the 5' UTR, hCFTR start codon, hCFTR stop codon, and 3' UTR of the mRNA of SEQ ID NO: 29 are as set forth in **Table B.** In embodiments, the concentration of mRNA is about 0.6 mg/mL.

In embodiments, the nitrogen/phosphorus (N/P) ratio (*i.e.,* the ratio of positively-charged nitrogens within ICE and the negatively charged phosphodiester lipids with the mRNA) is about 4. In embodiments, the average particle size range for the LNP formulation is about 40-60 nm.

Exemplary compositions comprising the mRNA of SEQ ID NO: 29 also include those described in **Table E.**

**Table E. Exemplary Formulations of SEQ ID NO: 29**

| | **Formulation 5** | **Formulation 6** | **Formulation 7** | **Formulation 8** |
|---|---|---|---|---|
| CFTR mRNA | SEQ ID NO: 29 | SEQ ID NO: 29 | SEQ ID NO: 29 | SEQ ID NO: 29 |
| 5' Cap | m7G(5')ppp(5') (2'OMeG) | m7G(5')ppp(5') (2'OMeG) | m7G(5')ppp(5') (2'OMeG) | m7G(5')ppp(5') (2'OMeG) |
| 5' UTR | as described in Table B | as described in Table B | as described in Table B | as described in Table B |
| Start Codon hCFTR | AUG | AUG | AUG | AUG |
| Stop Codon hCFTR | UAA | UAA | UAA | UAA |
| 3'UTR | as described in Table B | as described in Table B | as described in Table B | as described in |
| Poly A Tail | ~ 200-1000 adenosine nucleotides | ~ 200-1000 adenosine nucleotides | ~ 200-1000 adenosine nucleotides | ~ 200-1000 adenosine nucleotides |
| ICE:DOPE: DMG-PEG-2K | 60:35:5 | 60:30:10 | 60:35:5 | 60:35:5 |
| Diluent | 10% trehalose | 10% trehalose | 10% sucrose | 10% glucose |
| Average particle size | 40-60 nm | 40-60 nm | 40-60 nm | 40-60 nm |
| N/P charge ratio | 4 | 4 | 4 | 4 |

In embodiments, a formulation is Formulation 5. In embodiments, Formulation 5 is further characterized by a concentration of the mRNA that is about 0.6 mg/ml.

In embodiments, a formulation is Formulation 6. In embodiments, Formulation 6 is further characterized by a concentration of the mRNA that is about 0.6 mg/ml.

In embodiments, a formulation is Formulation 7. In embodiments, Formulation 7 is further characterized by a concentration of the mRNA that is about 0.6 mg/ml.

In embodiments, a formulation is Formulation 8. In embodiments, Formulation 8 is further characterized by a concentration of the mRNA that is about 0.6 mg/ml.

### Compositions Comprising SEQ ID NO: 30

In embodiments, a composition comprises:
a. an mRNA encoding the CFTR protein, wherein the mRNA comprises:
   i. the sequence as set out in SEQ ID NO: 30;
   ii. a 5' cap structure, wherein the 5' cap structure is 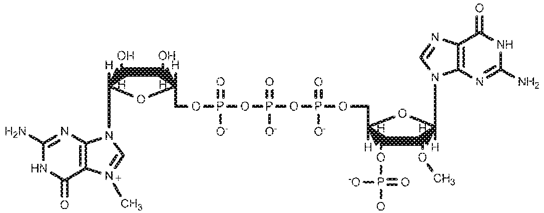 ("m7G(5')ppp(5')(2'OMeG)"); and
   iii. a poly A tail of between 200 and 1000 adenosine nucleotides;
b. imidazole cholesterol ester (ICE);
c. 1,2-dioleoyl-SN-glycero-3-phosphoethanolamine (DOPE)
d. 1,2-dimyristoyl-rac-glycero-3-methylpolyoxyethylene (DMG-PEG-2K); and
e. trehalose.

In embodiments, the mRNA of SEQ ID NO: 30 has an average molecular weight of about 1.63 megadaltons. In embodiments, the 5' UTR, hCFTR start codon, hCFTR stop codon, and 3' UTR of the mRNA of SEQ ID NO: 30 are as set forth in **Table C.** In embodiments, the concentration of mRNA is about 0.6 mg/mL.

In embodiments, the nitrogen/phosphorus (N/P) ratio (*i.e.,* the ratio of positively-charged nitrogens within ICE and the negatively charged phosphodiester lipids with the mRNA) is about 4. In embodiments, the average particle size range for the LNP formulation is about 40-60 nm.

Exemplary compositions comprising the mRNA of SEQ ID NO: 30 also include those described in **Table F**.

**Table F. Exemplary Formulations of SEQ ID NO: 30**

| | **Formulation 9** | **Formulation 10** | **Formulation 11** | **Formulation 12** |
|---|---|---|---|---|
| CFTR mRNA | SEQ ID NO: 30 | SEQ ID NO: 30 | SEQ ID NO: 30 | SEQ ID NO: 30 |
| 5' Cap | m7G(5')ppp(5') (2'OMeG) | m7G(5')ppp(5') (2'OMeG) | m7G(5')ppp(5') (2'OMeG) | m7G(5')ppp(5') (2'OMeG) |
| 5' UTR | as described in Table C | as described in Table C | as described in Table C | as described in Table C |
| Start Codon hCFTR | AUG | AUG | AUG | AUG |
| Stop Codon hCFTR | UGA | UGA | UGA | UGA |
| 3'UTR | as described in Table C | as described in Table C | as described in Table C | as described in Table C |
| PolyA Tail | ~ 200-1000 adenosine nucleotides | ~ 200-1000 adenosine nucleotides | ~ 200-1000 adenosine nucleotides | ~ 200-1000 adenosine nucleotides |
| ICE:DOPE: DMG-PEG-2K | 60:35:5 | 60:30:10 | 60:35:5 | 60:35:5 |
| Diluent | 10% trehalose | 10% trehalose | 10% sucrose | 10% glucose |
| Average particle size | 40-60 nm | 40-60 nm | 40-60 nm | 40-60 nm |
| N/P charge ratio | 4 | 4 | 4 | 4 |

In embodiments, a formulation is Formulation 9. In embodiments, Formulation 9 is further characterized by a concentration of the mRNA that is about 0.6 mg/ml.

In embodiments, a formulation is Formulation 10. In embodiments, Formulation 10 is further characterized by a concentration of the mRNA that is about 0.6 mg/ml.

In embodiments, a formulation is Formulation 11. In embodiments, Formulation 11 is further characterized by a concentration of the mRNA that is about 0.6 mg/ml.

In embodiments, a formulation is Formulation 12. In embodiments, Formulation 12 is further characterized by a concentration of the mRNA that is about 0.6 mg/ml.

### Assessment of Formulation Characteristics

The formulation may be assessed for one or more of the following characteristics: appearance, identity, quantity, concentration, presence of impurities, microbiological assessment, pH level and activity.

In some embodiments, acceptable appearance of the formulation includes a clear, colorless solution, essentially free of visible particulates.

In some embodiments, the identity of the CFTR mRNA is assessed by sequencing methods. The sequencing methods are performed to confirm the correct sequence of the desired CFTR mRNA.

In some embodiments, the concentration of the CFTR mRNA is assessed by a suitable method, such as UV spectrophotometry. In some embodiments, a suitable concentration is between about 90% and 110% nominal (0.9-1.1 mg/mL). Accordingly, in some embodiments, a suitable concentration is about 90% nominal (0.9 mg/mL). In some embodiments, a suitable concentration is about 91% nominal (0.91 mg/mL). In some embodiments, a suitable concentration is about 92% nominal (0.92 mg/mL). In some embodiments, a suitable concentration is about 93% nominal (0.93 mg/mL). In some embodiments, a suitable concentration is about 94% nominal (0.94 mg/mL). In some embodiments, a suitable concentration is about 95% nominal (0.95 mg/mL). In some embodiments, a suitable concentration is about 96% nominal (0.96 mg/mL). In some embodiments, a suitable concentration is about 97% nominal (0.97 mg/mL). In some embodiments, a suitable concentration is about 98% nominal (0.98 mg/mL). In some embodiments, a suitable concentration is about 99% nominal (0.99 mg/mL). In some embodiments, a suitable concentration is about 100% nominal (1.0 mg/mL). In some embodiments, a suitable concentration is about 101% nominal (1.01 mg/mL). In some embodiments, a suitable concentration is about 102% nominal (1.02 mg/mL). In some embodiments, a suitable concentration is about 103% nominal (1.03 mg/mL). In some embodiments, a suitable concentration is about 104% nominal (1.04 mg/mL). In some embodiments, a suitable concentration is about 105% nominal (1.05 mg/mL). In some embodiments, a suitable concentration is about 106% nominal (1.06 mg/mL). In some embodiments, a suitable concentration is about 107% nominal (1.07 mg/mL). In some embodiments, a suitable concentration is about 108% nominal (1.08 mg/mL). In some embodiments, a suitable concentration is about 109% nominal (1.09 mg/mL). In some embodiments, a suitable concentration is about 110% nominal (1.10 mg/mL).

In some embodiments, the formulation is assessed to determine CFTR mRNA integrity, to determine whether there is residual plasmid DNA, and to determine the presence of residual solvent. In some embodiments, CFTR mRNA integrity is assessed by agarose gel electrophoresis. The gels are analyzed to determine whether the banding pattern and apparent nucleotide length is consistent with an analytical reference standard. For example, gels are assessed to determine whether banding pattern and apparent nucleotide length is consistent with an analytical reference standard and is oriented between the 7,000 nt and 3,000 nt bands. Additional methods to assess CFTR mRNA integrity include, for example, assessment of the purified mRNA using capillary gel electrophoresis (CGE). In some embodiments, acceptable purity of the CFTR mRNA in the formulation as determined by CGE is that the main peak is not less than about 55%, 50%, 45%, 40%, 35%, or 30%. Accordingly, in some embodiments, acceptable purity of the CFTR mRNA in the formulation is a CGE with a main peak not less than about 55%. In some embodiments, acceptable purity of the CFTR mRNA in the formulation is a CGE with a main peak not less than about 50%. In some embodiments, acceptable purity of the CFTR mRNA in the formulation is a CGE with a main peak not less than about 45%. In some embodiments, acceptable purity of the CFTR mRNA in the formulation is a CGE with a main peak not less than about 40%. In some embodiments, acceptable purity of the CFTR mRNA in the formulation is a CGE with a main peak not less than about 35%. In some embodiments, acceptable purity of the CFTR mRNA in the formulation is a CGE with a main peak not less than about 30%.

The formulation can also be assessed for the presence of any residual plasmid DNA. Various methods can be used to assess the presence of residual plasmid DNA, for example qPCR. In some embodiments, less than 10 pg/mg (e.g., less than 10 pg/mg, less than 9 pg/mg, less than 8 pg/mg, less than 7 pg/mg, less than 6 pg/mg, less than 5 pg/mg, less than 4 pg/mg, less than 3 pg/mg, less than 2 pg/mg, or less than 1 pg/mg) is an acceptable level of residual plasmid DNA. Accordingly, in some embodiments, the formulation has less than 10 pg/mg of residual plasmid DNA. In some embodiments, the formulation has less than 9 pg/mg of residual plasmid DNA. In some embodiments, the formulation has less than 8 pg/mg of residual plasmid DNA. In some embodiments, the formulation has less than 7 pg/mg of residual plasmid DNA. In some embodiments, the formulation has less than 6 pg/mg of residual plasmid DNA. In some embodiments, the formulation has less than 5 pg/mg of residual plasmid DNA. In some embodiments, the formulation has less than 4 pg/mg of residual plasmid DNA. In some embodiments, the formulation has less than 3 pg/mg of residual plasmid DNA. In some embodiments, the formulation has less than 2 pg/mg of residual plasmid DNA. In some embodiments, the formulation has less than 1 pg/mg of residual plasmid DNA.

The formulation can also be assessed for the presence of any residual solvents. Various methods can be used to determine the presence of residual solvent. In some embodiments, acceptable residual solvent levels are not more than 10,000 ppm. In some embodiments, acceptable residual solvent levels are not more than 9,000 ppm. In some embodiments, acceptable residual solvent levels are not more than 8,000 ppm. In some embodiments, acceptable residual solvent levels are not more than 7,000 ppm. In some embodiments, acceptable residual solvent levels are not more than 6,000 ppm. In some embodiments, acceptable residual solvent levels are not more than 5,000 ppm. In some embodiments, acceptable residual solvent levels are not more than 4,000 ppm. In some embodiments, acceptable residual solvent levels are not more than 3,000 ppm. In some embodiments, acceptable residual solvent levels are not more than 2,000 ppm. In some embodiments, acceptable residual solvent levels are not more than 1,000 ppm. In some embodiments, the residual solvent is, for example, ethanol.

The formulation can also be assessed for the presence of bacterial endotoxins. In some embodiments, bacterial endotoxins are < 0.5 EU/mL, <0.4 EU/mL, <0.3 EU/mL, <0.2 EU/mL or <0.1 EU/mL. Accordingly, in some embodiments, bacterial endotoxins in the purified mRNA are < 0.5 EU/mL. In some embodiments, bacterial endotoxins in the purified mRNA are < 0.4 EU/mL. In some embodiments, bacterial endotoxins in the purified mRNA are < 0.3 EU/mL. In some embodiments, bacterial endotoxins in the purified mRNA are < 0.2 EU/mL. In some embodiments, bacterial endotoxins in the purified mRNA are < 0.2 EU/mL. In some embodiments, bacterial endotoxins in the purified mRNA are < 0.1 EU/mL.

The formulation can also be assessed for microbial contaminants (e.g., "bioburden testing"). The tests can include for example an assessment of total aerobic microbial count ("TAMC") and/or an assessment of total yeast/mold count ("TYMC"). In some embodiments, the purified mRNA has not more than 1 CFU/10mL, 1 CFU/25mL, 1 CFU/50mL, 1CFU/75mL, or not more than 1 CFU/100mL. Accordingly, in some embodiments, the purified mRNA has not more than 1 CFU/10 mL. In some embodiments, the purified mRNA has not more than 1 CFU/25 mL. In some embodiments, the purified mRNA has not more than 1 CFU/50 mL. In some embodiments, the purified mRNA has not more than 1 CFR/75 mL. In some embodiments, the purified mRNA has 1 CFU/100 mL.

The pH of the formulation can also be assessed. In some embodiments, acceptable pH of the formulation is between 5 and 8. Accordingly, in some embodiments, the formulation has a pH of about 5. In some embodiments, the formulation has a pH of about 6. In some embodiments, the formulation has a pH of about 7. In some embodiments, the formulation has a pH of about 7. In some embodiments, the formulation has a pH of about 8.

The formulation can also be assessed for translational fidelity of the CFTR mRNA. The translational fidelity can be assessed by various methods such as, for example, transfection and Western blot analysis. Acceptable characteristics of the purified mRNA includes banding pattern on a Western blot that migrates at a similar molecular weight as a reference standard. For example, the sample main band migrates at a similar apparent molecular weight as the reference standard and is oriented between the 100 kDa and 250 kDa markers.

The formulation can also be assessed for conductance. In some embodiments, acceptable characteristics of the purified mRNA include a conductance of between about 50% and 1 50% of a reference standard. Accordingly, in some embodiments, the formulation has a conductance of about 50% of a reference standard. In some embodiments, the formulation has a conductance of about 55% of a reference standard. In some embodiments, the formulation has a conductance of about 60% of a reference standard. In some embodiments, the formulation has a conductance of about 65% of a reference standard. In some embodiments, the formulation has a conductance of about 70% of a reference standard. In some embodiments, the formulation has a conductance of about 75% of a reference standard. In some embodiments, the formulation has a conductance of about 80% of a reference standard. In some embodiments, the formulation has a conductance of about 85% of a reference standard. In some embodiments, the formulation has a conductance of about 90% of a reference standard. In some embodiments, the formulation has a conductance of about 95% of a reference standard. In some embodiments, the formulation has a conductance of about 100% of a reference standard. In some embodiments, the formulation has a conductance of about 105% of a reference standard. In some embodiments, the formulation has a conductance of about 110% of a reference standard. In some embodiments, the formulation has a conductance of about 115% of a reference standard. In some embodiments, the formulation has a conductance of about 120% of a reference standard. In some embodiments, the formulation has a conductance of about 125% of a reference standard. In some embodiments, the formulation has a conductance of about 130% of a reference standard. In some embodiments, the formulation has a conductance of about 135% of a reference standard. In some embodiments, the formulation has a conductance of about 140% of a reference standard. In some embodiments, the formulation has a conductance of about 145% of a reference standard. In some embodiments, the formulation has a conductance of about 150% of a reference standard.

The CFTR mRNA in the formulation can also be assessed for Cap percentage. Various methods can be used to assess Cap percentage, for example Ultra Performance Liquid Chromatography ("UPLC"). In some embodiments, an acceptable Cap percentage includes Cap1, % area of not less than about 80%, 85%, 90%, or 95%. Accordingly, in some embodiments, an acceptable Cap percentage includes Cap1, % area of not less than about 80%. In some embodiments, an acceptable Cap percentage includes Cap1, % area of not less than about 85%. In some embodiments, an acceptable Cap percentage includes Cap1, % area of not less than about 90%. In some embodiments, an acceptable Cap percentage includes Cap1, % area of not less than about 95%.

Furthermore, the CFTR mRNA in the formulation can be assessed for PolyA tail length. Various methods can be used to assess PolyA tail length, for example capillary electrophoresis. In some embodiments, an acceptable PolyA tail length is about 100 -1500 nucleotides (e.g., 100, 150, 200, 250, 300, 350, 400, 450, 500, 550, 600, 650, 700, 750, 800, 850, 900, 950, and 1000, 1100, 1200, 1300, 1400, or 1500 nucleotides). Accordingly, in some embodiments an acceptable PolyA tail length is about 100 nucleotides. In some embodiments, an acceptable PolyA tail length is about 200 nucleotides. In some embodiments, an acceptable PolyA tail length is about 250 nucleotides. In some embodiments, an acceptable PolyA tail length is about 300 nucleotides. In some embodiments, an acceptable PolyA tail length is about 350 nucleotides. In some embodiments, an acceptable PolyA tail length is about 400 nucleotides. In some embodiments, an acceptable PolyA tail length is about 450 nucleotides. In some embodiments, an acceptable PolyA tail length is about 500 nucleotides. In some embodiments, an acceptable PolyA tail length is about 550 nucleotides. In some embodiments, an acceptable PolyA tail length is about 600 nucleotides. In some embodiments, an acceptable PolyA tail length is about 650 nucleotides. In some embodiments, an acceptable PolyA tail length is about 700 nucleotides. In some embodiments, an acceptable PolyA tail length is about 750 nucleotides. In some embodiments, an acceptable PolyA tail length is about 800 nucleotides. In some embodiments, an acceptable PolyA tail length is about 850 nucleotides. In some embodiments, an acceptable Poly A tail length is about 900 nucleotides. In some embodiments, an acceptable PolyA tail length is about 950 nucleotides. In some embodiments, an acceptable Poly A tail length is about 1000 nucleotides. In some embodiments, an acceptable PolyA tail length is about 1100 nucleotides. In some embodiments, an acceptable PolyA tail length is about 1200 nucleotides. In some embodiments, an acceptable PolyA tail length is about 1300 nucleotides. In some embodiments, an acceptable PolyA tail length is about 1400 nucleotides. In some embodiments, an acceptable PolyA tail length is about 1500 nucleotides. In some embodiments, an acceptable PolyA tail length is between about 200 - 1000 nt. In some embodiments, an acceptable PolyA tail length is between about 300 - 900 nt. In some embodiments, an acceptable PolyA tail length is between about 400 and 800 nt.

### Pulmonary Delivery

A CFTR mRNA may be formulated for delivery via different administration routes including, but not limited to, oral, rectal, vaginal, transmucosal, or intestinal administration; parenteral delivery, including intradermal, transdermal (topical), intramuscular, subcutaneous, intramedullary injections, as well as intrathecal, direct intraventricular, intravenous, intraperitoneal, and/or intranasal administration.

In some embodiments, a CFTR mRNA is formulated for pulmonary delivery. As used herein, pulmonary delivery refers to delivery to lung via, *e.g.,* nasal cavity, trachea, bronchi, bronchioles, and/or other pulmonary system. In particular embodiments, a CFTR mRNA is formulated for nebulization. In these embodiments, the delivery vehicle may be in an aerosolized composition which can be inhaled. In some embodiments, pulmonary delivery involves inhalation (*e.g.,* for nasal, tracheal, or bronchial delivery). In some embodiments, the CFTR mRNA formulation is nebulized prior to inhalation.

### Nebulization

Inhaled aerosol droplets of a particle size of 1-5 µm can penetrate into the narrow branches of the lower airways. Aerosol droplets with a larger diameter are typically absorbed by the epithelia cells lining the oral cavity, and are unlikely to reach the lower airway epithelium and the deep alveolar lung tissue.

Particle size in an aerosol is commonly described in reference to the Mass Median Aerodynamic Diameter (MMAD). MMAD, together with the geometric standard deviation (GSD), describes the particle size distribution of any aerosol statistically, based on the weight and size of the particles. Means of calculating the MMAD of an aerosol are well known in the art.

A specific method of calculating the MMAD using a cascade impactor was first described in 1959 by Mitchell *et al.* The cascade impactor for measuring particle sizes is constructed of a succession of jets, each followed by an impaction slide, and is based on the principle that particles in a moving air stream impact on a slide placed in their path, if their momentum is sufficient to overcome the drag exerted by the air stream as it moves around the slide. As each jet is smaller than the preceding one, the velocity of the air stream and therefore that of the dispersed particles are increased as the aerosol advances through the impactor. Consequently, smaller particles eventually acquire enough momentum to impact on a slide, and a complete particle size classification of the aerosol is achieved. The improved Next Generation Impactor, used herein to measure the MMAD of the pharmaceutical composition of the invention, was first described by Marple *et al.* in 2003 and has been widely used in the pharmacopoeia since.

Another parameter to describe particle size in an aerosol is the Volume Median Diameter (VMD). VMD also describes the particle size distribution of an aerosol based on the volume of the particles. Means of calculating the VMD of an aerosol are well known in the art. A specific method used for determining the VMD is laser diffraction, which is used herein to measure the VMD of the pharmaceutical composition of the invention (*see, e.g.,* Clark, 1995, Int J Pharm. 115:69-78).

In some embodiments, the mean particle size of the nebulized CFTR mRNA formulation of the invention is between about 4 µm and 6 µm, *e.g.*, about 4 µm, about 4.5 µm, about 5 µm, about 5.5 µm, or about 6 µm.

The Fine Particle Fraction (FPF) is defined as the proportion of particles in an aerosol which have an MMAD or a VMD smaller than a specified value. In some embodiments, the FPF of the nebulized CFTR mRNA formulation of the invention with a particle size <5 µm is at least about 30%, more typically at least about 40%, *e.g.,* at least about 50%, more typically at least about 60%.

In some embodiments, nebulization is performed in such a manner that the mean respirable emitted dose (*i.e.,* the percentage of FPF with a particle size < 5 µm; *e.g.*, as determined by next generation impactor with 15 L/min extraction) is at least about 30% of the emitted dose, *e.g.,* at least about 31%, at least about 32%, at least about 33%, at least about 34%, or at least about 35% the emitted dose. In some embodiments, nebulization is performed in such a manner that the mean respirable delivered dose (*i.e.,* the percentage of FPF with a particle size < 5 µm; *e.g.*, as determined by next generation impactor with 15 L/min extraction) is at least about 15% of the emitted dose, e.g., at least 16% or 16.5% of the emitted dose.

### Nebulizer

Nebulization can be achieved by any nebulizer known in the art. A nebulizer transforms a liquid to a mist so that it can be inhaled more easily into the lungs. Nebulizers are effective for infants, children and adults. Nebulizers are able to nebulize large doses of inhaled medications. Typically, a nebulizer for use with the invention comprises a mouthpiece that is detachable. This is important because only clean mouthpieces that are RNase free should be used when administering the CFTR mRNA formulation of the invention.

In some embodiments, the reservoir volume of the nebulizer ranges from about 5.0 mL to about 8.0 mL. In some embodiments, the reservoir volume of the nebulizer is about 5.0 mL. In some embodiments, the reservoir volume of the nebulizer is about 6.0 mL. In some embodiments, the reservoir volume of the nebulizer is about 7.0 mL. In some embodiments, the reservoir volume of the nebulizer is about 8.0 mL.

One type of nebulizer is a jet nebulizer, which comprises tubing connected to a compressor, which causes compressed air or oxygen to flow at a high velocity through a liquid medicine to turn it into an aerosol, which is then inhaled by the patient.

Another type of nebulizer is the ultrasonic wave nebulizer, which comprises an electronic oscillator that generates a high frequency ultrasonic wave, which causes the mechanical vibration of a piezoelectric element, which is in contact with a liquid reservoir. The high frequency vibration of the liquid is sufficient to produce a vapor mist. Exemplary ultrasonic wave nebulizers are the Omron NE-U17 and the Beurer Nebulizer IH30.

A third type of nebulizer comprises vibrating mesh technology (VMT). A VMT nebulizer typically comprises a mesh/membrane with 1000-7000 holes that vibrates at the top of a liquid reservoir and thereby pressures out a mist of very fine aerosol droplets through the holes in the mesh/membrane. VMT nebulizers suitable for delivery of the CFTR mRNA formulation include any of the following: eFlow (PARI Medical Ltd.), i-Neb (Respironics Respiratory Drug Delivery Ltd), Nebulizer IH50 (Beurer Ltd.), AeroNeb Go (Aerogen Ltd.), InnoSpire Go (Respironics Respiratory Drug Delivery Ltd), Mesh Nebulizer (Shenzhen Homed Medical Device Co, Ltd.), Portable Nebulizer (Microbase Technology Corporation) and Airworks (Convexity Scientific LLC). In some embodiments, the mesh or membrane of the VMT nebulizer is made to vibrate by a piezoelectric element. In some embodiments, the mesh or membrane of the VMT nebulizer is made to vibrate by ultrasound.

VMT nebulizers have been found to be particularly suitable for practicing the invention because they do not affect the mRNA integrity of the CFTR mRNA formulation of the invention. Typically, at least about 60%, *e.g.,* at least about 65% or at least about 70%, of the mRNA in the CFTR mRNA formulation of the invention maintains its integrity after nebulization.

In some embodiments, nebulization is continuous during inhalation and exhalation. More typically, nebulization is breath-actuated. Suitable nebulizers for use with the invention have nebulization rate of >0.2 mL/min. In some embodiments, the nebulization rate is >0.25 mL/min. In other embodiment, the nebulization rate is >0.3 mL/min. In certain embodiments, the nebulization rate is >0.45 mL/min. In a typical embodiment, the nebulization rate ranges between 0.2 mL/minute and 0.5 mL/minute.

In some embodiments, the nebulization volume is at a volume ranging from 13.0 mL to 42.0 mL, *e.g.*, between 14 mL and 28 mL. In some embodiments, the nebulization volume is at a volume less than or equal to 13.9 mL. In some embodiments, the nebulization volume is at a volume less than or equal to 16.0 mL. In some embodiments, the nebulization volume is at a volume less than or equal to 18.0 mL. In some embodiments, the nebulization volume is at a volume less than or equal to 20.0 mL. In some embodiments, the nebulization volume is at a volume less than or equal to 22.0 mL. In some embodiments, the nebulization volume is at a volume less than or equal to 24.0 mL. In some embodiments, the nebulization volume is at a volume less than or equal to 26.0 mL. In some embodiments, the nebulization volume is at a volume less than or equal to 27.9 mL. In some embodiments, the nebulization volume is at a volume less than or equal to 30.0 mL. In some embodiments, the nebulization volume is at a volume less than or equal to 32.0 mL. In some embodiments, the nebulization volume is at a volume less than or equal to 34.0 mL. In some embodiments, the nebulization volume is at a volume less than or equal to 36.0 mL. In some embodiments, the nebulization volume is at a volume less than or equal to 38.0 mL. In some embodiments, the nebulization volume is at a volume less than or equal to 40.0 mL. In some embodiments, the nebulization volume is at a volume less than or equal to 41.8 mL.

A human subject may display adverse effects during treatment, when the nebulization volume exceeds 10 mL. In particular, such adverse effects may be more common when volumes greater than 20 mL are administered. In some embodiments, the nebulization volume does not exceed 20 mL.

In some embodiments, a single dose of the CO-hCFTR mRNA composition of the invention can be administered with only a one or two refills per nebulization treatment. For example, if the total volume of the CO-hCFTR mRNA composition that is to be administered to the patient is 13 mL, then only a single refill is required to administer the entire volume when using a nebulizer with an 8 mL reservoir, but two refills are required to administer the same volume when using a nebulizer with a 5 mL reservoir. In another embodiment, at least three refills are required per nebulization treatment, e.g., to administer a volume of 26 mL, at least three refills are required when using a nebulizer with an 8 mL reservoir. In yet a further embodiment, at least four refills are required. For example, to deliver 42 mL with a nebulizer having a 5 mL reservoir, at least eight refills are required. Typically, no more than 1-3 refills will be required to administer the CO-hCFTR mRNA composition of the invention.

Typically, the duration of nebulization is between 30 and 300 minutes. An average nebulization session may exceed 30 minutes, *e.g.,* it may last for at least 35 minutes or more, at least 45 minutes or more, or at least 1 hour or more. For example, most patients are treated with a nebulization session that last between about 45 minutes to about 110 minutes, although some patients may require nebulization sessions that may last from about 100 minutes to about 180 minutes. Longer treatment may last for 1 hour, 1.5 hours, 2 hours or 2.5 hours. Accordingly, in some embodiments, the nebulization session is about 45 minutes, about 60 minutes, about 70 minutes, about 75 minutes, about 90 minutes, about 105 minutes, about 110 minutes, about 120 minutes, about 135 minutes, about 150 minutes, about 165 minutes, or about 180 minutes. In some embodiments, the nebulization session is about 45 minutes. In some embodiments, nebulization is about 90 minutes. In some embodiments, nebulization is about 2 hours and 15 minutes. In some embodiments, patients may require nebulization sessions that may last from about 150 minutes to about 300 minutes, *e.g.*, between 3 hours and 4.5 hours.

In a typical embodiment of the invention, the duration of nebulization of a human subject with a CFTR mRNA composition of the invention is less than 120 minutes. For example, nebulization with the CFTR mRNA composition of the invention for 110 minutes or less, e.g. for about 45 minutes to about 110 minutes, can be sufficient to observe an improvement of ppFEV1 (forced expiratory volume in one second) from baseline ppFEV1 at two days following administration. In order to achieve such durations, the composition of the invention is typically nebulized at a rate ranging from 0.2 mL/minute to 0.5 mL/minute. A concentration of 0.5 mg/ml to 0.8 mg/ml of the CFTR mRNA (e.g. about 0.6 mg/ml) has been found to be particularly suitable, in particular when administered with a vibrating mesh nebulizer.

In some embodiments, the number of nebulizers used during a single nebulization session ranges from 2-8. In some embodiments, 1 nebulizer is used during a single nebulization session. In some embodiments, 2 nebulizers are used during a single nebulization session. In some embodiments, 3 nebulizers are used during a single nebulization session. In some embodiments, 4 nebulizers are used during a single nebulization session. In some embodiments, 5 nebulizers are used during a single nebulization session. In some embodiments, 6 nebulizers are used during a single nebulization session. In some embodiments, 7 nebulizers are used during a single nebulization session. In some embodiments, 8 nebulizers are used during a single nebulization session.

### Therapeutic Efficacy

According to the present invention, a CFTR mRNA is delivered to a CF patient in need of treatment at a therapeutically effective dose and an administration interval for a treatment period sufficient to improve, stabilize or reduce one or more symptoms of cystic fibrosis relative to a control. The terms "treat" or "treatment", as used in the context of cystic fibrosis herein, refers to amelioration of one or more symptoms associated with cystic fibrosis, prevention or delay of the onset of one or more symptoms of cystic fibrosis, and/or lessening of the severity or frequency of one or more symptoms of cystic fibrosis. Particularly, the administration of the composition of the present invention by nebulization to the human CF patient results in improved lung function, as measured by an increase in absolute change in ppFEV1 from baseline ppFEV1.

In some embodiments, a suitable administration interval of the treatment is daily, twice a week, weekly, once every two weeks, once every three weeks, once every four weeks, monthly, once every two months, once every three months, once every 6 months, yearly, once every two years, or once every five years. Typically, weekly administration of a therapeutically effective dose of a CFTR mRNA in accordance with the invention is sufficient to effectively reduce the severity of one or more symptoms in a cystic fibrosis patient. For example, a nominal dose of 7-25 mg of a CFTR mRNA (*e.g.*, a nominal dose of 6-30 mg, *e.g.*, 8 mg, 16 mg, 20 mg or 24 mg) administered weekly by nebulization is effective in providing the human subject with a at least 3% increase in absolute change in ppFEV1 from baseline ppFEV1. In some embodiments, administration of a therapeutically effective dose of a CFTR mRNA every two weeks may also be effective.

In some embodiments, a human subject may be administered a composition of the invention comprising the CFTR mRNA at a concentration of 0.5 mg/ml to 0.8 mg/ml for a duration of 135 minutes or less in order to receive a dose that is effective in providing the human subject with an increase in absolute change in ppFEV1 from baseline ppFEV1. For example, nebulization of a human subject with the CFTR mRNA composition of the invention at said concentration for 100 minutes or less, e.g., for about 65 minutes to about 115 minutes, in particular for about 70 minutes to about 90 minutes, can be adequate to observe an improvement of ppFEV1 (forced expiratory volume in one second) from baseline ppFEV1 at two days following administration. In some embodiments, the duration of nebulization is at least 60 minutes, at least 65 minutes, at least 70 minutes, at least 75 minutes, at least 80 minutes, at least 85 minutes, at least 90 minutes, at least 95 minutes, at least 100 minutes, at least 105 minutes, at least 110 minutes, at least 115 minutes, or at least 120 minutes. For example, the duration of nebulization may be between 45 minutes and 135 minutes, between 65 minutes and 115 minutes, or between 70 minutes and 90 minutes.

In some embodiments, the present invention provides a method of treating cystic fibrosis (CF) in a human subject comprising administration of a composition comprising an mRNA encoding a Cystic Fibrosis Transmembrane Conductance Regulator (CFTR) protein at a dose between 7 mg and 25 mg via nebulization for a duration of less than 135 min. Typically, administration is repeated every week or every two weeks. In a particular embodiment, the CFTR mRNA is provided in a solution at a concentration of 0.5 mg/ml to 0.8 mg/ml. In a suitable composition, the CFTR mRNA is encapsulated in a liposome.

In some embodiments, the treatment period or how long the patient is administered a therapeutically effective dose of a CFTR mRNA is for the life of the patient. In some embodiments, a suitable treatment period is at least two weeks, three weeks, four weeks, a month, two months, three months, four months, five months, six months, seven months, eight months, nine months, ten months, eleven months, twelve months, 1 year, 2 years, 3 years, 4 years, 5 years, 10 years, 20 year, 30 years or 50 years.

Typically, the therapeutic effect of administration of a CFTR mRNA on a cystic fibrosis patient is measured relative to a control. In some embodiments, a control is the severity of one or more symptoms in the same patient before the treatment. In some embodiments, a control is indicative of a historical reference level of one or more symptoms in CF patients. In some embodiments, a control is indicative of a normal level of ability, physical conditions or biomarker corresponding to the one or more symptoms being measured.

In some embodiments, the therapeutic effect of administration of a CFTR mRNA according to the present invention is measured by a score on a Cystic Fibrosis Questionnaire Revise (CFQ-R) respiratory domain. In some embodiments, the therapeutic effect of administration of a CFTR mRNA according to the present invention is measured by a sweat chloride value. In some embodiments, the therapeutic effect of administration of a CFTR mRNA according to the present invention is measured by a body mass index and/or body weight. In some embodiments, the therapeutic effect of administration of a CFTR mRNA according to the present invention is measured by onset or severity of pulmonary exacerbation.

In some embodiments, the therapeutic effect of administration of a CFTR mRNA according to the present invention is measured by minute volume, respiratory rate, and/or tidal volume. In some embodiments, the therapeutic effect of administration of a CFTR mRNA according to the present invention on the respiratory system is determined by performing spirometry and assessing the following parameters: forced expiratory volume in 1 second (FEV₁): absolute volume (L) and percent based on the patient's age, gender, and height, forced vital capacity (FVC): absolute volume (L) and percent based on the patient's age, gender, and height, FEV₁/FVC: ratio and percent based on the patient's age, gender, and height, and/or forced expiratory flow over the middle one-half of the FVC (FEF_{25-75%}): absolute volume (L) and percent based on the patient's age, gender, and height. In some embodiments, the parameters can be normalized using the ERS Global Lung Function Initiative (GLI) prediction equations. In some embodiments, the therapeutic effect of administration of a CFTR mRNA according to the present invention on the respiratory system is determined by chest x-ray.

In some embodiments, the therapeutic effect of administration of a composition comprising an mRNA encoding CFTR protein to a human subject by nebulization at an effective dose is measured by an increase in absolute change in ppFEV1 from baseline ppFEV1. Accordingly, a suitable dose for use in the methods of the invention is selected on the basis that it provides the human subject with at least a 3% increase in absolute change in ppFEV1 (percent predicted forced expiratory volume in one second) from baseline ppFEV1 at two days following the administration. In a specific embodiment, the dose is selected to provide the human subject with at least a 5% increase in absolute change in ppFEV1 from baseline ppFEV1 at two days following the administration. For example, the dose may be selected to provide the human subject with at least a 10% increase in absolute change in ppFEV1 from baseline ppFEV1 at two days following the administration.

An additional or alternative consideration is selecting a dose for use in the method of the invention is whether it provides an increase in absolute change in ppFEV1 from baseline ppFEV1 at one week following the administration. In some embodiments, the dose is selected to provide the human subject with at least a 2% increase in absolute change in ppFEV1 from baseline ppFEV1 at one week following the administration. For instance, the dose may be selected to provide the human subject with at least a 7% increase in absolute change in ppFEV1 from baseline ppFEV1 through one week following administration. In some embodiments, the dose is selected to provide the human subject with at least a 8% increase in absolute change in ppFEV1 from baseline ppFEV1 at one week following the administration. In a specific embodiment, the dose is selected to provide the human subject with at least a 12% increase in absolute change in ppFEV1 from baseline ppFEV1 through one week following administration.

In some embodiments, the dose is selected additionally or alternatively on the basis that it provides the human subject with at least a 4% maximum increase in absolute change in ppFEV1 from baseline ppFEV1 through one week following administration. For instance, the dose may be selected to provide the human subject with at least a 6% maximum increase in absolute change in ppFEV1 from baseline ppFEV1 through one week following administration. In a specific embodiment, the dose is selected to provide the human subject with at least a 8% maximum increase in absolute change in ppFEV1 from baseline ppFEV1 through one week following administration.

In some embodiments, the administration of a composition comprising an mRNA encoding CFTR protein by nebulization at a dose greater than 9 mg provides the human subject with at least 5% increase in absolute change in ppFEV1 from baseline ppFEV1 at two days following the administration. In some embodiments, the administration of a composition comprising an mRNA encoding CFTR protein by nebulization at a dose of about 16 mg provides the human subject with at least 5% increase in absolute change in ppFEV1 from baseline ppFEV1 at two days following the administration. In some embodiments, the administration of a composition comprising an mRNA encoding CFTR protein by nebulization at a dose of about 24 mg provides the human subject with at least 5% increase in absolute change in ppFEV1 from baseline ppFEV1 at two days following the administration. In some embodiments, the administration of a composition comprising an mRNA encoding CFTR protein by nebulization at a dose between 11 mg and 17 mg provides the human subject with at least 5% increase in absolute change in ppFEV1 from baseline ppFEV1 at two days following the administration. In some embodiments, the administration of a composition comprising an mRNA encoding CFTR protein by nebulization at a dose of about 12 mg provides the human subject with at least 5% increase in absolute change in ppFEV1 from baseline ppFEV1 at two days following the administration. In some embodiments, the administration of a composition comprising an mRNA encoding CFTR protein by nebulization at a dose between 17 mg and 24 mg provides the human subject with at least 5% increase in absolute change in ppFEV1 from baseline ppFEV1 at two days following the administration. In some embodiments, the administration of a composition comprising an mRNA encoding CFTR protein by nebulization at a dose greater than 20 mg provides the human subject with at least 5% increase in absolute change in ppFEV1 from baseline ppFEV1 at two days following the administration. In some embodiments, the administration of a composition comprising an mRNA encoding CFTR protein by nebulization at a dose of about 12 mg provides the human subject with at least 5% increase in the absolute change in ppFEV1 from baseline ppFEV1 after two days following the administration. In some embodiments, the administration of a composition comprising an mRNA encoding CFTR protein by nebulization at a dose of about 20 mg provides the human subject with at least 5% increase in the absolute change in ppFEV1 from baseline ppFEV1 after two days following the administration. In some embodiments, the administration of a composition comprising an mRNA encoding CFTR protein by nebulization at a dose of about 12 mg provides the human subject with at least 5% maximum increase in absolute change in ppFEV1 from baseline ppFEV1 through one week following administration. In some embodiments, the administration of a composition comprising an mRNA encoding CFTR protein by nebulization at a dose of about 20 mg provides the human subject with at least 5% maximum increase in absolute change in ppFEV1 from baseline ppFEV1 through one week following administration.

The inventors found that administration of a composition comprising an mRNA encoding CFTR protein by nebulization at a dose between 9 mg and 25 mg can result in an increase in absolute change in ppFEV1 (forced expiratory volume in one second) from baseline ppFEV1 at two days as well as one week following the administration. A single nominal dose of 12 mg, 16 mg or 20 mg, or 24 mg of CFTR mRNA may therefore be particularly suitable for use in the methods of the invention. At the same efficacy level, lower doses (e.g., 12 mg or 16 mg) are generally preferred. The maximum increase in absolute change in ppFEV1 from baseline ppFEV1 through one week following administration was observed at a dose between 13 mg and 19 mg. Accordingly, a single nominal dose of 16 mg of CFTR mRNA may be particularly suitable for use in the methods of the invention.

In some embodiments, administration of a CFTR mRNA according to the present invention results in a change in the CFQ-R respiratory domain score by at least 1, at least 2, at least 3, at least 4, at least 5, at least 6, at least 7, at least 8, at least 9, at least 10, at least 11, at least 12, at least 13, at least 14, or at least 15 points relative to a control. In some embodiments, administration of a CFTR mRNA according to the present invention results in a change in the CFQ-R respiratory domain score by at least 1%, 2%, 3%, 4%, 5%, 6%, 7%, 8%, 9%, 10%, 11%, 12%, 13%, 14%, 15%, 16%, 17%, 18%, 19%, or 20% relative to a control.

In some embodiments, administration of a CFTR mRNA according to the present invention results in amelioration, prevention or delay in onset of pulmonary exacerbation. As used herein, pulmonary exacerbation refers to one or more of the following sino-pulmonary signs/symptoms: change in sputum, new or increased hemoptysis, increased cough, increased dyspnea, malaise/fatigue/lethargy, temperature >38°C (~100.4°F), anorexia/weight loss, sinus pain/tenderness, change in sinus discharge, change in physical chest exam, decrease in pulmonary function and radiographic indication of pulmonary infection.

In some embodiments, administration of a CFTR mRNA according to the present invention results in prevention or reduced inflammation associated with pulmonary exacerbation. For example, administration of a CFTR mRNA according to the present invention results in reduced expression of markers of inflammation and/or lung damage, including but not limited to, C-reactive protein, white cell counts, interleukin-8, neutrophil elastase alpha 1-antiprotease complexes and matrix metalloproteins, in blood or serum as compared to a control indicative of the corresponding level of relevant markers in a CF patient without treatment. Additionally or alternatively, administration of a CFTR mRNA according to the present invention results in reduced sputum concentrations of bioactive lipid mediators, such as the cysteinyl leukotrienes and prostaglandin-E2, or sputum cell counts as compared to a control indicative of the corresponding level of relevant markers in a CF patient without treatment.

In some embodiments, administration of a CFTR mRNA according to the present invention results in a weight gain of at least 1 pound, at least 2 pounds, at least 3 pounds, at least 4 pounds, at least 5 pounds, at least 6 pounds, at least 7 pounds, at least 8 pounds, at least 9 pounds, at least 10 pounds, at least 11 pounds, at least 12 pounds, at least 13 pounds, at least 14 pounds or at least 15 pounds as compared to pre-treatment body weight.

In some embodiments, a CFTR mRNA is administered in combination with one or more CFTR potentiators and/or correctors. Suitable CFTR potentiators and/or correctors include ivacaftor (trade name Kalydeco^{®}), lumacaftor (trade name Orkambi^{®}) or the combination of ivacaftor and lumacaftor. In some embodiments, a CFTR mRNA is administered in combination with one or more other CF treatment such as hormone replacement therapies, thyroid hormone replacement therapy, non-steroidal inflammatory drugs, and prescription dronabinol (Marinol^{®}) during treatment.

In some embodiments, the CF patient receives a concomitant CFTR modulator therapy. In some embodiments, the concomitant CFTR modulator therapy is given during the CFTR mRNA treatment regimen. In some embodiments, the concomitant CFTR modulator therapy is given before commencing the CFTR mRNA treatment regimen. Typically, the baseline ppFEV1 is measured in the CF patient following prior administration of the concomitant CFTR modulator therapy. In some embodiments, the concomitant CFTR modulator therapy is commenced after the CFTR mRNA treatment regimen.

Not all CF patients respond to CFTR modulator therapy that is available or in development. Accordingly, CF patients that are not eligible for treatment with one or more of ivacaftor, lumacaftor, tezacaftor, VX-659, VX-445, VX-152, VX-440, VX-371, VX-561, VX-659 particularly benefit from the compositions and methods of the invention.

In some embodiments, CFTR potentiators and/or correctors and/or other cystic fibrosis treatments may be administered prior to, concurrently or subsequent to the administration of a CFTR mRNA according to the present invention. For example, CFTR potentiators and/or correctors and/or other cystic fibrosis treatments may be administered at 1 hour or longer, at 2 hours or longer, at 4 hours or longer, at 6 hours or longer, at 8 hours or longer, at 10 hours or longer, at 12 hours or longer, at 18 hours or longer, at 24 hours or longer, at 36 hours or longer, at 48 hours or longer, at 72 hours or longer, at 1 week or longer, at 2 weeks or longer, at 3 weeks or longer, or at 1 month or longer prior to or following administration of a CFTR mRNA according to the invention.

### Pharmacokinetics and Tissue Distribution

According to the present invention, administration of a formulation comprising a CFTR mRNA results in delivery of the mRNA and encoded CFTR protein in various targets tissues described herein. In particular, administration of a formulation comprising a CFTR mRNA according to the present invention results in a therapeutically or clinically effective level or activity of CFTR in the target tissue. In various embodiments, a target tissue includes lung, pancreas, kidney, liver, spleen, testes/ovaries, salivary glands, sweat glands, heart and brain. In some embodiments, a target tissue is lung. In some embodiments, a target tissue is the upper (*i.e.,* superior) lobe of the right or left lung. In some embodiments, a target tissue is the lower (*i.e.,* inferior) lobe of the right or left lung. In some embodiments, a target tissue is the middle lobe of the right lung.

In some embodiments, a target tissue is the apical segment of the right lung or the apicoposterior segment of the left lung. In some embodiments, a target tissue is the posterior segment of the right lung. In some embodiments, a target tissue is the anterior segment of the right or left lung. In some embodiments, a target tissue is the superior segment of the right or left lung. In some embodiments, a target tissue is the lateral basal segment of the right or left lung. In some embodiments, a target tissue is the anterior basal segment of the right lung. In some embodiments, a target tissue is the anteromedial basal segment of the left lung. In some embodiments, a target tissue is the lateral segment of the right lung. In some embodiments, a target tissue is the medial segment of the right lung. In some embodiments, a target tissue is the superior lingular segment of the left lung. In some embodiments, a target tissue is the inferior lingular segment of the left lung. In some embodiments, a target tissue is the posterior basal segment of the right or left lung. In some embodiments, a target tissue is the medial basal segment of the right lung.

In particular embodiments, a target tissue is epithelial cells in the lung. In some embodiments, a target tissue is smooth muscle cells in the lung. In some embodiment, a target tissue is pancreatic duct epithelial cells. In some embodiment, a target tissue is bile-duct epithelial cells. In some embodiment, a target tissue is epithelial cells of the salivary glands. In some embodiment, a target tissue is renal epithelial cells. In some embodiment, a target tissue is beta-S cells in sweat gland secretory coils of sweat glands. In some embodiment, a target tissue is epithelial cells of the reproductive tract.

In some embodiments, a CFTR mRNA delivered according to the present invention achieves a level of CFTR protein expression or activity that is at least 5%, 10%, 20%, 30%, 40%, 50%, 60%, 70%, 80%, 90%, or 95% of the normal level of CFTR protein expression or activity in a target tissue described herein. In some embodiments, a CFTR mRNA delivered according to the present invention achieves a level of CFTR protein expression or activity that is increased by at least 1-fold, 2-fold, 3-fold, 4-fold, 5-fold, 6- fold, 7-fold, 8-fold, 9-fold or 10-fold as compared to a control (e.g., endogenous level of protein or activity without or before the treatment according to the invention, or a historical reference level) in a target tissue described herein.

In general, a CFTR mRNA delivered according to the present invention have sufficiently long half time in a target tissue described herein. In some embodiments, a CFTR mRNA delivered according to the present invention has a half-life of at least approximately 30 minutes, 45 minutes, 60 minutes, 90 minutes, 2 hours, 3 hours, 4 hours, 5 hours, 6 hours, 7 hours, 8 hours, 9 hours, 10 hours, 12 hours, 16 hours, 18 hours, 20 hours, 25 hours, 30 hours, 35 hours, 40 hours, 3 days, 7 days, 14 days, 21 days, or a month. In some embodiments, a CFTR mRNA delivered according to the present invention results in detectable CFTR protein level or activity in a target tissue (*e.g.,* the lung) or bloodstream after 12 hours, 24 hours, 30 hours, 36 hours, 42 hours, 48 hours, 54 hours, 60 hours, 66 hours, 72 hours, 78 hours, 84 hours, 90 hours, 96 hours, 102 hours, a week, two weeks, three weeks, or a month following administration. Detectable level or activity may be determined using various methods known in the art.

In some embodiments, a CFTR mRNA delivered according to the present invention results in increased CFTR protein level or activity in upper lobe lung tissue by *e.g.,* at least approximately 10%, 20%, 30%, 40%, 50%, 60%, 70%, 80%, 90%, 1-fold, 2-fold, 3-fold, 4-fold, 5-fold, 10-fold, 20-fold, 30-fold, 40-fold, 50-fold, 100-fold, 500-fold, 1000-fold, or 1500-fold as compared to a control (*e.g.,* endogenous level of protein or activity without or before the treatment according to the invention, or a historical reference level).

In some embodiments, a CFTR mRNA delivered according to the present invention results in increased CFTR protein level or activity in lower lobe lung tissue by *e.g.,* at least approximately 10%, 20%, 30%, 40%, 50%, 60%, 70%, 80%, 90%, 1-fold, 2-fold, 3-fold, 4-fold, 5-fold, 10-fold, 20-fold, 30-fold, 40-fold, 50-fold, 100-fold, 500-fold, 1000-fold, or 1500-fold as compared to a control (*e.g.,* endogenous level of protein or activity without or before the treatment according to the invention, or a historical reference level).

In some embodiments, a CFTR mRNA delivered according to the present invention results in increased CFTR protein level or activity in middle lobe lung tissue by *e.g.,* at least approximately 10%, 20%, 30%, 40%, 50%, 60%, 70%, 80%, 90%, 1-fold, 2-fold, 3-fold, 4-fold, 5-fold, 10-fold, 20-fold, 30-fold, 40-fold, 50-fold, 100-fold, 500-fold, 1000-fold, or 1500-fold as compared to a control (*e.g.,* endogenous level of protein or activity without or before the treatment according to the invention, or a historical reference level).

In some embodiments, a CFTR mRNA delivered according to the present invention results in increased CFTR protein level or activity in distal lung tissues by, *e.g.,* at least approximately 10%, 20%, 30%, 40%, 50%, 60%, 70%, 80%, 90%, 1-fold, 2-fold, 3-fold, 4-fold, 5-fold, 10-fold, 20-fold, 30-fold, 40-fold, 50-fold, 100-fold, 200-fold, 300-fold, 400-fold, or 500-fold as compared to a control (*e.g.,* endogenous level of protein or activity without or before the treatment according to the invention, or a historical reference level).

In some embodiments, a CFTR mRNA delivered according to the present invention results in increased CFTR protein level or activity in distal peripheral lung tissue by *e.g.,* at least approximately 10%, 20%, 30%, 40%, 50%, 60%, 70%, 80%, 90%, 1-fold, 2-fold, 3-fold, 4-fold, 5-fold, 10-fold, 20-fold, 30-fold, 40-fold, 50-fold, 100-fold, 200-fold, or 300-fold as compared to a control (*e.g.,* endogenous level of protein or activity without or before the treatment according to the invention, or a historical reference level).

In some embodiments, a CFTR mRNA delivered according to the present invention results in increased CFTR protein level or activity in lateral peripheral lung tissue by *e.g.,* at least approximately 10%, 20%, 30%, 40%, 50%, 60%, 70%, 80%, 90%, 1-fold, 2-fold, 3-fold, 4-fold, 5-fold, 10-fold, 20-fold, 30-fold, 40-fold, 50-fold, 100-fold, 500-fold, 1000-fold, or 1500-fold as compared to a control (*e.g.,* endogenous level of protein or activity without or before the treatment according to the invention, or a historical reference level).

In some embodiments, a CFTR mRNA delivered according to the present invention results in increased CFTR protein level or activity in medial peripheral lung tissue by *e.g.,* at least approximately 10%, 20%, 30%, 40%, 50%, 60%, 70%, 80%, 90%, 1-fold, 2-fold, 3-fold, 4-fold, 5-fold, 10-fold, 20-fold, 30-fold, 40-fold, 50-fold, 100-fold, 500-fold, or 1000-fold as compared to a control (*e.g.,* endogenous level of protein or activity without or before the treatment according to the invention, or a historical reference level).

In some embodiments, a CFTR mRNA delivered according to the present invention results in increased CFTR protein level or activity in middle lung tissue by *e.g.,* at least approximately 10%, 20%, 30%, 40%, 50%, 60%, 70%, 80%, 90%, 1-fold, 2-fold, 3-fold, 4-fold, 5-fold, 10-fold, 20-fold, 30-fold, 40-fold, 50-fold, 100-fold, 150-fold, 200-fold, 250-fold, 300-fold, 350-fold, 400-fold, 450-fold, or 500-fold as compared to a control (*e.g.,* endogenous level of protein or activity without or before the treatment according to the invention, or a historical reference level).

In some embodiments, a CFTR mRNA delivered according to the present invention results in increased CFTR protein level or activity in proximal lung tissue by, *e.g.,* at least approximately 10%, 20%, 30%, 40%, 50%, 60%, 70%, 80%, 90%, 1-fold, 2-fold, 3-fold, 4-fold, 5-fold, 10-fold, 20-fold, 30-fold, 40-fold, 50-fold, 100-fold, 500-fold, 1000-fold, or 1500-fold as compared to a control (*e.g.*, endogenous level of protein or activity without or before the treatment according to the invention, or a historical reference level).

In some embodiments, a CFTR mRNA delivered according to the present invention results in detectable CFTR protein or activity in the larynx, trachea, nasal turbinate, and/or bronchoalveolar lavage fluid (BALF). In some embodiments, a CFTR mRNA delivered according to the present invention results in detectable CFTR protein or activity in blood. In some embodiments, a CFTR mRNA delivered according to the present invention results in detectable CFTR protein or activity in lung, pancreas, kidney, liver, spleen, testes/ovaries, salivary glands, sweat glands, heart and brain.

In some embodiments, a CFTR mRNA delivered according to the present invention results in increased CFTR protein level or activity in larynx, trachea, tracheobronchial lymph node, and/or blood by, *e.g.,* at least approximately 10%, 20%, 30%, 40%, 50%, 60%, 70%, 80%, 90%, 1-fold, 2-fold, 3-fold, 4-fold, 5-fold, 10-fold, 20-fold, 30-fold, 40-fold, 50-fold, 100-fold, 500-fold, 1000-fold, or 1500-fold as compared to a control (*e.g.,* endogenous level of protein or activity without or before the treatment according to the invention, or a historical reference level).

The CFTR mRNA expression may be detected or quantified by qPCR on RNA purified from tissue samples. The CFTR protein expression may be determined by measuring immune responses to CFTR protein. In some embodiments, IgG antibody to CFTR protein is measured by an enzyme-linked immunosorbent assay in collected serum samples. In some embodiments, CFTR-specific T cell responses are assessed using collected peripheral blood mononuclear cells. In some embodiments, T cell responses to CFTR are measured by a human interferon-y enzyme-linked immunospot assay as described by Calcedo *et al.* (Calcedo et al., Hum Gene Ther Clin Dev. (2013) 24:108-15). Qualitative assessment of CFTR protein may also be performed by Western blot analysis. The CFTR protein activity may be measured by CFTR chloride channel activity in appropriate tissue cells. A stable potential with the mean value of a 10 second scoring interval after perfusion of solution is recorded. CFTR activity is estimated by the change in potential difference following perfusion with chloride-free isoproterenol. Various other methods are known in the art and may be used to determine the CFTR mRNA and CFTR protein expression or activity.

### EXAMPLES

While certain compounds, compositions and methods of the present invention have been described with specificity in accordance with certain embodiments, the following examples serve only to illustrate the compounds of the invention and are not intended to limit the same.

### Example 1. Formulation of hCFTR-mRNA LNP Composition

The drug product used in the clinical studies described in Examples 2-4 is a codon-optimized (CO) hCFTR mRNA encapsulated within a lipid nanoparticle (LNP) comprising ICE, DOPE, and DMG-PEG-2K formulated in 10% trehalose (see Formulation 1 in Table D).

**Table 3. Key characteristics of the drug product used in the clinical studies (Formulation 1)**

| | | | | | |
|---|---|---|---|---|---|
| CFTR mRNA | 5' Cap | ICE: DOPE: DMG-PEG-2K | Diluent | Average particle size | N/P ratio |
| SEQ ID NO:28 | m7G(5')ppp(5') (2'OMeG) | 60:35:5 | 10% trehalose | 40-60 nm | 4 |

Prior to its administration, the drug product was prepared by reconstituting a lyophilized dry powder into an aqueous solution that can be nebulized.

ICE is an ionizable lipid that affords a positively charged environment at low pH to facilitate efficient encapsulation of the negatively charged mRNA drug substance; it may also play a key role in cell surface interaction to allow for cellular uptake. DOPE is a zwitterionic lipid that has been reported to have fusogenic properties to enhance uptake and release of the drug payload; DMG-PEG-2K is a PEGylated lipid that provides control over particle size and stability of the nanoparticles and may provide enhanced mucopenetrating properties for lung uptake. The relatively high molar ratio of the PEGylated-lipid relative to the other lipid components, ICE and DOPE (5% versus 60% and 35%, respectively), may further promote mucopenetration of the LNPs.

### Example 2. Clinical Trial Design to evaluate the efficacy of hCFTR-mRNA LNPs in treating Cystic Fibrosis

This example shows an exemplary clinical trial design of first-in-human study to evaluate the efficacy of hCFTR mRNA-loaded LNPs in patients with cystic fibrosis.

The randomized, double-blind, placebo-controlled clinical trial was designed to assess safety and efficacy of delivering the hCFTR mRNA by nebulization. A clinical trial was conducted with 12 cystic fibrosis patients with Class I and/or Class II mutations. The majority of patients in the study had at least one F508del mutation and several had heterozygous F508del mutations. Other patients had other Class I or other Class II mutations, including G542X (Class I), R553X (Class I), CFTRdele2,3 (Class I), G542X (Class I), or N1303K (Class II). One patient had two non-F508del mutations and was not amenable to treatment with any small molecule modulators, e.g., KALYDECO^{®} (ivacaftor), ORKAMBI^{®} (lumacaftor/ivacaftor combination) or SYMDEKO^{®} (tezacaftor/ivacaftor combination). Patients who were receiving treatment with lumacaftor/ivacaftor combination drug (ORKAMBI^{®}) or tezacaftor/ivacaftor combination drug (SYMDEKO^{®}) were allowed in the study and were able to continue on their modulator treatment, provided that they had received such medication for at least 28 days prior to the screening visit and remained on it for the duration of the study at a stable dose. The patients were assigned to one of four treatment groups: 8 mg dose, 16 mg dose, 24 mg dose and placebo.

All doses were administered via a hand-held, vibrating-mesh nebulizer in a clinic setting and patients were followed for at least 1 month after the dose before unblinding and analysis.

### Example 3. Efficacy and safety of treating Cystic Fibrosis with a single dose of hCFTR mRNA LNPs by pulmonary delivery

This examples describes a first-in-human study of treating CF patients with hCFTR mRNA-loaded LNPs via nebulization, in accordance with the clinical trial design described in Example 2.

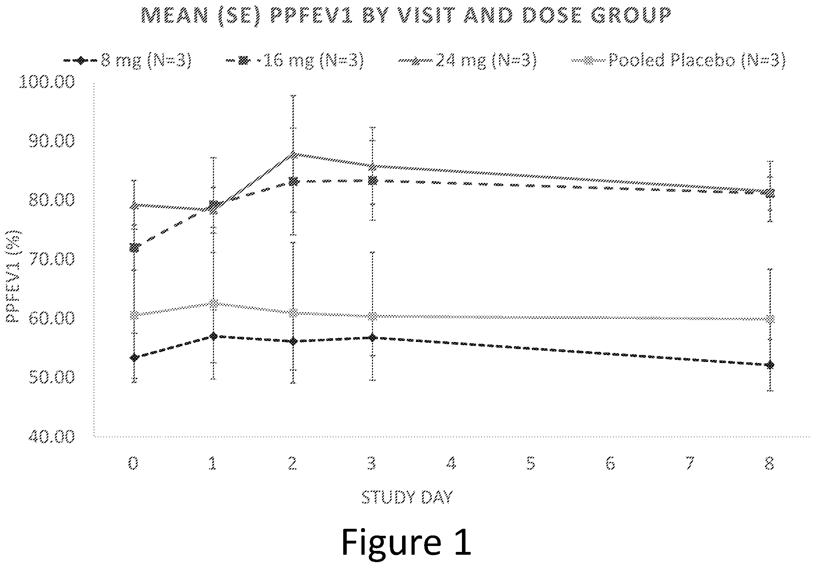

A single dose of hCFTR mRNA (8 mg, 16 mg, 24 mg, or placebo) was administered to the patients by pulmonary delivery via nebulization in accordance with the study design in Table 4 and in Example 2. For placebo group, saline was administered. To evaluate the efficacy of hCFTR mRNA in treating patients with cystic fibrosis, percent predicted forced expiratory volume in one second (ppFEV1), which is a primary measure of lung function, was monitored at pre-defined timepoints throughout 29 days post administration. The ppFEV1 values measured at each time point were compared to the baseline ppFEV1 to determine absolute change in ppFEV1 at each pre-defined timepoint. The mean ppFEV1 for each dose group by visit through day 8 is shown in Figure 1. Mean absolute change from baseline in ppFEV1 by visit and dose group through day 29 is summarized in Table 4.

**Table 4. Summary of Absolute Change in ppFEV1 from Baseline ppFEV1.**

| **Dose** | **Mean Baseline ppFEV1 (SE)** | **Absolute Change from Baseline Mean (SE) ppFEV1** | | | | | | **Maximum Change from Baseline through Day 8 Mean (SE) ppFEV1** | **Maximum Change from Baseline through Day 29 Mean (SE) ppFEV1** |
|---|---|---|---|---|---|---|---|---|---|
| | | **Day 1 *** | **Day 2** | **Day 3** | **Day 8** | **Day 15** | **Day 29** | | |
| **8 mg** | 53.3 (4.2) | 3.66 (0.54) | 2.78 (1.06) | 3.42 (1.32) | -1.24 (1.75) | -0.24 (2.10) | 3.10 (2.83) | 4.45 (0.37) | 5.82 (1.33) |
| **16 mg** | 72.0 (3.8) | 7.22 (4.21) | 11.21 (5.96) | 11.38 (2.97) | 9.19 (1.03) | 5.56 (0.70) | 4.66 (2.69) | 15.65 (3.35) | 15.65 (3.35) |
| **24 mg** | 79.0 (4.1) | 2.45 (0.31) | 8.62 (6.47) | 6.58 (2.78) | 2.29 (1.82) | 0.48 (3.03) | -5.26 (7.54) | 9.68 (5.87) | 10.29 (5.62) |
| **Placebo** | 60.5 (10.7) | 2.03 (2.17) | 0.40 (1.41) | -0.17 (0.83) | -0.61 (2.30) | 0.02 (0.79) | 0.75 (2.47) | 3.22 (1.55) | 4.49 (1.33) |

| | | | | | | | | | |
|---|---|---|---|---|---|---|---|---|---|
| *8 hours post dose | | | | | | | | | |

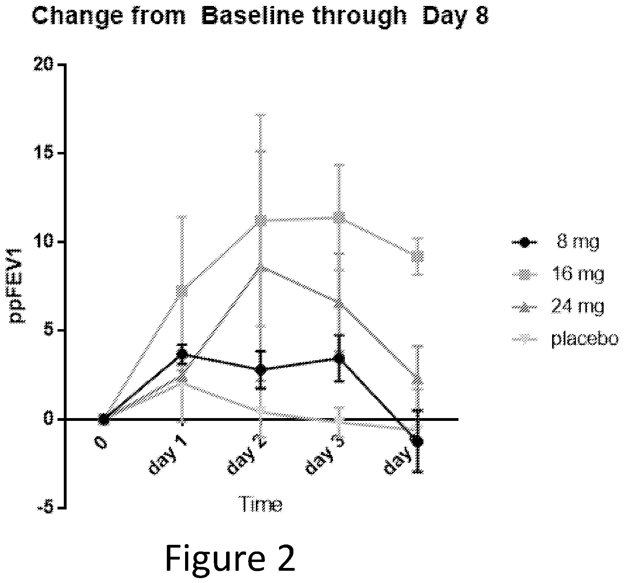

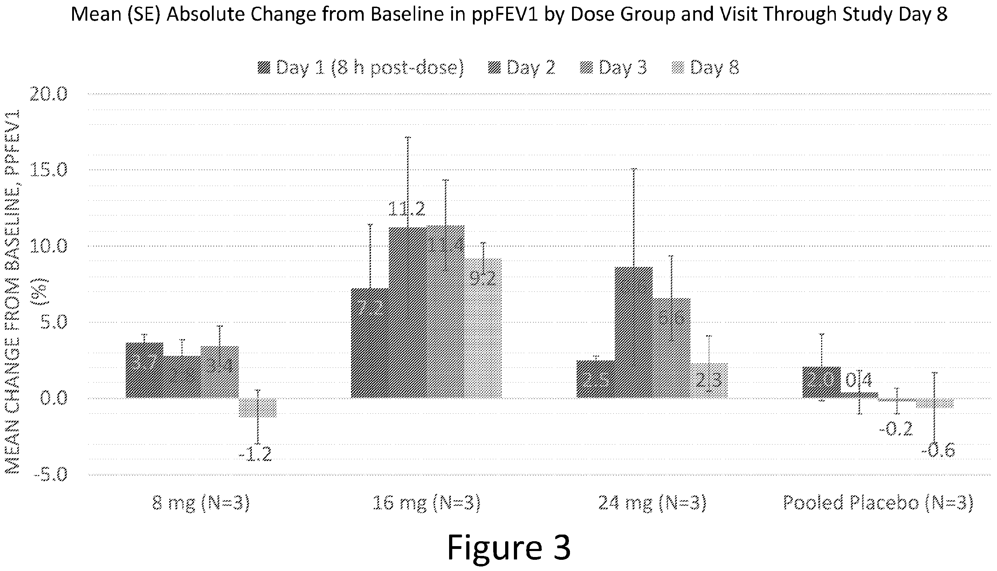

As shown in Figures 2 and 3, in several patients, mainly in the 16 mg dose group, increases in ppFEV1 were observed during the 8 days after treatment. Notably, an increase in ppFEV1 in a patient with a mutation non amendable to currently available modulators was observed. Moreover, ppFEV1 increases in patients who were already taking modulators (over any increase already achieved by the modulators) were observed, indicating the effectiveness of hCFTR mRNA LNP in improving lung function. Early improvement in ppFEV1 suggests that the LNP formulation is crossing the mucus layer in these patients following a single dose and enables the production of functional CFTR protein. Additionally, the treatment was generally well tolerated at the low (8 mg) and mid (16 mg) dose levels. At 24 mg dose, certain patients experienced mild to moderate febrile reactions that were transient and self-limiting, and also provided further evidence of successful delivery of the drug product thought the mucus to the epithelium. No serious adverse events occurred at any dose level.

Overall, this example shows that administration of the hCFTR mRNA LNPs via nebulization to CF patients according to the present invention is effective in improving the patients' lung function without serious side effects.

### Example 4. Efficacy of Multiple Doses of Inhaled CFTR mRNA Therapeutic in Adult CF Patients

The study in this Example is designed to evaluate the safety and efficacy of multiple ascending doses of the drug product of Example 1.

The CF patients are assigned to one of five treatment groups: 8 mg dose, 12 mg dose, 16 mg dose, or 20 mg dose (nominal dose of mRNA), and placebo. A total of five doses are administered to the patients, with each dose administered weekly via nebulization. Testing of the 20 mg dose will be contingent on the 20 mg dose being well tolerated in the study similar to that described in Example 3. Safety, tolerability and efficacy are evaluated as described in Example 3.

## Claims

1. A composition comprising an mRNA encoding a Cystic Fibrosis Transmembrane Conductance Regulator (CFTR) protein for use in a method of treating cystic fibrosis (CF) in a human subject, wherein the method comprises administration of the composition by nebulization at a dose between 13 mg and 19 mg that provides the human subject with at least a 3% increase in absolute change in ppFEV1 (percent predicted forced expiratory volume in one second) from baseline ppFEV1 at two days following the administration, wherein the mRNA is encapsulated in lipid nanoparticles.

2. The composition for use according to claim 1, wherein the composition is nebulized at a dose of 16 mg.

3. The composition for use according to claim 1 or claim 2, wherein:
a. the human subject is suffering from or at risk of chronic obstructive pulmonary disorder (COPD); or
b. the human subject is at risk of cystic fibrosis; or
c. the human subject is suffering from cystic fibrosis.

4. The composition for use according to any preceding claim, wherein:
a. the human subject has a class I mutation; or
b. the human subject has a class II mutation; optionally wherein the human subject has an F508del mutation, optionally wherein the F508del mutation is heterozygous or homozygous;
or
c. the human subject has a class I mutation and a class II mutation; optionally wherein the human subject has an F508del mutation, optionally wherein the F508del mutation is heterozygous or homozygous.

5. The composition for use according to any one of claims 1-4, wherein the human subject does not have an F508del mutation.

6. The composition for use according to any one of the preceding claims, wherein the human subject has a CFTR gene mutation selected from:
a. a class I mutation resulting in defective protein synthesis, e.g., a non-sense, frameshift or aberrant splicing mutation, selected from 1078delT, 1154 insTC, 1525-2A > G, 1717-1G > A, 1898+1G > A, 2184delA, 2184 insA, 3007delG, 3120+1G > A, 3659delC, 3876delA, 3905insT, 394delTT, 4010del4, 4016insT, 4326delTC, 4374+1G > T, 441delA, 556delA, 621+1G > T, 621-1G > T, 711+1G > T, 875+1G > C, E1104X, E585X, E60X, E822X, G542X, G551D/R553X, Q493X, Q552X, Q814X, R1066C, R1162X, R553X, V520F, W1282X, Y1092X;
b. a class II mutation resulting in abnormal processing and trafficking selected from A559T, D979A, ΔF508 (including F508del), ΔI507, G480C, G85E, N1303K, S549I, S549N, S549R;
c. a class III mutation resulting in defective channel regulation/gating selected from G1244E, G1349D, G551D, G551S, G85E, H199R, I1072T, I48T, L1077P, R560T, S1255P, S549N (R75Q);
d. a class IV mutation resulting in decreased channel conductance selected from A800G, D1152H, D1154G, D614G, delM1140, E822K, G314E, G576A, G622D, G85E, H620Q, I1139V, I1234V, L1335P, M1137V, P67L, R117C, R117P, R117H, R334W, R347H, R347P, R347P/R347H, R792G, S1251N, V232D; or
e. a class V mutation resulting in reduced synthesis and/or trafficking selected from 2789+5G > A, 3120G > A, 3272-26A > G, 3849+10kbC > T, 5T variant, 621+3A > G, 711+3A > G, A445E, A455E, IVS8 poly T, P574H, 875+1G > C.

7. The composition for use according to any one of the preceding claims, wherein:
a. the method first includes a step of selecting the human subject for treatment based on the presence of a class I and/or class II mutation; and/or
b. the method first includes a step of selecting the human subject for treatment based on the absence of an F508del mutation.

8. The composition for use according to any one of the preceding claims, wherein the human subject receives concomitant CFTR modulator therapy, optionally wherein:
a. the concomitant CFTR modulator therapy is selected from ivacaftor, lumacaftor, tezacaftor, or a combination thereof; or
b. the concomitant CFTR modulator therapy comprises ivacaftor; or
c. the concomitant CFTR modulator therapy comprises lumacaftor; or
d. the concomitant CFTR modulator therapy comprises tezacaftor; or
e. the concomitant CFTR modulator therapy comprises VX-659; or
f. the concomitant CFTR modulator therapy comprises VX-445; or
g. the concomitant CFTR modulator therapy comprises VX-152; or
h. the concomitant CFTR modulator therapy comprises VX-440; or
i. the concomitant CFTR modulator therapy comprises VX-371; or
j. the concomitant CFTR modulator therapy comprises VX-561.

9. The composition for use according to claim 8, wherein the baseline ppFEV1 is measured in the human subject following prior administration to the human subject of the concomitant CFTR modulator therapy.

10. The composition for use according to any one of claims 1-7, wherein the human subject is not eligible for treatment with one or more of ivacaftor, lumacaftor, tezacaftor, VX-659, VX-445, VX-152, VX-440, VX-371, VX-561, VX-659.

11. The composition for use according to any one of the preceding claims, wherein the human subject has the baseline ppFEV1 of between about 50% and 80% of predicted normal, optionally wherein the human subject has the baseline ppFEV1 of between about 50% and 60%, about 60% and 70%, or about 70% and 80% of predicted normal.

12. The composition for use according to any one of the preceding claims, wherein:
a. the mRNA comprises a nucleotide sequence of SEQ ID NO:28;
b. the mRNA comprises a 5' Cap with a structure of 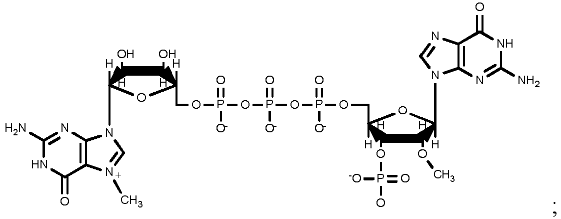
c. the mRNA has a capping level of at least 70%; and/or
d. the mRNA is unmodified.

13. The composition for use according to any one of the preceding claims, wherein:
a. each lipid nanoparticle comprises a PEG-modified lipid, optionally wherein lipid nanoparticle comprises the PEG-modified lipid at a molar ratio of 3% or greater of the total lipid content of the lipid nanoparticle, optionally wherein lipid nanoparticle comprises the PEG-modified lipid at a molar ratio of 4% or greater of the total lipid content of the lipid nanoparticle, optionally wherein lipid nanoparticle comprise the PEG-modified lipid at a molar ratio of 5% or greater of the total lipid content of the lipid nanoparticle; or
b. the lipid nanoparticles have an encapsulation level of at least 80%; and/or
c. the lipid nanoparticles have an average size ranging from 40 nm to 60 nm.

14. The composition for use according to any one of the preceding claims, wherein the composition is an aqueous solution comprising the lipid nanoparticles, optionally wherein:
a. the concentration of the mRNA encoding the CFTR protein ranges from 0.5 mg/mL to 0.8 mg/mL, optionally wherein the concentration is 0.6 mg/mL; and/or
b. the method comprises first reconstituting lyophilized dry powder into the aqueous solution prior to nebulization; and/or
c. each lipid nanoparticle has only three lipid components, optionally wherein:
i. the three lipid components are a cationic lipid, a helper lipid and a PEG-modified lipid, optionally wherein the molar ratio of cationic lipid:helper lipid:PEG-modified lipid in each lipid nanoparticle is 60:35:5; or
ii. the cationic lipid is imidazole cholesterol ester (ICE), the helper lipid is 1,2-dioleoyl-sn-glycero-3-phosphoethanolamine (DOPE), and the PEG-modified lipid is 1,2-dimyristoyl-sn-glycerol, methoxypolyethylene glycol (DMG-PEG-2K), optionally wherein the molar ratio of ICE:DOPE:DMG-PEG-2K in each lipid nanoparticle is 60:35:5.

15. The composition for use according to any one of the preceding claims, wherein:
a. the composition comprises trehalose, optionally wherein the trehalose is present at a concentration of at least 10% (w/v); and/or
b. the composition is nebulized at a rate ranging from 0.2 mL/minute to 0.5 mL/minute; and/or
c. the composition is nebulized using a vibrating mesh nebulizer.

## Patentansprüche

1. Zusammensetzung, umfassend eine mRNA, die ein Cystic Fibrosis Transmembrane Conductance Regulator (CFTR)-Protein codiert, zur Verwendung in einem Verfahren zum Behandeln von zystischer Fibrose (CF) in einem menschlichen Individuum, wobei das Verfahren die Verabreichung der Zusammensetzung durch Vernebelung bei einer Dosis zwischen 13 mg und 19 mg umfasst, welche bei dem menschlichen Individuum zumindest eine 3%ige Erhöhung der absoluten Änderung des ppFEV1 (prozentuales vorhergesagtes forciertes expiratorisches Volumen in einer Sekunde) gegenüber dem Basislinien-ppFEV1 zwei Tage nach der Verabreichung vorsieht, wobei die mRNA in Lipidnanopartikeln eingekapselt ist.

2. Zusammensetzung zur Verwendung nach Anspruch 1, wobei die Zusammensetzung bei einer Dosis von 16 mg vernebelt wird.

3. Zusammensetzung zur Verwendung nach Anspruch 1 oder Anspruch 2, wobei:
a. das menschliche Individuum an chronisch obstruktiver Lungenerkrankung (COPD) leidet oder hinsichtlich dieser gefährdet ist; oder
b. das menschliche Individuum hinsichtlich zystischer Fibrose gefährdet ist; oder
c. das menschliche Individuum an zystischer Fibrose leidet.

4. Zusammensetzung zur Verwendung nach irgendeinem vorhergehenden Anspruch, wobei:
a. das menschliche Individuum eine Klasse-I-Mutation aufweist; oder
b. das menschliche Individuum eine Klasse-II-Mutation aufweist; optional wobei das menschliche Individuum eine F508del-Mutation aufweist, optional wobei die F508del-Mutation heterozygot oder homozygot ist;
oder
c. das menschliche Individuum eine Klasse-I-Mutation und eine Klasse-II-Mutation aufweist; optional wobei das menschliche Individuum eine F508del-Mutation aufweist, optional wobei die F508del-Mutation heterozygot oder homozygot ist.

5. Zusammensetzung zur Verwendung nach einem der Ansprüche 1-4, wobei das menschliche Individuum keine F508del-Mutation aufweist.

6. Zusammensetzung zur Verwendung nach einem der vorhergehenden Ansprüche, wobei das menschliche Individuum eine CFTR-Genmutation aufweist, ausgewählt aus:
a. einer Klasse-I-Mutation, die zu mangelhafter Proteinsynthese führt, z. B. eine Nonsense-Mutation, Rasterschub-Mutation oder Aberrantes-Spleißen-Mutation, ausgewählt aus 1078delT, 1154 insTC, 1525-2A > G, 1717-1G > A, 1898+1G > A, 2184delA, 2184 insA, 3007delG, 3120+1G > A, 3659delC, 3876delA, 3905insT, 394delTT, 4010del4, 4016insT, 4326delTC, 4374+1G > T, 441delA, 556delA, 621+1G > T, 621-1G > T, 711+1G > T, 875+1G > C, E1104X, E585X, E60X, E822X, G542X, G551D/R553X, Q493X, Q552X, Q814X, R1066C, R1162X, R553X, V520F, W1282X, Y1092X;
b. einer Klasse-II-Mutation, die zu abnormaler Prozessierung und abnormalem Trafficking führt, ausgewählt aus A559T, D979A, ΔF508 (einschließlich F508del), ΔI507, G480C, G85E, N1303K, S549I, S549N, S549R;
c. einer Klasse-III-Mutation, die zu mangelhafter Kanal-Regulierung/Öffnung führt, ausgewählt aus G1244E, G1349D, G551D, G551S, G85E, H199R, I1072T, I48T, L1077P, R560T, S1255P, S549N (R75Q);
d. einer Klasse-IV-Mutation, die zu verringerter Kanal-Leitfähigkeit führt, ausgewählt aus A800G, D1152H, D1154G, D614G, delM1140, E822K, G314E, G576A, G622D, G85E, H620Q, I1139V, I1234V, L1335P, M1137V, P67L, R117C, R117P, R117H, R334W, R347H, R347P, R347P/R347H, R792G, S1251N, V232D; oder
e. einer Klasse-V-Mutation, die zu reduzierter Synthese und/oder reduziertem Trafficking führt, ausgewählt aus 2789+5G > A, 3120G > A, 3272-26A > G, 3849+10kbC > T, 5T-Variante, 621+3A > G, 711+3A > G, A445E, A455E, IVS8-poly-T, P574H, 875+1G > C.

7. Zusammensetzung zur Verwendung nach einem der vorhergehenden Ansprüche, wobei:
a. das Verfahren zuerst einen Schritt des Auswählens des menschlichen Individuums für die Behandlung basierend auf dem Vorhandensein einer Klasse-I- und/oder Klasse-II-Mutation umfasst; und/oder
b. das Verfahren zuerst einen Schritt des Auswählens des menschlichen Individuums für die Behandlung basierend auf dem Nichtvorhandensein einer F508del-Mutation umfasst.

8. Zusammensetzung zur Verwendung nach einem der vorhergehenden Ansprüche, wobei das menschliche Individuum eine begleitende CFTR-Modulator-Therapie erhält, optional wobei:
a. die begleitende CFTR-Modulator-Therapie ausgewählt ist aus Ivacaftor, Lumacaftor, Tezacaftor oder einer Kombination davon; oder
b. die begleitende CFTR-Modulator-Therapie Ivacaftor umfasst; oder
c. die begleitende CFTR-Modulator-Therapie Lumacaftor umfasst; oder
d. die begleitende CFTR-Modulator-Therapie Tezacaftor umfasst; oder
e. die begleitende CFTR-Modulator-Therapie VX-659 umfasst; oder
f. die begleitende CFTR-Modulator-Therapie VX-445 umfasst; oder
g. die begleitende CFTR-Modulator-Therapie VX-152 umfasst; oder
h. die begleitende CFTR-Modulator-Therapie VX-440 umfasst; oder
i. die begleitende CFTR-Modulator-Therapie VX-371 umfasst; oder
j. die begleitende CFTR-Modulator-Therapie VX-561 umfasst.

9. Zusammensetzung zur Verwendung nach Anspruch 8, wobei das Basislinien-ppFEV1 in dem menschlichen Individuum nach vorheriger Verabreichung der begleitenden CFTR-Modulator-Therapie an das menschliche Individuum gemessen wird.

10. Zusammensetzung zur Verwendung nach einem der Ansprüche 1-7, wobei das menschliche Individuum nicht für eine Behandlung mit einem oder mehreren von Ivacaftor, Lumacaftor, Tezacaftor, VX-659, VX-445, VX-152, VX-440, VX-371, VX-561, VX-659 in Frage kommt.

11. Zusammensetzung zur Verwendung nach einem der vorhergehenden Ansprüche, wobei bei dem menschlichen Individuum das Basislinien-ppFEV1 zwischen etwa 50 % und 80 % des vorhergesagten Normalwerts beträgt, optional wobei bei dem menschlichen Individuum das Basislinien-ppFEV1 zwischen etwa 50 % und 60 %, etwa 60 % und 70 % oder etwa 70 % und 80 % des vorhergesagten Normalwerts beträgt.

12. Zusammensetzung zur Verwendung nach einem der vorhergehenden Ansprüche, wobei:
a. die mRNA eine Nukleotidsequenz gemäß SEQ ID NO:28 umfasst;
b. die mRNA ein 5'-Cap umfasst, aufweisend eine Struktur von 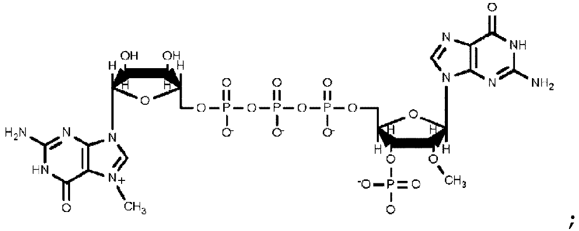
c. die mRNA einen Capping-Grad von mindestens 70 % aufweist; und/oder
d. die mRNA unmodifiziert ist.

13. Zusammensetzung zur Verwendung nach einem der vorhergehenden Ansprüche, wobei:
a. jedes Lipidnanopartikel ein PEG-modifiziertes Lipid umfasst, optional wobei das Lipidnanopartikel das PEG-modifizierte Lipid bei einem Molverhältnis von 3 % oder mehr des Gesamtlipidgehalts des Lipidnanopartikels umfasst, optional wobei das Lipidnanopartikel das PEG-modifizierte Lipid bei einem Molverhältnis von 4 % oder mehr des Gesamtlipidgehalts des Lipidnanopartikels umfasst, optional wobei das Lipidnanopartikel das PEG-modifizierte Lipid bei einem Molverhältnis von 5 % oder mehr des Gesamtlipidgehalts des Lipidnanopartikels umfasst; oder
b. die Lipidnanopartikel einen Einkapselungs-Grad von mindestens 80 % aufweisen; und/oder
c. die Lipidnanopartikel eine durchschnittliche Größe im Bereich von 40 nm bis 60 nm aufweisen.

14. Zusammensetzung zur Verwendung nach einem der vorhergehenden Ansprüche, wobei die Zusammensetzung eine wässrige Lösung ist, die die Lipidnanopartikel umfasst, optional wobei:
a. die Konzentration der mRNA, die das CFTR-Protein codiert, im Bereich von 0,5 mg/ml bis 0,8 mg/ml liegt, optional wobei die Konzentration 0,6 mg/ml beträgt; und/oder
b. das Verfahren zuerst das Rekonstituieren von lyophilisiertem Trockenpulver in die wässrige Lösung vor der Vernebelung umfasst; und/oder
c. jedes Lipidnanopartikel nur drei Lipidkomponenten aufweist, optional wobei:
i. es sich bei den drei Lipidkomponenten um ein kationisches Lipid, ein Helferlipid und ein PEG-modifiziertes Lipid handelt, wobei optional das Molverhältnis von kationischem Lipid:Helferlipid:PEG-modifiziertem Lipid in jedem Lipidnanopartikel 60:35:5 beträgt; oder
ii. das kationische Lipid Imidazol-Cholesterinester (ICE) ist, das Helferlipid 1,2-Dioleoyl-sn-glycero-3-phosphoethanolamin (DOPE) ist und das PEG-modifizierte Lipid 1,2-Dimyristoyl-sn-glycerol,methoxypolyethylenglycol (DMG-PEG-2K) ist, wobei optional das Molverhältnis von ICE:DOPE:DMG-PEG-2K in jedem Lipidnanopartikel 60:35:5 beträgt.

15. Zusammensetzung zur Verwendung nach einem der vorhergehenden Ansprüche, wobei:
a. die Zusammensetzung Trehalose umfasst, optional wobei die Trehalose bei einer Konzentration von mindestens 10 % (w/v) vorhanden ist; und/oder
b. die Zusammensetzung bei einer Rate im Bereich von 0,2 ml/Minute bis 0,5 ml/Minute vernebelt wird; und/oder
c. die Zusammensetzung mithilfe eines Rüttelgitterzerstäubers vernebelt wird.

## Revendications

1. Composition comprenant un ARNm codant pour une protéine régulatrice de conductance transmembranaire de la mucoviscidose (CFTR) pour une utilisation dans un procédé de traitement de la mucoviscidose (CF) chez un sujet humain, dans laquelle le procédé comprend l'administration de la composition par nébulisation à une dose comprise entre 13 mg et 19 mg qui procure au sujet humain une augmentation d'au moins 3 % de la variation absolue de ppFEV1 (pourcentage de volume expiratoire forcé prédit en une seconde) par rapport à la valeur de base de ppFEV1 deux jours après l'administration, dans laquelle l'ARNm est encapsulé dans des nanoparticules lipidiques.

2. Composition pour une utilisation selon la revendication 1, dans laquelle la composition est nébulisée à une dose de 16 mg.

3. Composition pour une utilisation selon la revendication 1 ou la revendication 2, dans laquelle :
a. le sujet humain souffre ou risque de souffrir de trouble pulmonaire obstructif chronique (COPD) ; ou
b. le sujet humain risque de souffrir de mucoviscidose ; ou
c. le sujet humain souffre de mucoviscidose.

4. Composition pour une utilisation selon l'une quelconque des revendications précédentes, dans laquelle :
a. le sujet humain présente une mutation de classe I ; ou
b. le sujet humain présente une mutation de classe II ; éventuellement, dans laquelle le sujet humain présente une mutation F508del, éventuellement dans laquelle la mutation F508del est hétérozygote ou homozygote ;
ou
c. le sujet humain présente une mutation de classe I et une mutation de classe II ; éventuellement dans laquelle le sujet humain présente une mutation F508del, éventuellement dans laquelle la mutation F508del est hétérozygote ou homozygote.

5. Composition pour une utilisation selon l'une quelconque des revendications 1 à 4, dans laquelle le sujet humain ne présente pas de mutation F508del.

6. Composition pour une utilisation selon l'une quelconque des revendications précédentes, dans laquelle le sujet humain présente une mutation du gène CFTR choisie parmi :
a. une mutation de classe I entraînant une synthèse protéique défectueuse, par exemple une mutation non-sens, à décalage de trame ou à épissage aberrant, choisie parmi 1078delT, 1154 insTC, 1525-2A > G, 1717-1G > A, 1898+1G > A, 2184delA, 2184 insA, 3007delG, 3120+1G > A, 3659delC, 3876delA, 3905insT, 394delTT, 4010del4, 4016insT, 4326delTC, 4374+1G > T, 441delA, 556delA, 621+1G > T, 621-1G > T, 711+1G > T, 875+1G > C, E1104X, E585X, E60X, E822X, G542X, G551D/R553X, Q493X, Q552X, Q814X, R1066C, R1162X, R553X, V520F, W1282X, Y1092X ;
b. une mutation de classe II entraînant un traitement et un trafic anormaux, choisie parmi A559T, D979A, ΔF508 (y compris F508del), ΔI507, G480C, G85E, N1303K, S549I, S549N, S549R ;
c. une mutation de classe III entraînant une régulation/synchronisation défectueuse des canaux choisie parmi G1244E, G1349D, G551D, G551S, G85E, H199R, I1072T, I48T, L1077P, R560T, S1255P, S549N (R75Q) ;
d. une mutation de classe IV entraînant une diminution de la conductance de canal choisie parmi A800G, D1152H, D1154G, D614G, delM1140, E822K, G314E, G576A, G622D, G85E, H620Q, I1139V, I1234V, L1335P, M1137V, P67L, R117C, R117P, R117H, R334W, R347H, R347P, R347P/R347H, R792G, S1251N, V232D ; ou
e. une mutation de classe V entraînant une réduction de la synthèse et/ou du trafic choisie parmi 2789+5G > A, 3120G > A, 3272-26A > G, 3849+10kbC > T, 5T variant, 621+3A > G, 711+3A > G, A445E, A455E, IVS8 poly T, P574H, 875+1G > C.

7. Composition pour une utilisation selon l'une quelconque des revendications précédentes, dans laquelle :
a. le procédé comprend d'abord une étape de sélection du sujet humain pour le traitement sur la base de la présence d'une mutation de classe I et/ou de classe II ; et/ou
b. le procédé comprend d'abord une étape de sélection du sujet humain pour le traitement sur la base de l'absence d'une mutation F508del.

8. Composition pour une utilisation selon l'une quelconque des revendications précédentes, dans laquelle le sujet humain reçoit un traitement concomitant par modulateur CFTR, éventuellement dans laquelle :
a. le traitement concomitant par modulateur CFTR est choisi parmi l'ivacaftor, le lumacaftor, le tézacaftor ou une combinaison de ceux-ci ; ou
b. le traitement concomitant par modulateur CFTR comprend l'ivacaftor ; ou
c. le traitement concomitant par modulateur CFTR comprend le lumacaftor ; ou
d. le traitement concomitant par modulateur CFTR comprend le tézacaftor ; ou
e. le traitement concomitant par modulateur CFTR comprend le VX-659 ; ou
f. le traitement concomitant par modulateur CFTR comprend le VX-445 ; ou
g. le traitement concomitant par modulateur CFTR comprend le VX-152 ; ou
h. le traitement concomitant par modulateur CFTR comprend le VX-440 ; ou
i. le traitement concomitant par modulateur CFTR comprend le VX-371 ; ou
j. le traitement concomitant par modulateur CFTR comprend le VX-561.

9. Composition pour une utilisation selon la revendication 8, dans laquelle le ppFEV1 de base est mesuré chez le sujet humain après administration préalable au sujet humain du traitement concomitant par modulateur CFTR.

10. Composition pour une utilisation selon l'une quelconque des revendications 1 à 7, dans laquelle le sujet humain n'est pas éligible pour un traitement par un ou plusieurs parmi ivacaftor, lumacaftor, tézacaftor, VX-659, VX-445, VX-152, VX-440, VX-371, VX-561, VX-659.

11. Composition pour une utilisation selon l'une quelconque des revendications précédentes, dans laquelle le sujet humain a le ppFEV1 de base compris entre environ 50 % et 80 % de la normale prédite, éventuellement dans laquelle le sujet humain a le ppFEV1 de base compris entre environ 50 % et 60 %, environ 60 % et 70 %, ou environ 70 % et 80 % de la normale prédite.

12. Composition pour une utilisation selon l'une quelconque des revendications précédentes, dans laquelle :
a. l'ARNm comprend une séquence nucléotidique de SEQ ID NO: 28 ;
b. l'ARNm comprend une coiffe en 5' ayant une structure de 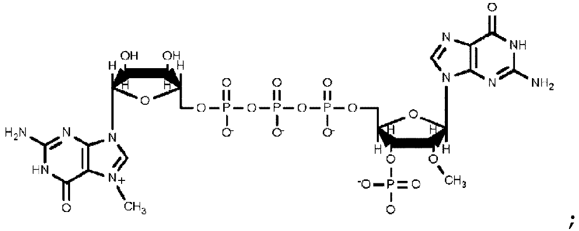
c. l'ARNm a un niveau de coiffage d'au moins 70 % ; et/ou
d. l'ARNm n'est pas modifié.

13. Composition pour une utilisation selon l'une quelconque des revendications précédentes, dans laquelle :
a. chaque nanoparticule lipidique comprend un lipide modifié par PEG, éventuellement dans laquelle la nanoparticule lipidique comprend le lipide modifié par PEG en un rapport molaire de 3 % ou plus de la teneur totale en lipides de la nanoparticule lipidique, éventuellement dans laquelle la nanoparticule lipidique comprend le lipide modifié par PEG en un rapport molaire de 4 % ou plus de la teneur totale en lipides de la nanoparticule lipidique, éventuellement dans laquelle la nanoparticule lipidique comprend le lipide modifié par PEG en un rapport molaire de 5 % ou plus de la teneur totale en lipides de la nanoparticule lipidique ; ou
b. les nanoparticules lipidiques ont un taux d'encapsulation d'au moins 80 % ; et/ou
c. les nanoparticules lipidiques ont une taille moyenne allant de 40 nm à 60 nm.

14. Composition pour une utilisation selon l'une quelconque des revendications précédentes, dans laquelle la composition est une solution aqueuse comprenant les nanoparticules lipidiques, éventuellement dans laquelle
a. la concentration de l'ARNm codant pour la protéine CFTR est comprise entre 0,5 mg/ml et 0,8 mg/ml, éventuellement dans laquelle la concentration est de 0,6 mg/ml ; et/ou
b. le procédé comprend d'abord la reconstitution de la poudre sèche lyophilisée dans la solution aqueuse avant la nébulisation ; et/ou
c. chaque nanoparticule lipidique a seulement trois composants lipidiques, éventuellement dans laquelle :
i. les trois composants lipidiques sont un lipide cationique, un lipide auxiliaire et un lipide modifié par PEG, éventuellement dans laquelle le rapport molaire lipide cationique : lipide auxiliaire : lipide modifié par PEG dans chaque nanoparticule lipidique est de 60:35:5 ; ou
ii. le lipide cationique est l'ester de cholestérol imidazole (ICE), le lipide auxiliaire est la 1,2-dioléoyl-sn-glycéro-3-phosphoéthanolamine (DOPE), et le lipide modifié par PEG est le 1,2-dimyristoyl-sn-glycérol, méthoxypolyéthylène glycol (DMG-PEG-2K), éventuellement dans laquelle le rapport molaire ICE:DOPE:DMG-PEG-2K dans chaque nanoparticule lipidique est de 60:35:5.

15. Composition pour une utilisation selon l'une quelconque des revendications précédentes, dans laquelle :
a. la composition comprend du tréhalose, éventuellement dans laquelle le tréhalose est présent à une concentration d'au moins 10 % (p/v) ; et/ou
b. la composition est nébulisée à une vitesse comprise entre 0,2 ml/minute et 0,5 ml/minute ; et/ou
c. la composition est nébulisée à l'aide d'un nébuliseur à mailles vibrantes.
